(19) Europäisches Patentamt
European Patent Office
Office européen des brevets

(11) EP 1 849 864 A1

(12) **EUROPEAN PATENT APPLICATION**
published in accordance with Art. 158(3) EPC

(43) Date of publication:
**31.10.2007 Bulletin 2007/44**

(21) Application number: **06714345.3**

(22) Date of filing: **16.02.2006**

(51) Int Cl.:
***C12N 15/00*** *(2006.01)* ***C12Q 1/68*** *(2006.01)*

(86) International application number:
**PCT/JP2006/303205**

(87) International publication number:
**WO 2006/088226 (24.08.2006 Gazette 2006/34)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **17.02.2005 JP 2005040698**
**26.04.2005 JP 2005128412**

(71) Applicant: **Takeda Pharmaceutical Company Limited**
**Osaka-shi,**
**Osaka 541-0045 (JP)**

(72) Inventors:
- **SAWADA, Hiroshi**
**TAKEDA PHARMACEUTICAL COMPANY LTD.**
**Osaka-shi, Osaka, 5328686 (JP)**
- **MORI, Ikuo**
**TAKEDA PHARMACEUTICAL COMPANY LTD.**
**Osaka-shi, Osaka, 5328686 (JP)**
- **TAKAMI, Kenji**
**TAKEDA PHARMACEUTICAL COMPANY LTD.**
**Osaka-shi, Osaka, 5328686 (JP)**

(74) Representative: **Keller, Günter et al**
**Lederer & Keller**
**Patentanwälte**
**Prinzregentenstrasse 16**
**80538 München (DE)**

# (54) METHOD FOR DETERMINING PHOSPHOLIPIDOSIS

(57) The present invention provides a rapid, convenient and noninvasive novel in vivo method for determining phospholipidosis (PLsis) by detecting a PLsis marker gene. More specifically, it is intended to provide a method for determining PLsis in a mammal comprising the step of detecting the expression variation of one or more genes showing expression variation in correlation with phospholipidosis expression in a sample from the mammal, in which at least one of the genes comprises the same or substantially the same base sequence as the base sequence shown in any of SEQ ID NO:n(wherein n is an odd number between 1 and 57, an integer number between 59 and 63, an even number between 64 and 98, 100 or 101).

EP 1 849 864 A1

SEQUENCE LISTING

<110> Takeda Pharmaceutical Company Limited

<120> Method for Diagnosing Phospholipidosis

<130> 09886

<150> JP 2005-40698
<151> 2005-02-17

<150> JP 2005-128412
<151> 2005-04-26

<160> 101

<170> PatentIn version 3.2

<210> 1
<211> 1380
<212> DNA
<213> Rattus norvegicus

<220>
<221> CDS
<222> (90)..(938)

<400> 1
aggtcgcgca ggcgcagacg gcgttggttc gagaagacct tcagcgttgc cttggtggag 60

cggtacgggc ccaccctcgg gttgtagat atg tgt agc aca cca act tac tgc 113
Met Cys Ser Thr Pro Thr Tyr Cys
1 5

gac cta gga aag gct gcc aag gat gtc ttt aac aaa ggg tat ggg ttt 161
Asp Leu Gly Lys Ala Ala Lys Asp Val Phe Asn Lys Gly Tyr Gly Phe
10 15 20

ggc atg gtc aaa ata gat ctg aaa acc aag tct tgt agt gga gtg gaa 209
Gly Met Val Lys Ile Asp Leu Lys Thr Lys Ser Cys Ser Gly Val Glu
25 30 35 40

ttt tct act tct ggt cat gct tat act gat aca ggg aaa gca tca ggc 257
Phe Ser Thr Ser Gly His Ala Tyr Thr Asp Thr Gly Lys Ala Ser Gly
45 50 55

aac cta gag acc aaa tat aag gtc tgt aac tac ggg ctc atc ttc acc 305
Asn Leu Glu Thr Lys Tyr Lys Val Cys Asn Tyr Gly Leu Ile Phe Thr
60 65 70

caa aag tgg aat aca gac aat act ctt ggg aca gaa atc tct tgg gag 353
Gln Lys Trp Asn Thr Asp Asn Thr Leu Gly Thr Glu Ile Ser Trp Glu
75 80 85

aat aag ttg gct gaa ggg ttg aaa ctg acg gtt gat acc ata ttt gta 401
Asn Lys Leu Ala Glu Gly Leu Lys Leu Thr Val Asp Thr Ile Phe Val
90 95 100

cca aac aca ggg aag aag agt ggg aaa tta aag gcc tcc tat aga cgg 449
Pro Asn Thr Gly Lys Lys Ser Gly Lys Leu Lys Ala Ser Tyr Arg Arg
105 110 115 120

gat tgt ttt agt gtg ggc agt aag gtt gac ata gat ttt tct gga ccg 497
Asp Cys Phe Ser Val Gly Ser Lys Val Asp Ile Asp Phe Ser Gly Pro
125 130 135

acc atc tat ggc tgg gcc gtg ttg gcc ttt gaa ggt tgg ctt gct ggc 545
Thr Ile Tyr Gly Trp Ala Val Leu Ala Phe Glu Gly Trp Leu Ala Gly
140 145 150

tac cag atg agt ttt gac aca gcc aaa tcc aaa ctg tgt cag aat aat 593
Tyr Gln Met Ser Phe Asp Thr Ala Lys Ser Lys Leu Cys Gln Asn Asn
155 160 165

ttt gct ctt ggt tac aag gct gaa gac ttc caa ctg cat act cat gta 641

```
Phe Ala Leu Gly Tyr Lys Ala Glu Asp Phe Gln Leu His Thr His Val
    170                 175                 180

aac gat ggc act gaa ttt gga ggc tcc atc tac cag aga gtt aat gag       689
Asn Asp Gly Thr Glu Phe Gly Gly Ser Ile Tyr Gln Arg Val Asn Glu
185                 190                 195                 200

aag atc gaa aca tca ata aac ctg gca tgg aca gct ggc agc aac aac       737
Lys Ile Glu Thr Ser Ile Asn Leu Ala Trp Thr Ala Gly Ser Asn Asn
                205                 210                 215

act cgt ttt ggc atc gct gct aag tat agg ctg gat tgt aga act tct       785
Thr Arg Phe Gly Ile Ala Ala Lys Tyr Arg Leu Asp Cys Arg Thr Ser
            220                 225                 230

ctg tct gcc aaa gta aac aat gcc agt tta att gga ctg ggt tat acg       833
Leu Ser Ala Lys Val Asn Asn Ala Ser Leu Ile Gly Leu Gly Tyr Thr
        235                 240                 245

cag agt ctc cga ccg gga gtc aaa ctg acc ctg tca gct tta gtg gat       881
Gln Ser Leu Arg Pro Gly Val Lys Leu Thr Leu Ser Ala Leu Val Asp
    250                 255                 260

gga aag aac ttc aat gca gga ggc cac aag gtt ggc ttg gga ttt gaa       929
Gly Lys Asn Phe Asn Ala Gly Gly His Lys Val Gly Leu Gly Phe Glu
265                 270                 275                 280

ctg gaa gct taatggggtt ttgagtagag tatcaattgt ccctggaaat               978
Leu Glu Ala

gaagagaaat gacaccacta tgttttggcc ttaaaattct gtgaaatttc aaaagtgaac    1038

tttttattct tccaaagaat tgtaattctt cccacaccga agtctagaga ttacagatcc    1098

atccagtggg aggtccttga aggcaatgcc tggaaattgt catgtttgtg ccacatttca    1158

gttgagttct gcagagttaa ttttaaatat gttcctcagc aacaacgtag tgtcacgtta    1218

aaggaagtga tctgccccggt ctgtacatcg tgtcctgcat gtcccaactc cactttccat   1278

gaccttttgt tacatcagtc tctgctctag tgagaccttt ggtttgcatc agagtaaaat    1338

aaacccatca catttggaac ataaaaaaaa aaaaaaaaaa aa                       1380
```

<210> 2
<211> 283
<212> PRT
<213> Rattus norvegicus

<400> 2

```
Met Cys Ser Thr Pro Thr Tyr Cys Asp Leu Gly Lys Ala Ala Lys Asp
1               5                   10                  15

Val Phe Asn Lys Gly Tyr Gly Phe Gly Met Val Lys Ile Asp Leu Lys
            20                  25                  30

Thr Lys Ser Cys Ser Gly Val Glu Phe Ser Thr Ser Gly His Ala Tyr
        35                  40                  45

Thr Asp Thr Gly Lys Ala Ser Gly Asn Leu Glu Thr Lys Tyr Lys Val
    50                  55                  60

Cys Asn Tyr Gly Leu Ile Phe Thr Gln Lys Trp Asn Thr Asp Asn Thr
65                  70                  75                  80

Leu Gly Thr Glu Ile Ser Trp Glu Asn Lys Leu Ala Glu Gly Leu Lys
                85                  90                  95
```

Leu Thr Val Asp Thr Ile Phe Val Pro Asn Thr Gly Lys Lys Ser Gly
100 105 110

Lys Leu Lys Ala Ser Tyr Arg Arg Asp Cys Phe Ser Val Gly Ser Lys
115 120 125

Val Asp Ile Asp Phe Ser Gly Pro Thr Ile Tyr Gly Trp Ala Val Leu
130 135 140

Ala Phe Glu Gly Trp Leu Ala Gly Tyr Gln Met Ser Phe Asp Thr Ala
145 150 155 160

Lys Ser Lys Leu Cys Gln Asn Asn Phe Ala Leu Gly Tyr Lys Ala Glu
165 170 175

Asp Phe Gln Leu His Thr His Val Asn Asp Gly Thr Glu Phe Gly Gly
180 185 190

Ser Ile Tyr Gln Arg Val Asn Glu Lys Ile Glu Thr Ser Ile Asn Leu
195 200 205

Ala Trp Thr Ala Gly Ser Asn Asn Thr Arg Phe Gly Ile Ala Ala Lys
210 215 220

Tyr Arg Leu Asp Cys Arg Thr Ser Leu Ser Ala Lys Val Asn Asn Ala
225 230 235 240

Ser Leu Ile Gly Leu Gly Tyr Thr Gln Ser Leu Arg Pro Gly Val Lys
245 250 255

Leu Thr Leu Ser Ala Leu Val Asp Gly Lys Asn Phe Asn Ala Gly Gly
260 265 270

His Lys Val Gly Leu Gly Phe Glu Leu Glu Ala
275 280

<210> 3
<211> 503
<212> DNA
<213> Rattus norvegicus

<220>
<221> CDS
<222> (114)..(317)

<400> 3
ctcgtgccga atcggcacga gcggcacgag tggtgttcgg gagaggcgtt ctagtcctcg 60

ccgccgcctg tgccgccgcc tgtgccgcct gtgccgcctg tgccgcgtca gtc atg 116
Met
1

ccg aag cac gag ttc tct gtg gac atg acc tgt gga ggc tgt gcg gaa 164
Pro Lys His Glu Phe Ser Val Asp Met Thr Cys Gly Gly Cys Ala Glu
5 10 15

gcc gtc tcc cga gtc ctc aac aag ctg gga gga gtg gag ttc aac att 212
Ala Val Ser Arg Val Leu Asn Lys Leu Gly Gly Val Glu Phe Asn Ile
20 25 30

gac ttg ccc aac aag aag gtc tgc atc gag tct gag cac agc tca gac 260
Asp Leu Pro Asn Lys Lys Val Cys Ile Glu Ser Glu His Ser Ser Asp

35 40 45

atc ctg ctg gca act ctc aac aaa aca gga aaa gcg gtc tcc tac ctt 308
Ile Leu Leu Ala Thr Leu Asn Lys Thr Gly Lys Ala Val Ser Tyr Leu
50 55 60 65

ggc ccc aag tagccaggac ctgggcgagt ccttccggat agaaactgaa 357
Gly Pro Lys

gaggcagact gttgatctgg tctcccccgc tgatctggaa caccaactgc tcagtccagc 417

tccgccatgg aattccagcc cagacaggcc ttccctgctg gctccctgcc agcttccttg 477

caattaaagt caagctgcat ttgttg 503

<210> 4
<211> 68
<212> PRT
<213> Rattus norvegicus

<400> 4

Met Pro Lys His Glu Phe Ser Val Asp Met Thr Cys Gly Gly Cys Ala
1 5 10 15

Glu Ala Val Ser Arg Val Leu Asn Lys Leu Gly Gly Val Glu Phe Asn
20 25 30

Ile Asp Leu Pro Asn Lys Lys Val Cys Ile Glu Ser Glu His Ser Ser
35 40 45

Asp Ile Leu Leu Ala Thr Leu Asn Lys Thr Gly Lys Ala Val Ser Tyr
50 55 60

Leu Gly Pro Lys
65

<210> 5
<211> 2090
<212> DNA
<213> Rattus norvegicus

<220>
<221> CDS
<222> (84)..(749)

<400> 5
cgggcggacg ccgcagagca gacgcgcggc tgctagcgaa cggccgtgtt ctgaggagag 60

cagcggtcgt gggcgcctca gca atg ttg tgt cgg gcg gcg tgc agc gcg ggc 113
Met Leu Cys Arg Ala Ala Cys Ser Ala Gly
1 5 10

aga aga ctg ggc ccc gcg gcc agt acc gcg ggc tcc cgg cac aag cac 161
Arg Arg Leu Gly Pro Ala Ala Ser Thr Ala Gly Ser Arg His Lys His
15 20 25

agc ctc cct gac ctg cct tac gac tat ggc gcg ctg gag ccg cac att 209
Ser Leu Pro Asp Leu Pro Tyr Asp Tyr Gly Ala Leu Glu Pro His Ile
30 35 40

aac gcg cag atc atg cag ctg cac cac agc aag cac cac gcg acc tac 257
Asn Ala Gln Ile Met Gln Leu His His Ser Lys His His Ala Thr Tyr
45 50 55

gtg aac aat ctg aac gtc acc gag gag aag tac cac gag gcg ctg gcc 305
Val Asn Asn Leu Asn Val Thr Glu Glu Lys Tyr His Glu Ala Leu Ala
60 65 70

```
aag gga gat gtt aca act cag gtt gct ctt cag cct gca ctg aag ttc      353
Lys Gly Asp Val Thr Thr Gln Val Ala Leu Gln Pro Ala Leu Lys Phe
75                  80                  85                  90

aat ggc ggg ggc cat atc aat cac agc att ttc tgg aca aac ctg agc      401
Asn Gly Gly Gly His Ile Asn His Ser Ile Phe Trp Thr Asn Leu Ser
                95                  100                 105

cct aag ggt ggt gga gaa ccc aaa gga gag ttg ctg gag gct atc aag      449
Pro Lys Gly Gly Gly Glu Pro Lys Gly Glu Leu Leu Glu Ala Ile Lys
            110                 115                 120

cgt gac ttt ggg tct ttt gag aag ttt aag gag aaa ctg aca gct gtg      497
Arg Asp Phe Gly Ser Phe Glu Lys Phe Lys Glu Lys Leu Thr Ala Val
        125                 130                 135

tct gtg gga gtc caa ggt tca ggc tgg ggc tgg ctt ggc ttc aat aag      545
Ser Val Gly Val Gln Gly Ser Gly Trp Gly Trp Leu Gly Phe Asn Lys
    140                 145                 150

gag caa ggt cgc tta cag att gcc gcc tgc tct aat cag gac cca ctg      593
Glu Gln Gly Arg Leu Gln Ile Ala Ala Cys Ser Asn Gln Asp Pro Leu
155                 160                 165                 170

caa gga acc aca ggc ctt att cca ctg ctg ggg att gat gtg tgg gag      641
Gln Gly Thr Thr Gly Leu Ile Pro Leu Leu Gly Ile Asp Val Trp Glu
                175                 180                 185

cac gct tac tat ctt cag tat aaa aac gtc aga cct gac tat ctg aaa      689
His Ala Tyr Tyr Leu Gln Tyr Lys Asn Val Arg Pro Asp Tyr Leu Lys
            190                 195                 200

gcc att tgg aat gta atc aac tgg gag aat gtt agc caa aga tac ata      737
Ala Ile Trp Asn Val Ile Asn Trp Glu Asn Val Ser Gln Arg Tyr Ile
        205                 210                 215

gtt tgc aag aag tgaagccctt ccgccaggct gtgtgtcagg cccgtggtgg          789
Val Cys Lys Lys
    220

gtgttttgta gtagtgtaga gcattgcagc actgtggctg agctgttgta atcttcattg    849

atgcctatcc acatatgtgt aagcatacag ttatgataat ttcttaatta aatgtattgt    909

taggcaactg tttgagaaca gtacatactt ggtgtgagct gctcttgatt gaacattttc    969

attagaggct tgaattgctt ggacgctgtc actgtcatca taaggccatc aaagatattc    1029

catctctgtg ttggggcctg tggggaggct gtaatcctgt tctactgcag ttaggaaaaa    1089

aaatgagtta cccccccccc cagaattgtt gaataataaa atagagaact gaatagttct    1149

cttttctgtt aaaaattgct atttttcata agtaatcctt tgtttagcgg atatcaccta    1209

gtggtcttta tttatggcca cagtttcata gaaacatcat tttttcactt gaaacgtgta    1269

actaggctaa ggatggatgg agtggtagag cctttgcctg tcttatgtga ggccctgggc    1329

tctacctcac tactgaacaa atcaacagac ccaagctagg ctcctgactg acaactgtta    1389

attcggagag gagtgacatt gtgcctctgg gtttttttat aggctgagat gcaaaaactg    1449

ttaccttgtc tattaaaacc gactgtgtat tgtatgaaag tgctcaagat ggacaaagta    1509

tgcagggcct gtcccatgat gtcaacatgg ggactaggcc acagggcatt cagggaagcc    1569

attcagcacc attaattgtg tatctcagga gaactggacc ctgggtgagg tggcaccttt    1629

ttcagtagag gcaaacgggt ttctgagtga ggtcagtagg gtgtcttgag aacatcagtt    1689

ttcagagaca tgtattttca ttttttctgc agtctcaaga acttatactc acaagagggc    1749

ctttgtattg ttattcaata gaaggacacc ctttccctga caaggtacac agtgtttctg    1809

attgtattcc tgtgcagcat ttgggtgtag agctgtgcac tgttgaaatg cctttgtgaa    1869
```

tttgcctttt cacatttcgt ctcaccatct tccttgtttt ctttactgac tatgtaatgt 1929

tttatcagtt ggatattact gaatagtttg tttgtgttta attgcctttt attaccaaaa 1989

aggctgtggt cagcaacccc aggcatgcac attacttctt aatggagtat tttttaaagg 2049

tttattttaa ttttaaaaaa aaaaaaaaaa aaaaaaaaaa a 2090

<210> 6
<211> 222
<212> PRT
<213> Rattus norvegicus

<400> 6

Met Leu Cys Arg Ala Ala Cys Ser Ala Gly Arg Arg Leu Gly Pro Ala
1               5                   10                  15

Ala Ser Thr Ala Gly Ser Arg His Lys His Ser Leu Pro Asp Leu Pro
            20                  25                  30

Tyr Asp Tyr Gly Ala Leu Glu Pro His Ile Asn Ala Gln Ile Met Gln
        35                  40                  45

Leu His His Ser Lys His His Ala Thr Tyr Val Asn Asn Leu Asn Val
    50                  55                  60

Thr Glu Glu Lys Tyr His Glu Ala Leu Ala Lys Gly Asp Val Thr Thr
65                  70                  75                  80

Gln Val Ala Leu Gln Pro Ala Leu Lys Phe Asn Gly Gly Gly His Ile
                85                  90                  95

Asn His Ser Ile Phe Trp Thr Asn Leu Ser Pro Lys Gly Gly Gly Glu
            100                 105                 110

Pro Lys Gly Glu Leu Leu Glu Ala Ile Lys Arg Asp Phe Gly Ser Phe
        115                 120                 125

Glu Lys Phe Lys Glu Lys Leu Thr Ala Val Ser Val Gly Val Gln Gly
    130                 135                 140

Ser Gly Trp Gly Trp Leu Gly Phe Asn Lys Glu Gln Gly Arg Leu Gln
145                 150                 155                 160

Ile Ala Ala Cys Ser Asn Gln Asp Pro Leu Gln Gly Thr Thr Gly Leu
                165                 170                 175

Ile Pro Leu Leu Gly Ile Asp Val Trp Glu His Ala Tyr Tyr Leu Gln
            180                 185                 190

Tyr Lys Asn Val Arg Pro Asp Tyr Leu Lys Ala Ile Trp Asn Val Ile
        195                 200                 205

Asn Trp Glu Asn Val Ser Gln Arg Tyr Ile Val Cys Lys Lys
    210                 215                 220

<210> 7
<211> 1689
<212> DNA

<213> Rattus norvegicus

<220>
<221> CDS
<222> (176)..(913)

<400> 7
```
tgcagccagc tagcgagaag gcgcgagcgg cggcgcagcc agcagcctcc cgccagccgg      60

cgagccagtg cgcgtgcgcg gcggcggcct cggcggcgac cgggaagcgg acgggcgggc     120

gaggcgagcg aggcaggcgg tgcgggcgtg cgaggcgagg ccgatcgcga gcgac atg      178
                                                             Met
                                                             1

ggg gac cga gag cag ctg ctg cag cgg gcg cga ctg gcg gag cag gcg       226
Gly Asp Arg Glu Gln Leu Leu Gln Arg Ala Arg Leu Ala Glu Gln Ala
            5                   10                  15

gag cgc tac gac gac atg gcc tcc gcc atg aag gcg gtg aca gag ctg       274
Glu Arg Tyr Asp Asp Met Ala Ser Ala Met Lys Ala Val Thr Glu Leu
        20                  25                  30

aat gaa cct cta tct aat gaa gat aga aat ctc ctc tct gtg gcc tac       322
Asn Glu Pro Leu Ser Asn Glu Asp Arg Asn Leu Leu Ser Val Ala Tyr
    35                  40                  45

aag aat gta gtt ggt gcc agg cga tct tct tgg agg gtt att agt agc       370
Lys Asn Val Val Gly Ala Arg Arg Ser Ser Trp Arg Val Ile Ser Ser
50                  55                  60                  65

att gag cag aaa acc atg gca gat ggg aat gag aag aag ctg gag aaa       418
Ile Glu Gln Lys Thr Met Ala Asp Gly Asn Glu Lys Lys Leu Glu Lys
                70                  75                  80

gtc aaa gcc tat cgg gag aag att gag aag gag ctg gag aca gtt tgc       466
Val Lys Ala Tyr Arg Glu Lys Ile Glu Lys Glu Leu Glu Thr Val Cys
            85                  90                  95

aat gat gtc ttg gct ctg ctc gac aag ttc ctt atc aag aac tgc aat       514
Asn Asp Val Leu Ala Leu Leu Asp Lys Phe Leu Ile Lys Asn Cys Asn
        100                 105                 110

gat ttt cag tac gag agc aag gtg ttc tac ctg aaa atg aag ggc gat       562
Asp Phe Gln Tyr Glu Ser Lys Val Phe Tyr Leu Lys Met Lys Gly Asp
    115                 120                 125

tac tac cgc tac ctg gca gag gtg gct tct ggg gag aag aaa aac agt       610
Tyr Tyr Arg Tyr Leu Ala Glu Val Ala Ser Gly Glu Lys Lys Asn Ser
130                 135                 140                 145

gtg gtt gaa gct tct gag gca gcg tat aag gaa gcc ttc gaa atc agc       658
Val Val Glu Ala Ser Glu Ala Ala Tyr Lys Glu Ala Phe Glu Ile Ser
                150                 155                 160

aaa gag cac atg cag cca aca cac ccc atc cgg ctt ggc ctg gcc ctc       706
Lys Glu His Met Gln Pro Thr His Pro Ile Arg Leu Gly Leu Ala Leu
            165                 170                 175

aat ttt tct gtg ttc tac tat gag atc cag aat gca cca gag cag gcc       754
Asn Phe Ser Val Phe Tyr Tyr Glu Ile Gln Asn Ala Pro Glu Gln Ala
        180                 185                 190

tgc ctc tta gcc aaa caa gcc ttc gat gat gct ata gct gag ctg gac       802
Cys Leu Leu Ala Lys Gln Ala Phe Asp Asp Ala Ile Ala Glu Leu Asp
    195                 200                 205

aca tta aac gag gat tcc tat aag gac tcc act ctc atc atg cag ttg       850
Thr Leu Asn Glu Asp Ser Tyr Lys Asp Ser Thr Leu Ile Met Gln Leu
210                 215                 220                 225

ctg cga gac aac ctc acc ctc tgg acg agc gac cag cag gat gaa gaa       898
Leu Arg Asp Asn Leu Thr Leu Trp Thr Ser Asp Gln Gln Asp Glu Glu
                230                 235                 240
```

```
gcc gga gaa ggc aac tgaagaccca tcaggtccct ggcccttcct ttacccacca      953
Ala Gly Glu Gly Asn
                245

cccccattat cactgattct tccttgccac aatcactata tctagtgcta aacctatctg   1013

tattggcagc acagctattc agatctgccc tcctgtccct tggaagcagt ttcagataaa   1073

ccttcatggg catttgctgg actgatggtt gctttgagcc acagagcgct ccctttttga   1133

attgtgcaga gaagtgtgtt ctgaacgagg cattttatta tgtctgttga tctgtagcaa   1193

atccatgtga tggtaattga gtgtagaaag gagaattagc caacacaggc tatggctgct   1253

atttaaaaca agctgatagt gtgttgttaa gcagtacatc tcgtgcatgc aaaaatgaat   1313

ttgaccctct caccccttct ttcagctaat ggaaactgac acacgacaac ttgttccttc   1373

accatcagct ttataaactg tttctcgtga gctttcaggc ccctgctgtg cctctttaaa   1433

ttatgatgtg cgcacacctt cttttcaatg caatgcatca gaggtttttg atatgtgtaa   1493

cttttttttt tggttgtgat taagaatcat ggatttattt tttgtaactc tttggctatt   1553

gttcttgtgt accctgacag catcatgtgt gtcaacctgt gtcaatcatg atgggtggtt   1613

atgaaatgcc agattgctaa aataaatgtt ttggacttaa aaagagtaaa taaatgctgc   1673

tttggggata ttaaaa                                                   1689


<210>  8
<211>  246
<212>  PRT
<213>  Rattus norvegicus

<400>  8

Met Gly Asp Arg Glu Gln Leu Leu Gln Arg Ala Arg Leu Ala Glu Gln
1               5                   10                  15

Ala Glu Arg Tyr Asp Asp Met Ala Ser Ala Met Lys Ala Val Thr Glu
            20                  25                  30

Leu Asn Glu Pro Leu Ser Asn Glu Asp Arg Asn Leu Leu Ser Val Ala
        35                  40                  45

Tyr Lys Asn Val Val Gly Ala Arg Arg Ser Ser Trp Arg Val Ile Ser
    50                  55                  60

Ser Ile Glu Gln Lys Thr Met Ala Asp Gly Asn Glu Lys Lys Leu Glu
65                  70                  75                  80

Lys Val Lys Ala Tyr Arg Glu Lys Ile Glu Lys Glu Leu Glu Thr Val
                85                  90                  95

Cys Asn Asp Val Leu Ala Leu Leu Asp Lys Phe Leu Ile Lys Asn Cys
            100                 105                 110

Asn Asp Phe Gln Tyr Glu Ser Lys Val Phe Tyr Leu Lys Met Lys Gly
        115                 120                 125

Asp Tyr Tyr Arg Tyr Leu Ala Glu Val Ala Ser Gly Glu Lys Lys Asn
    130                 135                 140

Ser Val Val Glu Ala Ser Glu Ala Ala Tyr Lys Glu Ala Phe Glu Ile
145                 150                 155                 160
```

```
Ser Lys Glu His Met Gln Pro Thr His Pro Ile Arg Leu Gly Leu Ala
                165                 170                 175

Leu Asn Phe Ser Val Phe Tyr Tyr Glu Ile Gln Asn Ala Pro Glu Gln
            180                 185                 190

Ala Cys Leu Leu Ala Lys Gln Ala Phe Asp Asp Ala Ile Ala Glu Leu
        195                 200                 205

Asp Thr Leu Asn Glu Asp Ser Tyr Lys Asp Ser Thr Leu Ile Met Gln
    210                 215                 220

Leu Leu Arg Asp Asn Leu Thr Leu Trp Thr Ser Asp Gln Gln Asp Glu
225                 230                 235                 240

Glu Ala Gly Glu Gly Asn
                245


<210>  9
<211>  667
<212>  DNA
<213>  Rattus norvegicus


<220>
<221>  CDS
<222>  (13)..(402)

<400>  9
cctgccttca cc atg aag tcc agc ggc ctg ttc cct ctc atg gtg ctc ctt      51
              Met Lys Ser Ser Gly Leu Phe Pro Leu Met Val Leu Leu
              1               5                   10

gct ctg ggt gtc ctg gca ccc tgg agt gtg gaa gga ggc aaa aat gat        99
Ala Leu Gly Val Leu Ala Pro Trp Ser Val Glu Gly Gly Lys Asn Asp
    15                  20                  25

gct atc aaa atc gga gcc tgc cct gct aga aag cct gcc cag tgc ctt       147
Ala Ile Lys Ile Gly Ala Cys Pro Ala Arg Lys Pro Ala Gln Cys Leu
30                  35                  40                  45

aaa cgt gag aaa cca gag tgc agt act gac tgg gga tgc cca gga aag       195
Lys Arg Glu Lys Pro Glu Cys Ser Thr Asp Trp Gly Cys Pro Gly Lys
                50                  55                  60

cag aga tgc tgc caa gat acc tgt ggt ttc aaa tgc ctg aat cct gtt       243
Gln Arg Cys Cys Gln Asp Thr Cys Gly Phe Lys Cys Leu Asn Pro Val
            65                  70                  75

ccc att cgt gga cca gtg aag aag cct ggg agg tgc ctc aaa ttt caa       291
Pro Ile Arg Gly Pro Val Lys Lys Pro Gly Arg Cys Leu Lys Phe Gln
        80                  85                  90

gga aaa tgt ctg atg ctt aac cct ccc aat aag tgc cag aat gac ggc       339
Gly Lys Cys Leu Met Leu Asn Pro Pro Asn Lys Cys Gln Asn Asp Gly
    95                  100                 105

cag tgt gat ggc aaa tac aaa tgt tgt gag ggt atg tgt ggg aaa gtc       387
Gln Cys Asp Gly Lys Tyr Lys Cys Cys Glu Gly Met Cys Gly Lys Val
110                 115                 120                 125

tgc ctt ccc cca gtg taagtctgat tcctgacatt tggaaatggc tctggacccg       442
Cys Leu Pro Pro Val
                130

tgctctatat gttctggaaa ctacttccct gctttcaggt ttcccggttc cgggttccgg     502

tgctctgggg tgcccaagct ttggacattt gaataatgtt tatttctgcc gccaacatat     562
```

cttttttgtcc catgtcagca ttcatcaagc ccctaaggaa atgctgttgg agacaaaata 622

aataaaggca atcatttctc taagcacaaa aaaaaaaaaa aaaaa 667

<210> 10
<211> 130
<212> PRT
<213> Rattus norvegicus

<400> 10

Met Lys Ser Ser Gly Leu Phe Pro Leu Met Val Leu Leu Ala Leu Gly
1 5 10 15

Val Leu Ala Pro Trp Ser Val Glu Gly Gly Lys Asn Asp Ala Ile Lys
20 25 30

Ile Gly Ala Cys Pro Ala Arg Lys Pro Ala Gln Cys Leu Lys Arg Glu
35 40 45

Lys Pro Glu Cys Ser Thr Asp Trp Gly Cys Pro Gly Lys Gln Arg Cys
50 55 60

Cys Gln Asp Thr Cys Gly Phe Lys Cys Leu Asn Pro Val Pro Ile Arg
65 70 75 80

Gly Pro Val Lys Lys Pro Gly Arg Cys Leu Lys Phe Gln Gly Lys Cys
85 90 95

Leu Met Leu Asn Pro Pro Asn Lys Cys Gln Asn Asp Gly Gln Cys Asp
100 105 110

Gly Lys Tyr Lys Cys Cys Glu Gly Met Cys Gly Lys Val Cys Leu Pro
115 120 125

Pro Val
130

<210> 11
<211> 1424
<212> DNA
<213> Rattus norvegicus

<220>
<221> CDS
<222> (237)..(1037)

<400> 11
cgcagcccag acaatgctta tccggaccgg actccagccc acttctgcca cctgaagggc 60

tccggttcgt gtgcaccggg aggcaccaga gtctaagggc atccgggaag ggactgttcg 120

gccggtcccc gggcagccgc tgggcgagag gaggaggctg cgccaaacag acagcaagca 180

agccttcgcc agcagttcat ctcctagacc tcatcagact ctgacccagt tcttga atg 239
Met
1

act ttg gag acc cag atg ttt cag aat gca cat tct gga agc caa tgg 287
Thr Leu Glu Thr Gln Met Phe Gln Asn Ala His Ser Gly Ser Gln Trp
5 10 15

cta ctc cca cca ctg aca atg ttg ctg ctg ttt gct ttt gca gac agg 335
Leu Leu Pro Pro Leu Thr Met Leu Leu Leu Phe Ala Phe Ala Asp Arg

```
            20                      25                      30

cag acg gga gat ctt ctg aag gct gtg gtg aaa cgt gat ccc cca tgg        383
Gln Thr Gly Asp Leu Leu Lys Ala Val Val Lys Arg Asp Pro Pro Trp
    35                  40                  45

atc cag gtg ctc aag gac gac act gtg acg ctg acg tgc gaa ggg acc        431
Ile Gln Val Leu Lys Asp Asp Thr Val Thr Leu Thr Cys Glu Gly Thr
50                  55                  60                  65

cac aat cct gga aac tct tct acc cag tgg ttc cac aac cag agc tcc        479
His Asn Pro Gly Asn Ser Ser Thr Gln Trp Phe His Asn Gln Ser Ser
                70                  75                  80

acc tgg ggc cag gtc caa gcc agc tac acg ttt aag gcc aca gtc aat        527
Thr Trp Gly Gln Val Gln Ala Ser Tyr Thr Phe Lys Ala Thr Val Asn
            85                  90                  95

gac agt gga gaa tac cgg tgc cga atg gcg cac acc agc ctc agc gac        575
Asp Ser Gly Glu Tyr Arg Cys Arg Met Ala His Thr Ser Leu Ser Asp
        100                 105                 110

ccc gta cat ctg gaa gtg att tct gac tgg ctg ctg ctc cag acc cct        623
Pro Val His Leu Glu Val Ile Ser Asp Trp Leu Leu Leu Gln Thr Pro
    115                 120                 125

caa ctg gtg ttt ctg gaa gga gaa aga atc aca ttg agg tgt cac ggc        671
Gln Leu Val Phe Leu Glu Gly Glu Arg Ile Thr Leu Arg Cys His Gly
130                 135                 140                 145

tgg aag agc ata cag ctg gcc agg att tca ttc ctc cag aat gga gaa        719
Trp Lys Ser Ile Gln Leu Ala Arg Ile Ser Phe Leu Gln Asn Gly Glu
                150                 155                 160

ttt gtg tca ttt cat cct tat aat gtt agc tac tcc atc tca aac gcc        767
Phe Val Ser Phe His Pro Tyr Asn Val Ser Tyr Ser Ile Ser Asn Ala
            165                 170                 175

aac cac agt cac agt ggg gac tac tac tgc aaa gca tat cta gga agg        815
Asn His Ser His Ser Gly Asp Tyr Tyr Cys Lys Ala Tyr Leu Gly Arg
        180                 185                 190

aca gaa cac gtg tcc aag cct gtc acc atc act gtc caa ggt tca gca        863
Thr Glu His Val Ser Lys Pro Val Thr Ile Thr Val Gln Gly Ser Ala
    195                 200                 205

acg gcg tcc acc agc tct cta gtg tgg ttc cat gcc gct ttc tgc cta        911
Thr Ala Ser Thr Ser Ser Leu Val Trp Phe His Ala Ala Phe Cys Leu
210                 215                 220                 225

gtg atg tgc ctc ctg ttt gca gtg gac acc ggc ctg tat ttc tgt gta        959
Val Met Cys Leu Leu Phe Ala Val Asp Thr Gly Leu Tyr Phe Cys Val
                230                 235                 240

cgg aga aat ctt caa acc tcg ggg gag gac tgg agg aga tcc ctg tca       1007
Arg Arg Asn Leu Gln Thr Ser Gly Glu Asp Trp Arg Arg Ser Leu Ser
            245                 250                 255

gtc gga aag tac aag gct cca cag gac aaa tgacatccca tcgtatggct         1057
Val Gly Lys Tyr Lys Ala Pro Gln Asp Lys
        260                 265

aaacagcggc agcttctttt cagccacacc gccacttcat ctacagcctt cctttgaaag    1117

caacttacca ccaggctgga tatttggtcc tgcaatcacc aactgctagg aggctgaggc    1177

aggaggatca ccaaaggctg cccgggtttt agagaagaga gagtgcaagt ctatcctgga    1237

taacccaata agaccctggg tttagatggc ttatcaggaa agagcaccca ttgctaagct    1297

caccagacaa gaccacagat tgatccctaa ggacacaggg caaagggaga gaacaaattc    1357

ctgaaagttg tcctctgacc tccacaggtc caccatggca gatgcacaca ataaacaaat    1417

taaaatg                                                               1424
```

<210> 12
<211> 267
<212> PRT
<213> Rattus norvegicus

<400> 12

Met Thr Leu Glu Thr Gln Met Phe Gln Asn Ala His Ser Gly Ser Gln
1 5 10 15

Trp Leu Leu Pro Pro Leu Thr Met Leu Leu Leu Phe Ala Phe Ala Asp
20 25 30

Arg Gln Thr Gly Asp Leu Leu Lys Ala Val Val Lys Arg Asp Pro Pro
35 40 45

Trp Ile Gln Val Leu Lys Asp Asp Thr Val Thr Leu Thr Cys Glu Gly
50 55 60

Thr His Asn Pro Gly Asn Ser Ser Thr Gln Trp Phe His Asn Gln Ser
65 70 75 80

Ser Thr Trp Gly Gln Val Gln Ala Ser Tyr Thr Phe Lys Ala Thr Val
85 90 95

Asn Asp Ser Gly Glu Tyr Arg Cys Arg Met Ala His Thr Ser Leu Ser
100 105 110

Asp Pro Val His Leu Glu Val Ile Ser Asp Trp Leu Leu Leu Gln Thr
115 120 125

Pro Gln Leu Val Phe Leu Glu Gly Glu Arg Ile Thr Leu Arg Cys His
130 135 140

Gly Trp Lys Ser Ile Gln Leu Ala Arg Ile Ser Phe Leu Gln Asn Gly
145 150 155 160

Glu Phe Val Ser Phe His Pro Tyr Asn Val Ser Tyr Ser Ile Ser Asn
165 170 175

Ala Asn His Ser His Ser Gly Asp Tyr Tyr Cys Lys Ala Tyr Leu Gly
180 185 190

Arg Thr Glu His Val Ser Lys Pro Val Thr Ile Thr Val Gln Gly Ser
195 200 205

Ala Thr Ala Ser Thr Ser Ser Leu Val Trp Phe His Ala Ala Phe Cys
210 215 220

Leu Val Met Cys Leu Leu Phe Ala Val Asp Thr Gly Leu Tyr Phe Cys
225 230 235 240

Val Arg Arg Asn Leu Gln Thr Ser Gly Glu Asp Trp Arg Arg Ser Leu
245 250 255

Ser Val Gly Lys Tyr Lys Ala Pro Gln Asp Lys
260 265

```
<210>  13
<211>  1396
<212>  DNA
<213>  Rattus norvegicus


<220>
<221>  CDS
<222>  (33)..(377)

<400>  13
ccccagacca gctgggcctg tccttcagga tg atg aga cat tcc aag act gcc        53
                                    Met Arg His Ser Lys Thr Ala
                                    1               5

att tca ttc ttt atc ctt gtg gcc gtc ctt ggg tcc cag gct ggg ctc       101
Ile Ser Phe Phe Ile Leu Val Ala Val Leu Gly Ser Gln Ala Gly Leu
        10                  15                  20

ata caa gat acg gta aaa gaa gat cgt ccc ttt aat cct aca ata att       149
Ile Gln Asp Thr Val Lys Glu Asp Arg Pro Phe Asn Pro Thr Ile Ile
    25                  30                  35

cac caa ggc ttt caa gac tct tca gac tgc tgc ttc tcc tat gcc tca       197
His Gln Gly Phe Gln Asp Ser Ser Asp Cys Cys Phe Ser Tyr Ala Ser
40                  45                  50                  55

cag atc cca tgt tca aga ttt ata tat tat ttt cca acc agt ggt ggg       245
Gln Ile Pro Cys Ser Arg Phe Ile Tyr Tyr Phe Pro Thr Ser Gly Gly
                60                  65                  70

tgc acc aag cca ggc atc atc ttt gtc acc agg aag agg aag cgg gtc       293
Cys Thr Lys Pro Gly Ile Ile Phe Val Thr Arg Lys Arg Lys Arg Val
            75                  80                  85

tgt gcc aac ccg agt gat cag aga gtt cag acg tgc ata agc acc ctg       341
Cys Ala Asn Pro Ser Asp Gln Arg Val Gln Thr Cys Ile Ser Thr Leu
        90                  95                  100

aag cta ggc cca aga tct ggg aac agt gcc att gct tgagaaggag            387
Lys Leu Gly Pro Arg Ser Gly Asn Ser Ala Ile Ala
    105                 110                 115

ggcgtactgc cacctgcatt cttctgtctc cccagtgacc acctaggaag cgctaagcat     447

ttgtttttat agatatttaa agcacgtatt cttctgtaca ggtttaaagc agcacatgta     507

gtccacagta ttcacatgga ttcagcctga catgattaga gccatctcga ggtgtaacca     567

tcatgagtgt ctttgggtaa ctgttgggtt ttcttgcata gttcctcagc agattataaa     627

tggataaact actagtcttt gggactttgg aatgtgtctg gttttgatgc aagcttaagc     687

caggtgtatc cagctgagat gaaatcaatt tttgccctaa gccatacata tgtccaactt     747

tgtggggacc cagctgttct tcctgccacc atagagcagt aagtgacccc aataaagtgc     807

actccatgta gctatgggtc tgtctctctt cagattcata gggctgtggg gccagtctga     867

aatcgggcta ttagaatgaa agtatcccac tcactcgctc gctcaccttg aatacagcag     927

ggagaaaggg atcatataac tgaatattct aaatgatata aatttctaat atataatatt     987

tctaaatata atttctaaat attctaatga tagaaatttt aaaaaaaaat taaaacacag    1047

ttttttccga tatattcctc tgagtttggt gcaaaatgga agaatgcctg ttccagcagg    1107

gcatcttctt ctctacctga ccttctctgg ggaacctgac ccccaaaccc tctaccaaag    1167

gaagttcaga tagctgtcga caaattgttc aacttccttt ttccagcaat tacctgggat    1227

tcccggaatg gctctccatg caaatgaggc attcccaata ctttaaactt catccaataa    1287

tttacatttt accccgaaaa ctcctaccca cactccaagg ttcatgtata ggcctggttc    1347

accccaaata aagtgtgcat gcgcaaaaaa aaaaaaaaaa aaaaaaaaa                1396
```

<210> 14
<211> 115
<212> PRT
<213> Rattus norvegicus

<400> 14

Met Arg His Ser Lys Thr Ala Ile Ser Phe Phe Ile Leu Val Ala Val
1 5 10 15

Leu Gly Ser Gln Ala Gly Leu Ile Gln Asp Thr Val Lys Glu Asp Arg
20 25 30

Pro Phe Asn Pro Thr Ile Ile His Gln Gly Phe Gln Asp Ser Ser Asp
35 40 45

Cys Cys Phe Ser Tyr Ala Ser Gln Ile Pro Cys Ser Arg Phe Ile Tyr
50 55 60

Tyr Phe Pro Thr Ser Gly Gly Cys Thr Lys Pro Gly Ile Ile Phe Val
65 70 75 80

Thr Arg Lys Arg Lys Arg Val Cys Ala Asn Pro Ser Asp Gln Arg Val
85 90 95

Gln Thr Cys Ile Ser Thr Leu Lys Leu Gly Pro Arg Ser Gly Asn Ser
100 105 110

Ala Ile Ala
115

<210> 15
<211> 604
<212> DNA
<213> Rattus norvegicus

<220>
<221> CDS
<222> (8)..(364)

<400> 15
cggcacg atg gct cgc tcg gtg acc gtg atc ttt ctg gtg ctt gtc tct 49
Met Ala Arg Ser Val Thr Val Ile Phe Leu Val Leu Val Ser
1 5 10

ctg gcc gtc gtg ctt gcc att cag aaa act ccc caa att caa gtg tac 97
Leu Ala Val Val Leu Ala Ile Gln Lys Thr Pro Gln Ile Gln Val Tyr
15 20 25 30

tct cgc cat cca ccg gag aat ggg aag ccc aac ttc ctc aac tgc tac 145
Ser Arg His Pro Pro Glu Asn Gly Lys Pro Asn Phe Leu Asn Cys Tyr
35 40 45

gtg tct cag ttc cac cca cct cag ata gaa att gag cta ctg aag aat 193
Val Ser Gln Phe His Pro Pro Gln Ile Glu Ile Glu Leu Leu Lys Asn
50 55 60

gga aag aag ata cca aat atc gag atg tca gat ctg tcc ttc agc aag 241
Gly Lys Lys Ile Pro Asn Ile Glu Met Ser Asp Leu Ser Phe Ser Lys
65 70 75

gac tgg tct ttc tac atc ctg gct cac act gaa ttc aca ccc acc gag 289
Asp Trp Ser Phe Tyr Ile Leu Ala His Thr Glu Phe Thr Pro Thr Glu
80 85 90

```
acc gat gta tat gct tgc aga gtt aaa cac gtc act ctg aag gag ccc      337
Thr Asp Val Tyr Ala Cys Arg Val Lys His Val Thr Leu Lys Glu Pro
95                  100                 105                 110

aaa acc gtc acc tgg gac cga gac atg taatcaagct ctatggagct            384
Lys Thr Val Thr Trp Asp Arg Asp Met
                115

ctgaatcatc tggaccagtt taactccaga tccggtttct aatatgctat acaatttatc    444

cacaaagtaa agaatagcaa tgagcacacc atcttcttca tatcttacct taaatatttt    504

atgcatgttt taaaaaaaat tggagactaa tatcctagat ttccggaata ataaagcttc    564

aatgagtgtt ttgatcagaa taataaatat ggttaagaac                          604


<210>  16
<211>  119
<212>  PRT
<213>  Rattus norvegicus

<400>  16

Met Ala Arg Ser Val Thr Val Ile Phe Leu Val Leu Val Ser Leu Ala
1               5                   10                  15


Val Val Leu Ala Ile Gln Lys Thr Pro Gln Ile Gln Val Tyr Ser Arg
            20                  25                  30


His Pro Pro Glu Asn Gly Lys Pro Asn Phe Leu Asn Cys Tyr Val Ser
        35                  40                  45


Gln Phe His Pro Pro Gln Ile Glu Ile Glu Leu Leu Lys Asn Gly Lys
    50                  55                  60


Lys Ile Pro Asn Ile Glu Met Ser Asp Leu Ser Phe Ser Lys Asp Trp
65                  70                  75                  80


Ser Phe Tyr Ile Leu Ala His Thr Glu Phe Thr Pro Thr Glu Thr Asp
                85                  90                  95


Val Tyr Ala Cys Arg Val Lys His Val Thr Leu Lys Glu Pro Lys Thr
            100                 105                 110


Val Thr Trp Asp Arg Asp Met
        115


<210>  17
<211>  1067
<212>  DNA
<213>  Rattus norvegicus


<220>
<221>  CDS
<222>  (111)..(710)

<400>  17
acggaggacc gtcagccagc aatcgcgtag ctcgatcgat cacccaggaa gagaaattgt     60

aagcagataa gaagaacgcg ttcggtttgg gacaccattt gcagctggaa atg ggg       116
                                                       Met Gly
                                                       1

aat gga ctg tca gac cag act tcc atc cta tcc agc ctg ccg tcc ttt      164
Asn Gly Leu Ser Asp Gln Thr Ser Ile Leu Ser Ser Leu Pro Ser Phe
        5                   10                  15
```

```
cag tcc ttt cac att gtt att ctg ggt ttg gac tgt gct gga aag aca      212
Gln Ser Phe His Ile Val Ile Leu Gly Leu Asp Cys Ala Gly Lys Thr
    20                  25                  30

aca gtt tta tac agg ctg cag ttc aac gaa ttt gta aat act gta cct      260
Thr Val Leu Tyr Arg Leu Gln Phe Asn Glu Phe Val Asn Thr Val Pro
35                  40                  45                  50

acc aaa gga ttt aac act gag aaa att aag gta acc ttg ggc aat tcc      308
Thr Lys Gly Phe Asn Thr Glu Lys Ile Lys Val Thr Leu Gly Asn Ser
                55                  60                  65

aaa aca gtc act ttt cac ttc tgg gat gta ggt ggt caa gag aaa tta      356
Lys Thr Val Thr Phe His Phe Trp Asp Val Gly Gly Gln Glu Lys Leu
            70                  75                  80

aga cca ctg tgg aag tca tat acc cga tgc aca gat ggc att gtg ttt      404
Arg Pro Leu Trp Lys Ser Tyr Thr Arg Cys Thr Asp Gly Ile Val Phe
        85                  90                  95

gtg gtg gac tct gtt gat gtt gag aga atg gaa gaa gcc aaa act gaa      452
Val Val Asp Ser Val Asp Val Glu Arg Met Glu Glu Ala Lys Thr Glu
    100                 105                 110

ctt cat aaa ata act agg ata tca gaa aat cag gga gtc cct gtg ctt      500
Leu His Lys Ile Thr Arg Ile Ser Glu Asn Gln Gly Val Pro Val Leu
115                 120                 125                 130

att gtt gct aac aaa caa gac ctg agg aac tca ctc tct ctc tca gag      548
Ile Val Ala Asn Lys Gln Asp Leu Arg Asn Ser Leu Ser Leu Ser Glu
                135                 140                 145

atc gag aag ttg tta gca atg ggt gaa ctg agc tca tcg act cct tgg      596
Ile Glu Lys Leu Leu Ala Met Gly Glu Leu Ser Ser Ser Thr Pro Trp
            150                 155                 160

cat tta cag ccc acc tgt gca atc ata gga gat ggg cta aag gaa gga      644
His Leu Gln Pro Thr Cys Ala Ile Ile Gly Asp Gly Leu Lys Glu Gly
        165                 170                 175

ctt gag aaa cta cat gat atg ata att aaa aga aga aaa atg ttg cgg      692
Leu Glu Lys Leu His Asp Met Ile Ile Lys Arg Arg Lys Met Leu Arg
    180                 185                 190

caa cag aaa aag aag aga tgaatggcag tacttttata tcggtgtgga             740
Gln Gln Lys Lys Lys Arg
195                 200

ataggtttta cttggtctga tttctgacaa gctgaagagt gtctacagcc tggtttgcct    800

gtctgccctc acggatgcta ttaaagcttt gttttgttga acagtcagat acccaactct    860

gttgccttgt ggaagatgag taaatgcaat gcttcttaaa ggggtctctt ctccgtgacc    920

cacaaatctt ttggtactac cattttggga agccaaaaaa ggctagtaat tgaccagaaa    980

acaattttgt ggaaatttga cctgaagtta gtgaaataaa actttgaaga gtgtaaaaaa   1040

aaaaaaaaaa aaaaaaaaaa aaaaaaa                                       1067


<210>  18
<211>  200
<212>  PRT
<213>  Rattus norvegicus

<400>  18

Met Gly Asn Gly Leu Ser Asp Gln Thr Ser Ile Leu Ser Ser Leu Pro
1               5                   10                  15


Ser Phe Gln Ser Phe His Ile Val Ile Leu Gly Leu Asp Cys Ala Gly
            20                  25                  30
```

```
Lys Thr Thr Val Leu Tyr Arg Leu Gln Phe Asn Glu Phe Val Asn Thr
        35                  40                  45

Val Pro Thr Lys Gly Phe Asn Thr Glu Lys Ile Lys Val Thr Leu Gly
    50                  55                  60

Asn Ser Lys Thr Val Thr Phe His Phe Trp Asp Val Gly Gly Gln Glu
65                  70                  75                  80

Lys Leu Arg Pro Leu Trp Lys Ser Tyr Thr Arg Cys Thr Asp Gly Ile
                85                  90                  95

Val Phe Val Val Asp Ser Val Asp Val Glu Arg Met Glu Glu Ala Lys
            100                 105                 110

Thr Glu Leu His Lys Ile Thr Arg Ile Ser Glu Asn Gln Gly Val Pro
        115                 120                 125

Val Leu Ile Val Ala Asn Lys Gln Asp Leu Arg Asn Ser Leu Ser Leu
    130                 135                 140

Ser Glu Ile Glu Lys Leu Leu Ala Met Gly Glu Leu Ser Ser Ser Thr
145                 150                 155                 160

Pro Trp His Leu Gln Pro Thr Cys Ala Ile Ile Gly Asp Gly Leu Lys
                165                 170                 175

Glu Gly Leu Glu Lys Leu His Asp Met Ile Ile Lys Arg Arg Lys Met
            180                 185                 190

Leu Arg Gln Gln Lys Lys Lys Arg
        195                 200
```

<210> 19
<211> 2524
<212> DNA
<213> Rattus norvegicus

<220>
<221> CDS
<222> (136)..(720)

<400> 19

```
ccctagtttc cctccccggg ccgcaggaga aacagtgccc tctcccttct tgacccctgg      60

cccttccctc ccttattccc tcttgccgcc gctgcttctg gcgctgccgc tcccggagga     120

gctcccgtca cggtg atg ggg tct cgg gcc tcc acg tta ctg cgg gac gaa      171
                 Met Gly Ser Arg Ala Ser Thr Leu Leu Arg Asp Glu
                 1               5                   10

gag ctc gag gag atc aag aag gag act ggc ttt tcc cac agt cag atc       219
Glu Leu Glu Glu Ile Lys Lys Glu Thr Gly Phe Ser His Ser Gln Ile
        15                  20                  25

aca cgc ctg tac agc cgc ttc acc agc ctg gac aaa ggg gag aac gga       267
Thr Arg Leu Tyr Ser Arg Phe Thr Ser Leu Asp Lys Gly Glu Asn Gly
    30                  35                  40

act ctc agc cgg gaa gat ttc cag agg att cca gaa ctt gcc atc aac       315
Thr Leu Ser Arg Glu Asp Phe Gln Arg Ile Pro Glu Leu Ala Ile Asn
45                  50                  55                  60
```

```
cca ctg ggg gac cgg atc atc aat gcc ttc ttc tca gag gga gag gat      363
Pro Leu Gly Asp Arg Ile Ile Asn Ala Phe Phe Ser Glu Gly Glu Asp
                65                  70                  75

cag gta aac ttc cga gga ttc atg aga act ttg gct cat ttc cga ccc      411
Gln Val Asn Phe Arg Gly Phe Met Arg Thr Leu Ala His Phe Arg Pro
            80                  85                  90

att gag gat aac gaa aag agc aaa gat gtg aat gga ccg gaa ccc ctc      459
Ile Glu Asp Asn Glu Lys Ser Lys Asp Val Asn Gly Pro Glu Pro Leu
        95                  100                 105

aac agc cgg agc aac aaa ctg cac ttt gct ttc aga ctt tat gac ttg      507
Asn Ser Arg Ser Asn Lys Leu His Phe Ala Phe Arg Leu Tyr Asp Leu
    110                 115                 120

gat aaa gat gac aag atc tcc cgc gat gag ctg tta cag gta ctg cga      555
Asp Lys Asp Asp Lys Ile Ser Arg Asp Glu Leu Leu Gln Val Leu Arg
125                 130                 135                 140

atg atg gtc gga gtg aat atc tca gat gag cag ctg ggc agt att gca      603
Met Met Val Gly Val Asn Ile Ser Asp Glu Gln Leu Gly Ser Ile Ala
                145                 150                 155

gac agg acc atc cag gag gct gac caa gat gga gac agt gcc ata tct      651
Asp Arg Thr Ile Gln Glu Ala Asp Gln Asp Gly Asp Ser Ala Ile Ser
            160                 165                 170

ttt aca gaa ttt gtt aag gtt ttg gag aag gtg gat gta gaa cag aag      699
Phe Thr Glu Phe Val Lys Val Leu Glu Lys Val Asp Val Glu Gln Lys
        175                 180                 185

atg agc atc cga ttt ctt cac taaagggcag agaccaaatt attcttgctt         750
Met Ser Ile Arg Phe Leu His
    190                 195

tctagtattt aagaactgaa acttccttct gtcctccagc cccaccccat cctgtcatcc    810

cccctttccca gaatactaca gctcctgcat gacaacctga agtctctcct gtccatacaa   870

acttgccttt ggtgtataaa cagggcgtta cagaatggta ccctgtctat ttctgttcgg    930

tatccaccca tcagtcttta ttgtaagtac caacttcccc tgttctactg ctcagtgcca    990

cgcatccgtc ctgtctgagg aaagaaacag ggactcatgt caagaaccag ccagcaaagc    1050

tcccactaga tgggaccata tccatgctgc cttcccttca tcagccctcc atcatggtcc    1110

tgtgtgctgt gtcctgtgtc ctgtgtctgt ttcttcctac ctctctgtca tccagccttc    1170

tctccattca gtctttcagc tgtcctcctg tcgttgagat tccctgcatt ctacttgact    1230

ctgggctctg ctggtaagcc tgtttctgat gtggcaggat agttagtggt ctggtgatct    1290

gtatctacct tctgatgtgg gatattgcct atccaggagc tattgctctg tcgtcacagg    1350

gcttatgagc agacatgttc ttaaaaggta gtcatggtga ggcttgtaga gccatgtctc    1410

ctaggtggct tcccaggtct ctctacctct gactttttat tttcaaatat atttatgtgg    1470

cttttctggg tacagtgttg acattcagaa aatagtctaa tgcctgccag tttgcttctc    1530

tttgggacat tgtcaccacc caagcggctg ttctgacaat atggtagagg gccttcttgt    1590

gttttctgac aaggatgggc accctggaga gctgcccaca tttgctccac agcatgggtt    1650

atgcctcagt gttctgtaca cttgaagatt cttcacattt ccaaacagaa agactagtgt    1710

tacagaagtg cctgacttac ctcagtccta aagaattaga ttgtcctcag tgtcttaaag    1770

ttttgcacaa aactctgaat gaattttata cttggcaaat tttaatgctg gaagatgtat    1830

agtcagttca taatcctggt ccaggcactg tatgcatcga atgtattgtc tcatgagctt    1890

cctaatgaca cgaccttcct agctccttgt gaatactata ctatgcagat ccctcagaag    1950
```

catgggacct gggactggtg gcaccaactg aggtgacatt ctctgccaat gtcagcttga 2010

tttagatgtc actgaatcaa gctgcttccg ccctgtgttc ctgtgaatct gctcttggct 2070

ggctttctct ggtgttgcct gcttgcttgc ttgctttgtt gctttggaag gctatccaag 2130

atatgtaagc aattctttag gaaataaatt gctgttaacc caaacaaaca aaaacactgc 2190

agacgagatg ggtaaaggcg agcagtgggg gcgtggctag gacagatggc tagggtctac 2250

atgaactggg gaattcttta tgaaatatgt cagactttgt gatcaaatga tgtgatataa 2310

gaattgtata aagggaataa ttgtctgata cttatctgtt tgagaagtac ttcagaagca 2370

tcttgattta attatttaaa gtttctaaga ttatgccccc cttggctgta tagcaaactg 2430

tttttatcca cagttgtgcc ttattgtccc attaaaattg tattcctcaa tccaccaata 2490

aattaattgt cgttaaaaca aaaaaaaaaa aaaa 2524

<210> 20
<211> 195
<212> PRT
<213> Rattus norvegicus

<400> 20

Met Gly Ser Arg Ala Ser Thr Leu Leu Arg Asp Glu Glu Leu Glu Glu
1 5 10 15

Ile Lys Lys Glu Thr Gly Phe Ser His Ser Gln Ile Thr Arg Leu Tyr
20 25 30

Ser Arg Phe Thr Ser Leu Asp Lys Gly Glu Asn Gly Thr Leu Ser Arg
35 40 45

Glu Asp Phe Gln Arg Ile Pro Glu Leu Ala Ile Asn Pro Leu Gly Asp
50 55 60

Arg Ile Ile Asn Ala Phe Phe Ser Glu Gly Glu Asp Gln Val Asn Phe
65 70 75 80

Arg Gly Phe Met Arg Thr Leu Ala His Phe Arg Pro Ile Glu Asp Asn
85 90 95

Glu Lys Ser Lys Asp Val Asn Gly Pro Glu Pro Leu Asn Ser Arg Ser
100 105 110

Asn Lys Leu His Phe Ala Phe Arg Leu Tyr Asp Leu Asp Lys Asp Asp
115 120 125

Lys Ile Ser Arg Asp Glu Leu Leu Gln Val Leu Arg Met Met Val Gly
130 135 140

Val Asn Ile Ser Asp Glu Gln Leu Gly Ser Ile Ala Asp Arg Thr Ile
145 150 155 160

Gln Glu Ala Asp Gln Asp Gly Asp Ser Ala Ile Ser Phe Thr Glu Phe
165 170 175

Val Lys Val Leu Glu Lys Val Asp Val Glu Gln Lys Met Ser Ile Arg
180 185 190

```
Phe Leu His
        195


<210>  21
<211>  913
<212>  DNA
<213>  Rattus norvegicus


<220>
<221>  CDS
<222>  (227)..(646)

<400>  21
aagctgctac cggcgcctaa ccgttccccg cctcctggac gtcggctctt caggcggagc      60

ctctaggagg aagaggagaa caagagagaa actcttctgg cccgggaccg cggcccgggc     120

gggacccggg gccgagtcgc ctaggcgacg ttgaccgaga ccgagcagtc actggatccc     180

taaggaggcg gcattctggc agaggcctct ggcagcagac ccagag atg gca tca        235
                                                   Met Ala Ser
                                                   1

gta gat ttc aag acc tat gtg gac cag gcc tgc aga gct gca gag gaa       283
Val Asp Phe Lys Thr Tyr Val Asp Gln Ala Cys Arg Ala Ala Glu Glu
    5                   10                  15

ttt gtc aac gtc tac tac act act atg gat aat cgg cgg cgg ctg ctg       331
Phe Val Asn Val Tyr Tyr Thr Thr Met Asp Asn Arg Arg Arg Leu Leu
20                  25                  30                  35

tct cgc ctg tac atg ggc acg gcc act tta gta tgg aat gga aat gcc       379
Ser Arg Leu Tyr Met Gly Thr Ala Thr Leu Val Trp Asn Gly Asn Ala
                40                  45                  50

gtt tcg gga caa gaa tcc ttg agt gag ttt ttt gag atg ttg cct tcc       427
Val Ser Gly Gln Glu Ser Leu Ser Glu Phe Phe Glu Met Leu Pro Ser
            55                  60                  65

agt gag ttc caa atc aac gtg gta gac tgc cag cct gtt cat gat gaa       475
Ser Glu Phe Gln Ile Asn Val Val Asp Cys Gln Pro Val His Asp Glu
        70                  75                  80

gct acg cca agc cag acc acg gtc ctt gtg gtc atc tgt gga aca gtg       523
Ala Thr Pro Ser Gln Thr Thr Val Leu Val Val Ile Cys Gly Thr Val
    85                  90                  95

aaa ttt gag ggc aac aaa cag cgg gac ttc aac cag aac ttc atc ctg       571
Lys Phe Glu Gly Asn Lys Gln Arg Asp Phe Asn Gln Asn Phe Ile Leu
100                 105                 110                 115

act gct cag gcg tca ccc agt aac aca gtg tgg aag ata gcg agt gac       619
Thr Ala Gln Ala Ser Pro Ser Asn Thr Val Trp Lys Ile Ala Ser Asp
                120                 125                 130

tgc ttc cgc ttc cag gac tgg gcc agc tagtgggggt gacaaaggca             666
Cys Phe Arg Phe Gln Asp Trp Ala Ser
            135                 140

gcttggcttt agccaagctc tgcagagaaa tgccagtctc aaatctcagg acgtggagaa     726

cactagttca tttttgttgt cacagcagca ctgcaaactg gatgttcgac tcctagaaac     786

ccctttcctg gttatatact ggtttgtcat agttgccttt taaaagtagt aaactttcta     846

tttttctact tactcagtag agaacctgtt tctggaaatt gacaaataaa tatgctgctt     906

ctctgcc                                                               913


<210>  22
<211>  140
<212>  PRT
<213>  Rattus norvegicus
```

<400> 22

Met Ala Ser Val Asp Phe Lys Thr Tyr Val Asp Gln Ala Cys Arg Ala
1               5                   10                  15

Ala Glu Glu Phe Val Asn Val Tyr Tyr Thr Thr Met Asp Asn Arg Arg
            20                  25                  30

Arg Leu Leu Ser Arg Leu Tyr Met Gly Thr Ala Thr Leu Val Trp Asn
        35                  40                  45

Gly Asn Ala Val Ser Gly Gln Glu Ser Leu Ser Glu Phe Phe Glu Met
    50                  55                  60

Leu Pro Ser Ser Glu Phe Gln Ile Asn Val Val Asp Cys Gln Pro Val
65                  70                  75                  80

His Asp Glu Ala Thr Pro Ser Gln Thr Thr Val Leu Val Val Ile Cys
                85                  90                  95

Gly Thr Val Lys Phe Glu Gly Asn Lys Gln Arg Asp Phe Asn Gln Asn
            100                 105                 110

Phe Ile Leu Thr Ala Gln Ala Ser Pro Ser Asn Thr Val Trp Lys Ile
        115                 120                 125

Ala Ser Asp Cys Phe Arg Phe Gln Asp Trp Ala Ser
    130                 135                 140

<210> 23
<211> 2623
<212> DNA
<213> Rattus norvegicus

<220>
<221> CDS
<222> (61)..(867)

<400> 23
gagctcttgg agggagttga gcaccaggca tccagcggtt atgtcaaggc ccctgccaac    60

atg gcc agc gaa atc aag aag aag ctc ttc tgg agg gct gtg gtg gct    108
Met Ala Ser Glu Ile Lys Lys Lys Leu Phe Trp Arg Ala Val Val Ala
1               5                   10                  15

gag ttc ctg gcc atg acc ctc ttc gtc ttc atc agc atc ggt tct gcc    156
Glu Phe Leu Ala Met Thr Leu Phe Val Phe Ile Ser Ile Gly Ser Ala
            20                  25                  30

cta ggc ttc aat tac cca ctg gag aga aac cag acg ctg gtc cag gac    204
Leu Gly Phe Asn Tyr Pro Leu Glu Arg Asn Gln Thr Leu Val Gln Asp
        35                  40                  45

aat gtg aag gtg tca ctg gcc ttt ggt ctg agc atc gct act ctg gcc    252
Asn Val Lys Val Ser Leu Ala Phe Gly Leu Ser Ile Ala Thr Leu Ala
    50                  55                  60

caa agt gtg ggt cac atc agt ggt gct cac ctc aac cca gcg gtc aca    300
Gln Ser Val Gly His Ile Ser Gly Ala His Leu Asn Pro Ala Val Thr
65                  70                  75                  80

ctg ggg ctt ctg ctc agc tgt cag atc agc atc ctc cgg gct gtc atg    348
Leu Gly Leu Leu Leu Ser Cys Gln Ile Ser Ile Leu Arg Ala Val Met
                85                  90                  95

```
tat atc atc gcc cag tgt gtg gga gcc atc gtt gcc tcc gcc atc ctc      396
Tyr Ile Ile Ala Gln Cys Val Gly Ala Ile Val Ala Ser Ala Ile Leu
        100                 105                 110

tcc ggc atc acc tcc tcc ctg ctc gag aac tca ctt ggc cga aat gac      444
Ser Gly Ile Thr Ser Ser Leu Leu Glu Asn Ser Leu Gly Arg Asn Asp
    115                 120                 125

ctg gct cga ggt gtg aac tcc ggc cag ggc ctg ggc att gag atc att      492
Leu Ala Arg Gly Val Asn Ser Gly Gln Gly Leu Gly Ile Glu Ile Ile
    130                 135                 140

ggc acc ctg cag ctg gtg ctg tgc gtt ctg gct acc act gac cgg agg      540
Gly Thr Leu Gln Leu Val Leu Cys Val Leu Ala Thr Thr Asp Arg Arg
145                 150                 155                 160

cgc cga gac tta ggt ggc tca gcc cca ctt gcc att ggc ttg tct gtg      588
Arg Arg Asp Leu Gly Gly Ser Ala Pro Leu Ala Ile Gly Leu Ser Val
            165                 170                 175

gct ctt gga cac ctg ctg gcc att gac tac act ggc tgt ggg atc aac      636
Ala Leu Gly His Leu Leu Ala Ile Asp Tyr Thr Gly Cys Gly Ile Asn
        180                 185                 190

cct gcc cgg tca ttt ggc tct gct gtg ctc acc cgc aac ttc tca aac      684
Pro Ala Arg Ser Phe Gly Ser Ala Val Leu Thr Arg Asn Phe Ser Asn
    195                 200                 205

cac tgg att ttc tgg gtg gga cca ttc att ggg agt gcc ctg gca gtg      732
His Trp Ile Phe Trp Val Gly Pro Phe Ile Gly Ser Ala Leu Ala Val
    210                 215                 220

ctg atc tat gac ttc atc ctg gcc cca cgc agc agc gac ttt aca gac      780
Leu Ile Tyr Asp Phe Ile Leu Ala Pro Arg Ser Ser Asp Phe Thr Asp
225                 230                 235                 240

cgc atg aag gtg tgg acc agt ggc caa gtg gag gag tat gac ctg gat      828
Arg Met Lys Val Trp Thr Ser Gly Gln Val Glu Glu Tyr Asp Leu Asp
            245                 250                 255

gct gat gat atc aac tcc agg gtg gag atg aag ccc aaa tagaggaggc       877
Ala Asp Asp Ile Asn Ser Arg Val Glu Met Lys Pro Lys
        260                 265

ttggcctggg catccgtatg ggggtggggc agggatgggc ggagggaggg gagggctgaa    937
ctcatattat agacactctg acaagctgac caaagtcact ccccaagacc tggctgtccc    997
acatggtcta gccttgtctg ggcagtaat atcactgtct ttctttctgt ttcctggtct    1057
cagagcttcc tggggcccaa gacttatcaa ctcagcacct cactcctttg acatcatgga   1117
ggagtgaaag ggagggaccc acctgctagt tgtcccctca gagtgtgcta gaaagtcccc   1177
ctcactccaa agttgcccag caagacaccc accttaggtg cctgggattc tgccattgtt   1237
actttgtgcc tttgatcatg gccttcctta ggtaggcacc ttagccccaa aggcactttg   1297
agggagagga agtcccttag ctttcccctt tggtctgact tacctccagg acccttcccc   1357
ttaaactcac tctaagacct tggaattttc cctatgctga ggcctcagtg atcacgtctg   1417
taaagtggca aggaagggac agctttgggg actctgtaag tgtggacaag ccacaggttc   1477
tagaagggag agtctagaca gcactgcata gtgcacctgg ctgagctctt tcccaacatc   1537
cacagcccat gagcacgtgt ggatgagcta cagagcggag aagcagtcca ggatgctggg   1597
agtgcctgtt cacacagaga gccttccttt aggcaggtct gttgcagcag agtaaaggtc   1657
atttcatggt ggaggaagag ctcacgaaat ctctcctagc tctagtgggt ctgctcatgt   1717
accatggcac caactggctg tttccctcta gtctcccttg cagtgtagat gtgacgtgtg   1777
tgtttattaa agagcactgg gctattgcag cgtcatgtct gaggaaagaa gcagctagac   1837
```

atgcaacaga ccccagacag atgcccatgc cgggcacaca ggggtttgga tgcccctagt 1897

tcatcatgat caaggaggcc actcttgtcc tggcttatga gctgctggct ggatgaattt 1957

gaccagagcc tggacaatct gaaggggctc actatgtgac tccaggcaca gtctccttat 2017

tgcaaggacc tgatgctgtg gcttctgcta tagcccaaga acatctcaga gtgcattcag 2077

ctcagggctt gcatttagct ctctgggtta ttctattcaa tcccaccaag ccagagcagc 2137

tctaccactg tgccgttaac catgtcgtga accgagagcc acattcttca ggtgcttaga 2197

agcagcagaa tagtcaggag gccattgacc actggcatag tacagctgca tactcttctc 2257

cattccctag caggcactat actcacttca caggtcagga cactgaggac ccatatgctc 2317

aactatatca tattgggtca agctcagcag tcaaagccat gtttggccac ttcatagtca 2377

atgaagtgga ctcctctcat ttccagcttc tccgtttctg cctggacaat ggctaagggc 2437

cagggatggg ggtaggggtg ggagtggagg tggtgagagc tgtgctggag gggggcaggc 2497

aggcccatcc cacctggctc tggccactgt gaaccaggac cctgcatctg tctgcctgtg 2557

tatgtttctt tgcaattgga attccatctt atggtaagaa aataaaggac aatgacttgt 2617

aaggtc 2623

<210> 24
<211> 269
<212> PRT
<213> Rattus norvegicus

<400> 24

Met Ala Ser Glu Ile Lys Lys Lys Leu Phe Trp Arg Ala Val Val Ala
1 5 10 15

Glu Phe Leu Ala Met Thr Leu Phe Val Phe Ile Ser Ile Gly Ser Ala
20 25 30

Leu Gly Phe Asn Tyr Pro Leu Glu Arg Asn Gln Thr Leu Val Gln Asp
35 40 45

Asn Val Lys Val Ser Leu Ala Phe Gly Leu Ser Ile Ala Thr Leu Ala
50 55 60

Gln Ser Val Gly His Ile Ser Gly Ala His Leu Asn Pro Ala Val Thr
65 70 75 80

Leu Gly Leu Leu Leu Ser Cys Gln Ile Ser Ile Leu Arg Ala Val Met
85 90 95

Tyr Ile Ile Ala Gln Cys Val Gly Ala Ile Val Ala Ser Ala Ile Leu
100 105 110

Ser Gly Ile Thr Ser Ser Leu Leu Glu Asn Ser Leu Gly Arg Asn Asp
115 120 125

Leu Ala Arg Gly Val Asn Ser Gly Gln Gly Leu Gly Ile Glu Ile Ile
130 135 140

Gly Thr Leu Gln Leu Val Leu Cys Val Leu Ala Thr Thr Asp Arg Arg
145 150 155 160

```
Arg Arg Asp Leu Gly Gly Ser Ala Pro Leu Ala Ile Gly Leu Ser Val
                165                 170                 175

Ala Leu Gly His Leu Leu Ala Ile Asp Tyr Thr Gly Cys Gly Ile Asn
            180                 185                 190

Pro Ala Arg Ser Phe Gly Ser Ala Val Leu Thr Arg Asn Phe Ser Asn
        195                 200                 205

His Trp Ile Phe Trp Val Gly Pro Phe Ile Gly Ser Ala Leu Ala Val
    210                 215                 220

Leu Ile Tyr Asp Phe Ile Leu Ala Pro Arg Ser Ser Asp Phe Thr Asp
225                 230                 235                 240

Arg Met Lys Val Trp Thr Ser Gly Gln Val Glu Glu Tyr Asp Leu Asp
                245                 250                 255

Ala Asp Asp Ile Asn Ser Arg Val Glu Met Lys Pro Lys
            260                 265


<210>  25
<211>  2070
<212>  DNA
<213>  Rattus norvegicus


<220>
<221>  CDS
<222>  (1)..(1113)

<400>  25
atg acg tac cag tta gga gct cgg atg gat cag aaa ctg agc ccc agg       48
Met Thr Tyr Gln Leu Gly Ala Arg Met Asp Gln Lys Leu Ser Pro Arg
1               5                   10                  15

gcc cgc act ccg ggc agt atc tcc atc aat tta agg ctt tgc tca ggc       96
Ala Arg Thr Pro Gly Ser Ile Ser Ile Asn Leu Arg Leu Cys Ser Gly
            20                  25                  30

acc atg gtc ccc atc tcc tcg gtg gcc att aca ctg agg tgg tca gtg      144
Thr Met Val Pro Ile Ser Ser Val Ala Ile Thr Leu Arg Trp Ser Val
        35                  40                  45

cct gac aag cct tgc acc gtc ccg aac ctg ggc gcg gct cac cag gtg      192
Pro Asp Lys Pro Cys Thr Val Pro Asn Leu Gly Ala Ala His Gln Val
    50                  55                  60

ccg gac gcc cgc gct tcc gct aag ccc cgc ccc cca gac caa tgg cgc      240
Pro Asp Ala Arg Ala Ser Ala Lys Pro Arg Pro Pro Asp Gln Trp Arg
65                  70                  75                  80

ggc gac tgc ggg ggc gac gag ctc cca ttg gtc gag agg gcg cgg cgg      288
Gly Asp Cys Gly Gly Asp Glu Leu Pro Leu Val Glu Arg Ala Arg Arg
                85                  90                  95

tgg gcg ccc att ggc aga gct gcg gag ggg cgg ggc caa gtg ctc cat      336
Trp Ala Pro Ile Gly Arg Ala Ala Glu Gly Arg Gly Gln Val Leu His
            100                 105                 110

ggc ccc ggc ctg cgg gcg gcg ctc agc gct cgc tgc gag gcg cgg gcg      384
Gly Pro Gly Leu Arg Ala Ala Leu Ser Ala Arg Cys Glu Ala Arg Ala
        115                 120                 125

gaa gtg gcg acg ctg gcg tcc cgg gag gcg gcg ggc ggc gac agc ccg      432
Glu Val Ala Thr Leu Ala Ser Arg Glu Ala Ala Gly Gly Asp Ser Pro
    130                 135                 140

ggc gcg gac gcc ctc ggc gcg ggc tgg ggc atc acc aag ggc ctc gac      480
```

```
Gly Ala Asp Ala Leu Gly Ala Gly Trp Gly Ile Thr Lys Gly Leu Asp
145                 150                 155                 160

ccc gaa atg gcg ctg ctg gcc gag cac ctg ctc aag ccg cta ccc gca        528
Pro Glu Met Ala Leu Leu Ala Glu His Leu Leu Lys Pro Leu Pro Ala
                165                 170                 175

gac cgg cag atc gag acc ggg ccc ttc ttg gag gcg gtg gcc cac ctg        576
Asp Arg Gln Ile Glu Thr Gly Pro Phe Leu Glu Ala Val Ala His Leu
            180                 185                 190

ccg ccc ttc ttc gat tgc ctt ggg tcc ccg gtg ttc acg ccc atc aag        624
Pro Pro Phe Phe Asp Cys Leu Gly Ser Pro Val Phe Thr Pro Ile Lys
        195                 200                 205

gca gac ata agc ggt aac atc acc aaa atc aaa gcc gtc tat gac act        672
Ala Asp Ile Ser Gly Asn Ile Thr Lys Ile Lys Ala Val Tyr Asp Thr
    210                 215                 220

gac ccg gcc aag ttc aag act ctg cag aac atc ctg gag gtg gag aag        720
Asp Pro Ala Lys Phe Lys Thr Leu Gln Asn Ile Leu Glu Val Glu Lys
225                 230                 235                 240

ggg atg tat ggg gcg gag tgg ccc aaa gtg ggg gcc acg ctg gca ctg        768
Gly Met Tyr Gly Ala Glu Trp Pro Lys Val Gly Ala Thr Leu Ala Leu
                245                 250                 255

ctg tgg ctg aaa agg ggc ctg cgc ttc atc cag gtg ttc ctg cag agc        816
Leu Trp Leu Lys Arg Gly Leu Arg Phe Ile Gln Val Phe Leu Gln Ser
            260                 265                 270

atc tgc gat ggc gaa cgg gac gag aac cac ccc aac ctc atc cgg gta        864
Ile Cys Asp Gly Glu Arg Asp Glu Asn His Pro Asn Leu Ile Arg Val
        275                 280                 285

aac gcc aac aag gcc tat gag atg gcg cta aag aag tat cac ggc tgg        912
Asn Ala Asn Lys Ala Tyr Glu Met Ala Leu Lys Lys Tyr His Gly Trp
    290                 295                 300

ctg gtg cag aag atc ttc aag gcc gca ctg tat gca gca cca tac aag        960
Leu Val Gln Lys Ile Phe Lys Ala Ala Leu Tyr Ala Ala Pro Tyr Lys
305                 310                 315                 320

tct gac ttc cta aag gcg ctc tcc aag ggg cag aac gtg acg gag gag       1008
Ser Asp Phe Leu Lys Ala Leu Ser Lys Gly Gln Asn Val Thr Glu Glu
                325                 330                 335

gag tgc ctg gag aag atc cgc ctc ttc ctg gtc aac tac acg gcc acc       1056
Glu Cys Leu Glu Lys Ile Arg Leu Phe Leu Val Asn Tyr Thr Ala Thr
            340                 345                 350

atc gac gcc atc tat gaa atg tat acc aag atg aac gcg gag ctt gac       1104
Ile Asp Ala Ile Tyr Glu Met Tyr Thr Lys Met Asn Ala Glu Leu Asp
        355                 360                 365

tac acc gtg taggcgccca gacacacagc cccacgtgga cacacagccc               1153
Tyr Thr Val
    370

acatggacac acagcccacg tggacacaca gcccccacgtg ggcacaagca gccccatcac    1213

tgtgaaacaa gctgggatcc ccagctcagc ctggcccgcc tggccagcgt gggtctgcag     1273

ggggcggcct tagcttgtta gaggtctttt gttttcttgt ttttgttatc ttggcctttt     1333

ctaatggagc aataaacacc ctccactcac tccagcttcc ccagcagctc aggctgaacc     1393

aacctccttc cagtgtggtt aaaaccaatt aactttgcta acgatgaatt cctagggttt     1453

tgtctttccc tcccctacct agctcaggag gagcccaact caggggagac atacacctca     1513

cccagcaaag aagccaggtc catccggccc aagggtgtct tggatgtttg aaggacaatg     1573

gaggcggatg ggaaattcag cagggcccaa ctgtggtgtc tgtttaaaag gcgagctgac     1633

agggtcctca cggggctgca catctggcat taccagccct gttctctctc tccctctgtc     1693
```

ttgtcagaca ggtttttttg tttctcctga attgttacca gcctttcaaa tgaccgccta 1753

ggcacacaca ggcgaccgtc cgtgactgaa ctcgcattct tctgcataat gctttcttcc 1813

tcagggttgg ctcttccaac ccaagaaact gcctgtcacg gggccctcca gctgcggaga 1873

cccaagaaac tgccttgctg ccttaaaccg tccttcagga gctaaaagag ctatattttt 1933

tttaaagtat cggggttaaa cacatcctga aagaattgat gtatgttaaa aaaaaaaaaa 1993

acccaatgag gaacaattgg tgatttttat gcagaaacta aataatcctt aataaataaa 2053

tctatatttt gaaatca 2070

<210> 26
<211> 371
<212> PRT
<213> Rattus norvegicus

<400> 26

Met Thr Tyr Gln Leu Gly Ala Arg Met Asp Gln Lys Leu Ser Pro Arg
1 5 10 15

Ala Arg Thr Pro Gly Ser Ile Ser Ile Asn Leu Arg Leu Cys Ser Gly
20 25 30

Thr Met Val Pro Ile Ser Ser Val Ala Ile Thr Leu Arg Trp Ser Val
35 40 45

Pro Asp Lys Pro Cys Thr Val Pro Asn Leu Gly Ala Ala His Gln Val
50 55 60

Pro Asp Ala Arg Ala Ser Ala Lys Pro Arg Pro Pro Asp Gln Trp Arg
65 70 75 80

Gly Asp Cys Gly Gly Asp Glu Leu Pro Leu Val Glu Arg Ala Arg Arg
85 90 95

Trp Ala Pro Ile Gly Arg Ala Ala Glu Gly Arg Gly Gln Val Leu His
100 105 110

Gly Pro Gly Leu Arg Ala Ala Leu Ser Ala Arg Cys Glu Ala Arg Ala
115 120 125

Glu Val Ala Thr Leu Ala Ser Arg Glu Ala Ala Gly Gly Asp Ser Pro
130 135 140

Gly Ala Asp Ala Leu Gly Ala Gly Trp Gly Ile Thr Lys Gly Leu Asp
145 150 155 160

Pro Glu Met Ala Leu Leu Ala Glu His Leu Leu Lys Pro Leu Pro Ala
165 170 175

Asp Arg Gln Ile Glu Thr Gly Pro Phe Leu Glu Ala Val Ala His Leu
180 185 190

Pro Pro Phe Phe Asp Cys Leu Gly Ser Pro Val Phe Thr Pro Ile Lys
195 200 205

Ala Asp Ile Ser Gly Asn Ile Thr Lys Ile Lys Ala Val Tyr Asp Thr

210 215 220

Asp Pro Ala Lys Phe Lys Thr Leu Gln Asn Ile Leu Glu Val Glu Lys
225 230 235 240

Gly Met Tyr Gly Ala Glu Trp Pro Lys Val Gly Ala Thr Leu Ala Leu
245 250 255

Leu Trp Leu Lys Arg Gly Leu Arg Phe Ile Gln Val Phe Leu Gln Ser
260 265 270

Ile Cys Asp Gly Glu Arg Asp Glu Asn His Pro Asn Leu Ile Arg Val
275 280 285

Asn Ala Asn Lys Ala Tyr Glu Met Ala Leu Lys Lys Tyr His Gly Trp
290 295 300

Leu Val Gln Lys Ile Phe Lys Ala Ala Leu Tyr Ala Ala Pro Tyr Lys
305 310 315 320

Ser Asp Phe Leu Lys Ala Leu Ser Lys Gly Gln Asn Val Thr Glu Glu
325 330 335

Glu Cys Leu Glu Lys Ile Arg Leu Phe Leu Val Asn Tyr Thr Ala Thr
340 345 350

Ile Asp Ala Ile Tyr Glu Met Tyr Thr Lys Met Asn Ala Glu Leu Asp
355 360 365

Tyr Thr Val
370

<210> 27
<211> 882
<212> DNA
<213> Rattus norvegicus

<220>
<221> CDS
<222> (56)..(652)

<400> 27
ggctcacggt tggttctgtt tgtgagacct gtagctcgac tctgctgata gcaag atg 58
Met
1

tct tca gga aat gca aaa att ggg cat cct gct ccc agc ttc aaa gcc 106
Ser Ser Gly Asn Ala Lys Ile Gly His Pro Ala Pro Ser Phe Lys Ala
5 10 15

acg gct gtt atg ccg gat gga caa ttc aaa gat atc agc cta agt gat 154
Thr Ala Val Met Pro Asp Gly Gln Phe Lys Asp Ile Ser Leu Ser Asp
20 25 30

tac aaa gga aaa tat gtt gta ttc ttt ttt tac cct ctt gac ttt act 202
Tyr Lys Gly Lys Tyr Val Val Phe Phe Phe Tyr Pro Leu Asp Phe Thr
35 40 45

ttt gtg tgt ccc acg gag atc att gct ttc agt gat aga gca gaa gaa 250
Phe Val Cys Pro Thr Glu Ile Ile Ala Phe Ser Asp Arg Ala Glu Glu
50 55 60 65

ttt aag aaa ctc aac tgc caa gtg att gga gct tct gtg gat tct cac 298
Phe Lys Lys Leu Asn Cys Gln Val Ile Gly Ala Ser Val Asp Ser His

70 75 80

ttc tgt cat ctg gca tgg att aac aca ccc aag aaa caa gga gga ttg 346
Phe Cys His Leu Ala Trp Ile Asn Thr Pro Lys Lys Gln Gly Gly Leu
85 90 95

gga ccc atg aac att ccc ttg gta tca gat ccc aag cgc acc att gct 394
Gly Pro Met Asn Ile Pro Leu Val Ser Asp Pro Lys Arg Thr Ile Ala
100 105 110

cag gat tat gga gtc tta aaa gct gat gaa ggt atc tct ttc agg ggc 442
Gln Asp Tyr Gly Val Leu Lys Ala Asp Glu Gly Ile Ser Phe Arg Gly
115 120 125

ctc ttt att att gat gat aaa ggt atc ctt cgc cag ata aca ata aat 490
Leu Phe Ile Ile Asp Asp Lys Gly Ile Leu Arg Gln Ile Thr Ile Asn
130 135 140 145

gat ctt cct gtt ggc cgc tct gtg gat gag att ctg aga cta gtc cag 538
Asp Leu Pro Val Gly Arg Ser Val Asp Glu Ile Leu Arg Leu Val Gln
150 155 160

gcc ttc cag ttc act gac aaa cat ggt gaa gtg tgc cca gct ggc tgg 586
Ala Phe Gln Phe Thr Asp Lys His Gly Glu Val Cys Pro Ala Gly Trp
165 170 175

aaa cct ggc agt gat acc atc aag cct gat gtc aat aag agt aaa gag 634
Lys Pro Gly Ser Asp Thr Ile Lys Pro Asp Val Asn Lys Ser Lys Glu
180 185 190

tat ttc tct aag cag aag tgagcactgg accagttttc tggcagacag 682
Tyr Phe Ser Lys Gln Lys
195

ctttgagcag ccagaagaaa tttgtactct acacatgacg tggtgtgatt ccagataagc 742

ctttcctaca agggctaggg gtggttagcc tttcttccac tattggtaag gggcagacca 802

tcttatatca gtcacagaaa ccaacctgtt aattctctct ctctcttttt tttttttaag 862

tatctattaa acgtgaattc 882

<210> 28
<211> 199
<212> PRT
<213> Rattus norvegicus

<400> 28

Met Ser Ser Gly Asn Ala Lys Ile Gly His Pro Ala Pro Ser Phe Lys
1 5 10 15

Ala Thr Ala Val Met Pro Asp Gly Gln Phe Lys Asp Ile Ser Leu Ser
20 25 30

Asp Tyr Lys Gly Lys Tyr Val Val Phe Phe Phe Tyr Pro Leu Asp Phe
35 40 45

Thr Phe Val Cys Pro Thr Glu Ile Ile Ala Phe Ser Asp Arg Ala Glu
50 55 60

Glu Phe Lys Lys Leu Asn Cys Gln Val Ile Gly Ala Ser Val Asp Ser
65 70 75 80

His Phe Cys His Leu Ala Trp Ile Asn Thr Pro Lys Lys Gln Gly Gly
85 90 95

Leu Gly Pro Met Asn Ile Pro Leu Val Ser Asp Pro Lys Arg Thr Ile
100 105 110

```
Ala Gln Asp Tyr Gly Val Leu Lys Ala Asp Glu Gly Ile Ser Phe Arg
        115                 120                 125

Gly Leu Phe Ile Ile Asp Asp Lys Gly Ile Leu Arg Gln Ile Thr Ile
    130                 135                 140

Asn Asp Leu Pro Val Gly Arg Ser Val Asp Glu Ile Leu Arg Leu Val
145                 150                 155                 160

Gln Ala Phe Gln Phe Thr Asp Lys His Gly Glu Val Cys Pro Ala Gly
                165                 170                 175

Trp Lys Pro Gly Ser Asp Thr Ile Lys Pro Asp Val Asn Lys Ser Lys
            180                 185                 190

Glu Tyr Phe Ser Lys Gln Lys
        195

<210>  29
<211>  494
<212>  DNA
<213>  Rattus norvegicus

<220>
<221>  CDS
<222>  (86)..(400)

<400>  29
gggggcaagc tccgttgggt gcctaggatc catttccatc gggttctgcc gaaactcgtg      60

tggctccctc cccgcaacag ccaaa atg gtg aag ctg atc gag agc aag gaa       112
                            Met Val Lys Leu Ile Glu Ser Lys Glu
                            1               5

gct ttt cag gag gcc ctg gcc gct gcg gga gac aag ctt gtg gta gtg       160
Ala Phe Gln Glu Ala Leu Ala Ala Ala Gly Asp Lys Leu Val Val Val
10                  15                  20                  25

gac ttc tct gcc acg tgg tgt gga cct tgc aaa atg atc aag ccc ttc       208
Asp Phe Ser Ala Thr Trp Cys Gly Pro Cys Lys Met Ile Lys Pro Phe
                30                  35                  40

ttt cat tcc ctc tgt gac aag tat tcc aat gtg gtg ttc ctt gaa gta       256
Phe His Ser Leu Cys Asp Lys Tyr Ser Asn Val Val Phe Leu Glu Val
            45                  50                  55

gac gtg gat gac tgc cag gat gtt gct gca gac tgt gaa gtc aaa tgc       304
Asp Val Asp Asp Cys Gln Asp Val Ala Ala Asp Cys Glu Val Lys Cys
        60                  65                  70

atg ccg acc ttc cag ttc tat aaa aag ggt caa aag gtt ggg gag ttc       352
Met Pro Thr Phe Gln Phe Tyr Lys Lys Gly Gln Lys Val Gly Glu Phe
    75                  80                  85

tct ggt gct aac aag gaa aag ctc gaa gcc act att acg gag ttt gcc       400
Ser Gly Ala Asn Lys Glu Lys Leu Glu Ala Thr Ile Thr Glu Phe Ala
90                  95                  100                 105

taatcatgct ctgaaaagtg tgaccagctc cagcaggtta aaacctgtac ctttttttaat    460

ttgcaaaaaa aaaaaaaaaa aaaaaaaaaa aaaa                                 494

<210>  30
<211>  105
<212>  PRT
<213>  Rattus norvegicus
```

<400> 30

```
Met Val Lys Leu Ile Glu Ser Lys Glu Ala Phe Gln Glu Ala Leu Ala
1               5                   10                  15

Ala Ala Gly Asp Lys Leu Val Val Val Asp Phe Ser Ala Thr Trp Cys
            20                  25                  30

Gly Pro Cys Lys Met Ile Lys Pro Phe Phe His Ser Leu Cys Asp Lys
        35                  40                  45

Tyr Ser Asn Val Val Phe Leu Glu Val Asp Val Asp Asp Cys Gln Asp
    50                  55                  60

Val Ala Ala Asp Cys Glu Val Lys Cys Met Pro Thr Phe Gln Phe Tyr
65                  70                  75                  80

Lys Lys Gly Gln Lys Val Gly Glu Phe Ser Gly Ala Asn Lys Glu Lys
                85                  90                  95

Leu Glu Ala Thr Ile Thr Glu Phe Ala
            100                 105
```

<210> 31
<211> 974
<212> DNA
<213> Rattus norvegicus

<220>
<221> CDS
<222> (293)..(889)

<400> 31

```
aattcggcac gagccaacct tgagcatctg atcctcttgg cttctccttc ctatcgctga      60

gctggtaggc tggagacagt tgtttgggtg ccaacatcaa caaacgattt ctgtagttta     120

gcgtttatga ccctggcgtg aagatttaag gtctggatta agcctgttga cttctccagc     180

tacttctaaa tttttgtgca taggtgctct ggtctctgtt gctgcttagt tcttccagcc     240

ttcctcaatg ccagttttat aatatgcagg tctctcccct cagtaatcca gg atg gct     298
                                                          Met Ala
                                                          1

act ctg atc ttt gtt gat aag gat aac gaa gag cca ggc agc cgt ttg       346
Thr Leu Ile Phe Val Asp Lys Asp Asn Glu Glu Pro Gly Ser Arg Leu
        5                   10                  15

gca tct aag gat gga ttg aag ctg ggc tct ggt gtc aaa gcc tta gat       394
Ala Ser Lys Asp Gly Leu Lys Leu Gly Ser Gly Val Lys Ala Leu Asp
    20                  25                  30

ggg aaa ttg cag gtt tca acg cca cga gtc ggc aaa gtg ttc ggt gcc       442
Gly Lys Leu Gln Val Ser Thr Pro Arg Val Gly Lys Val Phe Gly Ala
35                  40                  45                  50

cca ggc ttg cct aaa gcc agc agg aag gct ctg gga act gtc aac aga       490
Pro Gly Leu Pro Lys Ala Ser Arg Lys Ala Leu Gly Thr Val Asn Arg
                55                  60                  65

gtt act gaa aag cca gtg aag agt agt aaa ccc ctg caa tcg aaa cag       538
Val Thr Glu Lys Pro Val Lys Ser Ser Lys Pro Leu Gln Ser Lys Gln
            70                  75                  80

ccg act ctg agt gtg aaa aag atc acc gag aag tct act aag aca caa       586
Pro Thr Leu Ser Val Lys Lys Ile Thr Glu Lys Ser Thr Lys Thr Gln
```

85 90 95

ggc tct gct cct gct cct gat gat gcc tac cca gaa ata gaa aag ttc 634
Gly Ser Ala Pro Ala Pro Asp Asp Ala Tyr Pro Glu Ile Glu Lys Phe
100 105 110

ttc ccc ttc gat cct cta gat ttt gag agt ttt gac ctg cct gaa gag 682
Phe Pro Phe Asp Pro Leu Asp Phe Glu Ser Phe Asp Leu Pro Glu Glu
115 120 125 130

cac cag atc tca ctt ctc ccc ttg aat gga gtg cct ctc atg atc ctg 730
His Gln Ile Ser Leu Leu Pro Leu Asn Gly Val Pro Leu Met Ile Leu
135 140 145

aat gaa gag agg ggg ctt gag aag ctg ctg cac ctg gac ccc cct tcc 778
Asn Glu Glu Arg Gly Leu Glu Lys Leu Leu His Leu Asp Pro Pro Ser
150 155 160

cct ctg cag aag ccc ttc cta ccg tgg gaa tct gat ccg ttg ccg tct 826
Pro Leu Gln Lys Pro Phe Leu Pro Trp Glu Ser Asp Pro Leu Pro Ser
165 170 175

cct ccc agc gcc ctc tcc gct ctg gat gtt gaa ttg ccg cct gtt tgt 874
Pro Pro Ser Ala Leu Ser Ala Leu Asp Val Glu Leu Pro Pro Val Cys
180 185 190

tac gat gca gat att taaacgtctt actcctttat agtttatgta agttgtatta 929
Tyr Asp Ala Asp Ile
195

ataaagcatt tgtgtgtaaa aaaaaaaaaa aaaactcgag agtac 974

<210> 32
<211> 199
<212> PRT
<213> Rattus norvegicus

<400> 32

Met Ala Thr Leu Ile Phe Val Asp Lys Asp Asn Glu Glu Pro Gly Ser
1 5 10 15

Arg Leu Ala Ser Lys Asp Gly Leu Lys Leu Gly Ser Gly Val Lys Ala
20 25 30

Leu Asp Gly Lys Leu Gln Val Ser Thr Pro Arg Val Gly Lys Val Phe
35 40 45

Gly Ala Pro Gly Leu Pro Lys Ala Ser Arg Lys Ala Leu Gly Thr Val
50 55 60

Asn Arg Val Thr Glu Lys Pro Val Lys Ser Ser Lys Pro Leu Gln Ser
65 70 75 80

Lys Gln Pro Thr Leu Ser Val Lys Lys Ile Thr Glu Lys Ser Thr Lys
85 90 95

Thr Gln Gly Ser Ala Pro Ala Pro Asp Asp Ala Tyr Pro Glu Ile Glu
100 105 110

Lys Phe Phe Pro Phe Asp Pro Leu Asp Phe Glu Ser Phe Asp Leu Pro
115 120 125

Glu Glu His Gln Ile Ser Leu Leu Pro Leu Asn Gly Val Pro Leu Met
130 135 140

Ile Leu Asn Glu Glu Arg Gly Leu Glu Lys Leu Leu His Leu Asp Pro
145 150 155 160

Pro Ser Pro Leu Gln Lys Pro Phe Leu Pro Trp Glu Ser Asp Pro Leu
165 170 175

Pro Ser Pro Pro Ser Ala Leu Ser Ala Leu Asp Val Glu Leu Pro Pro
180 185 190

Val Cys Tyr Asp Ala Asp Ile
195

<210> 33
<211> 3069
<212> DNA
<213> Rattus norvegicus

<220>
<221> CDS
<222> (1)..(1755)

<400> 33
atg gga aag ggc ttc ctg cag tgc ttc tct gct tgg ctt cct gga aaa 48
Met Gly Lys Gly Phe Leu Gln Cys Phe Ser Ala Trp Leu Pro Gly Lys
1 5 10 15

act gga gct cga gtc aaa caa ggg gca gtt gtg acc gat gtc tat gtc 96
Thr Gly Ala Arg Val Lys Gln Gly Ala Val Val Thr Asp Val Tyr Val
20 25 30

ata ccc gca gcc atg aga ctc cag cac aag cag atg gtg ctt cca aca 144
Ile Pro Ala Ala Met Arg Leu Gln His Lys Gln Met Val Leu Pro Thr
35 40 45

gtt gtc tac ggg ggg gag tgg gtt tcc ccg gag cca tgg gca tcc atc 192
Val Val Tyr Gly Gly Glu Trp Val Ser Pro Glu Pro Trp Ala Ser Ile
50 55 60

ctg ggg ttc ctg aca agg tcc agc ttc aca ctg ttt ttt ttc ttc act 240
Leu Gly Phe Leu Thr Arg Ser Ser Phe Thr Leu Phe Phe Phe Phe Thr
65 70 75 80

gtt cag cga gca cca ggg ctt ttt gga ctc tct aga aat ggc tcc tcg 288
Val Gln Arg Ala Pro Gly Leu Phe Gly Leu Ser Arg Asn Gly Ser Ser
85 90 95

gtt cca cca caa cca tca gct ttg tgt ggt gga gca ttc atc gca gta 336
Val Pro Pro Gln Pro Ser Ala Leu Cys Gly Gly Ala Phe Ile Ala Val
100 105 110

tcc acc ctt caa cac aag aga agg tca caa gtt cga agc ctc tgg aac 384
Ser Thr Leu Gln His Lys Arg Arg Ser Gln Val Arg Ser Leu Trp Asn
115 120 125

att cca ttt ggg gga tcc ctc gtt acc aca cag tct aat ggc aac aaa 432
Ile Pro Phe Gly Gly Ser Leu Val Thr Thr Gln Ser Asn Gly Asn Lys
130 135 140

gcg ttt ggt gtg ctc att gta gtg aaa gga cca tca agt gcc aag caa 480
Ala Phe Gly Val Leu Ile Val Val Lys Gly Pro Ser Ser Ala Lys Gln
145 150 155 160

gta gga gcc ccg cag atg aca gtt gtg agg agc agc ttg cta agg aac 528
Val Gly Ala Pro Gln Met Thr Val Val Arg Ser Ser Leu Leu Arg Asn
165 170 175

cgt cag cgt tat gtg aag gaa gga agc att gag ttt caa gcc cag cgt 576
Arg Gln Arg Tyr Val Lys Glu Gly Ser Ile Glu Phe Gln Ala Gln Arg
180 185 190

gat gtc agg cca atg act aaa gaa tac aaa gaa ggt gca ggc ttc ttg 624

```
Asp Val Arg Pro Met Thr Lys Glu Tyr Lys Glu Gly Ala Gly Phe Leu
        195                 200                 205

tgt cct aca gac tct caa ctc cag agt agt gct ttc cgc ctt ggg atc      672
Cys Pro Thr Asp Ser Gln Leu Gln Ser Ser Ala Phe Arg Leu Gly Ile
    210                 215                 220

gtg cgc tgt gat cac ctc ctc tcc ctg agc gat gca cgt tgt cct gag      720
Val Arg Cys Asp His Leu Leu Ser Leu Ser Asp Ala Arg Cys Pro Glu
225                 230                 235                 240

atc ata aag agt tac aag gag att cca ata gaa ccg gga atg cct tgt      768
Ile Ile Lys Ser Tyr Lys Glu Ile Pro Ile Glu Pro Gly Met Pro Cys
                245                 250                 255

ggc cac aag aag tgg agg tgg gtt ttg gcc tac acg cct gct acc cac      816
Gly His Lys Lys Trp Arg Trp Val Leu Ala Tyr Thr Pro Ala Thr His
            260                 265                 270

act tgc tca cat gac tgg cac aca gcg gaa gaa agc tac ccc agt tac      864
Thr Cys Ser His Asp Trp His Thr Ala Glu Glu Ser Tyr Pro Ser Tyr
        275                 280                 285

gta acg aag ccc aag aag gcc acg tgt cac cag att aac acc agc gaa      912
Val Thr Lys Pro Lys Lys Ala Thr Cys His Gln Ile Asn Thr Ser Glu
    290                 295                 300

ccc tta caa gag cag atc aca aga ctc aga gaa cat atg cat ctg gaa      960
Pro Leu Gln Glu Gln Ile Thr Arg Leu Arg Glu His Met His Leu Glu
305                 310                 315                 320

act ggc cag cac tgg agc cac gcc ccc tcc ggg ctg ctc ctc tct gat     1008
Thr Gly Gln His Trp Ser His Ala Pro Ser Gly Leu Leu Leu Ser Asp
                325                 330                 335

tgg cct tct cgg ggg ggc gga acc cta gcg tgc aat agt tac ggc ggc     1056
Trp Pro Ser Arg Gly Gly Gly Thr Leu Ala Cys Asn Ser Tyr Gly Gly
            340                 345                 350

aga gtt tgg tcg caa agc agg tgc tct gca gtc agc gcc atg gct gcc     1104
Arg Val Trp Ser Gln Ser Arg Cys Ser Ala Val Ser Ala Met Ala Ala
        355                 360                 365

cgc gct cct gag ctc gcg cgc tcc ctg ctt gct gcc ctt ctg gct ccc     1152
Arg Ala Pro Glu Leu Ala Arg Ser Leu Leu Ala Ala Leu Leu Ala Pro
    370                 375                 380

gcg cta gtg gca cta ctg gtg tcg ccg gcc tgg gga cgc gga ggc cgg     1200
Ala Leu Val Ala Leu Leu Val Ser Pro Ala Trp Gly Arg Gly Gly Arg
385                 390                 395                 400

gac cac ggg gac tgg gac gtg gac agg cga ctg cct cca ctg cca ccc     1248
Asp His Gly Asp Trp Asp Val Asp Arg Arg Leu Pro Pro Leu Pro Pro
                405                 410                 415

cgc gat gac ggg ccg cgc gtg gcc cgc ttc gtg act cac gtc tcg gac     1296
Arg Asp Asp Gly Pro Arg Val Ala Arg Phe Val Thr His Val Ser Asp
            420                 425                 430

tgg ggc tcg ctg gcc act atc tcc acg aga gag gag gtg cgc ggc agg     1344
Trp Gly Ser Leu Ala Thr Ile Ser Thr Arg Glu Glu Val Arg Gly Arg
        435                 440                 445

ccc ttc gcg gac atc atc tca att agt gac ggt cct ccg gga gaa ggc     1392
Pro Phe Ala Asp Ile Ile Ser Ile Ser Asp Gly Pro Pro Gly Glu Gly
    450                 455                 460

tca ggc gag ccc tac atg tac ctg agt ccg ctg caa caa gca gtg agc     1440
Ser Gly Glu Pro Tyr Met Tyr Leu Ser Pro Leu Gln Gln Ala Val Ser
465                 470                 475                 480

gac ctg cag gga aat cca gag gct acg ctg act atg tct tta gca cag     1488
Asp Leu Gln Gly Asn Pro Glu Ala Thr Leu Thr Met Ser Leu Ala Gln
                485                 490                 495

act gcc tac tgt agg aac cat gga ttt gat ccc cag agt ccc ctc tgt     1536
```

```
Thr Ala Tyr Cys Arg Asn His Gly Phe Asp Pro Gln Ser Pro Leu Cys
            500                 505                 510

gtt cat ata atg atg tcc ggg act gtg atc aaa gta aat gag aca gaa      1584
Val His Ile Met Met Ser Gly Thr Val Ile Lys Val Asn Glu Thr Glu
        515                 520                 525

gag ggc tac gca cgg gac tcg cta ttc atc aga cac cct gag atg aaa      1632
Glu Gly Tyr Ala Arg Asp Ser Leu Phe Ile Arg His Pro Glu Met Lys
    530                 535                 540

cac tgg cct cct agc cat aag tgg ttc ttt gct aag tta aaa ata agc      1680
His Trp Pro Pro Ser His Lys Trp Phe Phe Ala Lys Leu Lys Ile Ser
545                 550                 555                 560

aat atc tgg gtt ttg gac tac ttt ggt gga ccg aaa gta gtg aca cct      1728
Asn Ile Trp Val Leu Asp Tyr Phe Gly Gly Pro Lys Val Val Thr Pro
                565                 570                 575

gaa gaa tat ttt aac gtc aca ctg cag tgaagcaaat acggtgaact           1775
Glu Glu Tyr Phe Asn Val Thr Leu Gln
            580                 585

ggatcacacc agcgacaggt cttccaaggc tcatgcacac agagggctcg accctgcctg    1835

gctgctagcc cactctgcca cctcttgttt cgatttcctt tgcttgtctt tgcagagtgt    1895

tgtccattag aagacgtgct tgctggattt aaaaaaaaaa aaaaaagtct tgaagccatt    1955

ttcagagaaa aatgcccttc tttatgatca aggaagccat gctcggtgct ttttcctcag    2015

tggtgccctc agtagggtga catataccag ctgtagaccg cccattggca ctggaggtcc    2075

agaggtgcag atgctctgtg cctgatggcc actgtgcctg aaggtgctgt gctcacccag    2135

ctgcctcagg agccagtgca gaggggccgt atgaatcctc cagtccagga cagcagtgac    2195

cacggatgca gcgtcccatt cacgtcaacc tgtatagctg atcagcacaa ggcaaagctg    2255

ggtcttctgt aaaactccat aaacgtctta cattctaaag tcatcgttac ccagaacaaa    2315

ataaatcaga aagattttcc tgcatccatt ctccatctaa gtagactgta cactcactcc    2375

tcatccttcg gaatgtgcac gcctgagctg gggcgcagct ttcttggcac agagcttgcc    2435

aagcatgaat gggaccataa cgtcgaaaca accagccagc cacagacgac atctttatgt    2495

gcatgaaaaa gcaacctcag aagaaaaagg atttcgcata tgttgggctc tttttcgtaa    2555

gacttaaccc tgtgccagcc gcaatccaag ccctcctccg gcattctgta tcttgaagct    2615

cctggtgctg ccgcctgcgt cctctgtggg atgtaccacg gtactgtggc ataaaaacaa    2675

actacaacaa caacaaaaca tgtctctgca ccagaggatg gctcagcttg taagatgact    2735

gctctagcag aggacccaag ttcagtaccc gcaaggggtc agacaggcac atatgcccag    2795

aactccagct ccaggagatc aaatacctct agcctccaag gtcacctgca ctggaataca    2855

cacacccaca catagacaca cagcgacaca atttcaaaca gtacatcttt tattccaaag    2915

aggtactact tagtttaatt acctgtttca tgctcttgga tgacataaga tttcttttaa    2975

cagagtgtgg gggaatcaag acactgactt aaacagccaa aaaaaaaaaa aaaaaagtta    3035

actatgaacc atcatgttgg agaaagagat ggta                                3069
```

<210> 34
<211> 585
<212> PRT
<213> Rattus norvegicus

<400> 34

```
Met Gly Lys Gly Phe Leu Gln Cys Phe Ser Ala Trp Leu Pro Gly Lys
```

```
1               5                   10                  15

Thr Gly Ala Arg Val Lys Gln Gly Ala Val Val Thr Asp Val Tyr Val
            20                  25                  30

Ile Pro Ala Ala Met Arg Leu Gln His Lys Gln Met Val Leu Pro Thr
        35                  40                  45

Val Val Tyr Gly Gly Glu Trp Val Ser Pro Glu Pro Trp Ala Ser Ile
    50                  55                  60

Leu Gly Phe Leu Thr Arg Ser Ser Phe Thr Leu Phe Phe Phe Phe Thr
65                  70                  75                  80

Val Gln Arg Ala Pro Gly Leu Phe Gly Leu Ser Arg Asn Gly Ser Ser
                85                  90                  95

Val Pro Pro Gln Pro Ser Ala Leu Cys Gly Gly Ala Phe Ile Ala Val
            100                 105                 110

Ser Thr Leu Gln His Lys Arg Arg Ser Gln Val Arg Ser Leu Trp Asn
        115                 120                 125

Ile Pro Phe Gly Gly Ser Leu Val Thr Thr Gln Ser Asn Gly Asn Lys
    130                 135                 140

Ala Phe Gly Val Leu Ile Val Val Lys Gly Pro Ser Ser Ala Lys Gln
145                 150                 155                 160

Val Gly Ala Pro Gln Met Thr Val Val Arg Ser Ser Leu Leu Arg Asn
                165                 170                 175

Arg Gln Arg Tyr Val Lys Glu Gly Ser Ile Glu Phe Gln Ala Gln Arg
            180                 185                 190

Asp Val Arg Pro Met Thr Lys Glu Tyr Lys Glu Gly Ala Gly Phe Leu
        195                 200                 205

Cys Pro Thr Asp Ser Gln Leu Gln Ser Ser Ala Phe Arg Leu Gly Ile
    210                 215                 220

Val Arg Cys Asp His Leu Leu Ser Leu Ser Asp Ala Arg Cys Pro Glu
225                 230                 235                 240

Ile Ile Lys Ser Tyr Lys Glu Ile Pro Ile Glu Pro Gly Met Pro Cys
                245                 250                 255

Gly His Lys Lys Trp Arg Trp Val Leu Ala Tyr Thr Pro Ala Thr His
            260                 265                 270

Thr Cys Ser His Asp Trp His Thr Ala Glu Glu Ser Tyr Pro Ser Tyr
        275                 280                 285

Val Thr Lys Pro Lys Lys Ala Thr Cys His Gln Ile Asn Thr Ser Glu
    290                 295                 300

Pro Leu Gln Glu Gln Ile Thr Arg Leu Arg Glu His Met His Leu Glu
```

305 310 315 320

Thr Gly Gln His Trp Ser His Ala Pro Ser Gly Leu Leu Leu Ser Asp
325 330 335

Trp Pro Ser Arg Gly Gly Gly Thr Leu Ala Cys Asn Ser Tyr Gly Gly
340 345 350

Arg Val Trp Ser Gln Ser Arg Cys Ser Ala Val Ser Ala Met Ala Ala
355 360 365

Arg Ala Pro Glu Leu Ala Arg Ser Leu Leu Ala Ala Leu Leu Ala Pro
370 375 380

Ala Leu Val Ala Leu Leu Val Ser Pro Ala Trp Gly Arg Gly Gly Arg
385 390 395 400

Asp His Gly Asp Trp Asp Val Asp Arg Arg Leu Pro Pro Leu Pro Pro
405 410 415

Arg Asp Asp Gly Pro Arg Val Ala Arg Phe Val Thr His Val Ser Asp
420 425 430

Trp Gly Ser Leu Ala Thr Ile Ser Thr Arg Glu Glu Val Arg Gly Arg
435 440 445

Pro Phe Ala Asp Ile Ile Ser Ile Ser Asp Gly Pro Pro Gly Glu Gly
450 455 460

Ser Gly Glu Pro Tyr Met Tyr Leu Ser Pro Leu Gln Gln Ala Val Ser
465 470 475 480

Asp Leu Gln Gly Asn Pro Glu Ala Thr Leu Thr Met Ser Leu Ala Gln
485 490 495

Thr Ala Tyr Cys Arg Asn His Gly Phe Asp Pro Gln Ser Pro Leu Cys
500 505 510

Val His Ile Met Met Ser Gly Thr Val Ile Lys Val Asn Glu Thr Glu
515 520 525

Glu Gly Tyr Ala Arg Asp Ser Leu Phe Ile Arg His Pro Glu Met Lys
530 535 540

His Trp Pro Pro Ser His Lys Trp Phe Phe Ala Lys Leu Lys Ile Ser
545 550 555 560

Asn Ile Trp Val Leu Asp Tyr Phe Gly Gly Pro Lys Val Val Thr Pro
565 570 575

Glu Glu Tyr Phe Asn Val Thr Leu Gln
580 585

<210> 35
<211> 3974
<212> DNA
<213> Rattus norvegicus

<220>
<221> CDS
<222> (131)..(529)

<400> 35
tcgactaccc atattcccca ccagccaacc ggttcctcac caagatgtgg cacccaaaca 60

tctatgaggt aagacactag aggcctgctt caggcacact ccaccacaca cttctctgcc 120

tcccttgcac atg ctc cac ccc tcc tct aac cct ggt cac tct tgc aga 169
Met Leu His Pro Ser Ser Asn Pro Gly His Ser Cys Arg
1 5 10

cag ggg acg tat gca tct cca tcc acc atc ccc cag ttg atg acc cac 217
Gln Gly Thr Tyr Ala Ser Pro Ser Thr Ile Pro Gln Leu Met Thr His
15 20 25

aga gtg gga gct acc ctc aga gcg gtg gaa ccc cac gca gaa tgt cag 265
Arg Val Gly Ala Thr Leu Arg Ala Val Glu Pro His Ala Glu Cys Gln
30 35 40 45

gtg aga att gcc atg gaa ctg cac cgt ggg cac ccg caa aca ccc tgt 313
Val Arg Ile Ala Met Glu Leu His Arg Gly His Pro Gln Thr Pro Cys
50 55 60

acc cta aga tcc acc ctt ccc tgc tcc agt cag ggg ttc cca gag tct 361
Thr Leu Arg Ser Thr Leu Pro Cys Ser Ser Gln Gly Phe Pro Glu Ser
65 70 75

ggg gag ctg agc ttg cac cta cgg ccc tac agg acc atc ctc ctg agt 409
Gly Glu Leu Ser Leu His Leu Arg Pro Tyr Arg Thr Ile Leu Leu Ser
80 85 90

gtg atc tcc ctg ctg aat gag ccc aac acc ttc tcg cct gcc aac gtg 457
Val Ile Ser Leu Leu Asn Glu Pro Asn Thr Phe Ser Pro Ala Asn Val
95 100 105

gac gcc tcg gtg atg tac aga aaa tgg aag gag agc aag ggg aag gac 505
Asp Ala Ser Val Met Tyr Arg Lys Trp Lys Glu Ser Lys Gly Lys Asp
110 115 120 125

cgc gag tac acg gac atc atc cgg tgagtgcttc ctactgtgaa gagtgatctg 559
Arg Glu Tyr Thr Asp Ile Ile Arg
130

gggtgtgggt ggggtgggga cttgttgtat gtttccattt tttgcatccc gggctggatg 619

tgtggaggac tctggggccc ccagagcacg gagttgacca cccagggtgg ccaagatccc 679

ctctgccttg ttgttgactt aactccatga gcaactgggc tctctgggcc acaccctcac 739

tgtctgacag agagcatcaa tcacttgcat ttcgtgcttc tgaaacttag agcttttttt 799

tttttttttg caaagtggga agggggctga gccccactgtg agtctgtcct cagttaggga 859

ccagggctgc gacctgttgc tgtctatgag tggcagtgtc cacacctatc atcgcttctt 919

agcaggagct cctggcctgt ctggggccag gcagtctccc ctacacccct cacgttgggt 979

atgaagttgc acttgagggg ttcagtcttt gtcgcttagc ttctgtgttt tctgagacag 1039

gctttcatat aactccgtat ctccttgtgg tccctctgat gtcaggatga caacacacat 1099

tcctcggaag tgaccccagt gcttgtctac acagtgaccc aaacacaggg cctgtcctgt 1159

ccccaagcct gccctctcag agttggtaca ggccgcagct cggtaggcag tgctggtcta 1219

gcatgtgcac acacacaggg ttagtctctg tcttaggcaa ggtggatgta gccagcatgg 1279

tccttcatgg aggtataagt tactcaggaa ggtctgagcg ctgcactacg gaggtgtccc 1339

cttcaccatg gtcaggtcag caacggtccc ggtcactgga agtccctgca gactgtcccc 1399

tttgttttct gttcagactg gctgaacaga actttgttcc tcctgctctt cctcagctga 1459

gaggaaggct ggctgcccta gctgttctat atagttctgg ctgtcctgga agaggagacc 1519
ctgggtctgg ccctgtcact gcactctcca ccctcctggc agcctttaat ccacttctgg 1579
ctgcttcctc cctggatccc tggcccagag caggagccca ggctattttt accattctga 1639
ctccaggagc cacttgctct gaccaggagg cctgtaccac tcctggaact caacctgagc 1699
cagtatctag tcatgtccct gcctgagcac aaggacagga tagctaggag cctgtgagaa 1759
ggaccttgag atggatctag gctattgggt gtgctgctgt cccacgtgcc cctccaccat 1819
agccttgtcc ccattcccct attccttctg ggcagctgaa aacagtgtct gctctgcttg 1879
actgcactgg gacctgtgcc ccaagactct ggcctgacgg tggcctggcc tctcaggcac 1939
ctagttaatt gctgcctgcc ctgtgcaagg aagcccttac atgttgcttc acccatggcc 1999
tgtgggacct ggaacctcag ttgttccgga actgcccttc atcatggtgc cctcaggtca 2059
ggctgcaatt tgttgttggt gtgaggaggt gcatgttacc tagcccacaa ctagggtagt 2119
gccccccaca gtctgggatt acagctgttt ctgtggtgct gggagctgga catgggtcct 2179
tatgcttaca gaaggagcac tctggctgcc cctgttgtcg gttatagccg aggtcagcct 2239
gaatctcata gtagttctcc tgcctccgcc tccaaggact gggtgacaga ccgtctccca 2299
cagctggctg tggttacatt tctgctggag ttggtcactg agccactcct tgtcgcctgg 2359
ccctctcctc ccacctgctc cctgcccccca ttcatctcat cgtcctgggc cgtggcctca 2419
cccgccctgt ctccccagga agcaggtcct ggggaccaag gtggacgcag agcgcgatgg 2479
cgtgaagtgc ccaccacgct ggccgagtac tgcgtgaaga ccaagcgccg gcgcccgacg 2539
agggctcaga cctcttctac gacgactact atgaggacgg tgaagtggag gaggccgaca 2599
gctgctttgg ggatgaagag gatgactctg gcaccgagga gtcctgaccc acgtccctga 2659
aataaactta caaattttac ctcagcagga cccgtgtggg cctcaggcga cagactacct 2719
caccgaggtt caatgtgggc tcccatccca tgatccttag gtcctcttac cctgtattcc 2779
ccatgggttt tcctcggctt tggagaagag ctgtggtgca gggcagacac cccactcagc 2839
cttcagagga tgacaagcct gggaggctgg gaaccgactg cccaggggag ccagagactc 2899
cagcccaaag agggccagcc ccacagtctc ctctctgctt ttggtgtgtg tgaaatccaa 2959
ataaaatagc tttctgagaa gctgagagaa gcagccagga cgccggtggc actgggtact 3019
gagggctacc tgggtaccca ggcagggctg tgggatgggc aggaagaggt agcgatggtg 3079
gcctgtttgt tggggctcag tagcatacag gagccccagg cccagaagaa acttgctgcc 3139
tgcaaatccc acccgcctgc atcgggcact gtggttgcgt ccaccccaag cctgtgttca 3199
ggaggcctgg gtcctgaggc ctacagggga ttggtattcc caacagtcct tgtgttctgg 3259
gcacttcctc agccctggaa actagtgctg cctgaggagt ccctctgata cctgagctgc 3319
agcctggtga ctccacagca ggggcaggct gagtgaattc tctgaccaca ccatgctgtg 3379
cagggcacct gcagtgaccc cgtgctgaga tagcagatct gctctgccac ctgcatgttt 3439
acgggtgctg tggaaagaaa gtgtcacttc ctcttatgcg ggcctgttaa tgtccaaagt 3499
tccagtcaac ccatgccagt cagaactgat cagagaagga cacatcggaa aaaggtgttc 3559
aggcgatcct ctgcccatac aatttgctgc cagcctgatc tacagttgca aaaccagcgg 3619
gaaggctcag tgggcaaagg cctgcaccaa gcctagcaga ggacccactt ggctccaggt 3679
ggtccctgtg gggggttgagg tcagttgaga caaatccccc cccccccccc ggagttcacc 3739

ccagtgctaa aaggagcagt gtgccaggag agggagcatc gcttggctgt agttgctgcc 3799

cccagacaca gggcaacttt gacagcagag ttgctttcta aaaccacaca gcagggtgtg 3859

aaggaggagg agctgacacg tgggtaaggt gggtggtgct tggaaggcca cttgtctcca 3919

ccggaagaga aagtgtgggg ccccttcccc cagggaccac cagtcctgtg ccagg 3974

<210> 36
<211> 133
<212> PRT
<213> Rattus norvegicus

<400> 36

```
Met Leu His Pro Ser Ser Asn Pro Gly His Ser Cys Arg Gln Gly Thr
1               5                   10                  15

Tyr Ala Ser Pro Ser Thr Ile Pro Gln Leu Met Thr His Arg Val Gly
            20                  25                  30

Ala Thr Leu Arg Ala Val Glu Pro His Ala Glu Cys Gln Val Arg Ile
        35                  40                  45

Ala Met Glu Leu His Arg Gly His Pro Gln Thr Pro Cys Thr Leu Arg
    50                  55                  60

Ser Thr Leu Pro Cys Ser Ser Gln Gly Phe Pro Glu Ser Gly Glu Leu
65                  70                  75                  80

Ser Leu His Leu Arg Pro Tyr Arg Thr Ile Leu Leu Ser Val Ile Ser
                85                  90                  95

Leu Leu Asn Glu Pro Asn Thr Phe Ser Pro Ala Asn Val Asp Ala Ser
            100                 105                 110

Val Met Tyr Arg Lys Trp Lys Glu Ser Lys Gly Lys Asp Arg Glu Tyr
        115                 120                 125

Thr Asp Ile Ile Arg
    130
```

<210> 37
<211> 1838
<212> DNA
<213> Rattus norvegicus

<220>
<221> CDS
<222> (169)..(1395)

<400> 37

```
cagctcctgc cccttcagtg acgggcggcc aagctgacag gagccggggc ggggcggcgg      60

cggccagcga aggagcgcgc ggcggcccgg ccccgcccct ggtcctcccg ccagccccag     120

tgaccttcac gggttcgggc ggcgggtgca ggcccgggcg gcggcgcg atg ttc gtg      177
                                                     Met Phe Val
                                                     1

cag gag gag aag atc ttt gcg ggc aag gtg ctt cgg ctg cac atc tgc      225
Gln Glu Glu Lys Ile Phe Ala Gly Lys Val Leu Arg Leu His Ile Cys
    5                   10                  15

gca gcc gac ggc tcc gag tgg ctg gag gag gcg acc gag gac aca tcg      273
```

```
Ala Ala Asp Gly Ser Glu Trp Leu Glu Glu Ala Thr Glu Asp Thr Ser
20                  25                  30                  35

gtg gag aag ctc aag gag agt tgc ctc aaa cat ggt gct cat ggg agc      321
Val Glu Lys Leu Lys Glu Ser Cys Leu Lys His Gly Ala His Gly Ser
                40                  45                  50

cta gaa gac ccc aag aat gtg act cac cat aag tta ata cat gct gct      369
Leu Glu Asp Pro Lys Asn Val Thr His His Lys Leu Ile His Ala Ala
            55                  60                  65

tca gag agg gtg ctg agt gac agc aaa acg atc ttg gaa gaa aac atc      417
Ser Glu Arg Val Leu Ser Asp Ser Lys Thr Ile Leu Glu Glu Asn Ile
        70                  75                  80

cag gac caa gac gtc ttg tta ttg ata aag aag cgt gct cct tcc cca      465
Gln Asp Gln Asp Val Leu Leu Leu Ile Lys Lys Arg Ala Pro Ser Pro
    85                  90                  95

atc cct aag atg gct gat gtc tca gca gaa gaa aag aag aaa caa gag      513
Ile Pro Lys Met Ala Asp Val Ser Ala Glu Glu Lys Lys Lys Gln Glu
100                 105                 110                 115

caa aag gct cca gac aaa gac gct att ctt cga gca acg gcc aac ctg      561
Gln Lys Ala Pro Asp Lys Asp Ala Ile Leu Arg Ala Thr Ala Asn Leu
                120                 125                 130

cct gcc tgt agc acg gac cgg act gct gtc cag acc acc atg aga gat      609
Pro Ala Cys Ser Thr Asp Arg Thr Ala Val Gln Thr Thr Met Arg Asp
            135                 140                 145

ttc cag aca gaa ctc cgg aag atc ctt gtg tct ctc att gag gtg gca      657
Phe Gln Thr Glu Leu Arg Lys Ile Leu Val Ser Leu Ile Glu Val Ala
        150                 155                 160

cag aag ctg tta gca ctg aat cct gat gca gtt gaa ttg ttt aag aag      705
Gln Lys Leu Leu Ala Leu Asn Pro Asp Ala Val Glu Leu Phe Lys Lys
    165                 170                 175

gca aat gct atg ttg gat gaa gac gag gat gag cga gtg gat gag acc      753
Ala Asn Ala Met Leu Asp Glu Asp Glu Asp Glu Arg Val Asp Glu Thr
180                 185                 190                 195

gcc ttg cgg cag ctc aca gag atg ggc ttt cct gag agc aga gcc tca      801
Ala Leu Arg Gln Leu Thr Glu Met Gly Phe Pro Glu Ser Arg Ala Ser
                200                 205                 210

aag gcc ctt cgg ctg aac cac atg tcg gtg cct cag gcc atg gag tgg      849
Lys Ala Leu Arg Leu Asn His Met Ser Val Pro Gln Ala Met Glu Trp
            215                 220                 225

ctg att gaa cat tcg gaa gat cca gct att gac aca cct ctc cca ggc      897
Leu Ile Glu His Ser Glu Asp Pro Ala Ile Asp Thr Pro Leu Pro Gly
        230                 235                 240

cat gcc gcc caa gcg gag gcg agt gct gca gct gcc acc tcc tcc tcc      945
His Ala Ala Gln Ala Glu Ala Ser Ala Ala Ala Ala Thr Ser Ser Ser
    245                 250                 255

tcc tct gaa gct gcc gtg gga act agt gtt gaa gat gag gag tcc cgg      993
Ser Ser Glu Ala Ala Val Gly Thr Ser Val Glu Asp Glu Glu Ser Arg
260                 265                 270                 275

gat gag ctc aca gaa atc ttc aag aag ata cgc agg aaa aag gaa ttc     1041
Asp Glu Leu Thr Glu Ile Phe Lys Lys Ile Arg Arg Lys Lys Glu Phe
                280                 285                 290

cga gcc gat gct cgg gct gtc att tcg ctg atg gag atg ggc ttt gat     1089
Arg Ala Asp Ala Arg Ala Val Ile Ser Leu Met Glu Met Gly Phe Asp
            295                 300                 305

gag aag gaa gtg att gat gcc ctc cga gtg aac aac aac cag cag aac     1137
Glu Lys Glu Val Ile Asp Ala Leu Arg Val Asn Asn Asn Gln Gln Asn
        310                 315                 320

gca gct tgt gaa tgg ctg ctg ggg gac cgg aag cca tcc cct gag gag     1185
```

Ala Ala Cys Glu Trp Leu Leu Gly Asp Arg Lys Pro Ser Pro Glu Glu
325 330 335

ttg gac cag ggc atc gat ccc aat agc cct ctc ttt cag gcc atc ctg 1233
Leu Asp Gln Gly Ile Asp Pro Asn Ser Pro Leu Phe Gln Ala Ile Leu
340 345 350 355

gac aac cca gtg gtg cag ctg ggt ttg aca aac cct aag aca ttg cta 1281
Asp Asn Pro Val Val Gln Leu Gly Leu Thr Asn Pro Lys Thr Leu Leu
360 365 370

gca ttt gaa gac atg ctg gag aac cca ctt aac agc acc cag tgg atg 1329
Ala Phe Glu Asp Met Leu Glu Asn Pro Leu Asn Ser Thr Gln Trp Met
375 380 385

aac gac cca gag acg ggc ccg gtc atg ctg cag atc tcg aga atc ttc 1377
Asn Asp Pro Glu Thr Gly Pro Val Met Leu Gln Ile Ser Arg Ile Phe
390 395 400

cag aca ctg aat cgc acg taggtggtgc ctactgtgct ccttggctgt 1425
Gln Thr Leu Asn Arg Thr
405

gcagtctcag cagtctgtga ggcggtgggc gcagggccca gccaggatct tgcctggatc 1485

acagcttcag tgactctcct ttcctgacct aacggcttgt catcttctgc cacatggttt 1545

gaaagttgca gtcccagctt ctgacaggtg tttacagaga gtggctattg cactgggaag 1605

gtggtgtttg ctgccccgtc ccctccagac actctgctgg gctcctgaag tgctacttga 1665

attgaccctt aggtgtttgc ttgtgtagcc caatgtattt taaagaaggc atgttctgat 1725

ccaaagtgtt atgtcaagtg tgctaattta actagactga ctcacagatg gcttctttaa 1785

taaataaacc cttttcctaa aaaaaaaaaa aaaaaaaaaa aaaaaaaaaa aaa 1838

<210> 38
<211> 409
<212> PRT
<213> Rattus norvegicus

<400> 38

Met Phe Val Gln Glu Glu Lys Ile Phe Ala Gly Lys Val Leu Arg Leu
1 5 10 15

His Ile Cys Ala Ala Asp Gly Ser Glu Trp Leu Glu Glu Ala Thr Glu
20 25 30

Asp Thr Ser Val Glu Lys Leu Lys Glu Ser Cys Leu Lys His Gly Ala
35 40 45

His Gly Ser Leu Glu Asp Pro Lys Asn Val Thr His His Lys Leu Ile
50 55 60

His Ala Ala Ser Glu Arg Val Leu Ser Asp Ser Lys Thr Ile Leu Glu
65 70 75 80

Glu Asn Ile Gln Asp Gln Asp Val Leu Leu Leu Ile Lys Lys Arg Ala
85 90 95

Pro Ser Pro Ile Pro Lys Met Ala Asp Val Ser Ala Glu Glu Lys Lys
100 105 110

Lys Gln Glu Gln Lys Ala Pro Asp Lys Asp Ala Ile Leu Arg Ala Thr
115 120 125

Ala Asn Leu Pro Ala Cys Ser Thr Asp Arg Thr Ala Val Gln Thr Thr
130 135 140

Met Arg Asp Phe Gln Thr Glu Leu Arg Lys Ile Leu Val Ser Leu Ile
145 150 155 160

Glu Val Ala Gln Lys Leu Leu Ala Leu Asn Pro Asp Ala Val Glu Leu
165 170 175

Phe Lys Lys Ala Asn Ala Met Leu Asp Glu Asp Glu Asp Glu Arg Val
180 185 190

Asp Glu Thr Ala Leu Arg Gln Leu Thr Glu Met Gly Phe Pro Glu Ser
195 200 205

Arg Ala Ser Lys Ala Leu Arg Leu Asn His Met Ser Val Pro Gln Ala
210 215 220

Met Glu Trp Leu Ile Glu His Ser Glu Asp Pro Ala Ile Asp Thr Pro
225 230 235 240

Leu Pro Gly His Ala Ala Gln Ala Glu Ala Ser Ala Ala Ala Ala Thr
245 250 255

Ser Ser Ser Ser Ser Glu Ala Ala Val Gly Thr Ser Val Glu Asp Glu
260 265 270

Glu Ser Arg Asp Glu Leu Thr Glu Ile Phe Lys Lys Ile Arg Arg Lys
275 280 285

Lys Glu Phe Arg Ala Asp Ala Arg Ala Val Ile Ser Leu Met Glu Met
290 295 300

Gly Phe Asp Glu Lys Glu Val Ile Asp Ala Leu Arg Val Asn Asn Asn
305 310 315 320

Gln Gln Asn Ala Ala Cys Glu Trp Leu Leu Gly Asp Arg Lys Pro Ser
325 330 335

Pro Glu Glu Leu Asp Gln Gly Ile Asp Pro Asn Ser Pro Leu Phe Gln
340 345 350

Ala Ile Leu Asp Asn Pro Val Val Gln Leu Gly Leu Thr Asn Pro Lys
355 360 365

Thr Leu Leu Ala Phe Glu Asp Met Leu Glu Asn Pro Leu Asn Ser Thr
370 375 380

Gln Trp Met Asn Asp Pro Glu Thr Gly Pro Val Met Leu Gln Ile Ser
385 390 395 400

Arg Ile Phe Gln Thr Leu Asn Arg Thr
405

<210> 39
<211> 1099
<212> DNA

<213> Rattus norvegicus

<220>
<221> CDS
<222> (16)..(537)

<400> 39
```
gagctgggcc agaag atg gcg gca gcc gcc gag ctg agg ctt ctg gag aag     51
                 Met Ala Ala Ala Ala Glu Leu Arg Leu Leu Glu Lys
                 1               5                   10

agt ctg gga ctg aag ccg ggg aat aag tac agc gct cag ggc gaa cga      99
Ser Leu Gly Leu Lys Pro Gly Asn Lys Tyr Ser Ala Gln Gly Glu Arg
        15                  20                  25

cag gtt cca gtc cta caa aca aac aat ggc ccc agt cta atg gga ttg     147
Gln Val Pro Val Leu Gln Thr Asn Asn Gly Pro Ser Leu Met Gly Leu
    30                  35                  40

agc acc att gcg acc cat ctg gtc aag gag gcc aac aag gag cac ttg     195
Ser Thr Ile Ala Thr His Leu Val Lys Glu Ala Asn Lys Glu His Leu
45                  50                  55                  60

ctg ggg agc act gca gaa gaa aag gca cta gtt cag cag tgg tta gag     243
Leu Gly Ser Thr Ala Glu Glu Lys Ala Leu Val Gln Gln Trp Leu Glu
                65                  70                  75

ttc agg atc act cga gta gat gga cac tcc agt aaa gaa gac acc cag     291
Phe Arg Ile Thr Arg Val Asp Gly His Ser Ser Lys Glu Asp Thr Gln
            80                  85                  90

act ctg ctg aag gat ctc aac tcc tat ctg gaa gac aaa gtc tac ctt     339
Thr Leu Leu Lys Asp Leu Asn Ser Tyr Leu Glu Asp Lys Val Tyr Leu
        95                  100                 105

gca gga cat aac act acc ctg gcg gac atc ctc ctg tac tac ggg ctc     387
Ala Gly His Asn Thr Thr Leu Ala Asp Ile Leu Leu Tyr Tyr Gly Leu
    110                 115                 120

cac cgc ttt ata gtt gac ctg aca gtt caa gaa aag gag aaa tat ctc     435
His Arg Phe Ile Val Asp Leu Thr Val Gln Glu Lys Glu Lys Tyr Leu
125                 130                 135                 140

aat gtg tct cgc tgg ttt tgc cac att caa cat tac cca gac atc agg     483
Asn Val Ser Arg Trp Phe Cys His Ile Gln His Tyr Pro Asp Ile Arg
                145                 150                 155

cag cat ctg tct agt gtt gtc ttc atc aag aac aga ctc tat gcc aac     531
Gln His Leu Ser Ser Val Val Phe Ile Lys Asn Arg Leu Tyr Ala Asn
            160                 165                 170

tcc cac taggagcccc cagcagcagg atagaagtga aggggaaagt gtgttctgga      587
Ser His

ccatggtact aatgtgaact gatatgacgt cattgtccct tgtgaaagtg taagctcaag   647

tctgagtgtt ttacttgtcc cttagttgag gctttgtaat ttttaatgta aaagttcagc   707

tgttatccaa gcaaatcacg atcaaattac aaatctcgct ttttatgagc gaagttgatt   767

ttaataacag ttgcaaatca atggctcaac tttaaaaaaa cattgctgtt ggtgtcacat   827

ctggctgttc actttttcat gtttttaaaa ttcatttttc tcttaggtat ttattgtagt   887

ttatttacta gaacctacga caaattatgt tgagcaatca gaaagggaag atagatatat   947

ttgttctcaa aatatttatg tgaactagta aatgtggttt taaaaatcac cttttgttat  1007

ctttcctgta gtcagtgttt actgagcccc ccactgctag ggaattaaat gtgtttgttc  1067

ctcttaaaaa caaaacaaca acaacaaaaa ct                                 1099
```

<210> 40

<211> 174
<212> PRT
<213> Rattus norvegicus

<400> 40

Met Ala Ala Ala Ala Glu Leu Arg Leu Leu Glu Lys Ser Leu Gly Leu
1 5 10 15

Lys Pro Gly Asn Lys Tyr Ser Ala Gln Gly Glu Arg Gln Val Pro Val
20 25 30

Leu Gln Thr Asn Asn Gly Pro Ser Leu Met Gly Leu Ser Thr Ile Ala
35 40 45

Thr His Leu Val Lys Glu Ala Asn Lys Glu His Leu Leu Gly Ser Thr
50 55 60

Ala Glu Glu Lys Ala Leu Val Gln Gln Trp Leu Glu Phe Arg Ile Thr
65 70 75 80

Arg Val Asp Gly His Ser Ser Lys Glu Asp Thr Gln Thr Leu Leu Lys
85 90 95

Asp Leu Asn Ser Tyr Leu Glu Asp Lys Val Tyr Leu Ala Gly His Asn
100 105 110

Thr Thr Leu Ala Asp Ile Leu Leu Tyr Tyr Gly Leu His Arg Phe Ile
115 120 125

Val Asp Leu Thr Val Gln Glu Lys Glu Lys Tyr Leu Asn Val Ser Arg
130 135 140

Trp Phe Cys His Ile Gln His Tyr Pro Asp Ile Arg Gln His Leu Ser
145 150 155 160

Ser Val Val Phe Ile Lys Asn Arg Leu Tyr Ala Asn Ser His
165 170

<210> 41
<211> 4568
<212> DNA
<213> Rattus norvegicus

<220>
<221> CDS
<222> (1)..(3009)

<400> 41
atg gcg gcg ggg ggt gct gcg ggg ctg cgg gag gag cag cgc tac ggg 48
Met Ala Ala Gly Gly Ala Ala Gly Leu Arg Glu Glu Gln Arg Tyr Gly
1 5 10 15

ctg gcg tgc ggg cgg ctg ggg cag gac aac atc acc gtg cta cat gtg 96
Leu Ala Cys Gly Arg Leu Gly Gln Asp Asn Ile Thr Val Leu His Val
20 25 30

aag ctc acc gag acg gcg atc cgg gcg ctg gag act tac cag agc caa 144
Lys Leu Thr Glu Thr Ala Ile Arg Ala Leu Glu Thr Tyr Gln Ser Gln
35 40 45

aag tgg ggc gct ggc tcg ggc tgc tgc ggc ggc acc cgc cta ggg gag 192
Lys Trp Gly Ala Gly Ser Gly Cys Cys Gly Gly Thr Arg Leu Gly Glu
50 55 60

```
gga ggc cag ccg gag tct ccg ggc cgt ggt ccg ggg cca ctg gat cga      240
Gly Gly Gln Pro Glu Ser Pro Gly Arg Gly Pro Gly Pro Leu Asp Arg
65                  70                  75                  80

gga tgc ggg cgg gcg gcc cct ggt ggc ggg gtc cgc ctg gca ggg gct      288
Gly Cys Gly Arg Ala Ala Pro Gly Gly Gly Val Arg Leu Ala Gly Ala
                85                  90                  95

gcg cgg ccg ctg cgg gac ttt ctg acg gcg ccc gcg ggg ttc cta gct      336
Ala Arg Pro Leu Arg Asp Phe Leu Thr Ala Pro Ala Gly Phe Leu Ala
            100                 105                 110

tgt ttg gtc ccg aga agg gac cgg gtg gag gag aga gaa gtg cgg agg      384
Cys Leu Val Pro Arg Arg Asp Arg Val Glu Glu Arg Glu Val Arg Arg
        115                 120                 125

ggg cgc gcg cag cta cca gga ctc gga gac act ggc act ccg agt gta      432
Gly Arg Ala Gln Leu Pro Gly Leu Gly Asp Thr Gly Thr Pro Ser Val
    130                 135                 140

ggc gtg agt gta cac ggc gta cct ggg aag gcc ggg aca ctg gca ctg      480
Gly Val Ser Val His Gly Val Pro Gly Lys Ala Gly Thr Leu Ala Leu
145                 150                 155                 160

aga gga ggg gga ggg gga cac acc ttg cac tct cag gga ctc tgt gcc      528
Arg Gly Gly Gly Gly Gly His Thr Leu His Ser Gln Gly Leu Cys Ala
                165                 170                 175

ttg ggt tcc cgg aga cta gca gcg tac cat gtg gcc aca aac cga cac      576
Leu Gly Ser Arg Arg Leu Ala Ala Tyr His Val Ala Thr Asn Arg His
            180                 185                 190

cgc atc tcg ggg aga gac cca gaa gac tca aaa aca act aga gcc tgg      624
Arg Ile Ser Gly Arg Asp Pro Glu Asp Ser Lys Thr Thr Arg Ala Trp
        195                 200                 205

aac aag acg gca ggt tct ggg gaa agg aat tct cag aag ctc ggc gta      672
Asn Lys Thr Ala Gly Ser Gly Glu Arg Asn Ser Gln Lys Leu Gly Val
    210                 215                 220

gcc gaa agg cgc gcc gtg tcc ggt gct gac tcg ctg tct gtt ggc gag      720
Ala Glu Arg Arg Ala Val Ser Gly Ala Asp Ser Leu Ser Val Gly Glu
225                 230                 235                 240

gag cga gcc cga aga atg gaa cac gag tcc atg gaa caa tca ggt gta      768
Glu Arg Ala Arg Arg Met Glu His Glu Ser Met Glu Gln Ser Gly Val
                245                 250                 255

gat gtc agc atg aat aca cac ctc aaa gct gtg aca atg acc ctg aag      816
Asp Val Ser Met Asn Thr His Leu Lys Ala Val Thr Met Thr Leu Lys
            260                 265                 270

aac aga gag cct gta cag ttg gaa aca ttg ggt att cga ggc agt aac      864
Asn Arg Glu Pro Val Gln Leu Glu Thr Leu Gly Ile Arg Gly Ser Asn
        275                 280                 285

att cgg tat ttt att cta cct gac agt tta cct cta gat aca cta ctt      912
Ile Arg Tyr Phe Ile Leu Pro Asp Ser Leu Pro Leu Asp Thr Leu Leu
    290                 295                 300

gtg gat gtt gaa cct aaa gtg aaa tct aag aaa aga gaa gct gtt gca      960
Val Asp Val Glu Pro Lys Val Lys Ser Lys Lys Arg Glu Ala Val Ala
305                 310                 315                 320

gga aga ggc cga ggc cgg gag ttt aat tcc ttt tca acc ttc aat tca     1008
Gly Arg Gly Arg Gly Arg Glu Phe Asn Ser Phe Ser Thr Phe Asn Ser
                325                 330                 335

gtt cca agg act cca agg gct gtt aat gag agt gac agt cag gaa gac     1056
Val Pro Arg Thr Pro Arg Ala Val Asn Glu Ser Asp Ser Gln Glu Asp
            340                 345                 350

att aga gaa gtc ctt ttg gag ctg aat ttg aag ggt tgc cag gac ttg     1104
Ile Arg Glu Val Leu Leu Glu Leu Asn Leu Lys Gly Cys Gln Asp Leu
        355                 360                 365
```

```
gtt ggt gag aaa aga ggt aaa tac gtt ttg ggc ctg cgc ttt cac agc      1152
Val Gly Glu Lys Arg Gly Lys Tyr Val Leu Gly Leu Arg Phe His Ser
    370                 375                 380

atg agg aaa ggg caa aca gtc tgt ctt caa aga gga gaa tgg gaa ctc      1200
Met Arg Lys Gly Gln Thr Val Cys Leu Gln Arg Gly Glu Trp Glu Leu
385                 390                 395                 400

aca ttt cca ttg gac agt gaa cag gtg ctg gct tgt tta acc cag gtg      1248
Thr Phe Pro Leu Asp Ser Glu Gln Val Leu Ala Cys Leu Thr Gln Val
                405                 410                 415

tct gaa agt tct gta ttt ggc tat aaa gat gtt cag ctt atg aaa att      1296
Ser Glu Ser Ser Val Phe Gly Tyr Lys Asp Val Gln Leu Met Lys Ile
            420                 425                 430

ccc aaa aat gat ccc ttc aat gaa gtc cac aac ttt aac ttc tat ttg      1344
Pro Lys Asn Asp Pro Phe Asn Glu Val His Asn Phe Asn Phe Tyr Leu
        435                 440                 445

tca aac gtg ggg aaa gat aac cct cag ggc agc ttt gac tgc atc cag      1392
Ser Asn Val Gly Lys Asp Asn Pro Gln Gly Ser Phe Asp Cys Ile Gln
    450                 455                 460

caa aca ctc tct agc tct gga gcc tct cag ctc aat tgc ctg gga ttt      1440
Gln Thr Leu Ser Ser Ser Gly Ala Ser Gln Leu Asn Cys Leu Gly Phe
465                 470                 475                 480

ata cag gat aaa att aca gtg tgt gca aca aat gat tcc tat caa atg      1488
Ile Gln Asp Lys Ile Thr Val Cys Ala Thr Asn Asp Ser Tyr Gln Met
                485                 490                 495

act cga gaa aga atg acc cag gcc gag gag gaa tcc cgt aat cga agc      1536
Thr Arg Glu Arg Met Thr Gln Ala Glu Glu Glu Ser Arg Asn Arg Ser
            500                 505                 510

aca aaa gtt atc aaa ccc ggt gga cca tat gta ggg aaa aga gtg caa      1584
Thr Lys Val Ile Lys Pro Gly Gly Pro Tyr Val Gly Lys Arg Val Gln
        515                 520                 525

att cgt aaa gca cct caa gcc atc tcg gat gca gtg cct gag agg aaa      1632
Ile Arg Lys Ala Pro Gln Ala Ile Ser Asp Ala Val Pro Glu Arg Lys
    530                 535                 540

agg tca acg ccc atg aac cct gca aat aca att cga aag atg cat ggt      1680
Arg Ser Thr Pro Met Asn Pro Ala Asn Thr Ile Arg Lys Met His Gly
545                 550                 555                 560

ggc agc agt gtc tct cag agg ccg tac agg gac aga gtc att cat tta      1728
Gly Ser Ser Val Ser Gln Arg Pro Tyr Arg Asp Arg Val Ile His Leu
                565                 570                 575

ctg gca cta aaa gcc tat aag aaa cct gag ctg ctt gcc cga ctg cag      1776
Leu Ala Leu Lys Ala Tyr Lys Lys Pro Glu Leu Leu Ala Arg Leu Gln
            580                 585                 590

aaa gat ggt gtc aat caa aaa gac aag aac tcc ttg gga gca att ttg      1824
Lys Asp Gly Val Asn Gln Lys Asp Lys Asn Ser Leu Gly Ala Ile Leu
        595                 600                 605

caa cag gtg gcc aat ctg aat cct aag gac ctc tcc tat act tta aag      1872
Gln Gln Val Ala Asn Leu Asn Pro Lys Asp Leu Ser Tyr Thr Leu Lys
    610                 615                 620

gat tat gta ttt aag gag ctc cag cgg gac tgg cca ggt tac agt gag      1920
Asp Tyr Val Phe Lys Glu Leu Gln Arg Asp Trp Pro Gly Tyr Ser Glu
625                 630                 635                 640

aca gac aaa cag aca ctg gat ttg gtg ctc tct aga aaa tta aat cca      1968
Thr Asp Lys Gln Thr Leu Asp Leu Val Leu Ser Arg Lys Leu Asn Pro
                645                 650                 655

tct cag aat gct ggc act agc cgg cca gag tct ccc atg tgt tcc agc      2016
Ser Gln Asn Ala Gly Thr Ser Arg Pro Glu Ser Pro Met Cys Ser Ser
            660                 665                 670
```

```
aaa gat gct gcc tca tct cct cag aaa cga cct ttg gat tca gat ttc      2064
Lys Asp Ala Ala Ser Ser Pro Gln Lys Arg Pro Leu Asp Ser Asp Phe
        675                 680                 685

att gat cct tta acg aac aaa aaa gct cga ata tct cac ctg aca aac      2112
Ile Asp Pro Leu Thr Asn Lys Lys Ala Arg Ile Ser His Leu Thr Asn
    690                 695                 700

aga gtc cca ccg aca tta aat ggc cat ttg aat ccc acc aat gaa aaa      2160
Arg Val Pro Pro Thr Leu Asn Gly His Leu Asn Pro Thr Asn Glu Lys
705                 710                 715                     720

tct tgt gca agc ctt cta ccg ccc cct gca gct gct gcc atc ccc acc      2208
Ser Cys Ala Ser Leu Leu Pro Pro Pro Ala Ala Ala Ala Ile Pro Thr
                725                 730                 735

ccc tca cca ctg cct tca acc cac ctg cct gtc tcc aat cct acc cag      2256
Pro Ser Pro Leu Pro Ser Thr His Leu Pro Val Ser Asn Pro Thr Gln
            740                 745                 750

act gta aat tcc aac tcc aat tcc cct agc act cca gaa ggc cgg ggg      2304
Thr Val Asn Ser Asn Ser Asn Ser Pro Ser Thr Pro Glu Gly Arg Gly
        755                 760                 765

act caa gac cta cct gtt gac agt ttt agt caa aac agt agc atc ttt      2352
Thr Gln Asp Leu Pro Val Asp Ser Phe Ser Gln Asn Ser Ser Ile Phe
    770                 775                 780

gag gac cag caa gac aaa tat acc tca agg act tgt ctg gag gaa aca      2400
Glu Asp Gln Gln Asp Lys Tyr Thr Ser Arg Thr Cys Leu Glu Glu Thr
785                 790                 795                     800

tta ccc tct agc tca gct tta cta aag tgt cca aag ccc atg gaa gaa      2448
Leu Pro Ser Ser Ser Ala Leu Leu Lys Cys Pro Lys Pro Met Glu Glu
                805                 810                 815

gaa cat cca gtg tct cac aaa aag tcc aaa aag aag tct aaa aaa cac      2496
Glu His Pro Val Ser His Lys Lys Ser Lys Lys Lys Ser Lys Lys His
            820                 825                 830

aag gaa aag gac caa ata aag aaa cat gac att gag agc agg gag gaa      2544
Lys Glu Lys Asp Gln Ile Lys Lys His Asp Ile Glu Ser Arg Glu Glu
        835                 840                 845

aag gag aaa gac ctt cag aga gaa gaa act gcc aag ctg agt aat gac      2592
Lys Glu Lys Asp Leu Gln Arg Glu Glu Thr Ala Lys Leu Ser Asn Asp
    850                 855                 860

agt cca aat ccc aat gaa gga gtt aaa gaa gag tgc aca gct tcc atg      2640
Ser Pro Asn Pro Asn Glu Gly Val Lys Glu Glu Cys Thr Ala Ser Met
865                 870                 875                     880

gag cct tct tca aca att gaa ctc cca gat tat ttg ata aaa tat att      2688
Glu Pro Ser Ser Thr Ile Glu Leu Pro Asp Tyr Leu Ile Lys Tyr Ile
                885                 890                 895

gct att gtc tct tat gaa caa cgc cag aat tac aag gat gac ttc aat      2736
Ala Ile Val Ser Tyr Glu Gln Arg Gln Asn Tyr Lys Asp Asp Phe Asn
            900                 905                 910

gct gag tat gat gaa tat aga gct ttg cat gcc agg atg gag act gta      2784
Ala Glu Tyr Asp Glu Tyr Arg Ala Leu His Ala Arg Met Glu Thr Val
        915                 920                 925

gcc agg aga ttt att aaa ctg gat gca caa cga aaa cgc ctt tct cca      2832
Ala Arg Arg Phe Ile Lys Leu Asp Ala Gln Arg Lys Arg Leu Ser Pro
    930                 935                 940

ggt tca aaa gag tat cag aat gtt cat gaa gaa gtc tta caa gaa tat      2880
Gly Ser Lys Glu Tyr Gln Asn Val His Glu Glu Val Leu Gln Glu Tyr
945                 950                 955                     960

cag aag atc aag cag tcc agt ccc aat tac cat gaa gaa aaa tac aga      2928
Gln Lys Ile Lys Gln Ser Ser Pro Asn Tyr His Glu Glu Lys Tyr Arg
                965                 970                 975
```

```
tgc gag tat ctt cat aac aag ctg gct cac atc aaa aga cta ata ggt      2976
Cys Glu Tyr Leu His Asn Lys Leu Ala His Ile Lys Arg Leu Ile Gly
            980                 985                 990

gaa ttt gac caa cag caa gca gag  tcg tgg cac tagggctctg              3019
Glu Phe Asp Gln Gln Gln Ala Glu  Ser Trp His
        995                 1000

cttgaagcag aagatgtgaa taaacttaag cttatttatt taaaattcca gatgagttgc   3079
tctggactct aaacgatgac actttgttgg acgtttcagg attagtaaac ttgagttact   3139
tctgttcctc tccattttac tttgcttccc tgaatttgaa gctgcttttt ttctggtcct   3199
ctccctgact tcccaccccca atctttgtta atagaagcaa ttttttagag attggagatc  3259
cagtgtctat gcttcaaatc aattttcctt gaaatcttgt gtttgtcatt agatatgatt   3319
tttgctagat atcagggatg caaagtccac acttaaaaga gttgtgtgtg tttaaaacaa   3379
gacaaaatag taatgtgcaa ggtgataagc ttttgaataa acataagcct attttctgac   3439
ctttcttaat gcaaactctt tgccttaaat ggtagaatat ttaaacattt gcacaaagta   3499
caaaaaaatc aaaacattgt ttttaaaggg ttgaaaaaag gttgtgtgaa tgtgtgtagt   3559
tttctatgca catatgcata catacatata tacatatata cacacataca tacatacata   3619
catacataca tacatacata catacgggct gtctggctcc agagtgttaa atctgcccta   3679
caagttagac ctccattgca atggctgcct taaggtgttt gctacatcat gtggttaatc   3739
agtagctaca ctgcttccca gtgataacaa ccctgggaag catactgtgg cctaccagca   3799
tcatcagaaa gtgtgtgttc tgcttgtatg tctgcagagc tgctgtggat gtgagctgcg   3859
tgaaggctaa gaagctgctg ctctcaggcc agacactcag gttaaagaac tgatgagtat   3919
tgcagtagct tatttggtgt gtgtgtgtgt gtgtgtgtgt gtgtgtgtgt gtgtgtgtgt   3979
gtgtgtggtg tgtttctgtc aggttgctac ttctattgtg gaagacaaaa gcatttccat   4039
ttcaacagtt tgtcagcttt attaatgttg ggcaaaaaat gatatgttat aaaaatgaaa   4099
cagatcttat agttttgggg caaaatgaca aaaataaatg tgtaggtaat ctatttatat   4159
actgctataa agtatttttt gaagagagat atgcaagaaa ctattaccta catacatcag   4219
atgtatattt aaagatttct ttcatcctgc ataccaggaa tataaaaaag tagatatata   4279
taaccataac atactgtgat tcatcaaata gcacaaactt tcatttcatg gattttatct   4339
gttaacgttg atttaaacta tcacttgtct tatcatgtgg gagcataagt tatgtggtca   4399
aaatagaagg attttgaact aaagttgatt caggttgtct tgcaccgttt caaagtgctg   4459
ccagttgaaa agggaagcat tatgtttaca aatctgtttt aaaaagtttg ccaaaatttt   4519
ggtagtatct ttaataaaga tgtttgtctt cagcatccaa aaaagtgaa                4568

<210>  42
<211>  1003
<212>  PRT
<213>  Rattus norvegicus

<400>  42

Met Ala Ala Gly Gly Ala Ala Gly Leu Arg Glu Glu Gln Arg Tyr Gly
1               5                   10                  15

Leu Ala Cys Gly Arg Leu Gly Gln Asp Asn Ile Thr Val Leu His Val
            20                  25                  30
```

Lys Leu Thr Glu Thr Ala Ile Arg Ala Leu Glu Thr Tyr Gln Ser Gln
35 40 45

Lys Trp Gly Ala Gly Ser Gly Cys Cys Gly Gly Thr Arg Leu Gly Glu
50 55 60

Gly Gly Gln Pro Glu Ser Pro Gly Arg Gly Pro Gly Pro Leu Asp Arg
65 70 75 80

Gly Cys Gly Arg Ala Ala Pro Gly Gly Gly Val Arg Leu Ala Gly Ala
85 90 95

Ala Arg Pro Leu Arg Asp Phe Leu Thr Ala Pro Ala Gly Phe Leu Ala
100 105 110

Cys Leu Val Pro Arg Arg Asp Arg Val Glu Glu Arg Glu Val Arg Arg
115 120 125

Gly Arg Ala Gln Leu Pro Gly Leu Gly Asp Thr Gly Thr Pro Ser Val
130 135 140

Gly Val Ser Val His Gly Val Pro Gly Lys Ala Gly Thr Leu Ala Leu
145 150 155 160

Arg Gly Gly Gly Gly Gly His Thr Leu His Ser Gln Gly Leu Cys Ala
165 170 175

Leu Gly Ser Arg Arg Leu Ala Ala Tyr His Val Ala Thr Asn Arg His
180 185 190

Arg Ile Ser Gly Arg Asp Pro Glu Asp Ser Lys Thr Thr Arg Ala Trp
195 200 205

Asn Lys Thr Ala Gly Ser Gly Glu Arg Asn Ser Gln Lys Leu Gly Val
210 215 220

Ala Glu Arg Arg Ala Val Ser Gly Ala Asp Ser Leu Ser Val Gly Glu
225 230 235 240

Glu Arg Ala Arg Arg Met Glu His Glu Ser Met Glu Gln Ser Gly Val
245 250 255

Asp Val Ser Met Asn Thr His Leu Lys Ala Val Thr Met Thr Leu Lys
260 265 270

Asn Arg Glu Pro Val Gln Leu Glu Thr Leu Gly Ile Arg Gly Ser Asn
275 280 285

Ile Arg Tyr Phe Ile Leu Pro Asp Ser Leu Pro Leu Asp Thr Leu Leu
290 295 300

Val Asp Val Glu Pro Lys Val Lys Ser Lys Lys Arg Glu Ala Val Ala
305 310 315 320

Gly Arg Gly Arg Gly Arg Glu Phe Asn Ser Phe Ser Thr Phe Asn Ser
325 330 335

Val Pro Arg Thr Pro Arg Ala Val Asn Glu Ser Asp Ser Gln Glu Asp
340 345 350

Ile Arg Glu Val Leu Leu Glu Leu Asn Leu Lys Gly Cys Gln Asp Leu
355 360 365

Val Gly Glu Lys Arg Gly Lys Tyr Val Leu Gly Leu Arg Phe His Ser
370 375 380

Met Arg Lys Gly Gln Thr Val Cys Leu Gln Arg Gly Glu Trp Glu Leu
385 390 395 400

Thr Phe Pro Leu Asp Ser Glu Gln Val Leu Ala Cys Leu Thr Gln Val
405 410 415

Ser Glu Ser Ser Val Phe Gly Tyr Lys Asp Val Gln Leu Met Lys Ile
420 425 430

Pro Lys Asn Asp Pro Phe Asn Glu Val His Asn Phe Asn Phe Tyr Leu
435 440 445

Ser Asn Val Gly Lys Asp Asn Pro Gln Gly Ser Phe Asp Cys Ile Gln
450 455 460

Gln Thr Leu Ser Ser Ser Gly Ala Ser Gln Leu Asn Cys Leu Gly Phe
465 470 475 480

Ile Gln Asp Lys Ile Thr Val Cys Ala Thr Asn Asp Ser Tyr Gln Met
485 490 495

Thr Arg Glu Arg Met Thr Gln Ala Glu Glu Glu Ser Arg Asn Arg Ser
500 505 510

Thr Lys Val Ile Lys Pro Gly Gly Pro Tyr Val Gly Lys Arg Val Gln
515 520 525

Ile Arg Lys Ala Pro Gln Ala Ile Ser Asp Ala Val Pro Glu Arg Lys
530 535 540

Arg Ser Thr Pro Met Asn Pro Ala Asn Thr Ile Arg Lys Met His Gly
545 550 555 560

Gly Ser Ser Val Ser Gln Arg Pro Tyr Arg Asp Arg Val Ile His Leu
565 570 575

Leu Ala Leu Lys Ala Tyr Lys Lys Pro Glu Leu Leu Ala Arg Leu Gln
580 585 590

Lys Asp Gly Val Asn Gln Lys Asp Lys Asn Ser Leu Gly Ala Ile Leu
595 600 605

Gln Gln Val Ala Asn Leu Asn Pro Lys Asp Leu Ser Tyr Thr Leu Lys
610 615 620

Asp Tyr Val Phe Lys Glu Leu Gln Arg Asp Trp Pro Gly Tyr Ser Glu
625 630 635 640

Thr Asp Lys Gln Thr Leu Asp Leu Val Leu Ser Arg Lys Leu Asn Pro
645 650 655

Ser Gln Asn Ala Gly Thr Ser Arg Pro Glu Ser Pro Met Cys Ser Ser
660 665 670

Lys Asp Ala Ala Ser Ser Pro Gln Lys Arg Pro Leu Asp Ser Asp Phe
675 680 685

Ile Asp Pro Leu Thr Asn Lys Lys Ala Arg Ile Ser His Leu Thr Asn
690 695 700

Arg Val Pro Pro Thr Leu Asn Gly His Leu Asn Pro Thr Asn Glu Lys
705 710 715 720

Ser Cys Ala Ser Leu Leu Pro Pro Pro Ala Ala Ala Ala Ile Pro Thr
725 730 735

Pro Ser Pro Leu Pro Ser Thr His Leu Pro Val Ser Asn Pro Thr Gln
740 745 750

Thr Val Asn Ser Asn Ser Asn Ser Pro Ser Thr Pro Glu Gly Arg Gly
755 760 765

Thr Gln Asp Leu Pro Val Asp Ser Phe Ser Gln Asn Ser Ser Ile Phe
770 775 780

Glu Asp Gln Gln Asp Lys Tyr Thr Ser Arg Thr Cys Leu Glu Glu Thr
785 790 795 800

Leu Pro Ser Ser Ser Ala Leu Leu Lys Cys Pro Lys Pro Met Glu Glu
805 810 815

Glu His Pro Val Ser His Lys Lys Ser Lys Lys Lys Ser Lys Lys His
820 825 830

Lys Glu Lys Asp Gln Ile Lys Lys His Asp Ile Glu Ser Arg Glu Glu
835 840 845

Lys Glu Lys Asp Leu Gln Arg Glu Glu Thr Ala Lys Leu Ser Asn Asp
850 855 860

Ser Pro Asn Pro Asn Glu Gly Val Lys Glu Glu Cys Thr Ala Ser Met
865 870 875 880

Glu Pro Ser Ser Thr Ile Glu Leu Pro Asp Tyr Leu Ile Lys Tyr Ile
885 890 895

Ala Ile Val Ser Tyr Glu Gln Arg Gln Asn Tyr Lys Asp Asp Phe Asn
900 905 910

Ala Glu Tyr Asp Glu Tyr Arg Ala Leu His Ala Arg Met Glu Thr Val
915 920 925

Ala Arg Arg Phe Ile Lys Leu Asp Ala Gln Arg Lys Arg Leu Ser Pro
930 935 940

```
Gly Ser Lys Glu Tyr Gln Asn Val His Glu Glu Val Leu Gln Glu Tyr
945                 950                 955                 960

Gln Lys Ile Lys Gln Ser Ser Pro Asn Tyr His Glu Glu Lys Tyr Arg
                965                 970                 975

Cys Glu Tyr Leu His Asn Lys Leu Ala His Ile Lys Arg Leu Ile Gly
            980                 985                 990

Glu Phe Asp Gln Gln Gln Ala Glu  Ser Trp His
        995                 1000
```

<210> 43
<211> 1904
<212> DNA
<213> Rattus norvegicus

<220>
<221> CDS
<222> (62)..(1078)

<400> 43

```
gagcgactgc gggcccaggt tgtcgcgctg cgctcggtga gtacaggatc cagcttccag      60

g atg tgg tgg tcc ttg atc cct ctc tct tgt ctg ctg gca ctg acc agt     109
  Met Trp Trp Ser Leu Ile Pro Leu Ser Cys Leu Leu Ala Leu Thr Ser
  1               5                   10                  15

gcc cat gac aag cct tcc tca cac cca ctg tcg gac gac atg att aac       157
Ala His Asp Lys Pro Ser Ser His Pro Leu Ser Asp Asp Met Ile Asn
            20                  25                  30

tat atc aac aaa cag aat aca aca tgg cag gct gga cgc aac ttc tac       205
Tyr Ile Asn Lys Gln Asn Thr Thr Trp Gln Ala Gly Arg Asn Phe Tyr
        35                  40                  45

aat gtt gac ata agc tat ctg aag aag ctg tgt gga act gtc ctg ggt       253
Asn Val Asp Ile Ser Tyr Leu Lys Lys Leu Cys Gly Thr Val Leu Gly
    50                  55                  60

gga ccc aac ctg ccg gaa agg gtt ggg ttc agc gag gac ata aat cta       301
Gly Pro Asn Leu Pro Glu Arg Val Gly Phe Ser Glu Asp Ile Asn Leu
65                  70                  75                  80

cct gaa tcc ttt gat gca cgg gaa cag tgg tcc aat tgc ccg acc atc       349
Pro Glu Ser Phe Asp Ala Arg Glu Gln Trp Ser Asn Cys Pro Thr Ile
                85                  90                  95

gca cag atc aga gac cag ggg tcc tgt ggc tct tgt tgg gca ttt ggg       397
Ala Gln Ile Arg Asp Gln Gly Ser Cys Gly Ser Cys Trp Ala Phe Gly
            100                 105                 110

gca gtg gaa gcc atg tct gac cga atc tgc att cac acc aat ggc cga       445
Ala Val Glu Ala Met Ser Asp Arg Ile Cys Ile His Thr Asn Gly Arg
        115                 120                 125

gtc aat gtg gag gtg tct gct gag gac ctg ctt acc tgc tgt ggt atc       493
Val Asn Val Glu Val Ser Ala Glu Asp Leu Leu Thr Cys Cys Gly Ile
    130                 135                 140

cag tgt ggg gat ggc tgt aat ggt ggc tat ccc tct gga gca tgg aac       541
Gln Cys Gly Asp Gly Cys Asn Gly Gly Tyr Pro Ser Gly Ala Trp Asn
145                 150                 155                 160

ttc tgg act aga aaa ggc ctg gtt tct ggt gga gta tac aat tct cat       589
Phe Trp Thr Arg Lys Gly Leu Val Ser Gly Gly Val Tyr Asn Ser His
                165                 170                 175

ata ggc tgc tta ccc tac acc atc cct ccc tgt gaa cac cat gtc aat       637
Ile Gly Cys Leu Pro Tyr Thr Ile Pro Pro Cys Glu His His Val Asn
```

```
            180                 185                 190

ggc tcc cgt ccc cca tgc act gga gag gga gat act ccc aag tgc aac         685
Gly Ser Arg Pro Pro Cys Thr Gly Glu Gly Asp Thr Pro Lys Cys Asn
        195                 200                 205

aag atg tgt gag gct ggc tac tcc aca tcc tac aag gaa gat aag cac         733
Lys Met Cys Glu Ala Gly Tyr Ser Thr Ser Tyr Lys Glu Asp Lys His
    210                 215                 220

tat ggg tac act tcc tac agt gtg tct gac agc gag aag gag atc atg         781
Tyr Gly Tyr Thr Ser Tyr Ser Val Ser Asp Ser Glu Lys Glu Ile Met
225                 230                 235                 240

gcg gaa atc tac aaa aat ggc cca gtg gag ggt gct ttt act gtg ttt         829
Ala Glu Ile Tyr Lys Asn Gly Pro Val Glu Gly Ala Phe Thr Val Phe
                245                 250                 255

tct gac ttc ttg act tac aaa tca ggc gta tac aag cat gaa gcc ggt         877
Ser Asp Phe Leu Thr Tyr Lys Ser Gly Val Tyr Lys His Glu Ala Gly
            260                 265                 270

gat gtg atg gga ggc cat gcc atc cgc att ctg ggc tgg gga ata gag         925
Asp Val Met Gly Gly His Ala Ile Arg Ile Leu Gly Trp Gly Ile Glu
        275                 280                 285

aat gga gta ccc tac tgg ctg gta gca aac tcc tgg aac gtt gac tgg         973
Asn Gly Val Pro Tyr Trp Leu Val Ala Asn Ser Trp Asn Val Asp Trp
    290                 295                 300

ggt gat aat ggt ttc ttt aaa atc ctc aga gga gag aac cac tgt gga        1021
Gly Asp Asn Gly Phe Phe Lys Ile Leu Arg Gly Glu Asn His Cys Gly
305                 310                 315                 320

att gaa tca gaa atc gtg gct gga atc cca cgc act cag cag tac tgg        1069
Ile Glu Ser Glu Ile Val Ala Gly Ile Pro Arg Thr Gln Gln Tyr Trp
                325                 330                 335

gga aga ttc taatctgctg ggacttgatt gtatagtcct taggagactt                1118
Gly Arg Phe

tttccaaaat ttagcaatgc aggcagggag tgaggcagat aggagtcttt gattctttga     1178

gttcacctaa catgcatgaa gctttcagca aggtttgggt gactggacca gagctgcccc     1238

ccatcagagc caccctacct ggctcccttc tagcctatcc catgacagtg atcacagcct     1298

acatcctagc ctctccctag actagtgctg tgtgtagcgc ctgctgctgt ggctctgtct     1358

gccatccctc ccaccccac ctcagcatcc ccagaccaag ggctttggct gcaggatttc      1418

caaaggaatg aacaccaact acatgatgag aggacagatg ccacccgcca gctgcacctt     1478

gaagctagtc acttctagga ctgtggcagg ctttgatggc ccaatgcagt tctctggaga     1538

aaactacctt tccccaaggc atctgcaccc attgacaatg gtaatgtgcc catctctcct     1598

tggtcctgcc ctcaaccgat gcttttccag tcagggtttt gtttttgtt ttgttttgtg      1658

tacctcaact gagttatgaa gatttgtact ggttttacag atcatctcat cgtatggaaa     1718

agaacaagct tcgtggtcag tttactgggt gaccggcaga caccacaatc aaactagtct     1778

ggggaaaacc tgcttttttg ttgtaggtgc cacgtaaccc tgtcagtttg acaaggaatg     1838

accgtgccaa taaaccaatt ctccctctgc ttggaaaaaa aaaaaaaaaa aaaaaaaaaa     1898

aaaaaa                                                                1904
```

<210> 44
<211> 339
<212> PRT
<213> Rattus norvegicus

<400> 44

Met Trp Trp Ser Leu Ile Pro Leu Ser Cys Leu Leu Ala Leu Thr Ser
1 5 10 15

Ala His Asp Lys Pro Ser Ser His Pro Leu Ser Asp Asp Met Ile Asn
20 25 30

Tyr Ile Asn Lys Gln Asn Thr Thr Trp Gln Ala Gly Arg Asn Phe Tyr
35 40 45

Asn Val Asp Ile Ser Tyr Leu Lys Lys Leu Cys Gly Thr Val Leu Gly
50 55 60

Gly Pro Asn Leu Pro Glu Arg Val Gly Phe Ser Glu Asp Ile Asn Leu
65 70 75 80

Pro Glu Ser Phe Asp Ala Arg Glu Gln Trp Ser Asn Cys Pro Thr Ile
85 90 95

Ala Gln Ile Arg Asp Gln Gly Ser Cys Gly Ser Cys Trp Ala Phe Gly
100 105 110

Ala Val Glu Ala Met Ser Asp Arg Ile Cys Ile His Thr Asn Gly Arg
115 120 125

Val Asn Val Glu Val Ser Ala Glu Asp Leu Leu Thr Cys Cys Gly Ile
130 135 140

Gln Cys Gly Asp Gly Cys Asn Gly Gly Tyr Pro Ser Gly Ala Trp Asn
145 150 155 160

Phe Trp Thr Arg Lys Gly Leu Val Ser Gly Gly Val Tyr Asn Ser His
165 170 175

Ile Gly Cys Leu Pro Tyr Thr Ile Pro Pro Cys Glu His His Val Asn
180 185 190

Gly Ser Arg Pro Pro Cys Thr Gly Glu Gly Asp Thr Pro Lys Cys Asn
195 200 205

Lys Met Cys Glu Ala Gly Tyr Ser Thr Ser Tyr Lys Glu Asp Lys His
210 215 220

Tyr Gly Tyr Thr Ser Tyr Ser Val Ser Asp Ser Glu Lys Glu Ile Met
225 230 235 240

Ala Glu Ile Tyr Lys Asn Gly Pro Val Glu Gly Ala Phe Thr Val Phe
245 250 255

Ser Asp Phe Leu Thr Tyr Lys Ser Gly Val Tyr Lys His Glu Ala Gly
260 265 270

Asp Val Met Gly Gly His Ala Ile Arg Ile Leu Gly Trp Gly Ile Glu
275 280 285

Asn Gly Val Pro Tyr Trp Leu Val Ala Asn Ser Trp Asn Val Asp Trp
290 295 300

Gly Asp Asn Gly Phe Phe Lys Ile Leu Arg Gly Glu Asn His Cys Gly
305 310 315 320

Ile Glu Ser Glu Ile Val Ala Gly Ile Pro Arg Thr Gln Gln Tyr Trp
325 330 335

Gly Arg Phe

<210> 45
<211> 3247
<212> DNA
<213> Rattus norvegicus

<220>
<221> CDS
<222> (36)..(2891)

<400> 45
gtcgctgccg cctccgctac cgctagtgga agaag atg gcg gaa ggc gga gcg 53
Met Ala Glu Gly Gly Ala
1 5

gcg gac ctg gac acc cag cgt tct gac atc gcg acg ctg ctc aaa acc 101
Ala Asp Leu Asp Thr Gln Arg Ser Asp Ile Ala Thr Leu Leu Lys Thr
10 15 20

tcg ctc cgg aaa ggg gac acc tgg tat cta gta gat agt cgg tgg ttc 149
Ser Leu Arg Lys Gly Asp Thr Trp Tyr Leu Val Asp Ser Arg Trp Phe
25 30 35

aaa cag tgg aaa aaa tat gtt ggc ttt gac agt tgg gac aaa tac cag 197
Lys Gln Trp Lys Lys Tyr Val Gly Phe Asp Ser Trp Asp Lys Tyr Gln
40 45 50

atg gga gat caa aat gtc tat cct gga ccc atc gat aac tct gga ctt 245
Met Gly Asp Gln Asn Val Tyr Pro Gly Pro Ile Asp Asn Ser Gly Leu
55 60 65 70

ctc aaa gat ggc gat gct cag tca ctt aag gaa cac ctt atc gac gag 293
Leu Lys Asp Gly Asp Ala Gln Ser Leu Lys Glu His Leu Ile Asp Glu
75 80 85

ttg gat tac ata ctg ttg ccg act gag ggc tgg aat aaa ctt gtc agc 341
Leu Asp Tyr Ile Leu Leu Pro Thr Glu Gly Trp Asn Lys Leu Val Ser
90 95 100

tgg tac aca ctg atg gaa ggc caa gag cca ata gca aga aag gtg gta 389
Trp Tyr Thr Leu Met Glu Gly Gln Glu Pro Ile Ala Arg Lys Val Val
105 110 115

gaa cag ggc atg ttt gta aag cac tgc aaa gtg gaa gtc tac ctc aca 437
Glu Gln Gly Met Phe Val Lys His Cys Lys Val Glu Val Tyr Leu Thr
120 125 130

gaa ctg aag ctc tgc gag aat ggg aac atg aac aat gtt gta act cgg 485
Glu Leu Lys Leu Cys Glu Asn Gly Asn Met Asn Asn Val Val Thr Arg
135 140 145 150

aga ttt agc aaa gct gac aca ata gac aca att gaa aag gaa ata aga 533
Arg Phe Ser Lys Ala Asp Thr Ile Asp Thr Ile Glu Lys Glu Ile Arg
155 160 165

aag atc ttc aat att cca gat gaa aag gag gcc aga ctg tgg aac aag 581
Lys Ile Phe Asn Ile Pro Asp Glu Lys Glu Ala Arg Leu Trp Asn Lys
170 175 180

tat atg agt aac aca ttt gag cca ctg aat aag cca gac agc acc atc 629
Tyr Met Ser Asn Thr Phe Glu Pro Leu Asn Lys Pro Asp Ser Thr Ile
185 190 195

```
cag gat gcc ggc cta tac caa gga cag gtg tta gtg ata gaa caa aaa        677
Gln Asp Ala Gly Leu Tyr Gln Gly Gln Val Leu Val Ile Glu Gln Lys
    200                 205                 210

aat gaa gac gga acg tgg ccc agg ggt ccc tct gct cct aat gtg aaa        725
Asn Glu Asp Gly Thr Trp Pro Arg Gly Pro Ser Ala Pro Asn Val Lys
215                 220                 225                 230

aac tcc aat tat tgt ctc ccg tca tat act gct tac aag aac tat gat        773
Asn Ser Asn Tyr Cys Leu Pro Ser Tyr Thr Ala Tyr Lys Asn Tyr Asp
                235                 240                 245

tat tcg gaa cct gga aga aac aat gaa cag cca ggc ctg tgt ggc cta        821
Tyr Ser Glu Pro Gly Arg Asn Asn Glu Gln Pro Gly Leu Cys Gly Leu
            250                 255                 260

agt aac ttg gga aac aca tgt ttc atg aac tca gcc att cag tgt ttg        869
Ser Asn Leu Gly Asn Thr Cys Phe Met Asn Ser Ala Ile Gln Cys Leu
        265                 270                 275

agc aac aca cct cca ctt act gaa tat ttc ctc aat gat aag tac caa        917
Ser Asn Thr Pro Pro Leu Thr Glu Tyr Phe Leu Asn Asp Lys Tyr Gln
    280                 285                 290

gaa gag ctg aat ttt gac aat ccc tta gga atg aga ggt gaa ata gct        965
Glu Glu Leu Asn Phe Asp Asn Pro Leu Gly Met Arg Gly Glu Ile Ala
295                 300                 305                 310

aaa tca tat gct gaa ctc atc aag cag atg tgg tct gga aag ttt agc       1013
Lys Ser Tyr Ala Glu Leu Ile Lys Gln Met Trp Ser Gly Lys Phe Ser
                315                 320                 325

tat gtc act cca aga gca ttt aag aca caa gta gga cgt ttt gca cct       1061
Tyr Val Thr Pro Arg Ala Phe Lys Thr Gln Val Gly Arg Phe Ala Pro
            330                 335                 340

cag ttc tct gga tat caa cag caa gac tgc caa gaa cta cta gct ttc       1109
Gln Phe Ser Gly Tyr Gln Gln Gln Asp Cys Gln Glu Leu Leu Ala Phe
        345                 350                 355

cta tta gat gga tta cat gag gat ttg aat aga att agg aaa aaa cca       1157
Leu Leu Asp Gly Leu His Glu Asp Leu Asn Arg Ile Arg Lys Lys Pro
    360                 365                 370

tat atc cag tta aaa gat gct gat ggg agg cca gac aag gtg gtt gct       1205
Tyr Ile Gln Leu Lys Asp Ala Asp Gly Arg Pro Asp Lys Val Val Ala
375                 380                 385                 390

gaa gaa gcc tgg gaa aac cat tta aaa aga aat gat tct atc ata gta       1253
Glu Glu Ala Trp Glu Asn His Leu Lys Arg Asn Asp Ser Ile Ile Val
                395                 400                 405

gat ata ttt cat ggc ctt ttc aag tct acg tta gtg tgt cct gag tgt       1301
Asp Ile Phe His Gly Leu Phe Lys Ser Thr Leu Val Cys Pro Glu Cys
            410                 415                 420

gct aag atc tca gtg acc ttc gac cct ttc tgt tac ttg aca ctt cca       1349
Ala Lys Ile Ser Val Thr Phe Asp Pro Phe Cys Tyr Leu Thr Leu Pro
        425                 430                 435

ttg cct atg aaa aag gaa cgt agc tta gaa gtt tat tta gtt aga atg       1397
Leu Pro Met Lys Lys Glu Arg Ser Leu Glu Val Tyr Leu Val Arg Met
    440                 445                 450

gat cca ctt gct aaa cct atg caa tac aaa gtc att gtg ccc aaa att       1445
Asp Pro Leu Ala Lys Pro Met Gln Tyr Lys Val Ile Val Pro Lys Ile
455                 460                 465                 470

gga aat ata cta gac ctt tgt aca gca ctg tct gct ttg tcg gga gta       1493
Gly Asn Ile Leu Asp Leu Cys Thr Ala Leu Ser Ala Leu Ser Gly Val
                475                 480                 485

cct gca gat aag atg ata gtc act gac ata tat aat cat aga ttt cac       1541
Pro Ala Asp Lys Met Ile Val Thr Asp Ile Tyr Asn His Arg Phe His
            490                 495                 500
```

```
agg ata ttt gca atg gat gag aac ctg agt agt att atg gag cgg gat        1589
Arg Ile Phe Ala Met Asp Glu Asn Leu Ser Ser Ile Met Glu Arg Asp
        505                 510                 515

gat att tat gtg ttt gaa att aac atc aac agg aca gaa gac aca gaa        1637
Asp Ile Tyr Val Phe Glu Ile Asn Ile Asn Arg Thr Glu Asp Thr Glu
    520                 525                 530

cat gtg gtt att cct gtt tgc cta aga gaa aag ttc aga cac tca agt        1685
His Val Val Ile Pro Val Cys Leu Arg Glu Lys Phe Arg His Ser Ser
535                 540                 545                 550

tac act cac cac act ggc tct tca ctg ttc ggc cag cct ttt ctt atg        1733
Tyr Thr His His Thr Gly Ser Ser Leu Phe Gly Gln Pro Phe Leu Met
                555                 560                 565

gcc gtg cca cgg aac aat act gaa gat aaa ctc tac aat ctc ctg ctc        1781
Ala Val Pro Arg Asn Asn Thr Glu Asp Lys Leu Tyr Asn Leu Leu Leu
            570                 575                 580

ttg aga atg tgc cgg tat gtc aag atg tct act gaa act gag gaa act        1829
Leu Arg Met Cys Arg Tyr Val Lys Met Ser Thr Glu Thr Glu Glu Thr
        585                 590                 595

gat gga ccc ctg cgt tgc tgt gag gac cag aat att aat ggg aat ggt        1877
Asp Gly Pro Leu Arg Cys Cys Glu Asp Gln Asn Ile Asn Gly Asn Gly
    600                 605                 610

cca aat ggc ata cat gaa gaa ggc tca cca agt gag atg gag aca gat        1925
Pro Asn Gly Ile His Glu Glu Gly Ser Pro Ser Glu Met Glu Thr Asp
615                 620                 625                 630

gaa cca gat gat gag tcc agc cag gat caa gag ctt ccc tca gaa aat        1973
Glu Pro Asp Asp Glu Ser Ser Gln Asp Gln Glu Leu Pro Ser Glu Asn
                635                 640                 645

gag aac agt cag tct gaa gat tca gtc gga gga gat aac gat tct gaa        2021
Glu Asn Ser Gln Ser Glu Asp Ser Val Gly Gly Asp Asn Asp Ser Glu
            650                 655                 660

aat gga ttg tgt act gaa gaa aca tgc aaa ggt cga ctg acg gga cac        2069
Asn Gly Leu Cys Thr Glu Glu Thr Cys Lys Gly Arg Leu Thr Gly His
        665                 670                 675

aaa aag cga ttg ttt aca ttc cag ttc aac aac tta ggc aac act gat        2117
Lys Lys Arg Leu Phe Thr Phe Gln Phe Asn Asn Leu Gly Asn Thr Asp
    680                 685                 690

atc aac tat atc aaa gac gac acc agg cat ata aga ttt gat gat agg        2165
Ile Asn Tyr Ile Lys Asp Asp Thr Arg His Ile Arg Phe Asp Asp Arg
695                 700                 705                 710

cag ctt cgg cta gat gaa aga tct ttt ctc gcc ttg gac tgg gac cct        2213
Gln Leu Arg Leu Asp Glu Arg Ser Phe Leu Ala Leu Asp Trp Asp Pro
                715                 720                 725

gac ttg aag aag aga tat ttt gat gag aat gct gca gag gat ttt gaa        2261
Asp Leu Lys Lys Arg Tyr Phe Asp Glu Asn Ala Ala Glu Asp Phe Glu
            730                 735                 740

aaa cat gaa agc gtg gaa tat aaa cct cca aaa aga ccc ttt gtg aag        2309
Lys His Glu Ser Val Glu Tyr Lys Pro Pro Lys Arg Pro Phe Val Lys
        745                 750                 755

tta aaa gac tgc att gaa ctt ttc aca aca aaa gag aag cta ggt gct        2357
Leu Lys Asp Cys Ile Glu Leu Phe Thr Thr Lys Glu Lys Leu Gly Ala
    760                 765                 770

gaa gac ccc tgg tat tgt cca aat tgt aaa gag cat cag caa gcc acc        2405
Glu Asp Pro Trp Tyr Cys Pro Asn Cys Lys Glu His Gln Gln Ala Thr
775                 780                 785                 790

aag aaa tta gat ctg tgg tcc ctg cct ccc gtt ctt gtg gtg cat ctc        2453
Lys Lys Leu Asp Leu Trp Ser Leu Pro Pro Val Leu Val Val His Leu
                795                 800                 805
```

```
aag aga ttc tcc tat agt cgc tac atg aga gac aag cta gat aca tta      2501
Lys Arg Phe Ser Tyr Ser Arg Tyr Met Arg Asp Lys Leu Asp Thr Leu
            810                 815                 820

gtc gat ttt ccc atc agt gac ttg gat atg tcg gaa ttc cta att aac      2549
Val Asp Phe Pro Ile Ser Asp Leu Asp Met Ser Glu Phe Leu Ile Asn
        825                 830                 835

cca aat gca ggc cct tgc cgc tat aat ttg att gct gtt tcc aat cac      2597
Pro Asn Ala Gly Pro Cys Arg Tyr Asn Leu Ile Ala Val Ser Asn His
    840                 845                 850

tat gga ggg atg gga gga gga cat tat act gct ttt gca aag aac aaa      2645
Tyr Gly Gly Met Gly Gly Gly His Tyr Thr Ala Phe Ala Lys Asn Lys
855                 860                 865                 870

gat gat gga aag tgg tac tat ttt gat gac agt agc gtc tcc agt gca      2693
Asp Asp Gly Lys Trp Tyr Tyr Phe Asp Asp Ser Ser Val Ser Ser Ala
            875                 880                 885

tct gaa gac caa att gtg tcc aaa gca gca tac gtg ctc ttc tac cag      2741
Ser Glu Asp Gln Ile Val Ser Lys Ala Ala Tyr Val Leu Phe Tyr Gln
            890                 895                 900

aga cag gac act ttc agt gga act ggc ttc ttc cct ctc gac cga gaa      2789
Arg Gln Asp Thr Phe Ser Gly Thr Gly Phe Phe Pro Leu Asp Arg Glu
        905                 910                 915

act aaa ggt gct tca gct gcc aca ggt gtc ccc ctg gaa agt gac gaa      2837
Thr Lys Gly Ala Ser Ala Ala Thr Gly Val Pro Leu Glu Ser Asp Glu
    920                 925                 930

gac agc aat gat aat gac aat gat cta gaa aat gaa aac tgt atg cac      2885
Asp Ser Asn Asp Asn Asp Asn Asp Leu Glu Asn Glu Asn Cys Met His
935                 940                 945                 950

act aat taatgaaagt ccaagaagcc atacgagaga gagagaaaga gagagagaga      2941
Thr Asn

ctttcctgcc ggtggtatct gtggagacgg agttacccac cgcattaaac agaggtttga    3001

gaggaggcgt ttcagatacc cgaatgtaaa tcctttatca gattttaaat tctgcagtac    3061

ttgccgtgaa acacaatgaa aactttaaca gaaattgttt tttaaaacat ttacagtttt    3121

gtatttacaa gctaaatata tataggaaat cacaaataaa tccctttttaa gttaaaaaaa   3181

aaaaaaaaaa aaaaaaaaaa aaaaaaaaaa aaaaaaaaaa aaaaaaaaaa aaaaaaaaaa    3241

aaaaaa                                                               3247


<210>  46
<211>  952
<212>  PRT
<213>  Rattus norvegicus

<400>  46

Met Ala Glu Gly Gly Ala Ala Asp Leu Asp Thr Gln Arg Ser Asp Ile
1               5                   10                  15


Ala Thr Leu Leu Lys Thr Ser Leu Arg Lys Gly Asp Thr Trp Tyr Leu
            20                  25                  30


Val Asp Ser Arg Trp Phe Lys Gln Trp Lys Lys Tyr Val Gly Phe Asp
        35                  40                  45


Ser Trp Asp Lys Tyr Gln Met Gly Asp Gln Asn Val Tyr Pro Gly Pro
    50                  55                  60
```

Ile Asp Asn Ser Gly Leu Leu Lys Asp Gly Asp Ala Gln Ser Leu Lys
65 70 75 80

Glu His Leu Ile Asp Glu Leu Asp Tyr Ile Leu Leu Pro Thr Glu Gly
85 90 95

Trp Asn Lys Leu Val Ser Trp Tyr Thr Leu Met Glu Gly Gln Glu Pro
100 105 110

Ile Ala Arg Lys Val Val Glu Gln Gly Met Phe Val Lys His Cys Lys
115 120 125

Val Glu Val Tyr Leu Thr Glu Leu Lys Leu Cys Glu Asn Gly Asn Met
130 135 140

Asn Asn Val Val Thr Arg Arg Phe Ser Lys Ala Asp Thr Ile Asp Thr
145 150 155 160

Ile Glu Lys Glu Ile Arg Lys Ile Phe Asn Ile Pro Asp Glu Lys Glu
165 170 175

Ala Arg Leu Trp Asn Lys Tyr Met Ser Asn Thr Phe Glu Pro Leu Asn
180 185 190

Lys Pro Asp Ser Thr Ile Gln Asp Ala Gly Leu Tyr Gln Gly Gln Val
195 200 205

Leu Val Ile Glu Gln Lys Asn Glu Asp Gly Thr Trp Pro Arg Gly Pro
210 215 220

Ser Ala Pro Asn Val Lys Asn Ser Asn Tyr Cys Leu Pro Ser Tyr Thr
225 230 235 240

Ala Tyr Lys Asn Tyr Asp Tyr Ser Glu Pro Gly Arg Asn Asn Glu Gln
245 250 255

Pro Gly Leu Cys Gly Leu Ser Asn Leu Gly Asn Thr Cys Phe Met Asn
260 265 270

Ser Ala Ile Gln Cys Leu Ser Asn Thr Pro Pro Leu Thr Glu Tyr Phe
275 280 285

Leu Asn Asp Lys Tyr Gln Glu Glu Leu Asn Phe Asp Asn Pro Leu Gly
290 295 300

Met Arg Gly Glu Ile Ala Lys Ser Tyr Ala Glu Leu Ile Lys Gln Met
305 310 315 320

Trp Ser Gly Lys Phe Ser Tyr Val Thr Pro Arg Ala Phe Lys Thr Gln
325 330 335

Val Gly Arg Phe Ala Pro Gln Phe Ser Gly Tyr Gln Gln Gln Asp Cys
340 345 350

Gln Glu Leu Leu Ala Phe Leu Leu Asp Gly Leu His Glu Asp Leu Asn
355 360 365

Arg Ile Arg Lys Lys Pro Tyr Ile Gln Leu Lys Asp Ala Asp Gly Arg
370 375 380

Pro Asp Lys Val Val Ala Glu Glu Ala Trp Glu Asn His Leu Lys Arg
385 390 395 400

Asn Asp Ser Ile Ile Val Asp Ile Phe His Gly Leu Phe Lys Ser Thr
405 410 415

Leu Val Cys Pro Glu Cys Ala Lys Ile Ser Val Thr Phe Asp Pro Phe
420 425 430

Cys Tyr Leu Thr Leu Pro Leu Pro Met Lys Lys Glu Arg Ser Leu Glu
435 440 445

Val Tyr Leu Val Arg Met Asp Pro Leu Ala Lys Pro Met Gln Tyr Lys
450 455 460

Val Ile Val Pro Lys Ile Gly Asn Ile Leu Asp Leu Cys Thr Ala Leu
465 470 475 480

Ser Ala Leu Ser Gly Val Pro Ala Asp Lys Met Ile Val Thr Asp Ile
485 490 495

Tyr Asn His Arg Phe His Arg Ile Phe Ala Met Asp Glu Asn Leu Ser
500 505 510

Ser Ile Met Glu Arg Asp Asp Ile Tyr Val Phe Glu Ile Asn Ile Asn
515 520 525

Arg Thr Glu Asp Thr Glu His Val Val Ile Pro Val Cys Leu Arg Glu
530 535 540

Lys Phe Arg His Ser Ser Tyr Thr His His Thr Gly Ser Ser Leu Phe
545 550 555 560

Gly Gln Pro Phe Leu Met Ala Val Pro Arg Asn Asn Thr Glu Asp Lys
565 570 575

Leu Tyr Asn Leu Leu Leu Leu Arg Met Cys Arg Tyr Val Lys Met Ser
580 585 590

Thr Glu Thr Glu Glu Thr Asp Gly Pro Leu Arg Cys Cys Glu Asp Gln
595 600 605

Asn Ile Asn Gly Asn Gly Pro Asn Gly Ile His Glu Glu Gly Ser Pro
610 615 620

Ser Glu Met Glu Thr Asp Glu Pro Asp Asp Glu Ser Ser Gln Asp Gln
625 630 635 640

Glu Leu Pro Ser Glu Asn Glu Asn Ser Gln Ser Glu Asp Ser Val Gly
645 650 655

Gly Asp Asn Asp Ser Glu Asn Gly Leu Cys Thr Glu Glu Thr Cys Lys
660 665 670

Gly Arg Leu Thr Gly His Lys Lys Arg Leu Phe Thr Phe Gln Phe Asn
675 680 685

Asn Leu Gly Asn Thr Asp Ile Asn Tyr Ile Lys Asp Asp Thr Arg His
690 695 700

Ile Arg Phe Asp Asp Arg Gln Leu Arg Leu Asp Glu Arg Ser Phe Leu
705 710 715 720

Ala Leu Asp Trp Asp Pro Asp Leu Lys Lys Arg Tyr Phe Asp Glu Asn
725 730 735

Ala Ala Glu Asp Phe Glu Lys His Glu Ser Val Glu Tyr Lys Pro Pro
740 745 750

Lys Arg Pro Phe Val Lys Leu Lys Asp Cys Ile Glu Leu Phe Thr Thr
755 760 765

Lys Glu Lys Leu Gly Ala Glu Asp Pro Trp Tyr Cys Pro Asn Cys Lys
770 775 780

Glu His Gln Gln Ala Thr Lys Lys Leu Asp Leu Trp Ser Leu Pro Pro
785 790 795 800

Val Leu Val Val His Leu Lys Arg Phe Ser Tyr Ser Arg Tyr Met Arg
805 810 815

Asp Lys Leu Asp Thr Leu Val Asp Phe Pro Ile Ser Asp Leu Asp Met
820 825 830

Ser Glu Phe Leu Ile Asn Pro Asn Ala Gly Pro Cys Arg Tyr Asn Leu
835 840 845

Ile Ala Val Ser Asn His Tyr Gly Gly Met Gly Gly Gly His Tyr Thr
850 855 860

Ala Phe Ala Lys Asn Lys Asp Asp Gly Lys Trp Tyr Tyr Phe Asp Asp
865 870 875 880

Ser Ser Val Ser Ser Ala Ser Glu Asp Gln Ile Val Ser Lys Ala Ala
885 890 895

Tyr Val Leu Phe Tyr Gln Arg Gln Asp Thr Phe Ser Gly Thr Gly Phe
900 905 910

Phe Pro Leu Asp Arg Glu Thr Lys Gly Ala Ser Ala Ala Thr Gly Val
915 920 925

Pro Leu Glu Ser Asp Glu Asp Ser Asn Asp Asn Asp Asn Asp Leu Glu
930 935 940

Asn Glu Asn Cys Met His Thr Asn
945 950

<210> 47
<211> 1458
<212> DNA

<213> Rattus norvegicus

<220>
<221> CDS
<222> (91)..(1074)

<400> 47
ttaccagcaa gtgtgcatag ggacaaacgg agaccgatcg cagcaggctg agggtgcaat 60

aacgacctcc tggaccgacc cgcccgcgcc atg cgc tcg cac act ggc ttg agg 114
Met Arg Ser His Thr Gly Leu Arg
1 5

gcg ctt gtg gcg cct ggt tgc tcg ctc ctc ctt cta tat ctt ctg gcg 162
Ala Leu Val Ala Pro Gly Cys Ser Leu Leu Leu Leu Tyr Leu Leu Ala
10 15 20

gcg acg cgc ccg gat cgg gct gtg ggc gat ccc gca gac tca gcg ttt 210
Ala Thr Arg Pro Asp Arg Ala Val Gly Asp Pro Ala Asp Ser Ala Phe
25 30 35 40

aca agt cta cct gtt cga gag gaa atg atg gct aag tac gca aat ctg 258
Thr Ser Leu Pro Val Arg Glu Glu Met Met Ala Lys Tyr Ala Asn Leu
45 50 55

tcc ttg gag acc tat aat atc tca ctg aca gaa cag acc aga gta tca 306
Ser Leu Glu Thr Tyr Asn Ile Ser Leu Thr Glu Gln Thr Arg Val Ser
60 65 70

gaa caa aac ata act ttg gaa aga cct tct cac ctg gaa ctc gaa tgc 354
Glu Gln Asn Ile Thr Leu Glu Arg Pro Ser His Leu Glu Leu Glu Cys
75 80 85

acg ttc acg gca act gag gat gtg atg tca atg aat gtg act tgg aag 402
Thr Phe Thr Ala Thr Glu Asp Val Met Ser Met Asn Val Thr Trp Lys
90 95 100

aaa gat gac gcg ctc ctt gag act act gat ggt ttc aat aca act aaa 450
Lys Asp Asp Ala Leu Leu Glu Thr Thr Asp Gly Phe Asn Thr Thr Lys
105 110 115 120

atg ggg gac acc tta tac agt caa tac agg ttc acc gtt ttt aat agc 498
Met Gly Asp Thr Leu Tyr Ser Gln Tyr Arg Phe Thr Val Phe Asn Ser
125 130 135

aaa caa atg gga aaa tac tct tgt ttc ctt gga gaa gag ctc aga gga 546
Lys Gln Met Gly Lys Tyr Ser Cys Phe Leu Gly Glu Glu Leu Arg Gly
140 145 150

acc ttt aat atc aga gta ccc aaa gtt cat ggg aaa aac aag cct ttg 594
Thr Phe Asn Ile Arg Val Pro Lys Val His Gly Lys Asn Lys Pro Leu
155 160 165

atc act tac gtg ggg gat tct act gtg ctg aag tgt gaa tgt caa aat 642
Ile Thr Tyr Val Gly Asp Ser Thr Val Leu Lys Cys Glu Cys Gln Asn
170 175 180

tgt ctt cct tta aat tgg act tgg tac atg agt aat gga act gca cag 690
Cys Leu Pro Leu Asn Trp Thr Trp Tyr Met Ser Asn Gly Thr Ala Gln
185 190 195 200

gtt ccc atc gat gtt cac gtg aat gat aag ttt gat ata aat ggt tcc 738
Val Pro Ile Asp Val His Val Asn Asp Lys Phe Asp Ile Asn Gly Ser
205 210 215

tat gct aac gaa aca aag ctc aag gta aag cac ctt ttg gag gaa gat 786
Tyr Ala Asn Glu Thr Lys Leu Lys Val Lys His Leu Leu Glu Glu Asp
220 225 230

gga gga tcc tac tgg tgc cgg gca gcc ttc ccg tta ggg gag agt gag 834
Gly Gly Ser Tyr Trp Cys Arg Ala Ala Phe Pro Leu Gly Glu Ser Glu
235 240 245

gaa cac att aag ctg gtt gtg ctg agt ttc atg gtg cct ctc aag cca 882
Glu His Ile Lys Leu Val Val Leu Ser Phe Met Val Pro Leu Lys Pro

250 255 260

ttc ctt gcc ata att gct gaa gtc atc ctc tta gtg gcc atc atc ctg 930
Phe Leu Ala Ile Ile Ala Glu Val Ile Leu Leu Val Ala Ile Ile Leu
265 270 275 280

ctt tgt gaa gtg tac acg cag aag aaa aag aat gac cca gat gat ggg 978
Leu Cys Glu Val Tyr Thr Gln Lys Lys Lys Asn Asp Pro Asp Asp Gly
285 290 295

aaa gaa ttt gaa caa atc gaa cag ttg aaa tca gat gat agc aat gga 1026
Lys Glu Phe Glu Gln Ile Glu Gln Leu Lys Ser Asp Asp Ser Asn Gly
300 305 310

ata gaa aac aat gtc ccc cgg tac aga aaa acg gac tct ggg gat cag 1074
Ile Glu Asn Asn Val Pro Arg Tyr Arg Lys Thr Asp Ser Gly Asp Gln
315 320 325

tgaatccaaa aagaactttt tgccttggga agacgtacag cactggagtt tgaatttcag 1134

cattcattgg agaacatccg agttttagat ttttccgaag acaaagatgc cctcactgtg 1194

cccaggagta gtttcgtaaa caatatagac catctcctac ctaaagatgt attttcattg 1254

tgtaattgct tttcattaaa gcaaggtaca ttgtttcagc tgtgttctgt gagctgtaac 1314

ctagataata aagtttaccc tctctgggag atcaggggga cagggcagac accagcaaaa 1374

tcagttaata acccacaaag taaatattgc taaagatctg tttattacca aagaatttat 1434

taaagagaaa gtgtttgcca aaaa 1458

<210> 48
<211> 328
<212> PRT
<213> Rattus norvegicus

<400> 48

Met Arg Ser His Thr Gly Leu Arg Ala Leu Val Ala Pro Gly Cys Ser
1 5 10 15

Leu Leu Leu Leu Tyr Leu Leu Ala Ala Thr Arg Pro Asp Arg Ala Val
20 25 30

Gly Asp Pro Ala Asp Ser Ala Phe Thr Ser Leu Pro Val Arg Glu Glu
35 40 45

Met Met Ala Lys Tyr Ala Asn Leu Ser Leu Glu Thr Tyr Asn Ile Ser
50 55 60

Leu Thr Glu Gln Thr Arg Val Ser Glu Gln Asn Ile Thr Leu Glu Arg
65 70 75 80

Pro Ser His Leu Glu Leu Glu Cys Thr Phe Thr Ala Thr Glu Asp Val
85 90 95

Met Ser Met Asn Val Thr Trp Lys Lys Asp Asp Ala Leu Leu Glu Thr
100 105 110

Thr Asp Gly Phe Asn Thr Thr Lys Met Gly Asp Thr Leu Tyr Ser Gln
115 120 125

Tyr Arg Phe Thr Val Phe Asn Ser Lys Gln Met Gly Lys Tyr Ser Cys
130 135 140

Phe Leu Gly Glu Glu Leu Arg Gly Thr Phe Asn Ile Arg Val Pro Lys
145 150 155 160

Val His Gly Lys Asn Lys Pro Leu Ile Thr Tyr Val Gly Asp Ser Thr
165 170 175

Val Leu Lys Cys Glu Cys Gln Asn Cys Leu Pro Leu Asn Trp Thr Trp
180 185 190

Tyr Met Ser Asn Gly Thr Ala Gln Val Pro Ile Asp Val His Val Asn
195 200 205

Asp Lys Phe Asp Ile Asn Gly Ser Tyr Ala Asn Glu Thr Lys Leu Lys
210 215 220

Val Lys His Leu Leu Glu Glu Asp Gly Gly Ser Tyr Trp Cys Arg Ala
225 230 235 240

Ala Phe Pro Leu Gly Glu Ser Glu Glu His Ile Lys Leu Val Val Leu
245 250 255

Ser Phe Met Val Pro Leu Lys Pro Phe Leu Ala Ile Ile Ala Glu Val
260 265 270

Ile Leu Leu Val Ala Ile Ile Leu Leu Cys Glu Val Tyr Thr Gln Lys
275 280 285

Lys Lys Asn Asp Pro Asp Asp Gly Lys Glu Phe Glu Gln Ile Glu Gln
290 295 300

Leu Lys Ser Asp Asp Ser Asn Gly Ile Glu Asn Asn Val Pro Arg Tyr
305 310 315 320

Arg Lys Thr Asp Ser Gly Asp Gln
325

<210> 49
<211> 4157
<212> DNA
<213> Rattus norvegicus

<220>
<221> CDS
<222> (2884)..(4002)

<400> 49
acgagccgca ttgcaggtgc ggggcagcgc cccggggtcc ggcccccacgc cggcgggatg 60

ctgcgggcct cagctccccg cccgcgtcac acctgccgcc tcccggaccc gcagccctgg 120

cgaccgggtg gctccgcgtc acctggatgt cagcatggca gccacaaacc tggagaacca 180

gctgcacagt gcgcagaaga acctgctctt cctccagcgg gaacatgcca gcacactcaa 240

ggggctgcac gctgagatcc ggcggctgca gcagcactgc acagatctaa catatgagct 300

gacactcaaa agtttcgaac tgacagagct ggcactccag agacgctgac cccagctgac 360

gctcaagctg ttttaacttc agcaccttca gattctggag acgttctttt acgcagagac 420

ggctcttcca gaacgacgga gctgaagagg cgctgtgaag agctggaagc ccagctgaag 480

gccaaagagg aggaaaaccg caagctgctt caagaactgg agcagaagaa tgccgccatt 540

gctgtgctgg agaacacggt ccgcgagaga gagaagaagt acctggagga gctgaagatg 600
aagagccaca agctgagcat gctgtcgggc gagctggagc agcgcgccag caccgtggcc 660
tacctcacct cacagctgca tgcggccaag aagaagttgc tgagctccag tggcacctcg 720
gatgccagcc ccgccggcag ccctgcactg gccagctaca agcccacacc ccccaaggac 780
aagctgcctg agacaccccg ccgccgcatg aagaagagcc tgtcagcccc actgcaccct 840
gagtttgagg aggtctacag gtttggagct gagagccgaa aactcctcct tcgagagcct 900
gtggatgcca tgcctgaccc caccccattc ctgctggccc gggagtctgc tgaggtccag 960
ctcaaggagc ggcctcttgt catccctccc atcgcctcag atcgtggcac cagtgggcag 1020
catagcccag cccgagacaa gccacacaag acccacattg ggtggctcca ccgcattcac 1080
catgccactc catcacaggc ccagcccgag ggagagacga gggccgtgga ccaggtgaat 1140
gcagggaagg tggtgcgaaa gcactcaggg acagaccgga ctgtgtgaag cctgcggcct 1200
gtcaccgcag ccatccacgc actgtgagcg ccacagggaa cctcgcccac atcttcctcg 1260
cctacccatg ccaaatgttt cctcctgttg aagcctggct cctggctcca tagtgacagt 1320
gctacaatgg tgtccccgct gaagatattt actggcactg tggccaatgc ctgcatgcca 1380
atagcttgct tccagcccca cgccatgaga tctcggcaca ctcttatatg gccatggctg 1440
aatcactacg ccacacagaa tcagaatcgc ctgaagtcga agcaaaaata aggagctctc 1500
tctctctttc cctctctctc tctttctctc cctccccagt tccagggcac ctacgagagc 1560
agagcagcag cttctgcttc cccctggaga ctcctgcctt gcacagtccc tttacaagca 1620
gctatgtctc atgatcttag caagatgacc tcaaccctga tgtcgcccca acctgaatgt 1680
gtgttcataa tttttttttg ttagtttcat ccagatgttc aggatccaac aaatgttatt 1740
ttctaaaccc gccctccttt tcctctgcgt ctttttgaag caccaaggga ctggaggagg 1800
tggctgggga ggaccaagca ggtggggtgg gagtacttgc tgcccagagc tgttgctgag 1860
atggatgtct gcgtttctgg cagaagctgc attccgtcct gcccaggctt cccactgcct 1920
gcctgtctcc agctggccag cccagtctca cagtgtaaga ggcacagcca gtggctgttt 1980
gtcctgaagc cacacacagc cctggctgag aagaggtctg gagctcgggc aggaactgaa 2040
tgcttgagcc ttcagtgctc ttgccaagaa agggggtgtg tgctggagca gacgagtgga 2100
accccaccag tggcgtagca ggaagggaga gagaacgcac ttgccttaaa acctgttctg 2160
gcctgtgctc tgagaagctg cagccaattc cagcacccgc gtggcagctc acaattagct 2220
gtaactccaa ggcccacctt acatgtggta cacggatatt caggcaggct ctcacacata 2280
cacagaaata aaatcttaaa aaaataaaaa aaagcggggc cgggcggagg cagcagcagc 2340
acgctgtgcc tcccgccgtc gctccactct cgccgtccgg cctctgtcct cgccggggta 2400
tggccccttg ggagaggcct tgagatctgc gggggtccga gagtcggggc gccgactttc 2460
cggttatttt agtgggaagg gctgcttctc aaagtggatt accccaactc ctccgggggc 2520
gggaagcggg gatcctcccc caaccgcaaa tactcaaaga aagcaccggc tgaagacata 2580
gacgatggag agtctcggtt ctcagagtcc ccttctaata ccttcggctt cgttgcctac 2640
tctgtgaaca acggggagaa gtcgagggct aaggttaaat cccaccagcc tacttattcc 2700
agcacctccc actccgaacg atctgggtcc cccatcccat cgccctcacc cagaaagcag 2760
cgttgcttag accagcgaga aatggagctg gaggagagga acgaacacta ctctaaaaag 2820

```
aggaggatta gagagtacaa cctcaatgta tttgattcac attggctggg aacaccttcc   2880

acc atg gcc acc tca gca agt tcc cac ttg aac aaa ggc atc aag cag     2928
    Met Ala Thr Ser Ala Ser Ser His Leu Asn Lys Gly Ile Lys Gln
    1               5                   10                  15

atg tac atg aac ctg ccc cag ggc gag aag atc caa ctc atg tat atc     2976
Met Tyr Met Asn Leu Pro Gln Gly Glu Lys Ile Gln Leu Met Tyr Ile
                20                  25                  30

tgg gtt gat ggt acc ggg gaa ggg cta cgc tgc aag acc cgt act ctg     3024
Trp Val Asp Gly Thr Gly Glu Gly Leu Arg Cys Lys Thr Arg Thr Leu
            35                  40                  45

gac tgt gac ccc aag tgt gta gaa gag tta ccc gag tgg aac ttt gat     3072
Asp Cys Asp Pro Lys Cys Val Glu Glu Leu Pro Glu Trp Asn Phe Asp
        50                  55                  60

ggt tct agt acg ttt cag tct gaa ggc tcc aac agc gac atg tac ctc     3120
Gly Ser Ser Thr Phe Gln Ser Glu Gly Ser Asn Ser Asp Met Tyr Leu
    65                  70                  75

cat cct gtg gcc atg ttt cga gac ccc ttc cgc aga gac ccc aac aag     3168
His Pro Val Ala Met Phe Arg Asp Pro Phe Arg Arg Asp Pro Asn Lys
80                  85                  90                  95

ctg gtg ttc tgc gaa gta ttc aag tat aac cgg aag ccc gca gag acc     3216
Leu Val Phe Cys Glu Val Phe Lys Tyr Asn Arg Lys Pro Ala Glu Thr
                100                 105                 110

aac ctg agg cac agc tgt aag cgt ata atg gac atg gtg agc agc cag     3264
Asn Leu Arg His Ser Cys Lys Arg Ile Met Asp Met Val Ser Ser Gln
            115                 120                 125

cac ccc tgg ttt gga atg gaa cag gag tat act ctc atg gga aca gac     3312
His Pro Trp Phe Gly Met Glu Gln Glu Tyr Thr Leu Met Gly Thr Asp
        130                 135                 140

ggc cac cct ttc ggc tgg cct tct aat ggc ttc cct gga ccc caa gga     3360
Gly His Pro Phe Gly Trp Pro Ser Asn Gly Phe Pro Gly Pro Gln Gly
    145                 150                 155

ccc tat tac tgc ggt gtg gga gct gac aag gct tat ggc cga gat atc     3408
Pro Tyr Tyr Cys Gly Val Gly Ala Asp Lys Ala Tyr Gly Arg Asp Ile
160                 165                 170                 175

gtg gag gct cac tac cgg gcc tgc ttg tat gct gga atc aag atc aca     3456
Val Glu Ala His Tyr Arg Ala Cys Leu Tyr Ala Gly Ile Lys Ile Thr
                180                 185                 190

ggg aca aat gcc gag gtt atg cct gcc cag tgg gaa ttc cag ata gga     3504
Gly Thr Asn Ala Glu Val Met Pro Ala Gln Trp Glu Phe Gln Ile Gly
            195                 200                 205

ccc tgc gaa ggg atc cgc atg gga gat cat ctc tgg gta gcc cgt ttt     3552
Pro Cys Glu Gly Ile Arg Met Gly Asp His Leu Trp Val Ala Arg Phe
        210                 215                 220

atc ttg cat cgg gta tgc gaa gac ttt ggg gtg ata gca acc ttt gac     3600
Ile Leu His Arg Val Cys Glu Asp Phe Gly Val Ile Ala Thr Phe Asp
    225                 230                 235

ccc aag ccc att cca ggg aac tgg aat ggg gca ggc tgc cac acc aac     3648
Pro Lys Pro Ile Pro Gly Asn Trp Asn Gly Ala Gly Cys His Thr Asn
240                 245                 250                 255

ttt agc acc aag gcc atg cgg gag gag aat ggt ctg agg tgc att gag     3696
Phe Ser Thr Lys Ala Met Arg Glu Glu Asn Gly Leu Arg Cys Ile Glu
                260                 265                 270

gag gcc att gat aaa ctg agc aag agg cac cag tac cac atc cgt gcc     3744
Glu Ala Ile Asp Lys Leu Ser Lys Arg His Gln Tyr His Ile Arg Ala
            275                 280                 285

tac gac ccc aag ggg ggc ctg gac aac gcc cgc cgt ctg act gga ttc     3792
```

```
Tyr Asp Pro Lys Gly Gly Leu Asp Asn Ala Arg Arg Leu Thr Gly Phe
        290                 295                 300

cac gaa acc tcc aac atc aac gac ttt tcc gct ggc gtt gcc aac cgc      3840
His Glu Thr Ser Asn Ile Asn Asp Phe Ser Ala Gly Val Ala Asn Arg
    305                 310                 315

agc gcc agt atc cgc att ccc cgg att gtc ggc cag gag aag aag ggt      3888
Ser Ala Ser Ile Arg Ile Pro Arg Ile Val Gly Gln Glu Lys Lys Gly
320                 325                 330                 335

tac ttt gaa gac cgt cgg cct tct gcc aat tgc gac ccc tat gcg gtg      3936
Tyr Phe Glu Asp Arg Arg Pro Ser Ala Asn Cys Asp Pro Tyr Ala Val
                340                 345                 350

acg gaa gcc atc gtc cgc acg tgt ctc ctc aac gaa act ggc gac gag      3984
Thr Glu Ala Ile Val Arg Thr Cys Leu Leu Asn Glu Thr Gly Asp Glu
            355                 360                 365

ccc ttc caa tac aag aac taagcggact cgacttccag tgatcttgag             4032
Pro Phe Gln Tyr Lys Asn
        370

cccttcctag ttcaccccac tcccaactgt tccctctccc actggtcccc actgtaactc    4092

aaaaggatgg aataccaagg tctttttatt ccttgaaaaa aaaaaaaaaa aaaaaaaaaa    4152

aaaaa                                                                4157


<210>  50
<211>  373
<212>  PRT
<213>  Rattus norvegicus

<400>  50

Met Ala Thr Ser Ala Ser Ser His Leu Asn Lys Gly Ile Lys Gln Met
1               5                   10                  15

Tyr Met Asn Leu Pro Gln Gly Glu Lys Ile Gln Leu Met Tyr Ile Trp
            20                  25                  30

Val Asp Gly Thr Gly Glu Gly Leu Arg Cys Lys Thr Arg Thr Leu Asp
        35                  40                  45

Cys Asp Pro Lys Cys Val Glu Glu Leu Pro Glu Trp Asn Phe Asp Gly
    50                  55                  60

Ser Ser Thr Phe Gln Ser Glu Gly Ser Asn Ser Asp Met Tyr Leu His
65                  70                  75                  80

Pro Val Ala Met Phe Arg Asp Pro Phe Arg Arg Asp Pro Asn Lys Leu
                85                  90                  95

Val Phe Cys Glu Val Phe Lys Tyr Asn Arg Lys Pro Ala Glu Thr Asn
            100                 105                 110

Leu Arg His Ser Cys Lys Arg Ile Met Asp Met Val Ser Ser Gln His
        115                 120                 125

Pro Trp Phe Gly Met Glu Gln Glu Tyr Thr Leu Met Gly Thr Asp Gly
    130                 135                 140

His Pro Phe Gly Trp Pro Ser Asn Gly Phe Pro Gly Pro Gln Gly Pro
145                 150                 155                 160
```

Tyr Tyr Cys Gly Val Gly Ala Asp Lys Ala Tyr Gly Arg Asp Ile Val
165 170 175

Glu Ala His Tyr Arg Ala Cys Leu Tyr Ala Gly Ile Lys Ile Thr Gly
180 185 190

Thr Asn Ala Glu Val Met Pro Ala Gln Trp Glu Phe Gln Ile Gly Pro
195 200 205

Cys Glu Gly Ile Arg Met Gly Asp His Leu Trp Val Ala Arg Phe Ile
210 215 220

Leu His Arg Val Cys Glu Asp Phe Gly Val Ile Ala Thr Phe Asp Pro
225 230 235 240

Lys Pro Ile Pro Gly Asn Trp Asn Gly Ala Gly Cys His Thr Asn Phe
245 250 255

Ser Thr Lys Ala Met Arg Glu Glu Asn Gly Leu Arg Cys Ile Glu Glu
260 265 270

Ala Ile Asp Lys Leu Ser Lys Arg His Gln Tyr His Ile Arg Ala Tyr
275 280 285

Asp Pro Lys Gly Gly Leu Asp Asn Ala Arg Arg Leu Thr Gly Phe His
290 295 300

Glu Thr Ser Asn Ile Asn Asp Phe Ser Ala Gly Val Ala Asn Arg Ser
305 310 315 320

Ala Ser Ile Arg Ile Pro Arg Ile Val Gly Gln Glu Lys Lys Gly Tyr
325 330 335

Phe Glu Asp Arg Arg Pro Ser Ala Asn Cys Asp Pro Tyr Ala Val Thr
340 345 350

Glu Ala Ile Val Arg Thr Cys Leu Leu Asn Glu Thr Gly Asp Glu Pro
355 360 365

Phe Gln Tyr Lys Asn
370

<210> 51
<211> 1869
<212> DNA
<213> Rattus norvegicus

<220>
<221> CDS
<222> (6)..(1406)

<400> 51
ccacc atg tgg gga tgt ccg cta ggg ctg cta ttg ctg ctg ctg gct ggc 50
Met Trp Gly Cys Pro Leu Gly Leu Leu Leu Leu Leu Leu Ala Gly
1 5 10 15

cag gct gcc ctg gag gcc cgg cgg agt cgt tgg cgc agg gag ctg gcg 98
Gln Ala Ala Leu Glu Ala Arg Arg Ser Arg Trp Arg Arg Glu Leu Ala
20 25 30

```
cca ggg ctg cac ctg cgg ggc atc cgg gac gcc ggt ggc aga tac tgc        146
Pro Gly Leu His Leu Arg Gly Ile Arg Asp Ala Gly Gly Arg Tyr Cys
            35                  40                  45

caa gag cag gac atg tgc tgc cgc ggc cgt gct gac gag tgt gct ttg        194
Gln Glu Gln Asp Met Cys Cys Arg Gly Arg Ala Asp Glu Cys Ala Leu
        50                  55                  60

ccc tac ctg gga gcc acc tgt tac tgt gac ctc ttc tgc aac cgc acc        242
Pro Tyr Leu Gly Ala Thr Cys Tyr Cys Asp Leu Phe Cys Asn Arg Thr
    65                  70                  75

gtc tct gac tgc tgc cct gac ttt tgg gac ttc tgc ctc ggg att cca        290
Val Ser Asp Cys Cys Pro Asp Phe Trp Asp Phe Cys Leu Gly Ile Pro
80                  85                  90                  95

ccc ccc ttc cct cct gtc caa ggt tgc atg cat gcg ggc cgg atc tac        338
Pro Pro Phe Pro Pro Val Gln Gly Cys Met His Ala Gly Arg Ile Tyr
                100                 105                 110

cca atc ttc gga acc tat tgg gaa aac tgc aat cgg tgc acc tgc cat        386
Pro Ile Phe Gly Thr Tyr Trp Glu Asn Cys Asn Arg Cys Thr Cys His
            115                 120                 125

gag aaa ggg cag tgg gaa tgt gac cag gag cca tgt cta gtg gac cca        434
Glu Lys Gly Gln Trp Glu Cys Asp Gln Glu Pro Cys Leu Val Asp Pro
        130                 135                 140

gcc atg att aaa gcc atc aac cgg ggc aac tac ggg tgg cag gct ggg        482
Ala Met Ile Lys Ala Ile Asn Arg Gly Asn Tyr Gly Trp Gln Ala Gly
    145                 150                 155

aac cac agt gcc ttc tgg ggc atg acc ctg gat gag ggc att cga tac        530
Asn His Ser Ala Phe Trp Gly Met Thr Leu Asp Glu Gly Ile Arg Tyr
160                 165                 170                 175

cgg ctg ggc aca atc cgc cca tct tcc tct gtc atg aat atg aat gaa        578
Arg Leu Gly Thr Ile Arg Pro Ser Ser Ser Val Met Asn Met Asn Glu
                180                 185                 190

att tat aca gtg ctg gga caa gga gaa gtg cta ccc act gcc ttt gag        626
Ile Tyr Thr Val Leu Gly Gln Gly Glu Val Leu Pro Thr Ala Phe Glu
            195                 200                 205

gct tcc gag aag tgg ccc aac ctg atc cat gag ccg ctg gac cag ggc        674
Ala Ser Glu Lys Trp Pro Asn Leu Ile His Glu Pro Leu Asp Gln Gly
        210                 215                 220

aac tgt gcg ggt tcc tgg gct ttc tcc aca gca gct gtc gca tct gac        722
Asn Cys Ala Gly Ser Trp Ala Phe Ser Thr Ala Ala Val Ala Ser Asp
    225                 230                 235

cga gtg tcc atc cat tct ttg gga cac atg aca ccc atc ctc tca ccg        770
Arg Val Ser Ile His Ser Leu Gly His Met Thr Pro Ile Leu Ser Pro
240                 245                 250                 255

cag aac ctg cta tcc tgt gat acc cac cac cag aag ggc tgc cga ggt        818
Gln Asn Leu Leu Ser Cys Asp Thr His His Gln Lys Gly Cys Arg Gly
                260                 265                 270

ggg cgt ctc gat ggt gct tgg tgg ttc ctg cgg cgc cga ggg gtg gtg        866
Gly Arg Leu Asp Gly Ala Trp Trp Phe Leu Arg Arg Arg Gly Val Val
            275                 280                 285

tct gat aac tgt tac cca ttc tcc ggc cgt gag cag aac gac gag gcc        914
Ser Asp Asn Cys Tyr Pro Phe Ser Gly Arg Glu Gln Asn Asp Glu Ala
        290                 295                 300

agc ccc act cct cga tgc atg atg cac agc cgc gcc atg ggg cgg ggc        962
Ser Pro Thr Pro Arg Cys Met Met His Ser Arg Ala Met Gly Arg Gly
    305                 310                 315

aag cgc cag gcc act tct cgc tgc ccc aat agt cag gtt gat tcc aat       1010
Lys Arg Gln Ala Thr Ser Arg Cys Pro Asn Ser Gln Val Asp Ser Asn
320                 325                 330                 335
```

gac atc tac cag gtc acg cct gtc tac cgc ctg gcc tcc gat gag aag 1058
Asp Ile Tyr Gln Val Thr Pro Val Tyr Arg Leu Ala Ser Asp Glu Lys
340 345 350

gag atc atg aag gag cta atg gaa aat ggc ccc gtt caa gcg ctt atg 1106
Glu Ile Met Lys Glu Leu Met Glu Asn Gly Pro Val Gln Ala Leu Met
355 360 365

gaa gta cac gaa gac ttc ttc ttg tac cag cga ggc atc tac agc cac 1154
Glu Val His Glu Asp Phe Phe Leu Tyr Gln Arg Gly Ile Tyr Ser His
370 375 380

aca cct gta agc cag ggg agg cca gag cag tac cgc cga cac ggg act 1202
Thr Pro Val Ser Gln Gly Arg Pro Glu Gln Tyr Arg Arg His Gly Thr
385 390 395

cac tct gtc aag atc aca ggg tgg gga gaa gag aca ctg cca gac gga 1250
His Ser Val Lys Ile Thr Gly Trp Gly Glu Glu Thr Leu Pro Asp Gly
400 405 410 415

agg acg atc aag tac tgg act gct gcc aac tcg tgg ggc cca tgg tgg 1298
Arg Thr Ile Lys Tyr Trp Thr Ala Ala Asn Ser Trp Gly Pro Trp Trp
420 425 430

ggt gag agg ggc cac ttc cga atc gtg cgt ggc atc aac gag tgt gac 1346
Gly Glu Arg Gly His Phe Arg Ile Val Arg Gly Ile Asn Glu Cys Asp
435 440 445

atc gag acc ttc gtg ctg ggc gtc tgg ggc cgc gta gga atg gag gac 1394
Ile Glu Thr Phe Val Leu Gly Val Trp Gly Arg Val Gly Met Glu Asp
450 455 460

atg ggg cac cac tgagtctcgg ccactaagct aggtgggatc cacagccacg 1446
Met Gly His His
465

gaagaggcct tgggggccac gccgatgagg ccttgggacc aggtgctaat cccctcagac 1506

tcagatccac acaaacgcag aaccccacct gggagctaat gtccctcagg gagcaacagt 1566

gaggctggag ggaaccctca gacatcacag ccggaactgg aaagggcccg gcttggaaac 1626

tgcagggagt aaaattccca ggcccctggt cagccagccc aagaccatgg gagctaagac 1686

accccaacct ctttatcctc ctacccaacc tcattcttat tttttcattc tctttggtgg 1746

attctgccca tccccctggc ctccgttctg gctggacctt tccatcctca acactgcttt 1806

cttactcttt aaaaatattt atttttcttt tcattaaaat aaaaccaaag tattgataat 1866

tgc 1869

<210> 52
<211> 467
<212> PRT
<213> Rattus norvegicus

<400> 52

Met Trp Gly Cys Pro Leu Gly Leu Leu Leu Leu Leu Leu Ala Gly Gln
1 5 10 15

Ala Ala Leu Glu Ala Arg Arg Ser Arg Trp Arg Arg Glu Leu Ala Pro
20 25 30

Gly Leu His Leu Arg Gly Ile Arg Asp Ala Gly Gly Arg Tyr Cys Gln
35 40 45

Glu Gln Asp Met Cys Cys Arg Gly Arg Ala Asp Glu Cys Ala Leu Pro
50 55 60

Tyr Leu Gly Ala Thr Cys Tyr Cys Asp Leu Phe Cys Asn Arg Thr Val
65 70 75 80

Ser Asp Cys Cys Pro Asp Phe Trp Asp Phe Cys Leu Gly Ile Pro Pro
85 90 95

Pro Phe Pro Pro Val Gln Gly Cys Met His Ala Gly Arg Ile Tyr Pro
100 105 110

Ile Phe Gly Thr Tyr Trp Glu Asn Cys Asn Arg Cys Thr Cys His Glu
115 120 125

Lys Gly Gln Trp Glu Cys Asp Gln Glu Pro Cys Leu Val Asp Pro Ala
130 135 140

Met Ile Lys Ala Ile Asn Arg Gly Asn Tyr Gly Trp Gln Ala Gly Asn
145 150 155 160

His Ser Ala Phe Trp Gly Met Thr Leu Asp Glu Gly Ile Arg Tyr Arg
165 170 175

Leu Gly Thr Ile Arg Pro Ser Ser Ser Val Met Asn Met Asn Glu Ile
180 185 190

Tyr Thr Val Leu Gly Gln Gly Glu Val Leu Pro Thr Ala Phe Glu Ala
195 200 205

Ser Glu Lys Trp Pro Asn Leu Ile His Glu Pro Leu Asp Gln Gly Asn
210 215 220

Cys Ala Gly Ser Trp Ala Phe Ser Thr Ala Ala Val Ala Ser Asp Arg
225 230 235 240

Val Ser Ile His Ser Leu Gly His Met Thr Pro Ile Leu Ser Pro Gln
245 250 255

Asn Leu Leu Ser Cys Asp Thr His His Gln Lys Gly Cys Arg Gly Gly
260 265 270

Arg Leu Asp Gly Ala Trp Trp Phe Leu Arg Arg Arg Gly Val Val Ser
275 280 285

Asp Asn Cys Tyr Pro Phe Ser Gly Arg Glu Gln Asn Asp Glu Ala Ser
290 295 300

Pro Thr Pro Arg Cys Met Met His Ser Arg Ala Met Gly Arg Gly Lys
305 310 315 320

Arg Gln Ala Thr Ser Arg Cys Pro Asn Ser Gln Val Asp Ser Asn Asp
325 330 335

Ile Tyr Gln Val Thr Pro Val Tyr Arg Leu Ala Ser Asp Glu Lys Glu
340 345 350

Ile Met Lys Glu Leu Met Glu Asn Gly Pro Val Gln Ala Leu Met Glu
355 360 365

Val His Glu Asp Phe Phe Leu Tyr Gln Arg Gly Ile Tyr Ser His Thr
370 375 380

Pro Val Ser Gln Gly Arg Pro Glu Gln Tyr Arg Arg His Gly Thr His
385 390 395 400

Ser Val Lys Ile Thr Gly Trp Gly Glu Glu Thr Leu Pro Asp Gly Arg
405 410 415

Thr Ile Lys Tyr Trp Thr Ala Ala Asn Ser Trp Gly Pro Trp Trp Gly
420 425 430

Glu Arg Gly His Phe Arg Ile Val Arg Gly Ile Asn Glu Cys Asp Ile
435 440 445

Glu Thr Phe Val Leu Gly Val Trp Gly Arg Val Gly Met Glu Asp Met
450 455 460

Gly His His
465

<210> 53
<211> 3628
<212> DNA
<213> Rattus norvegicus

<220>
<221> CDS
<222> (6)..(1085)

<400> 53
tgggg atg cta gtg cct act gcg ctg gct gct cgg ctg ctg agc ctg ttc 50
Met Leu Val Pro Thr Ala Leu Ala Ala Arg Leu Leu Ser Leu Phe
1 5 10 15

cag caa cag ctg ggt tcc ctc tgg agt ggc ctg gcc atg ctg ttc tgc 98
Gln Gln Gln Leu Gly Ser Leu Trp Ser Gly Leu Ala Met Leu Phe Cys
20 25 30

tgg ctg aga ata gca ttg ggg tgg cca gat cct gga aag gga cag cca 146
Trp Leu Arg Ile Ala Leu Gly Trp Pro Asp Pro Gly Lys Gly Gln Pro
35 40 45

cgg gtc cgg ggt gag ccc aag gag act cag gag acc cac gaa gat cca 194
Arg Val Arg Gly Glu Pro Lys Glu Thr Gln Glu Thr His Glu Asp Pro
50 55 60

ggc agt gct cag ccc aca acc ccc gtt agt gtc aac tac cac ttc acg 242
Gly Ser Ala Gln Pro Thr Thr Pro Val Ser Val Asn Tyr His Phe Thr
65 70 75

cgt cag tgc aac tac aaa tgt ggc ttc tgc ttc cac acg gcc aag aca 290
Arg Gln Cys Asn Tyr Lys Cys Gly Phe Cys Phe His Thr Ala Lys Thr
80 85 90 95

tcc ttc gtg ctg ccc ctg gag gag gcc aag cga gga ctg ctt ctg ctc 338
Ser Phe Val Leu Pro Leu Glu Glu Ala Lys Arg Gly Leu Leu Leu Leu
100 105 110

aaa cag gct ggt atg gag aag atc aac ttt tca gga gga gaa ccc ttc 386
Lys Gln Ala Gly Met Glu Lys Ile Asn Phe Ser Gly Gly Glu Pro Phe
115 120 125

cta cag gac agg ggt gaa tac ttg ggc aag ctc gtg aga ttc tgc aag 434
Leu Gln Asp Arg Gly Glu Tyr Leu Gly Lys Leu Val Arg Phe Cys Lys
130 135 140

gag gag cta gcc ctg ccc tct gtg agc ata gtg agc aat ggc agc ctt 482

Glu Glu Leu Ala Leu Pro Ser Val Ser Ile Val Ser Asn Gly Ser Leu
145 150 155

atc cga gag aga tgg ttc aag gac tat gga gac tat ttg gac att ctt 530
Ile Arg Glu Arg Trp Phe Lys Asp Tyr Gly Asp Tyr Leu Asp Ile Leu
160 165 170 175

gct atc tcc tgt gac agc ttt gat gag cag gtt aat gtt ctc att ggt 578
Ala Ile Ser Cys Asp Ser Phe Asp Glu Gln Val Asn Val Leu Ile Gly
180 185 190

cgt ggc caa ggg aaa aag aac cat gtg gaa aac ctt caa aag ctg cgg 626
Arg Gly Gln Gly Lys Lys Asn His Val Glu Asn Leu Gln Lys Leu Arg
195 200 205

aag tgg tgc agg gat tac aag gtg gct ttc aag atc aat tcc gtc att 674
Lys Trp Cys Arg Asp Tyr Lys Val Ala Phe Lys Ile Asn Ser Val Ile
210 215 220

aat cgc ttc aac gtg gac gaa gat atg aat gag cac atc aag gcg ctg 722
Asn Arg Phe Asn Val Asp Glu Asp Met Asn Glu His Ile Lys Ala Leu
225 230 235

agc cct gtc cgc tgg aag gtt ttc caa tgc ctc ctg att gag ggt gag 770
Ser Pro Val Arg Trp Lys Val Phe Gln Cys Leu Leu Ile Glu Gly Glu
240 245 250 255

aac tca gga gaa gat gcc ctg agg gaa gca gaa aga ttt ctt ata agc 818
Asn Ser Gly Glu Asp Ala Leu Arg Glu Ala Glu Arg Phe Leu Ile Ser
260 265 270

aat gaa gaa ttt gaa gca ttc tta cag cgt cac aaa gat gtg tcc tgc 866
Asn Glu Glu Phe Glu Ala Phe Leu Gln Arg His Lys Asp Val Ser Cys
275 280 285

ttg gtg cct gaa tct aac cag aag atg aaa gac tcc tac ctt atc ctg 914
Leu Val Pro Glu Ser Asn Gln Lys Met Lys Asp Ser Tyr Leu Ile Leu
290 295 300

gat gaa tat atg cgc ttt ttg aac tgt acc ggt ggc cgg aag gac cct 962
Asp Glu Tyr Met Arg Phe Leu Asn Cys Thr Gly Gly Arg Lys Asp Pro
305 310 315

tcc agg tcc atc ctg gat gtt ggc gtg gag gaa gcg atc aaa ttc agt 1010
Ser Arg Ser Ile Leu Asp Val Gly Val Glu Glu Ala Ile Lys Phe Ser
320 325 330 335

ggg ttt gat gag aag atg ttt ctg aag cgc ggg ggg aag tat gta tgg 1058
Gly Phe Asp Glu Lys Met Phe Leu Lys Arg Gly Gly Lys Tyr Val Trp
340 345 350

agt aag gct gac ctg aag cta gac tgg tgaggtgaga ttggacggag 1105
Ser Lys Ala Asp Leu Lys Leu Asp Trp
355 360

actctaacca gctacgagga gtccacgagc agccgctcgc caccacgcac atctggctgt 1165

ctacggactg ttgctactga gaatgcactc tatgttcttt ctttcagttt gactgaacta 1225

agaaaggagt aaaaccattc tgtgaaccct tatacagccg ataagggtcc tccacagggg 1285

aaggagttca gttcacagag gaacaaggtc cagcgatctc aaagaactca cattttattt 1345

gtattcgaat ttttgtatgg atcgttactg tgaatcccat gtttcctttt ccctttctgt 1405

tccccccatga gaaactgggt tattttctgc taactatgcc agttttgtca acaggaagga 1465

tcacaaaaca caccctgcga ttactgctga ctgttctaat gcttttctgc tttggaatct 1525

cggacttttt gctcagaaat atagaatcaa gaagtgtgtt atagagccgt acgtgagaac 1585

cttactcttt tgcaaggagc atatggaagg aggtacttct gaggacggtg tgttcatggc 1645

ctcagcctct gctgctgctg ctgcttcaag gatggctgtt ctatatcgta ccaattcctt 1705

caaggttctg gacaaagcca aggtacactt tacctttcat ggcatggccg tgaagatggc 1765

actgacagcc gcaaccaccc cagcactgag gctgcctgag tctgtcaggc taaaaggttg 1825
tcccactttt ctgaaaagct acaagggctc ttctgcacat ccagcagaat catgacagga 1885
tgggcatgga ggactcttca ggagtatgaa atgtggctgc tgctccttac catatttctc 1945
atgaaactct gaaataagac ctagtagtct aggtcaccat gagaaaataa tttcttctct 2005
acatcaaata agcaaaagag agaacatgaa ctagcccaaa tcttcttcct gtggaagcat 2065
tgcatgagtt tattcaacct agacagcctc actgcttgga aaataattcc ctggaaatta 2125
aataaatacc atggaaaaga aattaaactc ctctgcaatc ccactgtggc cactagaggg 2185
caccaggaag tcagacttca ttgtctcaga gacttttctt cagtcttttt ttagaatttg 2245
gtgaatttta attctgttcc ttatctgttt tgtctttgtt ttttgttttg gtgcctatta 2305
ctaaaattaa tactaaacat tttccaagga actggaattt tggccattta caaaaggtct 2365
tcattcttta gtgaggtcac tataattcaa ctaagacata ctctgtctca ttaaaaatag 2425
gtcaggtttt cctgtgtttt ctgtcttgtt tggttgtgtg tgtgtgttta cctatttttg 2485
cttgaatata gatataacta tgtatcccag gctagcctgg aactcatgat acctctgcct 2545
taacctcccc ctcttaagtg cccaggaatt acccaactcc tgtacagaga tttttaagag 2605
aagtttgagt cacattcccc cctgcagatt gtgatttttt tttttaagtt aagtcctgtg 2665
tacatagttc acatgggaaa ccatgacatc ccagaggaca tgggcagaca cccacttggt 2725
cacacgacag agggaagctg catgtttctt tccctaatta aagtagagca tgaagaaagt 2785
atgattggaa agtgagccat catttacatg tacccccaac cccttgtgca tgcactggcg 2845
gtgggagatc tctgtgctta aaatattgaa aagtcccctt tccttaaagg gaaaaattac 2905
gatattttac tatcgtagta caaatcaaac ttggattaga tcatacattg gtttgctttt 2965
tcagtgttaa atktgggtgt tcattttgta aagcatacgc tccctgctgg ggaaacaagg 3025
agtgatctag ttgacatttg ttaagtgtga ccagcagata ccttgtgtcc catgattgat 3085
ttatgcagag cagtaatact gggaaggggg atttacttaa gaacatcgac gggtaggaca 3145
tccaacactg atttcaaagt tttggcttct ttattataat gtgttcggcg gtaaaaaaaa 3205
gtcatgggtt gttttctttg aagctgaaag tctcatggtg ttatttacct aaattatctg 3265
aaaaatttaa aaggggccca ggtttcattc tgcgttggag tttgggggtc taattgttgc 3325
ataaataaac aggcaaccaa tcatcagagg ttgactcctc ccatcaataa ttcagctcgt 3385
gttttaccct tttgatggac tgatgttgtt tcataagcta ttagtagtcc aagaacaatc 3445
atgcaggcct catgtgaatg tgttcagatg aggtttgatg ttcgggatac atgtgcatgt 3505
atctccctgt agaatgacac tgtattttga ggcattgctc tagtgtatgg aaacgtgatc 3565
tgtgtattaa aatgttttat gcatgacctc aaaaaaaaaa aaaaaaaaaa aaaaaaaaaa 3625
aga 3628

<210> 54
<211> 360
<212> PRT
<213> Rattus norvegicus

<400> 54

Met Leu Val Pro Thr Ala Leu Ala Ala Arg Leu Leu Ser Leu Phe Gln
1 5 10 15

Gln Gln Leu Gly Ser Leu Trp Ser Gly Leu Ala Met Leu Phe Cys Trp
20 25 30

Leu Arg Ile Ala Leu Gly Trp Pro Asp Pro Gly Lys Gly Gln Pro Arg
35 40 45

Val Arg Gly Glu Pro Lys Glu Thr Gln Glu Thr His Glu Asp Pro Gly
50 55 60

Ser Ala Gln Pro Thr Thr Pro Val Ser Val Asn Tyr His Phe Thr Arg
65 70 75 80

Gln Cys Asn Tyr Lys Cys Gly Phe Cys Phe His Thr Ala Lys Thr Ser
85 90 95

Phe Val Leu Pro Leu Glu Glu Ala Lys Arg Gly Leu Leu Leu Leu Lys
100 105 110

Gln Ala Gly Met Glu Lys Ile Asn Phe Ser Gly Gly Glu Pro Phe Leu
115 120 125

Gln Asp Arg Gly Glu Tyr Leu Gly Lys Leu Val Arg Phe Cys Lys Glu
130 135 140

Glu Leu Ala Leu Pro Ser Val Ser Ile Val Ser Asn Gly Ser Leu Ile
145 150 155 160

Arg Glu Arg Trp Phe Lys Asp Tyr Gly Asp Tyr Leu Asp Ile Leu Ala
165 170 175

Ile Ser Cys Asp Ser Phe Asp Glu Gln Val Asn Val Leu Ile Gly Arg
180 185 190

Gly Gln Gly Lys Lys Asn His Val Glu Asn Leu Gln Lys Leu Arg Lys
195 200 205

Trp Cys Arg Asp Tyr Lys Val Ala Phe Lys Ile Asn Ser Val Ile Asn
210 215 220

Arg Phe Asn Val Asp Glu Asp Met Asn Glu His Ile Lys Ala Leu Ser
225 230 235 240

Pro Val Arg Trp Lys Val Phe Gln Cys Leu Leu Ile Glu Gly Glu Asn
245 250 255

Ser Gly Glu Asp Ala Leu Arg Glu Ala Glu Arg Phe Leu Ile Ser Asn
260 265 270

Glu Glu Phe Glu Ala Phe Leu Gln Arg His Lys Asp Val Ser Cys Leu
275 280 285

Val Pro Glu Ser Asn Gln Lys Met Lys Asp Ser Tyr Leu Ile Leu Asp
290 295 300

Glu Tyr Met Arg Phe Leu Asn Cys Thr Gly Gly Arg Lys Asp Pro Ser
305 310 315 320

```
Arg Ser Ile Leu Asp Val Gly Val Glu Glu Ala Ile Lys Phe Ser Gly
            325                 330                 335

Phe Asp Glu Lys Met Phe Leu Lys Arg Gly Gly Lys Tyr Val Trp Ser
        340                 345                 350

Lys Ala Asp Leu Lys Leu Asp Trp
    355                 360

<210>  55
<211>  1146
<212>  DNA
<213>  Rattus norvegicus

<220>
<221>  CDS
<222>  (456)..(1001)

<400>  55
ggcgggcggc ggccggggcg gcccaggggc tgccgcgggg actcggggcg ccgccgagcg      60

gccaggcgct ggggcgggcc gagcgcaccg cggggcccgg acacggcggg ttcgaggccg     120

ggcccccgcg cggggagctg cggggtggag actttgccgc gcggcgcgga gcaggcggga     180

gccggcgggg gcctcgggga gccccagggc ggccgggccc ggatcccggg ggagcctcgc     240

gagtccgggc cggaacctcg gtgtgcgcat cccctccggg gcccgggctg caggagcaca     300

gagcaaccgg gcctcgcgga gctcagagcg cgcatgaccc ggggcgcacc gtgggacccg     360

ggcgcggggc tggcggagag ctgccccgcg gggctagcag ccgcggagtg gcctacaggg     420

gccctccccc ggaggggccg ggtcggggcg gggag atg aac ggc ttc agc acg        473
                                       Met Asn Gly Phe Ser Thr
                                       1               5

gag gag gac agc cgc gaa ggg ccc cct gcc gcc ccc gcc gcc gcc ccg       521
Glu Glu Asp Ser Arg Glu Gly Pro Pro Ala Ala Pro Ala Ala Ala Pro
        10                  15                  20

ggc tac ggc cag agc tgc tgc ctc atc gcg gac ggc gag cgc tgc gtc       569
Gly Tyr Gly Gln Ser Cys Cys Leu Ile Ala Asp Gly Glu Arg Cys Val
    25                  30                  35

cgg ccc gcg ggc aac gcc tcc ttc agc aag agg gtg cag aaa agc atc       617
Arg Pro Ala Gly Asn Ala Ser Phe Ser Lys Arg Val Gln Lys Ser Ile
40                  45                  50

tcg cag aag aaa ctc aag ctg gac ata gac aag agc gta agg cac ctg       665
Ser Gln Lys Lys Leu Lys Leu Asp Ile Asp Lys Ser Val Arg His Leu
55                  60                  65                  70

tac atc tgc gac ttc cac aaa aat ttc atc cag agc gtc cga aat aaa       713
Tyr Ile Cys Asp Phe His Lys Asn Phe Ile Gln Ser Val Arg Asn Lys
                75                  80                  85

agg aag agg aag aca agt gat gat ggc gga gat tcc cct gag cat gat       761
Arg Lys Arg Lys Thr Ser Asp Asp Gly Gly Asp Ser Pro Glu His Asp
            90                  95                  100

gcc gac att cct gag gtt gat ctg ttc cag cta cag gtg aac act ctc       809
Ala Asp Ile Pro Glu Val Asp Leu Phe Gln Leu Gln Val Asn Thr Leu
        105                 110                 115

cga cgc tac aaa cgg cac tac aag ctg cag acc aga ccc ggc ttc aac       857
Arg Arg Tyr Lys Arg His Tyr Lys Leu Gln Thr Arg Pro Gly Phe Asn
    120                 125                 130

aag gcc cag cta gca gag act gtg agc cga cac ttc agg aac ata cca       905
Lys Ala Gln Leu Ala Glu Thr Val Ser Arg His Phe Arg Asn Ile Pro
```

135 140 145 150

gtg aat gag aaa gag acg ctt gcc tat ttc atc tac atg gtg aag agt 953
Val Asn Glu Lys Glu Thr Leu Ala Tyr Phe Ile Tyr Met Val Lys Ser
155 160 165

aac agg agc aga ctg gac cag aag cca gag ggc agc aag cag ctt gag 1001
Asn Arg Ser Arg Leu Asp Gln Lys Pro Glu Gly Ser Lys Gln Leu Glu
170 175 180

tgaggtgcag ccgcccagtg aggtggaagc cctgatgaga ggacagccat gcactgcttg 1061

atgacaggtg gcccctgcaa cgtctcagcc aataaggact gttcgtatct actgtaaata 1121

aagtttgaat gatggacact gtaaa 1146

<210> 56
<211> 182
<212> PRT
<213> Rattus norvegicus

<400> 56

Met Asn Gly Phe Ser Thr Glu Glu Asp Ser Arg Glu Gly Pro Pro Ala
1 5 10 15

Ala Pro Ala Ala Ala Pro Gly Tyr Gly Gln Ser Cys Cys Leu Ile Ala
20 25 30

Asp Gly Glu Arg Cys Val Arg Pro Ala Gly Asn Ala Ser Phe Ser Lys
35 40 45

Arg Val Gln Lys Ser Ile Ser Gln Lys Lys Leu Lys Leu Asp Ile Asp
50 55 60

Lys Ser Val Arg His Leu Tyr Ile Cys Asp Phe His Lys Asn Phe Ile
65 70 75 80

Gln Ser Val Arg Asn Lys Arg Lys Arg Lys Thr Ser Asp Asp Gly Gly
85 90 95

Asp Ser Pro Glu His Asp Ala Asp Ile Pro Glu Val Asp Leu Phe Gln
100 105 110

Leu Gln Val Asn Thr Leu Arg Arg Tyr Lys Arg His Tyr Lys Leu Gln
115 120 125

Thr Arg Pro Gly Phe Asn Lys Ala Gln Leu Ala Glu Thr Val Ser Arg
130 135 140

His Phe Arg Asn Ile Pro Val Asn Glu Lys Glu Thr Leu Ala Tyr Phe
145 150 155 160

Ile Tyr Met Val Lys Ser Asn Arg Ser Arg Leu Asp Gln Lys Pro Glu
165 170 175

Gly Ser Lys Gln Leu Glu
180

<210> 57
<211> 854
<212> DNA
<213> Rattus norvegicus

<220>
<221> CDS
<222> (402)..(740)

<400> 57
ctggtttgaa cctgaacagg aggagcctgt gggaaaaggg gcggaacttt atgggggacc 60

gatatctttg caaattagct ggaggtgtgg ggttggggtg tcgaagaggc gatcaacttg 120

gaaagtgggc caatccgtga cgcaaatctt tcagtcccac cctcaacccc gcctcccact 180

gctccgctcc tccgctccta attggttcta gtttctccgc ccgccagact tcttctccat 240

tcagcagaag ttttacaaag gcgtctgtaa gggaactagg agctaaaatg aaaatagctc 300

tgattgggca cctttggacc ttatttgcat gggcaattgg gattggttgg cttcttgggt 360

ggccagggcg gtgttggttc cgcgcgtgac cacccacagc t atg gcc ggg gta ggc 416
Met Ala Gly Val Gly
1 5

gct gct ttc cgc cgc cta ggt gcc ttg tcc gga gct ggg gcc ttg agt 464
Ala Ala Phe Arg Arg Leu Gly Ala Leu Ser Gly Ala Gly Ala Leu Ser
10 15 20

ttg gcc acc tac ggg gca cac ggc gca cag ttt ccg gat gcc tac ggg 512
Leu Ala Thr Tyr Gly Ala His Gly Ala Gln Phe Pro Asp Ala Tyr Gly
25 30 35

aag gag ctc ttt gac aag gcc aac aaa cac cac ttt ttg cac agc ctg 560
Lys Glu Leu Phe Asp Lys Ala Asn Lys His His Phe Leu His Ser Leu
40 45 50

gcc ctg tta ggg gta ccc tat tgc aga aaa cct gtc tgg gca ggg ttg 608
Ala Leu Leu Gly Val Pro Tyr Cys Arg Lys Pro Val Trp Ala Gly Leu
55 60 65

ctg cta gct tct gga act acc tta ttc tgc acc agc ttt tac tac cag 656
Leu Leu Ala Ser Gly Thr Thr Leu Phe Cys Thr Ser Phe Tyr Tyr Gln
70 75 80 85

gct ctg agt gga gac act agc atc cag act ctg ggg cct gtt gga ggg 704
Ala Leu Ser Gly Asp Thr Ser Ile Gln Thr Leu Gly Pro Val Gly Gly
90 95 100

agc ctg ctg atc ttg ggc tgg ctt gcc ttg gct ttt taaggtctct 750
Ser Leu Leu Ile Leu Gly Trp Leu Ala Leu Ala Phe
105 110

tgtttaagtg cccagtcctg gcttttctgg gtaattgggt gcagagggaa aggggaatgt 810

tgaagcagaa aagaaattaa aagaaaaagg cttacataaa aaaa 854

<210> 58
<211> 113
<212> PRT
<213> Rattus norvegicus

<400> 58

Met Ala Gly Val Gly Ala Ala Phe Arg Arg Leu Gly Ala Leu Ser Gly
1 5 10 15

Ala Gly Ala Leu Ser Leu Ala Thr Tyr Gly Ala His Gly Ala Gln Phe
20 25 30

Pro Asp Ala Tyr Gly Lys Glu Leu Phe Asp Lys Ala Asn Lys His His
35 40 45

Phe Leu His Ser Leu Ala Leu Leu Gly Val Pro Tyr Cys Arg Lys Pro

50 55 60

Val Trp Ala Gly Leu Leu Leu Ala Ser Gly Thr Thr Leu Phe Cys Thr
65 70 75 80

Ser Phe Tyr Tyr Gln Ala Leu Ser Gly Asp Thr Ser Ile Gln Thr Leu
85 90 95

Gly Pro Val Gly Gly Ser Leu Leu Ile Leu Gly Trp Leu Ala Leu Ala
100 105 110

Phe

<210> 59
<211> 338
<212> DNA
<213> Rattus norvegicus

<400> 59
tttttttttt tttttataat aatgctttta attactgatt tcaataacaa gatccttact 60

gtttagtggg ttttgactga gacgtcgttt gctggatttc tgttatgatt accatctctt 120

tctccaacat gatggttcat agttaacttt tttttttttt tttttggctg tttaagtcag 180

tgtcttgatt cccccacact ctgttaaaag aaatcttatg tcatccaaga gcatgaaaca 240

ggtaattaaa ctaagttgta cctctttgga ataaaagatg tactggttta aaatgggttg 300

ctgggtgtct atgtgtgggt gtgtgtattt ccaatgca 338

<210> 60
<211> 675
<212> DNA
<213> Rattus norvegicus

<400> 60
tttttttttt tttttttaa aatctgtgta ctagctttat taagccacac aaaataaata 60

aaaggaacgt tcaatttaat acatttttat tgagctccca cagggtgcat gaattaatga 120

tttataattt acacaggtag aaaagtgata tgttagcccc ttctcttttg aaatgcaaac 180

tgcatcttct ctttatagtt cgagtgaatt attcaatgtg agcaaggctc agtctgatgt 240

tgccataggt aaaagggaca gtacaaacat tttcccttct ccaaaaaagg gctttgtacc 300

agagtaaacc agcctgttca tctaacaaca gctggtcaaa aaggctagtg aatctggaag 360

aaaagatgct ctggaagcac tgtcctgaga aggattcatt ccccagagca cagtaaaatg 420

tagaagaaaa ttaaccaggt caaaagctca ctgagatcca gaaagtacca gtcacagttt 480

agaaggtggg aaagaagaga gaaggaaagg aagggaaagg agagccaaca ctggctgagc 540

tgggcataac tgacgttggt cgtgcgggtg ttctgatctg ggcagagagg gtcaattcgt 600

gccccatgaa cagagtggtt taactgatca ttctccagtg caccaactgt attttcaagt 660

ataatttgtc atttg 675

<210> 61
<211> 548
<212> DNA
<213> Rattus norvegicus

<400> 61
tttttttttt tttttttgc ctgcttttgt ttttactgga ggctcaggtg gcacatgaca 60

gttcataaag tggcttcaga ggtgattggg ggaggtcgaa gggaaacaaa aaataaatgg 120

gtggggaaga aaaacaatca ggaggaggta tgagctggct ggttccttct cagcctgact 180

taggggaggg aggtgatgcc tctgaacaat aaggatggat cccttccttc aacccatgct 240

aggggagagg ggaaaaaaag agaaaaaagc caaaggctgt tgctttggcc ctcctgagtc 300

tcaagggaaa agctgatgtt ttgatgtcat gaattatggg aaaggggga gggggatgct 360

gggtaaagtc agttggaaac tggcatatac cagatggtag ctctgggtgt cctctgagtt 420

ggaatcactc cagtatggtg gtggggggt ccccactgtt gataggtgct gaggtctccg 480

cagctatagg ctgccggggg ccatggggct gctgccgacg cctctggaag tatggacggt 540

ttcgtgga 548

<210> 62
<211> 472
<212> DNA
<213> Rattus norvegicus

<400> 62
aaaaaaaaat aataaatcca gtttatccat acggcttcag aggtcacagt aaaatcagtg 60

tagactgagt gaagggaaga cctgcgtgtg tgcacctgtg tcatgtcatt ctgggaagag 120

cagtccctcc acgggggtgg gggtggggcg gggacgagtg gcatgtggtg tgtggggggg 180

tataggagac tggtacacga ggacaaaggg ctaaactgat ttcacgccgt ttcccgttcc 240

ctcggtccag aaagatcgtg tgaatttctt acaaatgagt ttgaagtcaa aaatcaaaac 300

aaaacgcaaa caaacggatc aaccatccca gccacggtaa gcccttattc gtgtctgcac 360

ccgtgtgctc tttggggctc cttatccgct cccttctctc gccgcctttg tcctgattcc 420

cacagagatc catagtcgtt agaggcagga ctgaagactg taggtcatgc gt 472

<210> 63
<211> 686
<212> DNA
<213> Rattus norvegicus

<400> 63
gactaaattc acttttattt ttttttaaat acataggaaa caggactcta tcaaaagaga 60

acaaagaaga aacgtcagcc aagtgttgcc aaaagagagc atgcacaagt ctgcttcagg 120

tctgtctcca agcaccggct gtacaggacc tcagcacaca agtggagctg ctggaagagg 180

tgagccttac tcaccaaaca gaagaaaaca agaatgctag gaaacatccc ccagaggtgc 240

agtttccata ggcatcgtca gtatttacgt ctgaaggaat tccaatgcgg gctcgatggg 300

aacaaaatgt atcttaaaca gacattggag tataaaattg ctgagcccta tcagtcgttc 360

agtttctcaa aaggatttgc tatcaaaaag attagattta gtcacggaca ccgagtgcct 420

tgatcagccg ttccctggtg taatcccagg ggcagctctg actgtgcttc ttggttttaa 480

aaaggaacag aagaaattga agttaaagcc acaagtgata gaggagagag gaaatgtttg 540

gaccgtgtag tcgtcttgat tgtaaacagc agacattgtg ctttgtcgtc ttatgtaaaa 600

caaggaccca agcatggaag ggtaatggat ccagtgacag gaaaccagac acaagggtgc 660

tcaatgattg gcagcagcat ctgcat 686

<210> 64
<211> 1402

<212> DNA
<213> Rattus norvegicus

<220>
<221> CDS
<222> (62)..(1099)

<400> 64
```
cgagcaaagc ttctcttcag ttccctggaa gacaaggcaa tacaaagata ctttattaaa      60

a atg gca atg gta tca gaa ttc ctc aag cag gcc tgc tat att gaa aag     109
  Met Ala Met Val Ser Glu Phe Leu Lys Gln Ala Cys Tyr Ile Glu Lys
  1               5                   10                  15

caa gag cag gaa tat gtt caa gct gta aaa tcc tac aaa ggt ggt cct       157
Gln Glu Gln Glu Tyr Val Gln Ala Val Lys Ser Tyr Lys Gly Gly Pro
            20                  25                  30

gga tca gca gtg agc ccc tac cct tcc ttc aat ccg tcc tcg gat gtt       205
Gly Ser Ala Val Ser Pro Tyr Pro Ser Phe Asn Pro Ser Ser Asp Val
        35                  40                  45

gct gcc ttg cac aaa gct atc atg gtt aaa ggt gtg gat gag gca acc       253
Ala Ala Leu His Lys Ala Ile Met Val Lys Gly Val Asp Glu Ala Thr
    50                  55                  60

atc att gac atc ctt acc aag aga acc aat gct cag cgc cag cag atc       301
Ile Ile Asp Ile Leu Thr Lys Arg Thr Asn Ala Gln Arg Gln Gln Ile
65                  70                  75                  80

aag gca gca tac tta cag gag act ggg aag ccc ctg gat gaa acc ttg       349
Lys Ala Ala Tyr Leu Gln Glu Thr Gly Lys Pro Leu Asp Glu Thr Leu
                85                  90                  95

aaa aaa gcc ctt acg ggc cac ctg gag gag gtt gtt ttg gct atg ctc       397
Lys Lys Ala Leu Thr Gly His Leu Glu Glu Val Val Leu Ala Met Leu
            100                 105                 110

aag acc cca gct cag ttt gat gca gat gaa ctc cgt gct gcc atg aag       445
Lys Thr Pro Ala Gln Phe Asp Ala Asp Glu Leu Arg Ala Ala Met Lys
        115                 120                 125

gga ctt gga aca gat gaa gac act ctc att gag att ttg aca aca aga       493
Gly Leu Gly Thr Asp Glu Asp Thr Leu Ile Glu Ile Leu Thr Thr Arg
    130                 135                 140

tct aac cag caa atc aga gag att act aga gtc tac aga gaa gag ctg       541
Ser Asn Gln Gln Ile Arg Glu Ile Thr Arg Val Tyr Arg Glu Glu Leu
145                 150                 155                 160

aaa aga gat ctg gcc aaa gac atc act tcg gac aca tct gga gac ttt       589
Lys Arg Asp Leu Ala Lys Asp Ile Thr Ser Asp Thr Ser Gly Asp Phe
                165                 170                 175

cgt aat gcc ttg ctt gct ctc gcc aag ggt gat cgc tgt gag gat atg       637
Arg Asn Ala Leu Leu Ala Leu Ala Lys Gly Asp Arg Cys Glu Asp Met
            180                 185                 190

agt gtg aat caa gat ttg gct gat aca gat gcc agg gct ttg tat gaa       685
Ser Val Asn Gln Asp Leu Ala Asp Thr Asp Ala Arg Ala Leu Tyr Glu
        195                 200                 205

gct gga gaa agg aga aag ggg aca gac gtg aat gtg ttc aat aca att       733
Ala Gly Glu Arg Arg Lys Gly Thr Asp Val Asn Val Phe Asn Thr Ile
    210                 215                 220

ttg acc aca aga agc tat cct cat ctt cgg aaa gtg ttt cag aat tat       781
Leu Thr Thr Arg Ser Tyr Pro His Leu Arg Lys Val Phe Gln Asn Tyr
225                 230                 235                 240

aga aag tac agt caa cat gac atg aac aaa gcc ctg gat ctg gaa ctg       829
Arg Lys Tyr Ser Gln His Asp Met Asn Lys Ala Leu Asp Leu Glu Leu
                245                 250                 255

aag ggt gac att gag aag tgc ctc aca acc att gtg aag tgt gcc acc       877
```

Lys Gly Asp Ile Glu Lys Cys Leu Thr Thr Ile Val Lys Cys Ala Thr
260 265 270

agc act cca gct ttc ttt gct gag aaa ctg tat gaa gcc atg aag ggt 925
Ser Thr Pro Ala Phe Phe Ala Glu Lys Leu Tyr Glu Ala Met Lys Gly
275 280 285

gct gga act cgc cat aag aca ttg atc agg att atg gtc tcc cgt tcg 973
Ala Gly Thr Arg His Lys Thr Leu Ile Arg Ile Met Val Ser Arg Ser
290 295 300

gaa att gac atg aat gaa atc aaa gta ttt tac cag aag aag tac gga 1021
Glu Ile Asp Met Asn Glu Ile Lys Val Phe Tyr Gln Lys Lys Tyr Gly
305 310 315 320

atc cct ctc tgc caa gcc atc ctg gat gaa acc aaa gga gac tat gaa 1069
Ile Pro Leu Cys Gln Ala Ile Leu Asp Glu Thr Lys Gly Asp Tyr Glu
325 330 335

aaa atc ctg gtg gct ctg tgt gga gga aac taaacatccc aactgctctg 1119
Lys Ile Leu Val Ala Leu Cys Gly Gly Asn
340 345

taagattccg aggagaacat ctcttagccg ttgttttctt cctattgcaa ggcttaagta 1179

ggaaagttgc tttgtcagta agtctaatta ccttctttga ataatgtagc ctataaatat 1239

gttttagatc attcatctgt acaatagaga aatacttgtt ttgttaatta tgtttatccc 1299

aaattataaa tccctgtaag caagtcactt tggtaccatt cctgagaaag aagtttacat 1359

agaataaaat aaaacaattt tataagacaa aaaaaaaaa aaa 1402

<210> 65
<211> 346
<212> PRT
<213> Rattus norvegicus

<400> 65

Met Ala Met Val Ser Glu Phe Leu Lys Gln Ala Cys Tyr Ile Glu Lys
1 5 10 15

Gln Glu Gln Glu Tyr Val Gln Ala Val Lys Ser Tyr Lys Gly Gly Pro
20 25 30

Gly Ser Ala Val Ser Pro Tyr Pro Ser Phe Asn Pro Ser Ser Asp Val
35 40 45

Ala Ala Leu His Lys Ala Ile Met Val Lys Gly Val Asp Glu Ala Thr
50 55 60

Ile Ile Asp Ile Leu Thr Lys Arg Thr Asn Ala Gln Arg Gln Gln Ile
65 70 75 80

Lys Ala Ala Tyr Leu Gln Glu Thr Gly Lys Pro Leu Asp Glu Thr Leu
85 90 95

Lys Lys Ala Leu Thr Gly His Leu Glu Glu Val Val Leu Ala Met Leu
100 105 110

Lys Thr Pro Ala Gln Phe Asp Ala Asp Glu Leu Arg Ala Ala Met Lys
115 120 125

Gly Leu Gly Thr Asp Glu Asp Thr Leu Ile Glu Ile Leu Thr Thr Arg
130 135 140

Ser Asn Gln Gln Ile Arg Glu Ile Thr Arg Val Tyr Arg Glu Glu Leu
145                 150                 155                 160

Lys Arg Asp Leu Ala Lys Asp Ile Thr Ser Asp Thr Ser Gly Asp Phe
                165                 170                 175

Arg Asn Ala Leu Leu Ala Leu Ala Lys Gly Asp Arg Cys Glu Asp Met
            180                 185                 190

Ser Val Asn Gln Asp Leu Ala Asp Thr Asp Ala Arg Ala Leu Tyr Glu
        195                 200                 205

Ala Gly Glu Arg Arg Lys Gly Thr Asp Val Asn Val Phe Asn Thr Ile
    210                 215                 220

Leu Thr Thr Arg Ser Tyr Pro His Leu Arg Lys Val Phe Gln Asn Tyr
225                 230                 235                 240

Arg Lys Tyr Ser Gln His Asp Met Asn Lys Ala Leu Asp Leu Glu Leu
                245                 250                 255

Lys Gly Asp Ile Glu Lys Cys Leu Thr Thr Ile Val Lys Cys Ala Thr
            260                 265                 270

Ser Thr Pro Ala Phe Phe Ala Glu Lys Leu Tyr Glu Ala Met Lys Gly
        275                 280                 285

Ala Gly Thr Arg His Lys Thr Leu Ile Arg Ile Met Val Ser Arg Ser
    290                 295                 300

Glu Ile Asp Met Asn Glu Ile Lys Val Phe Tyr Gln Lys Lys Tyr Gly
305                 310                 315                 320

Ile Pro Leu Cys Gln Ala Ile Leu Asp Glu Thr Lys Gly Asp Tyr Glu
                325                 330                 335

Lys Ile Leu Val Ala Leu Cys Gly Gly Asn
            340                 345

<210> 66
<211> 2954
<212> DNA
<213> Rattus norvegicus

<220>
<221> CDS
<222> (111)..(647)

<400> 66
ccggctccgg agcaccgcga cttcgttcgg tccgactccc cggcggggtg gcggcggccg    60

ggtccccacg gtggcggccg gagcagcggc tgcaggagcc cggctctagg atg aag       116
                                                       Met Lys
                                                       1

ttc aag ccc aac cag acg cgg aca tac gac cgc gag ggc ttc aag aag      164
Phe Lys Pro Asn Gln Thr Arg Thr Tyr Asp Arg Glu Gly Phe Lys Lys
        5                   10                  15

cgg gcg gcc tgc ctg tgc ttc cgc agc gag cag gag gac gag gtg ctg      212
Arg Ala Ala Cys Leu Cys Phe Arg Ser Glu Gln Glu Asp Glu Val Leu

```
            20                      25                      30

ttg gtg agc agc agt cgg tac cca gac caa tgg atc gtg ccg gga gga       260
Leu Val Ser Ser Ser Arg Tyr Pro Asp Gln Trp Ile Val Pro Gly Gly
35                  40                  45                  50

ggg gtg gag ccg gag gag gag cct ggc ggt gct gct gca agg gaa gtg       308
Gly Val Glu Pro Glu Glu Glu Pro Gly Gly Ala Ala Ala Arg Glu Val
                55                  60                  65

tat gaa gag gct gga gtc aaa gga aaa tta ggc aga ctc ctg gga ata       356
Tyr Glu Glu Ala Gly Val Lys Gly Lys Leu Gly Arg Leu Leu Gly Ile
            70                  75                  80

ttt gag aat caa gac cgg aag cac aga aca tat gtt tat gtt ctg aca       404
Phe Glu Asn Gln Asp Arg Lys His Arg Thr Tyr Val Tyr Val Leu Thr
        85                  90                  95

gtc act gaa ata ttg gaa gac tgg gaa gac tct gtt aac ata ggg agg       452
Val Thr Glu Ile Leu Glu Asp Trp Glu Asp Ser Val Asn Ile Gly Arg
    100                 105                 110

aag aga gag tgg ttc aaa gtg gaa gac gca atc aag gtt ctt cag tgt       500
Lys Arg Glu Trp Phe Lys Val Glu Asp Ala Ile Lys Val Leu Gln Cys
115                 120                 125                 130

cac aag cct gtc cac gca gag tac ctg gaa agg ctg aag ctg ggg tgt       548
His Lys Pro Val His Ala Glu Tyr Leu Glu Arg Leu Lys Leu Gly Cys
                135                 140                 145

tct ccg acc aac ggg aat tcc acg gtc cct tcc ctc cca gat aac aat       596
Ser Pro Thr Asn Gly Asn Ser Thr Val Pro Ser Leu Pro Asp Asn Asn
            150                 155                 160

gcc ttg ttt gtg act gcc gca cca ccc tct ggg gtg cca tcc agt ata       644
Ala Leu Phe Val Thr Ala Ala Pro Pro Ser Gly Val Pro Ser Ser Ile
        165                 170                 175

aga tagagagctg gggcccctcc cccaccgtgt catggcacat gtcagaggga            697
Arg

gagaggcttt tttacttcta acacatctga ctgctgctgg cagactctag atttgccatg     757
caggggtttc aaataatttg catgtatttc agatgctttc aactctttta aaataaataa     817
caaaatagca aaaatacttt aagatgccaa agccatgtgg attttttta aagccttaat      877
tgtaagtcta ccccattact ttggttctca tttttataac ctttcttaaa atttcacttt     937
tgaacactca acattctatg gtttattttg acagatcaac tggttgttgg gtatgttttt     997
gccctaatat ttttttctta cagtatcaaa tagtcaccaa atctttggga attaatgaaa    1057
aatattcttg gtatttaaaa aaatatatat gtgccccaaa acttgtttaa gagagtcgca    1117
ttctctccac ttgtaagctg caggtttgcg ctgatttgta tggacgggcc agttgccttt    1177
gaccaagcac tcaagtttac tctatccgag aatttgtaca cagcaagaat tatgtcagtg    1237
cacaggcact gcctcccctc tgacacggtg attccatatg aatcactcca tttttaggga    1297
aaactgtcag gaacttgcct gtggcaccgc acagctcatt ctgaaactgt tcagagaagt    1357
ctgttcagca cgctctgcgg ctgcttcctc cctgctcctt ccgacacaga ctgtgttagg    1417
ggccacattg ctcccatggc cagtgctctt catattttca gtctagcaac aacactgttg    1477
accagtggat tagactctaa ccttagatac agcagtcact gactggcatc caaggttaga    1537
tcacactaaa caaaatggct gtcctaggcc taagacactg tcttgagtgt cagtaagcag    1597
cttgtcagat gagtgtgagg gaactagaac tatgttacct tgtctttgtg ctctctcaaa    1657
acgggccacc ccatggaaaa gtacctacat ttccttcagg aagtcatgga ggaagtgaag    1717
```

```
ggggaggggg gagaggaaaa gatctttcgc agtacagtta ggacatctgt aacctgttaa   1777
cctttaaatt gtaatgttgc tgctgccaat gtccttacct gtaaggaagg aaaatgagat   1837
ctcgtgccca aatgcagcag aactaaaaac attaggttac aaagcagaaa aacccaacca   1897
gtagtgttca ttctaaatta tgtagcgtaa acaaatgtga tcgtccagac cttttctatt   1957
tttctttgta cagaggtcat gtactctggg tgtgttttaa aagaaacatt ttaaagaaac   2017
ccacagattg acattttcta tcttttcgtt ttctcacgat tctctgcaat ttttccatgt   2077
actaaacaca acagaaactg gactgttttg tatagaatac tgcttccagc taaaaccatc   2137
acaagtgccc gggagtttat gtggacatga tagatactct gggcggtaat aatgcatgaa   2197
cctgttttat aaactcttga actgtgtatt gccatgtaaa atatgtacag tattaatgtc   2257
aaccatctct ttaaaagtta gcctatgaac attgttgtat aatttctttg gtcagaaaca   2317
tttggactaa gtcatgtcac ctgggtcagg attttcttca atgccgtgta gcaaaactgt   2377
ctttagtcta tgcaaatagc gagtcactga gtgtgacaga atgcaacttc actgttaaac   2437
ttcacctgag ggttcctcat tctggaatcc agactgcaag attataaagg aaaagaccta   2497
aggcaattca gttcttttg caaatcaatt gaatccacga gagatgtcta ccagcgagat   2557
gtttaccagc ccagccgcct gcagcctgca gcctgctgtg tgtgcttatt tgtgcgctga   2617
ataaaatggg gcagctaaat tctccagttc catatgcctc cgaagttcaa agaaaagaaa   2677
gcaaagtaac atgttagact tgacttgtgt ggcggggtaa agaaatggca tcttcccact   2737
aagaacgaac catccagttc ttttgtcagt cacactatga aacagggaag gtgaagggaa   2797
gaaatggtta tgtgtgcacg aatcgctttg catggtctca tgagatggct gcattcgaac   2857
tgttttaaga attgtaagga tcttgacttt tttacatttg gaaacatcaa ataaaaacaa   2917
acataatctg tgaaaaaaaa aaaaaaaaaa aaaaaaa                            2954
```

<210> 67
<211> 179
<212> PRT
<213> Rattus norvegicus

<400> 67

```
Met Lys Phe Lys Pro Asn Gln Thr Arg Thr Tyr Asp Arg Glu Gly Phe
1               5                   10                  15

Lys Lys Arg Ala Ala Cys Leu Cys Phe Arg Ser Glu Gln Glu Asp Glu
            20                  25                  30

Val Leu Leu Val Ser Ser Ser Arg Tyr Pro Asp Gln Trp Ile Val Pro
        35                  40                  45

Gly Gly Gly Val Glu Pro Glu Glu Glu Pro Gly Gly Ala Ala Ala Arg
    50                  55                  60

Glu Val Tyr Glu Glu Ala Gly Val Lys Gly Lys Leu Gly Arg Leu Leu
65                  70                  75                  80

Gly Ile Phe Glu Asn Gln Asp Arg Lys His Arg Thr Tyr Val Tyr Val
                85                  90                  95

Leu Thr Val Thr Glu Ile Leu Glu Asp Trp Glu Asp Ser Val Asn Ile
            100                 105                 110
```

Gly Arg Lys Arg Glu Trp Phe Lys Val Glu Asp Ala Ile Lys Val Leu
115 120 125

Gln Cys His Lys Pro Val His Ala Glu Tyr Leu Glu Arg Leu Lys Leu
130 135 140

Gly Cys Ser Pro Thr Asn Gly Asn Ser Thr Val Pro Ser Leu Pro Asp
145 150 155 160

Asn Asn Ala Leu Phe Val Thr Ala Ala Pro Pro Ser Gly Val Pro Ser
165 170 175

Ser Ile Arg

<210> 68
<211> 2050
<212> DNA
<213> Rattus norvegicus

<220>
<221> CDS
<222> (26)..(1945)

<400> 68
gaggggtggc ggacgagctg cagag atg tct ggc cag agc ctg acg gac cga 52
Met Ser Gly Gln Ser Leu Thr Asp Arg
1 5

atc acc gcg gcc cag cac agt gtc act ggc tcc gcg gta tct aag aca 100
Ile Thr Ala Ala Gln His Ser Val Thr Gly Ser Ala Val Ser Lys Thr
10 15 20 25

gta tgc aag gcc acg acc cac gag atc atg ggc ccc aag aaa aag cac 148
Val Cys Lys Ala Thr Thr His Glu Ile Met Gly Pro Lys Lys Lys His
30 35 40

ctg gac tac tta att cag tgt aca aat gag atg aat gtg aat atc cca 196
Leu Asp Tyr Leu Ile Gln Cys Thr Asn Glu Met Asn Val Asn Ile Pro
45 50 55

cag ttg gca gac agt ttg ttt gaa aga act act aat agt agt tgg gtg 244
Gln Leu Ala Asp Ser Leu Phe Glu Arg Thr Thr Asn Ser Ser Trp Val
60 65 70

gtg gtc ttc aaa tca ctc att aca act cat cat ttg atg gtg tat gga 292
Val Val Phe Lys Ser Leu Ile Thr Thr His His Leu Met Val Tyr Gly
75 80 85

aac gag cgt ttc att cag tat ttg gct tca aga aac aca ttg ttt aac 340
Asn Glu Arg Phe Ile Gln Tyr Leu Ala Ser Arg Asn Thr Leu Phe Asn
90 95 100 105

tta agc aac ttt ttg gat aaa agt gga ttg caa gga tat gat atg tct 388
Leu Ser Asn Phe Leu Asp Lys Ser Gly Leu Gln Gly Tyr Asp Met Ser
110 115 120

aca ttt att aga cga tat agt agg tac cta aat gaa aag gca gtt tca 436
Thr Phe Ile Arg Arg Tyr Ser Arg Tyr Leu Asn Glu Lys Ala Val Ser
125 130 135

tac aga caa gtt gca ttc gat ttc aca aaa gtg aag aga gga gct gat 484
Tyr Arg Gln Val Ala Phe Asp Phe Thr Lys Val Lys Arg Gly Ala Asp
140 145 150

gga gtt atg aga aca atg aac aca gaa aaa ctg tta aaa act gta cca 532
Gly Val Met Arg Thr Met Asn Thr Glu Lys Leu Leu Lys Thr Val Pro
155 160 165

```
att atc caa aat caa atg gat gca ctt ctt gat ttt aat gtt aat agt     580
Ile Ile Gln Asn Gln Met Asp Ala Leu Leu Asp Phe Asn Val Asn Ser
170                 175                 180                 185

aat gaa ctt aca aat ggg gta ata aat gct gcc ttc atg ctc ctg ttc     628
Asn Glu Leu Thr Asn Gly Val Ile Asn Ala Ala Phe Met Leu Leu Phe
                190                 195                 200

aaa gat gcc att aga cta ttt gca gca tac aat gaa gga att att aat     676
Lys Asp Ala Ile Arg Leu Phe Ala Ala Tyr Asn Glu Gly Ile Ile Asn
            205                 210                 215

tta ttg gaa aaa tat ttt gat atg aaa aag aac cag tgc aaa gaa ggt     724
Leu Leu Glu Lys Tyr Phe Asp Met Lys Lys Asn Gln Cys Lys Glu Gly
        220                 225                 230

ctt gac atc tat aag aag ttt ttg act agg atg aca aga atc tca gag     772
Leu Asp Ile Tyr Lys Lys Phe Leu Thr Arg Met Thr Arg Ile Ser Glu
    235                 240                 245

ttt ctg aaa gtt gca gag caa gtt gga att gac aga gga gat att cca     820
Phe Leu Lys Val Ala Glu Gln Val Gly Ile Asp Arg Gly Asp Ile Pro
250                 255                 260                 265

gat ctt tca cag gcc ccc agc agt ctt ctt gat gct tta gaa caa cat     868
Asp Leu Ser Gln Ala Pro Ser Ser Leu Leu Asp Ala Leu Glu Gln His
                270                 275                 280

tta gct tcc ttg gaa ggg aag aaa atc aaa gat tcc aca gct gca agc     916
Leu Ala Ser Leu Glu Gly Lys Lys Ile Lys Asp Ser Thr Ala Ala Ser
            285                 290                 295

agg gct aca aca ctt tcc aat gca gtc tct tct ttg gca agc act ggc     964
Arg Ala Thr Thr Leu Ser Asn Ala Val Ser Ser Leu Ala Ser Thr Gly
        300                 305                 310

cta tct ctg acc aaa gtg gat gaa agg gaa aag cag gca gca tta gag     1012
Leu Ser Leu Thr Lys Val Asp Glu Arg Glu Lys Gln Ala Ala Leu Glu
    315                 320                 325

gaa gaa cag gct cga tta aaa gca cta aag gaa cag cgt cta aaa gaa     1060
Glu Glu Gln Ala Arg Leu Lys Ala Leu Lys Glu Gln Arg Leu Lys Glu
330                 335                 340                 345

ctt gca aag aaa ccg cat acc tct tta aca act gca gcc tct cct gtg     1108
Leu Ala Lys Lys Pro His Thr Ser Leu Thr Thr Ala Ala Ser Pro Val
                350                 355                 360

tcc acc tca gca ggg gga ata atg act gca cca gcc atc gac ata ttt     1156
Ser Thr Ser Ala Gly Gly Ile Met Thr Ala Pro Ala Ile Asp Ile Phe
            365                 370                 375

tct acc cct agt tct tct aac agc aca tcc aag ctg cca aat gac ctg     1204
Ser Thr Pro Ser Ser Ser Asn Ser Thr Ser Lys Leu Pro Asn Asp Leu
        380                 385                 390

ctt gat ttg cag cag cca acc ttt cat cca tct gtc cat gct atg tca     1252
Leu Asp Leu Gln Gln Pro Thr Phe His Pro Ser Val His Ala Met Ser
    395                 400                 405

gct gct cct cag gta gca agt aca tgg gga gat gct gtt gat gat gcc     1300
Ala Ala Pro Gln Val Ala Ser Thr Trp Gly Asp Ala Val Asp Asp Ala
410                 415                 420                 425

att cca agc tta aat cct ttc ctc aca aaa agt agt ggt gat gtt cac     1348
Ile Pro Ser Leu Asn Pro Phe Leu Thr Lys Ser Ser Gly Asp Val His
                430                 435                 440

ctt cct att tct tca gat gta tcc act ttt act act agg aca cct act     1396
Leu Pro Ile Ser Ser Asp Val Ser Thr Phe Thr Thr Arg Thr Pro Thr
            445                 450                 455

cat gaa atg ttt gtt gga ttc agt cct tct ccg gtt aca cag cca cat     1444
His Glu Met Phe Val Gly Phe Ser Pro Ser Pro Val Thr Gln Pro His
        460                 465                 470
```

```
cct tca gct ggc ctt aat gtt gac ttt gaa tct gtg ttt gga aat aag      1492
Pro Ser Ala Gly Leu Asn Val Asp Phe Glu Ser Val Phe Gly Asn Lys
    475                 480                 485

tct acg aat gtt gct gta gat tct ggt ggt gga ctt ctc aaa cca aca      1540
Ser Thr Asn Val Ala Val Asp Ser Gly Gly Gly Leu Leu Lys Pro Thr
490                 495                 500                 505

gtg gcc tct cag aac cag agt ctt cct gtt gcc aaa ctt ccg cct aac      1588
Val Ala Ser Gln Asn Gln Ser Leu Pro Val Ala Lys Leu Pro Pro Asn
                510                 515                 520

aaa tta gtg tct gat gac ttg gat tca tct tta gcc aac ctt gtg ggc      1636
Lys Leu Val Ser Asp Asp Leu Asp Ser Ser Leu Ala Asn Leu Val Gly
            525                 530                 535

aat ctt ggc att gga aat gga acc act aag aat gat gta agt tgc agt      1684
Asn Leu Gly Ile Gly Asn Gly Thr Thr Lys Asn Asp Val Ser Cys Ser
        540                 545                 550

caa cca ggt gaa aag aag tta act gga gga tct aac tgg caa cca aag      1732
Gln Pro Gly Glu Lys Lys Leu Thr Gly Gly Ser Asn Trp Gln Pro Lys
    555                 560                 565

gtc gca cca aca act gcc tgg agt gct gca aca atg gca ccc cct gta      1780
Val Ala Pro Thr Thr Ala Trp Ser Ala Ala Thr Met Ala Pro Pro Val
570                 575                 580                 585

atg gcc tat cct gct act aca cca acg ggc atg ata gga tat gga att      1828
Met Ala Tyr Pro Ala Thr Thr Pro Thr Gly Met Ile Gly Tyr Gly Ile
                590                 595                 600

cct cct cag atg gga agt gta cct gta atg aca cag cca acc tta ata      1876
Pro Pro Gln Met Gly Ser Val Pro Val Met Thr Gln Pro Thr Leu Ile
            605                 610                 615

tac agc cag cct gtc atg aga ccg cca aac ccc ttt ggc cct gta cca      1924
Tyr Ser Gln Pro Val Met Arg Pro Pro Asn Pro Phe Gly Pro Val Pro
        620                 625                 630

gga gca cag ata cag ttt atg taactagatg gaagagaatg gaattactcc         1975
Gly Ala Gln Ile Gln Phe Met
    635                 640

aagaatagaa gtgcacaggt ggcgactcct tacttccagc aaaatccaaa ctgctgtctc    2035

taagactctt cctcc                                                     2050
```

<210> 69
<211> 640
<212> PRT
<213> Rattus norvegicus

<400> 69

```
Met Ser Gly Gln Ser Leu Thr Asp Arg Ile Thr Ala Ala Gln His Ser
1               5                   10                  15

Val Thr Gly Ser Ala Val Ser Lys Thr Val Cys Lys Ala Thr Thr His
            20                  25                  30

Glu Ile Met Gly Pro Lys Lys Lys His Leu Asp Tyr Leu Ile Gln Cys
        35                  40                  45

Thr Asn Glu Met Asn Val Asn Ile Pro Gln Leu Ala Asp Ser Leu Phe
    50                  55                  60

Glu Arg Thr Thr Asn Ser Ser Trp Val Val Val Phe Lys Ser Leu Ile
65                  70                  75                  80
```

Thr Thr His His Leu Met Val Tyr Gly Asn Glu Arg Phe Ile Gln Tyr
85 90 95

Leu Ala Ser Arg Asn Thr Leu Phe Asn Leu Ser Asn Phe Leu Asp Lys
100 105 110

Ser Gly Leu Gln Gly Tyr Asp Met Ser Thr Phe Ile Arg Arg Tyr Ser
115 120 125

Arg Tyr Leu Asn Glu Lys Ala Val Ser Tyr Arg Gln Val Ala Phe Asp
130 135 140

Phe Thr Lys Val Lys Arg Gly Ala Asp Gly Val Met Arg Thr Met Asn
145 150 155 160

Thr Glu Lys Leu Leu Lys Thr Val Pro Ile Ile Gln Asn Gln Met Asp
165 170 175

Ala Leu Leu Asp Phe Asn Val Asn Ser Asn Glu Leu Thr Asn Gly Val
180 185 190

Ile Asn Ala Ala Phe Met Leu Leu Phe Lys Asp Ala Ile Arg Leu Phe
195 200 205

Ala Ala Tyr Asn Glu Gly Ile Ile Asn Leu Leu Glu Lys Tyr Phe Asp
210 215 220

Met Lys Lys Asn Gln Cys Lys Glu Gly Leu Asp Ile Tyr Lys Lys Phe
225 230 235 240

Leu Thr Arg Met Thr Arg Ile Ser Glu Phe Leu Lys Val Ala Glu Gln
245 250 255

Val Gly Ile Asp Arg Gly Asp Ile Pro Asp Leu Ser Gln Ala Pro Ser
260 265 270

Ser Leu Leu Asp Ala Leu Glu Gln His Leu Ala Ser Leu Glu Gly Lys
275 280 285

Lys Ile Lys Asp Ser Thr Ala Ala Ser Arg Ala Thr Thr Leu Ser Asn
290 295 300

Ala Val Ser Ser Leu Ala Ser Thr Gly Leu Ser Leu Thr Lys Val Asp
305 310 315 320

Glu Arg Glu Lys Gln Ala Ala Leu Glu Glu Glu Gln Ala Arg Leu Lys
325 330 335

Ala Leu Lys Glu Gln Arg Leu Lys Glu Leu Ala Lys Lys Pro His Thr
340 345 350

Ser Leu Thr Thr Ala Ala Ser Pro Val Ser Thr Ser Ala Gly Gly Ile
355 360 365

Met Thr Ala Pro Ala Ile Asp Ile Phe Ser Thr Pro Ser Ser Ser Asn
370 375 380

```
Ser Thr Ser Lys Leu Pro Asn Asp Leu Leu Asp Leu Gln Gln Pro Thr
385                 390                 395                 400

Phe His Pro Ser Val His Ala Met Ser Ala Ala Pro Gln Val Ala Ser
                405                 410                 415

Thr Trp Gly Asp Ala Val Asp Asp Ala Ile Pro Ser Leu Asn Pro Phe
            420                 425                 430

Leu Thr Lys Ser Ser Gly Asp Val His Leu Pro Ile Ser Ser Asp Val
        435                 440                 445

Ser Thr Phe Thr Thr Arg Thr Pro Thr His Glu Met Phe Val Gly Phe
    450                 455                 460

Ser Pro Ser Pro Val Thr Gln Pro His Pro Ser Ala Gly Leu Asn Val
465                 470                 475                 480

Asp Phe Glu Ser Val Phe Gly Asn Lys Ser Thr Asn Val Ala Val Asp
                485                 490                 495

Ser Gly Gly Gly Leu Leu Lys Pro Thr Val Ala Ser Gln Asn Gln Ser
            500                 505                 510

Leu Pro Val Ala Lys Leu Pro Pro Asn Lys Leu Val Ser Asp Asp Leu
        515                 520                 525

Asp Ser Ser Leu Ala Asn Leu Val Gly Asn Leu Gly Ile Gly Asn Gly
    530                 535                 540

Thr Thr Lys Asn Asp Val Ser Cys Ser Gln Pro Gly Glu Lys Lys Leu
545                 550                 555                 560

Thr Gly Gly Ser Asn Trp Gln Pro Lys Val Ala Pro Thr Thr Ala Trp
                565                 570                 575

Ser Ala Ala Thr Met Ala Pro Pro Val Met Ala Tyr Pro Ala Thr Thr
            580                 585                 590

Pro Thr Gly Met Ile Gly Tyr Gly Ile Pro Pro Gln Met Gly Ser Val
        595                 600                 605

Pro Val Met Thr Gln Pro Thr Leu Ile Tyr Ser Gln Pro Val Met Arg
    610                 615                 620

Pro Pro Asn Pro Phe Gly Pro Val Pro Gly Ala Gln Ile Gln Phe Met
625                 630                 635                 640

<210>  70
<211>  361
<212>  DNA
<213>  Rattus norvegicus


<220>
<221>  CDS
<222>  (30)..(296)

<400>  70
```

```
gcacgaggtg aaaggaaatt tttatcgac atg gca act gaa ctg gag aag gcc      53
                                Met Ala Thr Glu Leu Glu Lys Ala
                                1               5

ttg agc aac gtc att gaa gtc tac cac aat tat tct ggt ata aaa ggg      101
Leu Ser Asn Val Ile Glu Val Tyr His Asn Tyr Ser Gly Ile Lys Gly
    10                  15                  20

aat cac cat gcc ctc tac agg gat gac ttc agg aaa atg gtc act act      149
Asn His His Ala Leu Tyr Arg Asp Asp Phe Arg Lys Met Val Thr Thr
25                  30                  35                  40

gag tgc cct cag ttt gtg cag aat aaa aat acc gaa agc ttg ttc aaa      197
Glu Cys Pro Gln Phe Val Gln Asn Lys Asn Thr Glu Ser Leu Phe Lys
                45                  50                  55

gaa ttg gac gtc aat agt gac aac gca att aac ttc gaa gag ttc ctt      245
Glu Leu Asp Val Asn Ser Asp Asn Ala Ile Asn Phe Glu Glu Phe Leu
            60                  65                  70

gcg ttg gtg ata agg gtg ggc gtg gca gct cat aaa gac agc cac aag      293
Ala Leu Val Ile Arg Val Gly Val Ala Ala His Lys Asp Ser His Lys
        75                  80                  85

gag taacagagct tctggcctgg gctgggtccc ttggatatgt ctacagaata           346
Glu

aagtcgtcat atctt                                                     361
```

<210> 71
<211> 89
<212> PRT
<213> Rattus norvegicus

<400> 71

```
Met Ala Thr Glu Leu Glu Lys Ala Leu Ser Asn Val Ile Glu Val Tyr
1               5                   10                  15

His Asn Tyr Ser Gly Ile Lys Gly Asn His His Ala Leu Tyr Arg Asp
            20                  25                  30

Asp Phe Arg Lys Met Val Thr Thr Glu Cys Pro Gln Phe Val Gln Asn
        35                  40                  45

Lys Asn Thr Glu Ser Leu Phe Lys Glu Leu Asp Val Asn Ser Asp Asn
    50                  55                  60

Ala Ile Asn Phe Glu Glu Phe Leu Ala Leu Val Ile Arg Val Gly Val
65                  70                  75                  80

Ala Ala His Lys Asp Ser His Lys Glu
                85
```

<210> 72
<211> 494
<212> DNA
<213> Rattus norvegicus

<220>
<221> CDS
<222> (32)..(370)

<400> 72

```
cggcacgagc tccttagctt tgagcaagaa g atg gct gcc aaa aca gga tct       52
                                   Met Ala Ala Lys Thr Gly Ser
                                   1               5
```

```
cag ctg gag cgc agc ata agc acc atc atc aat gtt ttc cat cag tac      100
Gln Leu Glu Arg Ser Ile Ser Thr Ile Ile Asn Val Phe His Gln Tyr
        10                  15                  20

tct agg aag tat gga cat cct gac acc ctg aac aag gcg gaa ttc aaa      148
Ser Arg Lys Tyr Gly His Pro Asp Thr Leu Asn Lys Ala Glu Phe Lys
    25                  30                  35

gaa atg gtg aat aag gac ttg cca aat ttt ctg aag agg gag aaa aga      196
Glu Met Val Asn Lys Asp Leu Pro Asn Phe Leu Lys Arg Glu Lys Arg
40                  45                  50                  55

aat gaa aat ctc cta aga gac atc atg gag gac ctg gac aca aac cag      244
Asn Glu Asn Leu Leu Arg Asp Ile Met Glu Asp Leu Asp Thr Asn Gln
                60                  65                  70

gac aat caa ctg tcc ttt gag gag tgt atg atg ctg atg gga aag ttg      292
Asp Asn Gln Leu Ser Phe Glu Glu Cys Met Met Leu Met Gly Lys Leu
            75                  80                  85

atc ttt gcc tgt cat gag aag ctg cat gag aac aac cca cgt ggg cat      340
Ile Phe Ala Cys His Glu Lys Leu His Glu Asn Asn Pro Arg Gly His
        90                  95                  100

gac cac agg cac ggc aaa ggc tgt ggg aag taattaagag gtcgccatgt        390
Asp His Arg His Gly Lys Gly Cys Gly Lys
    105                 110

aacatctgcc caaccaagtc taaagggaat agcttactaa atgaccttgg ttctggggct    450

gggaaataat ttaaaaatga ataaataaag tctttatcca ttcc                     494


<210>  73
<211>  113
<212>  PRT
<213>  Rattus norvegicus

<400>  73

Met Ala Ala Lys Thr Gly Ser Gln Leu Glu Arg Ser Ile Ser Thr Ile
1               5                   10                  15


Ile Asn Val Phe His Gln Tyr Ser Arg Lys Tyr Gly His Pro Asp Thr
            20                  25                  30


Leu Asn Lys Ala Glu Phe Lys Glu Met Val Asn Lys Asp Leu Pro Asn
        35                  40                  45


Phe Leu Lys Arg Glu Lys Arg Asn Glu Asn Leu Leu Arg Asp Ile Met
    50                  55                  60


Glu Asp Leu Asp Thr Asn Gln Asp Asn Gln Leu Ser Phe Glu Glu Cys
65                  70                  75                  80


Met Met Leu Met Gly Lys Leu Ile Phe Ala Cys His Glu Lys Leu His
                85                  90                  95


Glu Asn Asn Pro Arg Gly His Asp His Arg His Gly Lys Gly Cys Gly
            100                 105                 110


Lys


<210>  74
<211>  2320
<212>  DNA
```

<213> Rattus norvegicus

<220>
<221> CDS
<222> (178)..(1341)

<400> 74
```
gcctagggtg gcagcggcta cctacctcgg atctcgacct gctgccacca cggcctgagc      60

gctgtccctc ggctcccgga gctcagcgcg aagccctggc cccggcggct ggggcatgga     120

tcaggggcgc tgcgtgaggc ggcttcctgc acggccgtga cgtgcaccgg cttcagc        177

atg aag acc ctc atc gct gcc tac tcc ggg gtc ctg cgg ggt gag cgt       225
Met Lys Thr Leu Ile Ala Ala Tyr Ser Gly Val Leu Arg Gly Glu Arg
1               5                   10                  15

cgg gcc gaa gct gcc cgc agc gag aac aag aat aaa gga tct gcc ctg       273
Arg Ala Glu Ala Ala Arg Ser Glu Asn Lys Asn Lys Gly Ser Ala Leu
            20                  25                  30

tca cgc gag ggg tct ggg cga tgg ggc act ggc tcc agc atc ctc tcg       321
Ser Arg Glu Gly Ser Gly Arg Trp Gly Thr Gly Ser Ser Ile Leu Ser
        35                  40                  45

gcc ctc caa gac atc ttc tct gtc acc tgg ctc aac aga tcc aag gtg       369
Ala Leu Gln Asp Ile Phe Ser Val Thr Trp Leu Asn Arg Ser Lys Val
    50                  55                  60

gaa aaa cac cta cag gtc atc tca gtc cta cag tgg gtc cta tcc ttc       417
Glu Lys His Leu Gln Val Ile Ser Val Leu Gln Trp Val Leu Ser Phe
65                  70                  75                  80

ctg gtg cta gga gtg gcc tgc agt gtc atc ctc atg tac acc ttc tgc       465
Leu Val Leu Gly Val Ala Cys Ser Val Ile Leu Met Tyr Thr Phe Cys
                85                  90                  95

act gac tgc tgg ctg ata gct gct ctc tac ttc acc tgg ctg gca ttt       513
Thr Asp Cys Trp Leu Ile Ala Ala Leu Tyr Phe Thr Trp Leu Ala Phe
            100                 105                 110

gac tgg aac acg ccc aag aaa ggt ggc agg aga tca cag tgg gtg cga       561
Asp Trp Asn Thr Pro Lys Lys Gly Gly Arg Arg Ser Gln Trp Val Arg
        115                 120                 125

aac tgg gcc gtg tgg cgc tat ttt cga gac tac ttt ccc atc cag ctg       609
Asn Trp Ala Val Trp Arg Tyr Phe Arg Asp Tyr Phe Pro Ile Gln Leu
    130                 135                 140

gtg aag aca cac aac ctg ctg acc acc agg aac tat atc ttt gga tac       657
Val Lys Thr His Asn Leu Leu Thr Thr Arg Asn Tyr Ile Phe Gly Tyr
145                 150                 155                 160

cat ccc cat ggc atc atg ggc ctg ggt gcc ttc tgt aac ttc agc acg       705
His Pro His Gly Ile Met Gly Leu Gly Ala Phe Cys Asn Phe Ser Thr
                165                 170                 175

gag gcc acc gaa gtt agc aag aag ttc cct ggc ata agg cct tat ttg       753
Glu Ala Thr Glu Val Ser Lys Lys Phe Pro Gly Ile Arg Pro Tyr Leu
            180                 185                 190

gcc aca ttg gct ggc aac ttc cgg atg cct gtg ctt cgg gag tac ctg       801
Ala Thr Leu Ala Gly Asn Phe Arg Met Pro Val Leu Arg Glu Tyr Leu
        195                 200                 205

atg tct gga ggc atc tgc cct gtc aac aga gac acc ata gac tac ttg       849
Met Ser Gly Gly Ile Cys Pro Val Asn Arg Asp Thr Ile Asp Tyr Leu
    210                 215                 220

ctt tcc aag aat ggg agt ggt aat gcc att gtc atc gtg gtg gga ggt       897
Leu Ser Lys Asn Gly Ser Gly Asn Ala Ile Val Ile Val Val Gly Gly
225                 230                 235                 240

gca gct gaa tcc ctg agc tcc atg cct ggc aag aac gca gtc acc ctg       945
Ala Ala Glu Ser Leu Ser Ser Met Pro Gly Lys Asn Ala Val Thr Leu
```

```
                    245                 250                 255

cgg aac cgc aaa ggc ttt gta aag ctg gcc ctg cgc cat gga gct gat      993
Arg Asn Arg Lys Gly Phe Val Lys Leu Ala Leu Arg His Gly Ala Asp
            260                 265                 270

ctg gtt ccc acc tat tcc ttt gga gag aat gag gta tac aag cag gtg     1041
Leu Val Pro Thr Tyr Ser Phe Gly Glu Asn Glu Val Tyr Lys Gln Val
        275                 280                 285

atc ttt gag gag ggc tcc tgg ggc cga tgg gtc cag aag aag ttc cag     1089
Ile Phe Glu Glu Gly Ser Trp Gly Arg Trp Val Gln Lys Lys Phe Gln
    290                 295                 300

aag tat att ggt ttc gcc ccc tgc atc ttc cat ggc cga ggt ctc ttc     1137
Lys Tyr Ile Gly Phe Ala Pro Cys Ile Phe His Gly Arg Gly Leu Phe
305                 310                 315                 320

tcc tct gac acc tgg ggg ctg gtg ccc tac tcc aag ccc atc acc acc     1185
Ser Ser Asp Thr Trp Gly Leu Val Pro Tyr Ser Lys Pro Ile Thr Thr
                325                 330                 335

gtt gtg ggg gag ccc atc acc gtc cct aag ctg gag cac ccg acc cag     1233
Val Val Gly Glu Pro Ile Thr Val Pro Lys Leu Glu His Pro Thr Gln
            340                 345                 350

aaa gac atc gac ctg tac cac acc atg tac atg gag gcc ctg gtg aag     1281
Lys Asp Ile Asp Leu Tyr His Thr Met Tyr Met Glu Ala Leu Val Lys
        355                 360                 365

ctc ttt gac aat cac aag acc aaa ttc ggc ctt cca gag act gag gtg     1329
Leu Phe Asp Asn His Lys Thr Lys Phe Gly Leu Pro Glu Thr Glu Val
    370                 375                 380

ctg gag gtg aac tgacccagcc catgggtgcc agctcctgga aggaatgact         1381
Leu Glu Val Asn
385

gcaaatcctt ttctaccaag ttctcaagtg cattttgttc tgtaaatttg gaagcgtcat   1441

gggtgtctgt gggttattta aaagaaatta taatttgtta aaccattgca atgttagatc   1501

tttttaaga agggaagagt cagtatttta agctcacttc tagtgtgtcc tgcctaaggt    1561

ggtggctgac atttataggc cttgatggtt tctatccacc ccttctagtg ttccccaaac   1621

aacagacact tggccctagt taattgggga agggcagccc ttagtgactc agccatttaa   1681

tcctcttcgc tctagggatt ctcaagagac ggaggccacg tttaaaaaca cctccattcc   1741

cacccacaac acgtgactgc tggtcaggtt tttcttactt aggggaggat gagggggcat   1801

agctggttcc gcttggggag agtggtagat aacatctgga atgcccggct cgagtgtcct   1861

ctgctcccca cctactcctc ttctccaatc tgagcctacc ctggcctcct gtacactgtg   1921

ctagggacag ggctgtccca caggtgccat gttgggttat ctcgctgctg ttggctggtt   1981

ttactctgga gattggcatc gtgaacacag ctcagcgtca ttctggagat gttctcccag   2041

ccacctgagc tctcctgagc cacaccccaa gtctggtgtg aggagaggcc tctgttcttc   2101

acagaggtgc ctggcttcct gtgcagcaca ctgggtccag gacaggaggc cacccccaac   2161

caagcctcac ctgtgtgcct ttatgaggcg tcgggagaaa gccaccctcc tgtgtattct   2221

gctttctcca tgagattttg ccatgtcaca cttttgtata ttcctagact aataaatgga   2281

aacaagaaca gccaaaaaaa aaaaaaaaaa aaaaaaaaa                          2320

<210>  75
<211>  388
<212>  PRT
<213>  Rattus norvegicus
```

<400> 75

Met Lys Thr Leu Ile Ala Ala Tyr Ser Gly Val Leu Arg Gly Glu Arg
1               5                   10                  15

Arg Ala Glu Ala Ala Arg Ser Glu Asn Lys Asn Lys Gly Ser Ala Leu
            20                  25                  30

Ser Arg Glu Gly Ser Gly Arg Trp Gly Thr Gly Ser Ser Ile Leu Ser
        35                  40                  45

Ala Leu Gln Asp Ile Phe Ser Val Thr Trp Leu Asn Arg Ser Lys Val
    50                  55                  60

Glu Lys His Leu Gln Val Ile Ser Val Leu Gln Trp Val Leu Ser Phe
65                  70                  75                  80

Leu Val Leu Gly Val Ala Cys Ser Val Ile Leu Met Tyr Thr Phe Cys
                85                  90                  95

Thr Asp Cys Trp Leu Ile Ala Ala Leu Tyr Phe Thr Trp Leu Ala Phe
            100                 105                 110

Asp Trp Asn Thr Pro Lys Lys Gly Gly Arg Arg Ser Gln Trp Val Arg
        115                 120                 125

Asn Trp Ala Val Trp Arg Tyr Phe Arg Asp Tyr Phe Pro Ile Gln Leu
    130                 135                 140

Val Lys Thr His Asn Leu Leu Thr Thr Arg Asn Tyr Ile Phe Gly Tyr
145                 150                 155                 160

His Pro His Gly Ile Met Gly Leu Gly Ala Phe Cys Asn Phe Ser Thr
                165                 170                 175

Glu Ala Thr Glu Val Ser Lys Lys Phe Pro Gly Ile Arg Pro Tyr Leu
            180                 185                 190

Ala Thr Leu Ala Gly Asn Phe Arg Met Pro Val Leu Arg Glu Tyr Leu
        195                 200                 205

Met Ser Gly Gly Ile Cys Pro Val Asn Arg Asp Thr Ile Asp Tyr Leu
    210                 215                 220

Leu Ser Lys Asn Gly Ser Gly Asn Ala Ile Val Ile Val Val Gly Gly
225                 230                 235                 240

Ala Ala Glu Ser Leu Ser Ser Met Pro Gly Lys Asn Ala Val Thr Leu
                245                 250                 255

Arg Asn Arg Lys Gly Phe Val Lys Leu Ala Leu Arg His Gly Ala Asp
            260                 265                 270

Leu Val Pro Thr Tyr Ser Phe Gly Glu Asn Glu Val Tyr Lys Gln Val
        275                 280                 285

Ile Phe Glu Glu Gly Ser Trp Gly Arg Trp Val Gln Lys Lys Phe Gln
    290                 295                 300

```
Lys Tyr Ile Gly Phe Ala Pro Cys Ile Phe His Gly Arg Gly Leu Phe
305                 310                 315                 320

Ser Ser Asp Thr Trp Gly Leu Val Pro Tyr Ser Lys Pro Ile Thr Thr
                325                 330                 335

Val Val Gly Glu Pro Ile Thr Val Pro Lys Leu Glu His Pro Thr Gln
            340                 345                 350

Lys Asp Ile Asp Leu Tyr His Thr Met Tyr Met Glu Ala Leu Val Lys
        355                 360                 365

Leu Phe Asp Asn His Lys Thr Lys Phe Gly Leu Pro Glu Thr Glu Val
    370                 375                 380

Leu Glu Val Asn
385

<210>  76
<211>  1714
<212>  DNA
<213>  Rattus norvegicus

<220>
<221>  CDS
<222>  (56)..(1102)

<400>  76
cctctgaggc gtttggtgct ccggtagcta ccagggctgg agagcgagca ttgcc atg      58
                                                             Met
                                                             1

gaa ata tac act tcg gat aac tac tcc gaa gaa gta ggg tct gga gac      106
Glu Ile Tyr Thr Ser Asp Asn Tyr Ser Glu Glu Val Gly Ser Gly Asp
            5                   10                  15

tat gac tcc aac aag gaa ccc tgc ttc cgg gat gaa aac gaa aac ttc      154
Tyr Asp Ser Asn Lys Glu Pro Cys Phe Arg Asp Glu Asn Glu Asn Phe
        20                  25                  30

aac agg atc ttc ctg ccc acc atc tat ttt atc atc ttc ttg act ggc      202
Asn Arg Ile Phe Leu Pro Thr Ile Tyr Phe Ile Ile Phe Leu Thr Gly
    35                  40                  45

ata gtg ggc aat ggg ttg gta atc ctg gtc atg ggt tac cag aag aag      250
Ile Val Gly Asn Gly Leu Val Ile Leu Val Met Gly Tyr Gln Lys Lys
50                  55                  60                  65

ctg agg agc atg aca gac aag tac cgg ctg cac ctg tcc gtg gct gac      298
Leu Arg Ser Met Thr Asp Lys Tyr Arg Leu His Leu Ser Val Ala Asp
                70                  75                  80

ctc ctc ttt gtc atc aca ctc ccc ttc tgg gca gtg gac gcc atg gct      346
Leu Leu Phe Val Ile Thr Leu Pro Phe Trp Ala Val Asp Ala Met Ala
            85                  90                  95

gac tgg tac ttt ggg aaa ttt tta tgt aag gct gtg cat atc atc tac      394
Asp Trp Tyr Phe Gly Lys Phe Leu Cys Lys Ala Val His Ile Ile Tyr
        100                 105                 110

acc gtc aac ctt tac agc agt gtt ctc atc ctg gcc ttc atc agc ctg      442
Thr Val Asn Leu Tyr Ser Ser Val Leu Ile Leu Ala Phe Ile Ser Leu
    115                 120                 125

gac cgc tac ctt gcc att gtc cac gcc acc aac agc cag agg ccg agg      490
Asp Arg Tyr Leu Ala Ile Val His Ala Thr Asn Ser Gln Arg Pro Arg
130                 135                 140                 145
```

```
aag ctg ctg gct gaa aag gcc gtc tat gtg ggt gtc tgg atc ccc gcc      538
Lys Leu Leu Ala Glu Lys Ala Val Tyr Val Gly Val Trp Ile Pro Ala
                150                 155                 160

ctc ctc ctg act atc cct gac atc atc ttc gcc gat gtc agc cag ggg      586
Leu Leu Leu Thr Ile Pro Asp Ile Ile Phe Ala Asp Val Ser Gln Gly
            165                 170                 175

gac ggc agg tac atc tgt gac cgc ctt tac ccc gac agc ctg tgg atg      634
Asp Gly Arg Tyr Ile Cys Asp Arg Leu Tyr Pro Asp Ser Leu Trp Met
        180                 185                 190

gtg gtg ttc cag ttc cag cac atc atg gtg ggt ctc atc ctg ccg ggc      682
Val Val Phe Gln Phe Gln His Ile Met Val Gly Leu Ile Leu Pro Gly
    195                 200                 205

atc gtc atc ctg tcc tgt tac tgc atc atc atc tcc aag ctg tca cac      730
Ile Val Ile Leu Ser Cys Tyr Cys Ile Ile Ile Ser Lys Leu Ser His
210                 215                 220                 225

tcc aag ggc cac cag aag cgc aag gcc ctc aag act acg gtc atc ctt      778
Ser Lys Gly His Gln Lys Arg Lys Ala Leu Lys Thr Thr Val Ile Leu
                230                 235                 240

atc ctg gct ttc ttt gcc tgc tgg cta ccg tat tac gtg ggg atc agc      826
Ile Leu Ala Phe Phe Ala Cys Trp Leu Pro Tyr Tyr Val Gly Ile Ser
            245                 250                 255

atc gat tcc ttc atc ctt ttg gag gtc atc aag caa gga tgt gag ttc      874
Ile Asp Ser Phe Ile Leu Leu Glu Val Ile Lys Gln Gly Cys Glu Phe
        260                 265                 270

gag agc gtc gtg cac aag tgg atc tcc atc acg gag gcc ctc gcc ttc      922
Glu Ser Val Val His Lys Trp Ile Ser Ile Thr Glu Ala Leu Ala Phe
    275                 280                 285

ttc cac tgt tgc ctg aac ccc atc ctc tac gcc ttc ctc ggg gcc aaa      970
Phe His Cys Cys Leu Asn Pro Ile Leu Tyr Ala Phe Leu Gly Ala Lys
290                 295                 300                 305

ttc aag agc tcc gcg cag cat gca ctc aat tcc atg agc aga ggc tcc     1018
Phe Lys Ser Ser Ala Gln His Ala Leu Asn Ser Met Ser Arg Gly Ser
                310                 315                 320

agc ctc aag atc ctt tcc aaa ggg aaa cgg ggt gga cac tct tcc gtc     1066
Ser Leu Lys Ile Leu Ser Lys Gly Lys Arg Gly Gly His Ser Ser Val
            325                 330                 335

tcc aca gag tca gaa tcc tca agt ttt cac tcc agc taacacttac         1112
Ser Thr Glu Ser Glu Ser Ser Ser Phe His Ser Ser
        340                 345

gcaaagacat atataatata tatatatata tatatatatg acaaagaact tttttatgt    1172

tacacatttc ccagatataa aagactgacc aatcttgtac agttttttt attgactgtt     1232

ggagtttatg tttctctagt ttttgtgtgt gtgtgtgtgt gtgaggtttg acttaattta   1292

tataaatatt ggttttttc tgttgtttgt ctgtttcacg tgaatgagtg tctaggcagg    1352

acctgtggcc aagttcttag tcgctgtgtg tctgtgtgta ggactgtaga gctgtagagg   1412

aaggaactga acgctccaga atgtgtggta aatcggataa agctagaagt gaccctcagc   1472

tgtcgctgca taatctcttc gccccgccca ccccgttcct gaattgtttg gttgtgctgt   1532

gtggtttctc tgtagaagat ggcacttaaa accaaagcct gaaatggtat agaaatgctg   1592

ggttttttct ttcagttttc aagggtggat tgatttcagt acctacaaat gtacagtctt   1652

gtattatgtt gttaataaaa gtcaatggta aacttgaaaa aaaaaaaaaa aaaaaaaaaa   1712

aa                                                                  1714
```

<210> 77
<211> 349
<212> PRT
<213> Rattus norvegicus

<400> 77

Met Glu Ile Tyr Thr Ser Asp Asn Tyr Ser Glu Glu Val Gly Ser Gly
1 5 10 15

Asp Tyr Asp Ser Asn Lys Glu Pro Cys Phe Arg Asp Glu Asn Glu Asn
20 25 30

Phe Asn Arg Ile Phe Leu Pro Thr Ile Tyr Phe Ile Ile Phe Leu Thr
35 40 45

Gly Ile Val Gly Asn Gly Leu Val Ile Leu Val Met Gly Tyr Gln Lys
50 55 60

Lys Leu Arg Ser Met Thr Asp Lys Tyr Arg Leu His Leu Ser Val Ala
65 70 75 80

Asp Leu Leu Phe Val Ile Thr Leu Pro Phe Trp Ala Val Asp Ala Met
85 90 95

Ala Asp Trp Tyr Phe Gly Lys Phe Leu Cys Lys Ala Val His Ile Ile
100 105 110

Tyr Thr Val Asn Leu Tyr Ser Ser Val Leu Ile Leu Ala Phe Ile Ser
115 120 125

Leu Asp Arg Tyr Leu Ala Ile Val His Ala Thr Asn Ser Gln Arg Pro
130 135 140

Arg Lys Leu Leu Ala Glu Lys Ala Val Tyr Val Gly Val Trp Ile Pro
145 150 155 160

Ala Leu Leu Leu Thr Ile Pro Asp Ile Ile Phe Ala Asp Val Ser Gln
165 170 175

Gly Asp Gly Arg Tyr Ile Cys Asp Arg Leu Tyr Pro Asp Ser Leu Trp
180 185 190

Met Val Val Phe Gln Phe Gln His Ile Met Val Gly Leu Ile Leu Pro
195 200 205

Gly Ile Val Ile Leu Ser Cys Tyr Cys Ile Ile Ile Ser Lys Leu Ser
210 215 220

His Ser Lys Gly His Gln Lys Arg Lys Ala Leu Lys Thr Thr Val Ile
225 230 235 240

Leu Ile Leu Ala Phe Phe Ala Cys Trp Leu Pro Tyr Tyr Val Gly Ile
245 250 255

Ser Ile Asp Ser Phe Ile Leu Leu Glu Val Ile Lys Gln Gly Cys Glu
260 265 270

Phe Glu Ser Val Val His Lys Trp Ile Ser Ile Thr Glu Ala Leu Ala

```
        275                 280                 285

Phe Phe His Cys Cys Leu Asn Pro Ile Leu Tyr Ala Phe Leu Gly Ala
    290                 295                 300

Lys Phe Lys Ser Ser Ala Gln His Ala Leu Asn Ser Met Ser Arg Gly
305                 310                 315                 320

Ser Ser Leu Lys Ile Leu Ser Lys Gly Lys Arg Gly Gly His Ser Ser
                325                 330                 335

Val Ser Thr Glu Ser Glu Ser Ser Ser Phe His Ser Ser
            340                 345

<210>  78
<211>  1329
<212>  DNA
<213>  Rattus norvegicus

<220>
<221>  CDS
<222>  (77)..(880)

<400>  78
gggcggttca aggcataaca ggctcatctg ggatcctctc cagtcaggct tccttgtgca      60

agtgtctgaa gcagct atg gca act gtc cct gaa ctc aac tgt gaa ata gca    112
                  Met Ala Thr Val Pro Glu Leu Asn Cys Glu Ile Ala
                  1               5                   10

gct ttc gac agt gag gag aat gac ctg ttc ttt gag gct gac aga ccc       160
Ala Phe Asp Ser Glu Glu Asn Asp Leu Phe Phe Glu Ala Asp Arg Pro
        15                  20                  25

caa aag att aag gat tgc ttc caa gcc ctt gac ttg ggc tgt cca gat       208
Gln Lys Ile Lys Asp Cys Phe Gln Ala Leu Asp Leu Gly Cys Pro Asp
    30                  35                  40

gag agc atc cag ctt caa atc tca cag cag cat ctc gac aag agc ttc       256
Glu Ser Ile Gln Leu Gln Ile Ser Gln Gln His Leu Asp Lys Ser Phe
45                  50                  55                  60

agg aag gca gtg tca ctc att gtg gct gtg gag aag ctg tgg cag cta       304
Arg Lys Ala Val Ser Leu Ile Val Ala Val Glu Lys Leu Trp Gln Leu
                65                  70                  75

cct atg tct tgc ccg tgg agc ttc cag gat gag gac cca agc acc ttc       352
Pro Met Ser Cys Pro Trp Ser Phe Gln Asp Glu Asp Pro Ser Thr Phe
            80                  85                  90

ttt tcc ttc atc ttt gaa gaa gag ccc gtc ctc tgt gac tcg tgg gat       400
Phe Ser Phe Ile Phe Glu Glu Glu Pro Val Leu Cys Asp Ser Trp Asp
        95                  100                 105

gat gac gac ctg cta gtg tgt gat gtt ccc att aga cag ctg cac tgc       448
Asp Asp Asp Leu Leu Val Cys Asp Val Pro Ile Arg Gln Leu His Cys
    110                 115                 120

agg ctt cga gat gaa caa caa aaa tgc ctc gtg ctg tct gac cca tgt       496
Arg Leu Arg Asp Glu Gln Gln Lys Cys Leu Val Leu Ser Asp Pro Cys
125                 130                 135                 140

gag ctg aaa gct ctc cac ctc aat gga cag aac ata agc caa caa gtg       544
Glu Leu Lys Ala Leu His Leu Asn Gly Gln Asn Ile Ser Gln Gln Val
                145                 150                 155

gta ttc tcc atg agc ttt gta caa gga gag aca agc aac gac aaa atc       592
Val Phe Ser Met Ser Phe Val Gln Gly Glu Thr Ser Asn Asp Lys Ile
            160                 165                 170
```

```
cct gtg gcc ttg ggc ctc aag ggg ttg aat cta tac ctg tcc tgt gtg        640
Pro Val Ala Leu Gly Leu Lys Gly Leu Asn Leu Tyr Leu Ser Cys Val
        175                 180                 185

atg aaa gac ggc aca ccc acc ctg cag ctg gag agt gtg gat ccc aaa        688
Met Lys Asp Gly Thr Pro Thr Leu Gln Leu Glu Ser Val Asp Pro Lys
    190                 195                 200

caa tac cca aag aag aag atg gaa aag cgg ttt gtc ttc aac aag ata        736
Gln Tyr Pro Lys Lys Lys Met Glu Lys Arg Phe Val Phe Asn Lys Ile
205                 210                 215                 220

gaa gtc aag acc aaa gtg gag ttt gag tct gca cag ttc ccc aac tgg        784
Glu Val Lys Thr Lys Val Glu Phe Glu Ser Ala Gln Phe Pro Asn Trp
                225                 230                 235

tac atc agc acc tct caa gca gag cac aga cct gtc ttc cta gga aac        832
Tyr Ile Ser Thr Ser Gln Ala Glu His Arg Pro Val Phe Leu Gly Asn
            240                 245                 250

agc aat ggt cgg gac ata gtt gac ttc acc atg gaa ccc gtg tct tcc        880
Ser Asn Gly Arg Asp Ile Val Asp Phe Thr Met Glu Pro Val Ser Ser
        255                 260                 265

taaagatggc tgcactattc ctaatgcctt ccccaggaca tgctagggag cccccttgtc      940

gagaatgggc agtctccagg ggaagccttt gtcctctgcc aagtcaggtc tctcagagcc     1000

ataagaaaac cgtggcacat tctggtcaaa gaaaacgtgt gtttccctcc ctgcctctga     1060

caggcaacca cttacctatt tatttatgta tttattgatt ggttgatcta tttaagttga     1120

ttcagggggg tcacgaggca gcattgtcga cagaagaatc tagttgtccg tgtgtatggg     1180

atgaattgaa tttggaccag tgcacagcca gcactgagtt ctttcattga tgctgaaaat     1240

gaagagtttc atattgtgtg gatgagagtg tttatgaatg aagcacaagc acatcatttt     1300

gatgagtatg aaataaatgt cactaaaac                                       1329


<210>  79
<211>  268
<212>  PRT
<213>  Rattus norvegicus

<400>  79

Met Ala Thr Val Pro Glu Leu Asn Cys Glu Ile Ala Ala Phe Asp Ser
1               5                   10                  15

Glu Glu Asn Asp Leu Phe Phe Glu Ala Asp Arg Pro Gln Lys Ile Lys
            20                  25                  30

Asp Cys Phe Gln Ala Leu Asp Leu Gly Cys Pro Asp Glu Ser Ile Gln
        35                  40                  45

Leu Gln Ile Ser Gln Gln His Leu Asp Lys Ser Phe Arg Lys Ala Val
    50                  55                  60

Ser Leu Ile Val Ala Val Glu Lys Leu Trp Gln Leu Pro Met Ser Cys
65                  70                  75                  80

Pro Trp Ser Phe Gln Asp Glu Asp Pro Ser Thr Phe Phe Ser Phe Ile
                85                  90                  95

Phe Glu Glu Glu Pro Val Leu Cys Asp Ser Trp Asp Asp Asp Asp Leu
            100                 105                 110
```

```
Leu Val Cys Asp Val Pro Ile Arg Gln Leu His Cys Arg Leu Arg Asp
        115                 120                 125

Glu Gln Gln Lys Cys Leu Val Leu Ser Asp Pro Cys Glu Leu Lys Ala
    130                 135                 140

Leu His Leu Asn Gly Gln Asn Ile Ser Gln Gln Val Val Phe Ser Met
145                 150                 155                 160

Ser Phe Val Gln Gly Glu Thr Ser Asn Asp Lys Ile Pro Val Ala Leu
                165                 170                 175

Gly Leu Lys Gly Leu Asn Leu Tyr Leu Ser Cys Val Met Lys Asp Gly
            180                 185                 190

Thr Pro Thr Leu Gln Leu Glu Ser Val Asp Pro Lys Gln Tyr Pro Lys
        195                 200                 205

Lys Lys Met Glu Lys Arg Phe Val Phe Asn Lys Ile Glu Val Lys Thr
    210                 215                 220

Lys Val Glu Phe Glu Ser Ala Gln Phe Pro Asn Trp Tyr Ile Ser Thr
225                 230                 235                 240

Ser Gln Ala Glu His Arg Pro Val Phe Leu Gly Asn Ser Asn Gly Arg
                245                 250                 255

Asp Ile Val Asp Phe Thr Met Glu Pro Val Ser Ser
            260                 265


<210>  80
<211>  2924
<212>  DNA
<213>  Rattus norvegicus


<220>
<221>  CDS
<222>  (9)..(2174)

<400>  80
gcgggcgc atg cgc agg ggg tgc gcg ccg cct ctg ccc cgc ggc gag ggt      50
         Met Arg Arg Gly Cys Ala Pro Pro Leu Pro Arg Gly Glu Gly
         1               5                   10

gtc tat gga gag gcg gcg gcc gcg gct gct gag gcg gac gct gag gca      98
Val Tyr Gly Glu Ala Ala Ala Ala Ala Ala Glu Ala Asp Ala Glu Ala
15                  20                  25                  30

gtg gcg atg gcg ccc ttc ccc gat gaa gtg gac gtc ttc acc gcc cca     146
Val Ala Met Ala Pro Phe Pro Asp Glu Val Asp Val Phe Thr Ala Pro
                35                  40                  45

cac tgg cgg atg aag cag ctg gtg ggg cgc tac tgc gac aag ctt tct     194
His Trp Arg Met Lys Gln Leu Val Gly Arg Tyr Cys Asp Lys Leu Ser
            50                  55                  60

aaa acc aac ttt tcc aac aac aat gac ttc cgt gct ctt ctc cag tct     242
Lys Thr Asn Phe Ser Asn Asn Asn Asp Phe Arg Ala Leu Leu Gln Ser
        65                  70                  75

tta tat gct act ttc aag gag ttt aaa atg cat gaa caa att gaa aat     290
Leu Tyr Ala Thr Phe Lys Glu Phe Lys Met His Glu Gln Ile Glu Asn
    80                  85                  90

gaa tac att att ggt tta ctt cag caa cgc agt cag acc att tac aat     338
```

```
Glu Tyr Ile Ile Gly Leu Leu Gln Gln Arg Ser Gln Thr Ile Tyr Asn
95                  100                 105                 110

gtg cat tct gac aat aag ctc tcc gag atg ctt agt ctc ttt gaa aag      386
Val His Ser Asp Asn Lys Leu Ser Glu Met Leu Ser Leu Phe Glu Lys
                115                 120                 125

ggg ctg aag aat gtt aag aat gag tgt gaa cag ttg aat tat gca aag      434
Gly Leu Lys Asn Val Lys Asn Glu Cys Glu Gln Leu Asn Tyr Ala Lys
            130                 135                 140

cag ctg aag gag aga ttg gag gcc ttc acg aga gat ttc ctt ccc cac      482
Gln Leu Lys Glu Arg Leu Glu Ala Phe Thr Arg Asp Phe Leu Pro His
        145                 150                 155

atg aaa gag gag gag gag gtt ttt cag ccc atg tta atg gaa tat ttt      530
Met Lys Glu Glu Glu Glu Val Phe Gln Pro Met Leu Met Glu Tyr Phe
    160                 165                 170

acc tat gaa gag ctt aaa gat atc aaa aag aaa gtg atc gca caa cac      578
Thr Tyr Glu Glu Leu Lys Asp Ile Lys Lys Lys Val Ile Ala Gln His
175                 180                 185                 190

tgc tca cag aag gac aca gca gaa ctc ctc aga ggg ctc agt ctg tgg      626
Cys Ser Gln Lys Asp Thr Ala Glu Leu Leu Arg Gly Leu Ser Leu Trp
                195                 200                 205

aat caa gct gaa gag cga cag aaa gtt ttc aaa tat tct gtg gat gag      674
Asn Gln Ala Glu Glu Arg Gln Lys Val Phe Lys Tyr Ser Val Asp Glu
            210                 215                 220

aag tca gat gca gaa gcg gaa gtg tca gaa cac tcc aca ggt ata acc      722
Lys Ser Asp Ala Glu Ala Glu Val Ser Glu His Ser Thr Gly Ile Thr
        225                 230                 235

cat ctt cct cct gag gta atg ctg tcc att ttc agt tac ctt aat cct      770
His Leu Pro Pro Glu Val Met Leu Ser Ile Phe Ser Tyr Leu Asn Pro
    240                 245                 250

caa gaa ttg tgt cgg tgc agt caa gtc agc act aag tgg tct cag ctg      818
Gln Glu Leu Cys Arg Cys Ser Gln Val Ser Thr Lys Trp Ser Gln Leu
255                 260                 265                 270

gca aag aca gga tct ttg tgg aaa cat ctt tac cct gtt cat tgg gca      866
Ala Lys Thr Gly Ser Leu Trp Lys His Leu Tyr Pro Val His Trp Ala
                275                 280                 285

aga ggt gac tgg tat agt ggc cct gcc act gaa ctt gat act gaa cct      914
Arg Gly Asp Trp Tyr Ser Gly Pro Ala Thr Glu Leu Asp Thr Glu Pro
            290                 295                 300

gat gag gaa tgg gtg aga agt agg aaa gat gaa agt cgt gct ttc cag      962
Asp Glu Glu Trp Val Arg Ser Arg Lys Asp Glu Ser Arg Ala Phe Gln
        305                 310                 315

gaa tgg gac gaa gat gcc gat ata gat gaa tct gaa gag tct gca gag     1010
Glu Trp Asp Glu Asp Ala Asp Ile Asp Glu Ser Glu Glu Ser Ala Glu
    320                 325                 330

gaa tcg gtt gct atc agc att gca caa atg gaa aaa cgt tta ctc cat     1058
Glu Ser Val Ala Ile Ser Ile Ala Gln Met Glu Lys Arg Leu Leu His
335                 340                 345                 350

ggc tta att cat aat gtt ctg cca tat gtt ggt acc tct gta aaa act     1106
Gly Leu Ile His Asn Val Leu Pro Tyr Val Gly Thr Ser Val Lys Thr
                355                 360                 365

tta gtg tta gca tac agc tct gca gtc tcc agc aaa atg gtt agg cag     1154
Leu Val Leu Ala Tyr Ser Ser Ala Val Ser Ser Lys Met Val Arg Gln
            370                 375                 380

att tta gag ctt tgt cct aat ctg gaa cat ctg gat ctt acc cag act     1202
Ile Leu Glu Leu Cys Pro Asn Leu Glu His Leu Asp Leu Thr Gln Thr
        385                 390                 395

gac att tcc gat tct gca ttt gac agt tgg tct tgg ctt ggc tgc tgc     1250
```

Asp Ile Ser Asp Ser Ala Phe Asp Ser Trp Ser Trp Leu Gly Cys Cys
400 405 410

cag agt ctt cgg cat ctt gac ttg tct gga tgt gaa aaa atc aca gac 1298
Gln Ser Leu Arg His Leu Asp Leu Ser Gly Cys Glu Lys Ile Thr Asp
415 420 425 430

atg gct ctg gag aag att tct aga gct ctt gga gtt ctg aca tct cat 1346
Met Ala Leu Glu Lys Ile Ser Arg Ala Leu Gly Val Leu Thr Ser His
435 440 445

cag agt ggc gtt ctg aaa agt gca ggc aag act act tca act cca tgg 1394
Gln Ser Gly Val Leu Lys Ser Ala Gly Lys Thr Thr Ser Thr Pro Trp
450 455 460

aca aat aaa gac att acc atg cca tcc acc acg cag tat gcc tgt ttg 1442
Thr Asn Lys Asp Ile Thr Met Pro Ser Thr Thr Gln Tyr Ala Cys Leu
465 470 475

cac aat tta act aac aaa ggc gtt ggt gaa gaa atc gac agc gaa cac 1490
His Asn Leu Thr Asn Lys Gly Val Gly Glu Glu Ile Asp Ser Glu His
480 485 490

act tgg act gaa cct gtg tct tct gaa agt ttc act cct ccc tac gtg 1538
Thr Trp Thr Glu Pro Val Ser Ser Glu Ser Phe Thr Pro Pro Tyr Val
495 500 505 510

tgg atg cta gat gct gaa gat ttg gcc gat atc gaa gat gct gta gaa 1586
Trp Met Leu Asp Ala Glu Asp Leu Ala Asp Ile Glu Asp Ala Val Glu
515 520 525

tgg agg cac aga aac gtt gaa agt ctc tgt gtg atg gaa aca gcc tcc 1634
Trp Arg His Arg Asn Val Glu Ser Leu Cys Val Met Glu Thr Ala Ser
530 535 540

aac ttt ggt tgt tcc tcc tct ggt tgt tac agc aag gac att gtg gga 1682
Asn Phe Gly Cys Ser Ser Ser Gly Cys Tyr Ser Lys Asp Ile Val Gly
545 550 555

tta agg act agt gtc tgt tgg cag cag cat tgt gct tct cca gcc ttt 1730
Leu Arg Thr Ser Val Cys Trp Gln Gln His Cys Ala Ser Pro Ala Phe
560 565 570

gcg tat tgt ggt cat tca ttc tgt tgc aca ggg aca gct cta aga act 1778
Ala Tyr Cys Gly His Ser Phe Cys Cys Thr Gly Thr Ala Leu Arg Thr
575 580 585 590

atg tct gca ctc cca gcg gct tct gca gtg tgt aga aaa gca tta agg 1826
Met Ser Ala Leu Pro Ala Ala Ser Ala Val Cys Arg Lys Ala Leu Arg
595 600 605

act acg ttg ccc agg ggg aaa gac tta att tat ttt ggg agt gaa aag 1874
Thr Thr Leu Pro Arg Gly Lys Asp Leu Ile Tyr Phe Gly Ser Glu Lys
610 615 620

tct gac caa gag act gga cga gta ctt ttg ttc ctc agt ctg tct gga 1922
Ser Asp Gln Glu Thr Gly Arg Val Leu Leu Phe Leu Ser Leu Ser Gly
625 630 635

tgt tac cag atc aca gac cat ggt ctc agg gtt ctg act ctg gga gga 1970
Cys Tyr Gln Ile Thr Asp His Gly Leu Arg Val Leu Thr Leu Gly Gly
640 645 650

gga ctg cct tac ttg gag cac ctg aat ctc tct ggc tgt ctg act gta 2018
Gly Leu Pro Tyr Leu Glu His Leu Asn Leu Ser Gly Cys Leu Thr Val
655 660 665 670

act ggt gca gga ctg cag gat ttg gtc tca gca tgt cct tcc cta aat 2066
Thr Gly Ala Gly Leu Gln Asp Leu Val Ser Ala Cys Pro Ser Leu Asn
675 680 685

gac gaa tac ttc tac tac tgt gac aac att aac ggt cct cat gct gac 2114
Asp Glu Tyr Phe Tyr Tyr Cys Asp Asn Ile Asn Gly Pro His Ala Asp
690 695 700

acc gcc agt gga tgc cag aat ttg cag tgt ggt ttt cga gcc tgc tgc 2162

Thr Ala Ser Gly Cys Gln Asn Leu Gln Cys Gly Phe Arg Ala Cys Cys
705 710 715

cgc tct ggc gaa tgacccttga cttctgacct ttgtctactt catttagctg 2214
Arg Ser Gly Glu
720

agcaggcttc ctttcatgca ctttacttat agcacatttc ttgtgttaac ccattttggg 2274

tgtgtcttgt tttggcccca tttcttacag tctcagaaat ttaatttagc agtgaactgt 2334

acaatgttgt ttttctttgc aaaatatatt tctgtttaga aaagattagg attttttgta 2394

gggtgagacc agtcttctgg tttcttttaa atgaaatgct taaaaagaat agcactaata 2454

ccatgctatt taaaatattt tagatttatt tacatttagt caatgggggg ttattggttc 2514

tcttatgtgt aaacactgta ccaagttttg cagctccttt caaatataca tttctgaaga 2574

tttattttca aagtctgaac attttagaaa tgaatctctt ttcccctaat gttttcttta 2634

tttcaacaat tctcaggggc caattcaaac acacttagaa gcgagattct ttaagattat 2694

agaataagtt ggcctcttgg tgccggccca gtgagccagc agaacagcag tctgtttcta 2754

ttgctgtaga cacctgcttc cacggctgtc cctaaactgt tgaattcggg aggtattttt 2814

aaaaattcca caattgtttg aagaaatgta taaataaaat ctactttgag aactttacca 2874

agcaatatct tttgtgttgt gattttattt taaatctcaa tctaagttta 2924

<210> 81
<211> 722
<212> PRT
<213> Rattus norvegicus

<400> 81

Met Arg Arg Gly Cys Ala Pro Pro Leu Pro Arg Gly Glu Gly Val Tyr
1 5 10 15

Gly Glu Ala Ala Ala Ala Ala Ala Glu Ala Asp Ala Glu Ala Val Ala
20 25 30

Met Ala Pro Phe Pro Asp Glu Val Asp Val Phe Thr Ala Pro His Trp
35 40 45

Arg Met Lys Gln Leu Val Gly Arg Tyr Cys Asp Lys Leu Ser Lys Thr
50 55 60

Asn Phe Ser Asn Asn Asn Asp Phe Arg Ala Leu Leu Gln Ser Leu Tyr
65 70 75 80

Ala Thr Phe Lys Glu Phe Lys Met His Glu Gln Ile Glu Asn Glu Tyr
85 90 95

Ile Ile Gly Leu Leu Gln Gln Arg Ser Gln Thr Ile Tyr Asn Val His
100 105 110

Ser Asp Asn Lys Leu Ser Glu Met Leu Ser Leu Phe Glu Lys Gly Leu
115 120 125

Lys Asn Val Lys Asn Glu Cys Glu Gln Leu Asn Tyr Ala Lys Gln Leu
130 135 140

Lys Glu Arg Leu Glu Ala Phe Thr Arg Asp Phe Leu Pro His Met Lys

145 150 155 160

Glu Glu Glu Glu Val Phe Gln Pro Met Leu Met Glu Tyr Phe Thr Tyr
165 170 175

Glu Glu Leu Lys Asp Ile Lys Lys Lys Val Ile Ala Gln His Cys Ser
180 185 190

Gln Lys Asp Thr Ala Glu Leu Leu Arg Gly Leu Ser Leu Trp Asn Gln
195 200 205

Ala Glu Glu Arg Gln Lys Val Phe Lys Tyr Ser Val Asp Glu Lys Ser
210 215 220

Asp Ala Glu Ala Glu Val Ser Glu His Ser Thr Gly Ile Thr His Leu
225 230 235 240

Pro Pro Glu Val Met Leu Ser Ile Phe Ser Tyr Leu Asn Pro Gln Glu
245 250 255

Leu Cys Arg Cys Ser Gln Val Ser Thr Lys Trp Ser Gln Leu Ala Lys
260 265 270

Thr Gly Ser Leu Trp Lys His Leu Tyr Pro Val His Trp Ala Arg Gly
275 280 285

Asp Trp Tyr Ser Gly Pro Ala Thr Glu Leu Asp Thr Glu Pro Asp Glu
290 295 300

Glu Trp Val Arg Ser Arg Lys Asp Glu Ser Arg Ala Phe Gln Glu Trp
305 310 315 320

Asp Glu Asp Ala Asp Ile Asp Glu Ser Glu Glu Ser Ala Glu Glu Ser
325 330 335

Val Ala Ile Ser Ile Ala Gln Met Glu Lys Arg Leu Leu His Gly Leu
340 345 350

Ile His Asn Val Leu Pro Tyr Val Gly Thr Ser Val Lys Thr Leu Val
355 360 365

Leu Ala Tyr Ser Ser Ala Val Ser Ser Lys Met Val Arg Gln Ile Leu
370 375 380

Glu Leu Cys Pro Asn Leu Glu His Leu Asp Leu Thr Gln Thr Asp Ile
385 390 395 400

Ser Asp Ser Ala Phe Asp Ser Trp Ser Trp Leu Gly Cys Cys Gln Ser
405 410 415

Leu Arg His Leu Asp Leu Ser Gly Cys Glu Lys Ile Thr Asp Met Ala
420 425 430

Leu Glu Lys Ile Ser Arg Ala Leu Gly Val Leu Thr Ser His Gln Ser
435 440 445

Gly Val Leu Lys Ser Ala Gly Lys Thr Thr Ser Thr Pro Trp Thr Asn

450 455 460

Lys Asp Ile Thr Met Pro Ser Thr Thr Gln Tyr Ala Cys Leu His Asn
465 470 475 480

Leu Thr Asn Lys Gly Val Gly Glu Glu Ile Asp Ser Glu His Thr Trp
485 490 495

Thr Glu Pro Val Ser Ser Glu Ser Phe Thr Pro Pro Tyr Val Trp Met
500 505 510

Leu Asp Ala Glu Asp Leu Ala Asp Ile Glu Asp Ala Val Glu Trp Arg
515 520 525

His Arg Asn Val Glu Ser Leu Cys Val Met Glu Thr Ala Ser Asn Phe
530 535 540

Gly Cys Ser Ser Ser Gly Cys Tyr Ser Lys Asp Ile Val Gly Leu Arg
545 550 555 560

Thr Ser Val Cys Trp Gln Gln His Cys Ala Ser Pro Ala Phe Ala Tyr
565 570 575

Cys Gly His Ser Phe Cys Cys Thr Gly Thr Ala Leu Arg Thr Met Ser
580 585 590

Ala Leu Pro Ala Ala Ser Ala Val Cys Arg Lys Ala Leu Arg Thr Thr
595 600 605

Leu Pro Arg Gly Lys Asp Leu Ile Tyr Phe Gly Ser Glu Lys Ser Asp
610 615 620

Gln Glu Thr Gly Arg Val Leu Leu Phe Leu Ser Leu Ser Gly Cys Tyr
625 630 635 640

Gln Ile Thr Asp His Gly Leu Arg Val Leu Thr Leu Gly Gly Gly Leu
645 650 655

Pro Tyr Leu Glu His Leu Asn Leu Ser Gly Cys Leu Thr Val Thr Gly
660 665 670

Ala Gly Leu Gln Asp Leu Val Ser Ala Cys Pro Ser Leu Asn Asp Glu
675 680 685

Tyr Phe Tyr Tyr Cys Asp Asn Ile Asn Gly Pro His Ala Asp Thr Ala
690 695 700

Ser Gly Cys Gln Asn Leu Gln Cys Gly Phe Arg Ala Cys Cys Arg Ser
705 710 715 720

Gly Glu

<210> 82
<211> 630
<212> DNA
<213> Rattus norvegicus

<220>
<221> CDS
<222> (20)..(568)

<400> 82
```
cgtgtgcagc acgtccagc atg ttg ttt gcc tgg gct ccc ttc cct gcc ctc      52
                     Met Leu Phe Ala Trp Ala Pro Phe Pro Ala Leu
                     1               5                   10

ctg ggc ctg gca gac tcc tgc tgt ttc gtc gtg ccc cgc agt gag tgg      100
Leu Gly Leu Ala Asp Ser Cys Cys Phe Val Val Pro Arg Ser Glu Trp
            15                  20                  25

aag gcc ctg cca tcc gag tgc tcc aag ggc ctg aag aaa cca gtc cgc      148
Lys Ala Leu Pro Ser Glu Cys Ser Lys Gly Leu Lys Lys Pro Val Arg
        30                  35                  40

tac gtg gtg atc tca cac aca gcc ggc agc ttc tgc agc agc cca gac      196
Tyr Val Val Ile Ser His Thr Ala Gly Ser Phe Cys Ser Ser Pro Asp
    45                  50                  55

tcc tgt gaa cag cag gcc cgc aat gtg cag ctt tac caa atg aaa cag      244
Ser Cys Glu Gln Gln Ala Arg Asn Val Gln Leu Tyr Gln Met Lys Gln
60                  65                  70                  75

ctg ggc tgg tgc gat gta gcc tac aac ttc ctc att gga gag gat ggt      292
Leu Gly Trp Cys Asp Val Ala Tyr Asn Phe Leu Ile Gly Glu Asp Gly
                80                  85                  90

cat gtc tac gaa ggc cga ggc tgg acc atc aag ggt gac cac aca ggg      340
His Val Tyr Glu Gly Arg Gly Trp Thr Ile Lys Gly Asp His Thr Gly
            95                  100                 105

ccc atc tgg aac ccc atg tct atc ggc atc acc ttc atg ggt gac tac      388
Pro Ile Trp Asn Pro Met Ser Ile Gly Ile Thr Phe Met Gly Asp Tyr
        110                 115                 120

tca cac cgg gta cct gca aag cgg gct ctc cgt gct gcc cta aat ctt      436
Ser His Arg Val Pro Ala Lys Arg Ala Leu Arg Ala Ala Leu Asn Leu
    125                 130                 135

ctg aaa tgt ggg gtg tct gag ggc ttc ctg aga tct aac tat gag gtc      484
Leu Lys Cys Gly Val Ser Glu Gly Phe Leu Arg Ser Asn Tyr Glu Val
140                 145                 150                 155

aaa gga cat cga gat gtg caa agc act ctg tct cca ggt gac cag ctc      532
Lys Gly His Arg Asp Val Gln Ser Thr Leu Ser Pro Gly Asp Gln Leu
                160                 165                 170

tac gag atc atc caa agc tgg gac cac tac cga gag tgagatcttg          578
Tyr Glu Ile Ile Gln Ser Trp Asp His Tyr Arg Glu
            175                 180

agaccttgtg gagttcccac cctgaaatcc ctcctaccac ctgctgcttc cc           630
```

<210> 83
<211> 183
<212> PRT
<213> Rattus norvegicus

<400> 83
```
Met Leu Phe Ala Trp Ala Pro Phe Pro Ala Leu Leu Gly Leu Ala Asp
1               5                   10                  15

Ser Cys Cys Phe Val Val Pro Arg Ser Glu Trp Lys Ala Leu Pro Ser
            20                  25                  30

Glu Cys Ser Lys Gly Leu Lys Lys Pro Val Arg Tyr Val Val Ile Ser
        35                  40                  45
```

```
His Thr Ala Gly Ser Phe Cys Ser Ser Pro Asp Ser Cys Glu Gln Gln
    50                  55                  60

Ala Arg Asn Val Gln Leu Tyr Gln Met Lys Gln Leu Gly Trp Cys Asp
65                  70                  75                  80

Val Ala Tyr Asn Phe Leu Ile Gly Glu Asp Gly His Val Tyr Glu Gly
                85                  90                  95

Arg Gly Trp Thr Ile Lys Gly Asp His Thr Gly Pro Ile Trp Asn Pro
            100                 105                 110

Met Ser Ile Gly Ile Thr Phe Met Gly Asp Tyr Ser His Arg Val Pro
        115                 120                 125

Ala Lys Arg Ala Leu Arg Ala Ala Leu Asn Leu Leu Lys Cys Gly Val
    130                 135                 140

Ser Glu Gly Phe Leu Arg Ser Asn Tyr Glu Val Lys Gly His Arg Asp
145                 150                 155                 160

Val Gln Ser Thr Leu Ser Pro Gly Asp Gln Leu Tyr Glu Ile Ile Gln
                165                 170                 175

Ser Trp Asp His Tyr Arg Glu
            180


<210>  84
<211>  1393
<212>  DNA
<213>  Rattus norvegicus


<220>
<221>  CDS
<222>  (98)..(973)

<400>  84
ggggggcgtg attccgtcct gcgtgtctgt tctgcggaac agtaggcagt tgttttccgt      60

ccggcttctc tcacactcaa gtgcgcgcct ccacctc atg gaa gac tcg atg gac      115
                                         Met Glu Asp Ser Met Asp
                                         1               5

atg gac atg agc cct ctt agg cct cag aac tac ctt ttc ggt tgt gaa      163
Met Asp Met Ser Pro Leu Arg Pro Gln Asn Tyr Leu Phe Gly Cys Glu
            10                  15                  20

cta aag gct gac aaa gat tat cac ttt aaa gtg gat aat gat gaa aat      211
Leu Lys Ala Asp Lys Asp Tyr His Phe Lys Val Asp Asn Asp Glu Asn
        25                  30                  35

gag cac cag tta tca tta aga acg gtc agt tta gga gca ggg gca aaa      259
Glu His Gln Leu Ser Leu Arg Thr Val Ser Leu Gly Ala Gly Ala Lys
    40                  45                  50

gat gag ttg cac atc gta gag gca gaa gca atg aac tat gaa ggc agc      307
Asp Glu Leu His Ile Val Glu Ala Glu Ala Met Asn Tyr Glu Gly Ser
55                  60                  65                  70

cca att aaa gta aca ctg gca act ttg aaa atg tct gta caa cca aca      355
Pro Ile Lys Val Thr Leu Ala Thr Leu Lys Met Ser Val Gln Pro Thr
                75                  80                  85

gtt tcc ctt ggg ggc ttc gaa att aca cca cct gtg gtc ttg agg ttg      403
Val Ser Leu Gly Gly Phe Glu Ile Thr Pro Pro Val Val Leu Arg Leu
            90                  95                  100
```

```
aag tgt ggt tct ggg cct gtg cac ata agt gga cag cac cta gta gct      451
Lys Cys Gly Ser Gly Pro Val His Ile Ser Gly Gln His Leu Val Ala
        105                 110                 115

gta gag gaa gat gca gag tca gaa gat gaa gat gag gaa gat gta aaa      499
Val Glu Glu Asp Ala Glu Ser Glu Asp Glu Asp Glu Glu Asp Val Lys
    120                 125                 130

ctc tta ggc atg tct gga aag aga tct gct ccc gga ggt ggt aac aaa      547
Leu Leu Gly Met Ser Gly Lys Arg Ser Ala Pro Gly Gly Gly Asn Lys
135                 140                 145                 150

gtc cca cag aaa aaa gta aaa ctt gat gaa gat gat gat gag gat gat      595
Val Pro Gln Lys Lys Val Lys Leu Asp Glu Asp Asp Asp Glu Asp Asp
                155                 160                 165

gaa gat gat gag gat gat gaa gat gat gat gat gat gat ttt gat gaa      643
Glu Asp Asp Glu Asp Asp Glu Asp Asp Asp Asp Asp Asp Phe Asp Glu
            170                 175                 180

gag gaa act gaa gaa aag gtt cca gtg aag aaa tct gta cga gat acc      691
Glu Glu Thr Glu Glu Lys Val Pro Val Lys Lys Ser Val Arg Asp Thr
        185                 190                 195

cca gcc aaa aat gca caa aaa tca aac caa aat ggg aaa gat tta aaa      739
Pro Ala Lys Asn Ala Gln Lys Ser Asn Gln Asn Gly Lys Asp Leu Lys
    200                 205                 210

cca tca aca cca agg tca aag ggt caa gag tcc ttc aaa aaa cag gaa      787
Pro Ser Thr Pro Arg Ser Lys Gly Gln Glu Ser Phe Lys Lys Gln Glu
215                 220                 225                 230

aaa act ccc aaa aca ccc aaa gga cct agc tct gta gaa gac att aag      835
Lys Thr Pro Lys Thr Pro Lys Gly Pro Ser Ser Val Glu Asp Ile Lys
                235                 240                 245

gca aaa atg caa gca agt ata gaa aaa ggt ggt tct ctt ccc aaa gtg      883
Ala Lys Met Gln Ala Ser Ile Glu Lys Gly Gly Ser Leu Pro Lys Val
            250                 255                 260

gaa gcc aag ttc att aat tat gtg aag aat tgt ttc cgg atg act gac      931
Glu Ala Lys Phe Ile Asn Tyr Val Lys Asn Cys Phe Arg Met Thr Asp
        265                 270                 275

cag gag gct att caa gat ctc tgg cag tgg agg aag tct ctt              973
Gln Glu Ala Ile Gln Asp Leu Trp Gln Trp Arg Lys Ser Leu
    280                 285                 290

taagaaaatg gtttaaacag tttgaaataa ttctgtcttc atttctgtaa tagttgctat    1033

ctggctgtcc tttttataat gcaaagtgag aactttccct actgtgtttg ataaatgttg    1093

tccaggttca attgccaaga atgtgttgtc taaaatgcct gtttagtttt caaggatgga    1153

actccgccct ttacttggtt ttaagtatgt atggaatgtt atgataggac atagtagtag    1213

tggtggtcag atgtggaaat ggtagggaga caaatataca tgtgaaataa actcagtatt    1273

ttaataaagt aaaaaaaaaa aaaaaaaaaa aaaaaaaaaa aaaaaaaaaa aaaaaaaaaa    1333

aaaaaaaaaa aaaaaaaaaa aaaaaaaata aaaaaaaaaa aaaaaaaaaa aaaaaaaaaa    1393
```

<210> 85
<211> 292
<212> PRT
<213> Rattus norvegicus

<400> 85

```
Met Glu Asp Ser Met Asp Met Asp Met Ser Pro Leu Arg Pro Gln Asn
1               5                   10                  15

Tyr Leu Phe Gly Cys Glu Leu Lys Ala Asp Lys Asp Tyr His Phe Lys
```

20 25 30

Val Asp Asn Asp Glu Asn Glu His Gln Leu Ser Leu Arg Thr Val Ser
35 40 45

Leu Gly Ala Gly Ala Lys Asp Glu Leu His Ile Val Glu Ala Glu Ala
50 55 60

Met Asn Tyr Glu Gly Ser Pro Ile Lys Val Thr Leu Ala Thr Leu Lys
65 70 75 80

Met Ser Val Gln Pro Thr Val Ser Leu Gly Gly Phe Glu Ile Thr Pro
85 90 95

Pro Val Val Leu Arg Leu Lys Cys Gly Ser Gly Pro Val His Ile Ser
100 105 110

Gly Gln His Leu Val Ala Val Glu Glu Asp Ala Glu Ser Glu Asp Glu
115 120 125

Asp Glu Glu Asp Val Lys Leu Leu Gly Met Ser Gly Lys Arg Ser Ala
130 135 140

Pro Gly Gly Gly Asn Lys Val Pro Gln Lys Lys Val Lys Leu Asp Glu
145 150 155 160

Asp Asp Asp Glu Asp Asp Glu Asp Asp Glu Asp Asp Glu Asp Asp Asp
165 170 175

Asp Asp Asp Phe Asp Glu Glu Glu Thr Glu Glu Lys Val Pro Val Lys
180 185 190

Lys Ser Val Arg Asp Thr Pro Ala Lys Asn Ala Gln Lys Ser Asn Gln
195 200 205

Asn Gly Lys Asp Leu Lys Pro Ser Thr Pro Arg Ser Lys Gly Gln Glu
210 215 220

Ser Phe Lys Lys Gln Glu Lys Thr Pro Lys Thr Pro Lys Gly Pro Ser
225 230 235 240

Ser Val Glu Asp Ile Lys Ala Lys Met Gln Ala Ser Ile Glu Lys Gly
245 250 255

Gly Ser Leu Pro Lys Val Glu Ala Lys Phe Ile Asn Tyr Val Lys Asn
260 265 270

Cys Phe Arg Met Thr Asp Gln Glu Ala Ile Gln Asp Leu Trp Gln Trp
275 280 285

Arg Lys Ser Leu
290

<210> 86
<211> 1326
<212> DNA
<213> Rattus norvegicus

```
<220>
<221>  CDS
<222>  (10)..(1005)

<400>  86
ggagcagcc atg gcc tct tgg cag cat caa ggg aga acc ctg ttc ctc ctg      51
          Met Ala Ser Trp Gln His Gln Gly Arg Thr Leu Phe Leu Leu
          1               5                   10

gcc ctc ttg ggc ggg gtg tcg ggc ctc agg gaa tgc ttg ata ctg cag      99
Ala Leu Leu Gly Gly Val Ser Gly Leu Arg Glu Cys Leu Ile Leu Gln
15                  20                  25                  30

tcg gtt aag gga agc acg gtc tcc tgc cac ggt ccc act gag ttc ccg     147
Ser Val Lys Gly Ser Thr Val Ser Cys His Gly Pro Thr Glu Phe Pro
                35                  40                  45

agc tcc ctc ccg gcc gac act gtc cat ctg tcg gtg gaa ttc tcc agc     195
Ser Ser Leu Pro Ala Asp Thr Val His Leu Ser Val Glu Phe Ser Ser
            50                  55                  60

ctg act cag ctg ccc gcc gcc gcc ctg caa ggg tgt ccc ggt ctg cag     243
Leu Thr Gln Leu Pro Ala Ala Ala Leu Gln Gly Cys Pro Gly Leu Gln
        65                  70                  75

gaa ctg cac ctc tcc agc aac cgc ctg cag gaa ctg tcc ccc ggg ctg     291
Glu Leu His Leu Ser Ser Asn Arg Leu Gln Glu Leu Ser Pro Gly Leu
    80                  85                  90

ctg gcg ccc gtg ccc cgg ctg cgc gtc ctg gat ctg act cgc aat gct     339
Leu Ala Pro Val Pro Arg Leu Arg Val Leu Asp Leu Thr Arg Asn Ala
95                  100                 105                 110

ctc cgc agc ctg ccc ccc ggt ttg ttc cgc agc tcg gcc gcc ctg aac     387
Leu Arg Ser Leu Pro Pro Gly Leu Phe Arg Ser Ser Ala Ala Leu Asn
                115                 120                 125

acc ctg gtc ctg agg gag aac cag ctg cag gag gcg agc gcg cgc tgg     435
Thr Leu Val Leu Arg Glu Asn Gln Leu Gln Glu Ala Ser Ala Arg Trp
            130                 135                 140

ctg cag ggc ctg gac gcc ctg ggc tac ttg gac ctg gcg gag aac cgg     483
Leu Gln Gly Leu Asp Ala Leu Gly Tyr Leu Asp Leu Ala Glu Asn Arg
        145                 150                 155

ctg cgc tcg ctg ccc gct agg ctc ctt gcc aac cta ggc gcc ctg cac     531
Leu Arg Ser Leu Pro Ala Arg Leu Leu Ala Asn Leu Gly Ala Leu His
    160                 165                 170

acc ctc gac ctt ggg cac aac ctg ctt gag tcg ctg ccc gag gga ttc     579
Thr Leu Asp Leu Gly His Asn Leu Leu Glu Ser Leu Pro Glu Gly Phe
175                 180                 185                 190

ctg agg ggc ccc agg cgg cta cag cgc ctg cac ctg gaa ggg aac cgg     627
Leu Arg Gly Pro Arg Arg Leu Gln Arg Leu His Leu Glu Gly Asn Arg
                195                 200                 205

ctg caa agg ctg gag gct ggc tta ctt gcg ccc cag ccg ttc ctg ggc     675
Leu Gln Arg Leu Glu Ala Gly Leu Leu Ala Pro Gln Pro Phe Leu Gly
            210                 215                 220

gtc ctg ttc ctg aat gac aac cag cta acc gca gtg gcg gcc gac tcc     723
Val Leu Phe Leu Asn Asp Asn Gln Leu Thr Ala Val Ala Ala Asp Ser
        225                 230                 235

ttt cgg ggc cta aag cag ctg gat atg ttg gat ctg tcc aat aac tct     771
Phe Arg Gly Leu Lys Gln Leu Asp Met Leu Asp Leu Ser Asn Asn Ser
    240                 245                 250

ctg tcc agc act ccc ccg ggt ctg tgg gcg ttc ctg ggg aga ccg acc     819
Leu Ser Ser Thr Pro Pro Gly Leu Trp Ala Phe Leu Gly Arg Pro Thr
255                 260                 265                 270

cgc gat atg cag gat ggt ttc gat gtc tcc cac aac ccc tgg gtc tgt     867
Arg Asp Met Gln Asp Gly Phe Asp Val Ser His Asn Pro Trp Val Cys
```

```
                275                 280                 285

gac aag gac ctc gtg gac ctg tgc cgc tgg ctg gcc gcc aac cga cat        915
Asp Lys Asp Leu Val Asp Leu Cys Arg Trp Leu Ala Ala Asn Arg His
            290                 295                 300

aag atg ttc tcg cag aac gac aca ctc tgt gcg ggg ccc gag gcc gtg        963
Lys Met Phe Ser Gln Asn Asp Thr Leu Cys Ala Gly Pro Glu Ala Val
        305                 310                 315

agg gga cag cga ctg ctg gac gtg gca gag ctg ggg acc ttg               1005
Arg Gly Gln Arg Leu Leu Asp Val Ala Glu Leu Gly Thr Leu
    320                 325                 330

tgaggatggc aactggggtg cgagccaagg gtaccccgct tgccactgaa gcaatttggt    1065

cccatgtcag aatgcagatt cccagcatct gccattcccc attccctcag ccaggaatgc    1125

tattccctga ctctccctca ggctcctctc catttgcccc aactcttcca cctctcactg    1185

ttcctgtgct ggcccccagg ctaccatgtg tttatctagc tttgcctcat atgtttcagg    1245

gtcaccaaag cagttaataa aacagctccc ggctggctga gccgctcagc gtcaaaaaaa    1305

aaaaaaaaaa aaaaaaaaaa a                                               1326
```

<210> 87
<211> 332
<212> PRT
<213> Rattus norvegicus

<400> 87

```
Met Ala Ser Trp Gln His Gln Gly Arg Thr Leu Phe Leu Leu Ala Leu
1               5                   10                  15

Leu Gly Gly Val Ser Gly Leu Arg Glu Cys Leu Ile Leu Gln Ser Val
            20                  25                  30

Lys Gly Ser Thr Val Ser Cys His Gly Pro Thr Glu Phe Pro Ser Ser
        35                  40                  45

Leu Pro Ala Asp Thr Val His Leu Ser Val Glu Phe Ser Ser Leu Thr
    50                  55                  60

Gln Leu Pro Ala Ala Ala Leu Gln Gly Cys Pro Gly Leu Gln Glu Leu
65                  70                  75                  80

His Leu Ser Ser Asn Arg Leu Gln Glu Leu Ser Pro Gly Leu Leu Ala
                85                  90                  95

Pro Val Pro Arg Leu Arg Val Leu Asp Leu Thr Arg Asn Ala Leu Arg
            100                 105                 110

Ser Leu Pro Pro Gly Leu Phe Arg Ser Ser Ala Ala Leu Asn Thr Leu
        115                 120                 125

Val Leu Arg Glu Asn Gln Leu Gln Glu Ala Ser Ala Arg Trp Leu Gln
    130                 135                 140

Gly Leu Asp Ala Leu Gly Tyr Leu Asp Leu Ala Glu Asn Arg Leu Arg
145                 150                 155                 160

Ser Leu Pro Ala Arg Leu Leu Ala Asn Leu Gly Ala Leu His Thr Leu
                165                 170                 175
```

Asp Leu Gly His Asn Leu Leu Glu Ser Leu Pro Glu Gly Phe Leu Arg
180 185 190

Gly Pro Arg Arg Leu Gln Arg Leu His Leu Glu Gly Asn Arg Leu Gln
195 200 205

Arg Leu Glu Ala Gly Leu Leu Ala Pro Gln Pro Phe Leu Gly Val Leu
210 215 220

Phe Leu Asn Asp Asn Gln Leu Thr Ala Val Ala Ala Asp Ser Phe Arg
225 230 235 240

Gly Leu Lys Gln Leu Asp Met Leu Asp Leu Ser Asn Asn Ser Leu Ser
245 250 255

Ser Thr Pro Pro Gly Leu Trp Ala Phe Leu Gly Arg Pro Thr Arg Asp
260 265 270

Met Gln Asp Gly Phe Asp Val Ser His Asn Pro Trp Val Cys Asp Lys
275 280 285

Asp Leu Val Asp Leu Cys Arg Trp Leu Ala Ala Asn Arg His Lys Met
290 295 300

Phe Ser Gln Asn Asp Thr Leu Cys Ala Gly Pro Glu Ala Val Arg Gly
305 310 315 320

Gln Arg Leu Leu Asp Val Ala Glu Leu Gly Thr Leu
325 330

<210> 88
<211> 2216
<212> DNA
<213> Rattus norvegicus

<220>
<221> CDS
<222> (7)..(1995)

<400> 88
tgcaag atg ggt gtc tac cgc atc cgc gtc tcc acc gga gac tcc aag 48
Met Gly Val Tyr Arg Ile Arg Val Ser Thr Gly Asp Ser Lys
1 5 10

tac gcg ggc tcc aac aac gag gtc tac ctg tgg ttg gtt gga cag cat 96
Tyr Ala Gly Ser Asn Asn Glu Val Tyr Leu Trp Leu Val Gly Gln His
15 20 25 30

gga gag gca tct ctc ggg aag ctg cta cga ccc tgt cgg gac tcg gaa 144
Gly Glu Ala Ser Leu Gly Lys Leu Leu Arg Pro Cys Arg Asp Ser Glu
35 40 45

gca gaa ttc aaa gtg gat gtg tca gga tac ctt ggg cca ctg ctg ttc 192
Ala Glu Phe Lys Val Asp Val Ser Gly Tyr Leu Gly Pro Leu Leu Phe
50 55 60

gta aga gtg cag aaa tgg cat tat ctc acg gat gac gcc tgg ttc tgc 240
Val Arg Val Gln Lys Trp His Tyr Leu Thr Asp Asp Ala Trp Phe Cys
65 70 75

aac tgg att tct gtg aag ggc ccc gga gac caa gga tca gag tac atg 288
Asn Trp Ile Ser Val Lys Gly Pro Gly Asp Gln Gly Ser Glu Tyr Met
80 85 90

```
ttc ccc tgt tac cga tgg gtt cag ggc aga agc atc ctg agc ctc cct      336
Phe Pro Cys Tyr Arg Trp Val Gln Gly Arg Ser Ile Leu Ser Leu Pro
95                  100                 105                 110

gag ggc act ggc tgc acc gtg gtt gaa gat tct caa gga ctg ttc agg      384
Glu Gly Thr Gly Cys Thr Val Val Glu Asp Ser Gln Gly Leu Phe Arg
                115                 120                 125

aaa cat agg gaa gag gag ctt gaa gag agg agg agt ctg tac agg tgg      432
Lys His Arg Glu Glu Glu Leu Glu Glu Arg Arg Ser Leu Tyr Arg Trp
            130                 135                 140

ggc aac tgg aag gat ggc tca atc ctg aat gtg gcg gcg gcc agt ata      480
Gly Asn Trp Lys Asp Gly Ser Ile Leu Asn Val Ala Ala Ala Ser Ile
        145                 150                 155

tct gac ctc cct gta gac caa cga ttt cga gag gac aaa aga att gaa      528
Ser Asp Leu Pro Val Asp Gln Arg Phe Arg Glu Asp Lys Arg Ile Glu
    160                 165                 170

ttt gaa gct tca cag gtt ata ggg gta atg gat act gtt gtc aac ttt      576
Phe Glu Ala Ser Gln Val Ile Gly Val Met Asp Thr Val Val Asn Phe
175                 180                 185                 190

cct ata aac act gtg acc tgc tgg aaa agc cta gat gac ttc aac tgc      624
Pro Ile Asn Thr Val Thr Cys Trp Lys Ser Leu Asp Asp Phe Asn Cys
                195                 200                 205

gtt ttc aag agt ggc cat acc aaa atg gct gag cgg gtt cga aac tcc      672
Val Phe Lys Ser Gly His Thr Lys Met Ala Glu Arg Val Arg Asn Ser
            210                 215                 220

tgg aag gaa gat gcg ttc ttt ggg tac caa ttc ctc aat ggt gct aac      720
Trp Lys Glu Asp Ala Phe Phe Gly Tyr Gln Phe Leu Asn Gly Ala Asn
        225                 230                 235

ccc atg gtg ctg aag cgc tct act tgt ctt cct gcc cgc ctg gta ttc      768
Pro Met Val Leu Lys Arg Ser Thr Cys Leu Pro Ala Arg Leu Val Phe
    240                 245                 250

cct cca gga atg gag aag cta cag gcc cag ctg aac aag gag ctc cag      816
Pro Pro Gly Met Glu Lys Leu Gln Ala Gln Leu Asn Lys Glu Leu Gln
255                 260                 265                 270

aaa ggc act ctg ttt gaa gcg gat ttc ttc ctt ctg gat ggg atc aag      864
Lys Gly Thr Leu Phe Glu Ala Asp Phe Phe Leu Leu Asp Gly Ile Lys
                275                 280                 285

gcc aat gtc atc ctt tgt agc cag cag tac ctg gct gcc cct ctc gtc      912
Ala Asn Val Ile Leu Cys Ser Gln Gln Tyr Leu Ala Ala Pro Leu Val
            290                 295                 300

atg ctg aag ctg atg cct gat gga caa ctc ttg ccc ata gcc atc cag      960
Met Leu Lys Leu Met Pro Asp Gly Gln Leu Leu Pro Ile Ala Ile Gln
        305                 310                 315

ctt gaa ctt ccc aaa act ggg tct act cca cca cct att ttc acg ccc     1008
Leu Glu Leu Pro Lys Thr Gly Ser Thr Pro Pro Pro Ile Phe Thr Pro
    320                 325                 330

tcg gat ccc cca atg gac tgg ctc cta gcc aaa tgc tgg gtc cgg agc     1056
Ser Asp Pro Pro Met Asp Trp Leu Leu Ala Lys Cys Trp Val Arg Ser
335                 340                 345                 350

tcc gac tta cag ctc cat gag ctg cag gct cat ctt ctg agg gga cac     1104
Ser Asp Leu Gln Leu His Glu Leu Gln Ala His Leu Leu Arg Gly His
                355                 360                 365

ttg atg gct gag gtc ttt gct gtg gcc acc atg agg tgc ctg cct tcc     1152
Leu Met Ala Glu Val Phe Ala Val Ala Thr Met Arg Cys Leu Pro Ser
            370                 375                 380

gtg cac cct gtt ttt aag ctt cta gtt cct cat ctg ctt tac acc atg     1200
Val His Pro Val Phe Lys Leu Leu Val Pro His Leu Leu Tyr Thr Met
        385                 390                 395
```

gaa att aat gtc cgg gcc agg agt gac ctg atc tca gag aga ggc ttt 1248
Glu Ile Asn Val Arg Ala Arg Ser Asp Leu Ile Ser Glu Arg Gly Phe
400 405 410

ttt gac aag gca atg agc aca ggt ggg gga ggc cac ctg gat ctt ctc 1296
Phe Asp Lys Ala Met Ser Thr Gly Gly Gly Gly His Leu Asp Leu Leu
415 420 425 430

aag caa gct gga gcc ttt ctg acc tat tgc tca ttg tgt ccc ccc gat 1344
Lys Gln Ala Gly Ala Phe Leu Thr Tyr Cys Ser Leu Cys Pro Pro Asp
435 440 445

gac ttg gct gag cga gga ctc ttg gat atc gag act tgc ttc tat gct 1392
Asp Leu Ala Glu Arg Gly Leu Leu Asp Ile Glu Thr Cys Phe Tyr Ala
450 455 460

aaa gac gcc ctg cga ctc tgg cag atc atg aat cgg tac gtg gtg gga 1440
Lys Asp Ala Leu Arg Leu Trp Gln Ile Met Asn Arg Tyr Val Val Gly
465 470 475

atg ttc aat ctc cac tac aag acc gac aaa gct gtg caa gac gac tat 1488
Met Phe Asn Leu His Tyr Lys Thr Asp Lys Ala Val Gln Asp Asp Tyr
480 485 490

gaa ctg cag agc tgg tgt cga gag atc act gac att ggt ctt caa ggg 1536
Glu Leu Gln Ser Trp Cys Arg Glu Ile Thr Asp Ile Gly Leu Gln Gly
495 500 505 510

gcc cag gac aga ggc ttc cct acc tct ctt cag tcc cgg gct cag gct 1584
Ala Gln Asp Arg Gly Phe Pro Thr Ser Leu Gln Ser Arg Ala Gln Ala
515 520 525

tgc tac ttc atc acc atg tgc atc ttc acg tgc acc gca cag cac tct 1632
Cys Tyr Phe Ile Thr Met Cys Ile Phe Thr Cys Thr Ala Gln His Ser
530 535 540

tcc gtc cat ctt ggc cag ctg gat tgg ttc tac tgg gtt cct aat gca 1680
Ser Val His Leu Gly Gln Leu Asp Trp Phe Tyr Trp Val Pro Asn Ala
545 550 555

ccc tgc acc atg cgg ctg cca cca ccc acc acc aag gaa gca aca atg 1728
Pro Cys Thr Met Arg Leu Pro Pro Pro Thr Thr Lys Glu Ala Thr Met
560 565 570

gag aag ctg atg gct aca ctg ccc aac cct aat cag tct act ctc cag 1776
Glu Lys Leu Met Ala Thr Leu Pro Asn Pro Asn Gln Ser Thr Leu Gln
575 580 585 590

ata aat gtc gtt tgg ctc ctg ggc aga cgc cag gct gtt atg gtg ccc 1824
Ile Asn Val Val Trp Leu Leu Gly Arg Arg Gln Ala Val Met Val Pro
595 600 605

ctg ggc cag cat tca gag gaa cac ttt cca aac cct gag gcc aag gct 1872
Leu Gly Gln His Ser Glu Glu His Phe Pro Asn Pro Glu Ala Lys Ala
610 615 620

gtg ctg aag aag ttc aga gag gaa ctg gct gcc ttg gat aag gaa att 1920
Val Leu Lys Lys Phe Arg Glu Glu Leu Ala Ala Leu Asp Lys Glu Ile
625 630 635

gag att cgt aat aag agc ttg gac ata cct tat gag tac ctg cgg ccc 1968
Glu Ile Arg Asn Lys Ser Leu Asp Ile Pro Tyr Glu Tyr Leu Arg Pro
640 645 650

agc atg gtg gaa aac agc gtg gcc ata tgagcatccc cagactcctg 2015
Ser Met Val Glu Asn Ser Val Ala Ile
655 660

ctttgttgtt tagttaagac cacccaagta catccctgtg cttgagtggt ggcagtcctg 2075

ccctcccaag ccccgccctc tgaccctcaa agccccaccc cctgcccatg tgggaccctc 2135

ctccaagctg tccattgtct gctgcaatga atacattttg ctttggatgc attaaaaaaa 2195

aaaaaaaaaa aaaaaaaaaa a 2216

<210> 89
<211> 663
<212> PRT
<213> Rattus norvegicus

<400> 89

Met Gly Val Tyr Arg Ile Arg Val Ser Thr Gly Asp Ser Lys Tyr Ala
1 5 10 15

Gly Ser Asn Asn Glu Val Tyr Leu Trp Leu Val Gly Gln His Gly Glu
20 25 30

Ala Ser Leu Gly Lys Leu Leu Arg Pro Cys Arg Asp Ser Glu Ala Glu
35 40 45

Phe Lys Val Asp Val Ser Gly Tyr Leu Gly Pro Leu Leu Phe Val Arg
50 55 60

Val Gln Lys Trp His Tyr Leu Thr Asp Asp Ala Trp Phe Cys Asn Trp
65 70 75 80

Ile Ser Val Lys Gly Pro Gly Asp Gln Gly Ser Glu Tyr Met Phe Pro
85 90 95

Cys Tyr Arg Trp Val Gln Gly Arg Ser Ile Leu Ser Leu Pro Glu Gly
100 105 110

Thr Gly Cys Thr Val Val Glu Asp Ser Gln Gly Leu Phe Arg Lys His
115 120 125

Arg Glu Glu Glu Leu Glu Glu Arg Arg Ser Leu Tyr Arg Trp Gly Asn
130 135 140

Trp Lys Asp Gly Ser Ile Leu Asn Val Ala Ala Ala Ser Ile Ser Asp
145 150 155 160

Leu Pro Val Asp Gln Arg Phe Arg Glu Asp Lys Arg Ile Glu Phe Glu
165 170 175

Ala Ser Gln Val Ile Gly Val Met Asp Thr Val Val Asn Phe Pro Ile
180 185 190

Asn Thr Val Thr Cys Trp Lys Ser Leu Asp Asp Phe Asn Cys Val Phe
195 200 205

Lys Ser Gly His Thr Lys Met Ala Glu Arg Val Arg Asn Ser Trp Lys
210 215 220

Glu Asp Ala Phe Phe Gly Tyr Gln Phe Leu Asn Gly Ala Asn Pro Met
225 230 235 240

Val Leu Lys Arg Ser Thr Cys Leu Pro Ala Arg Leu Val Phe Pro Pro
245 250 255

Gly Met Glu Lys Leu Gln Ala Gln Leu Asn Lys Glu Leu Gln Lys Gly
260 265 270

Thr Leu Phe Glu Ala Asp Phe Phe Leu Leu Asp Gly Ile Lys Ala Asn
275 280 285

Val Ile Leu Cys Ser Gln Gln Tyr Leu Ala Ala Pro Leu Val Met Leu
290 295 300

Lys Leu Met Pro Asp Gly Gln Leu Leu Pro Ile Ala Ile Gln Leu Glu
305 310 315 320

Leu Pro Lys Thr Gly Ser Thr Pro Pro Pro Ile Phe Thr Pro Ser Asp
325 330 335

Pro Pro Met Asp Trp Leu Leu Ala Lys Cys Trp Val Arg Ser Ser Asp
340 345 350

Leu Gln Leu His Glu Leu Gln Ala His Leu Leu Arg Gly His Leu Met
355 360 365

Ala Glu Val Phe Ala Val Ala Thr Met Arg Cys Leu Pro Ser Val His
370 375 380

Pro Val Phe Lys Leu Leu Val Pro His Leu Leu Tyr Thr Met Glu Ile
385 390 395 400

Asn Val Arg Ala Arg Ser Asp Leu Ile Ser Glu Arg Gly Phe Phe Asp
405 410 415

Lys Ala Met Ser Thr Gly Gly Gly Gly His Leu Asp Leu Leu Lys Gln
420 425 430

Ala Gly Ala Phe Leu Thr Tyr Cys Ser Leu Cys Pro Pro Asp Asp Leu
435 440 445

Ala Glu Arg Gly Leu Leu Asp Ile Glu Thr Cys Phe Tyr Ala Lys Asp
450 455 460

Ala Leu Arg Leu Trp Gln Ile Met Asn Arg Tyr Val Val Gly Met Phe
465 470 475 480

Asn Leu His Tyr Lys Thr Asp Lys Ala Val Gln Asp Asp Tyr Glu Leu
485 490 495

Gln Ser Trp Cys Arg Glu Ile Thr Asp Ile Gly Leu Gln Gly Ala Gln
500 505 510

Asp Arg Gly Phe Pro Thr Ser Leu Gln Ser Arg Ala Gln Ala Cys Tyr
515 520 525

Phe Ile Thr Met Cys Ile Phe Thr Cys Thr Ala Gln His Ser Ser Val
530 535 540

His Leu Gly Gln Leu Asp Trp Phe Tyr Trp Val Pro Asn Ala Pro Cys
545 550 555 560

Thr Met Arg Leu Pro Pro Pro Thr Thr Lys Glu Ala Thr Met Glu Lys
565 570 575

Leu Met Ala Thr Leu Pro Asn Pro Asn Gln Ser Thr Leu Gln Ile Asn
580 585 590

Val Val Trp Leu Leu Gly Arg Arg Gln Ala Val Met Val Pro Leu Gly
595 600 605

Gln His Ser Glu Glu His Phe Pro Asn Pro Glu Ala Lys Ala Val Leu
610 615 620

Lys Lys Phe Arg Glu Glu Leu Ala Ala Leu Asp Lys Glu Ile Glu Ile
625 630 635 640

Arg Asn Lys Ser Leu Asp Ile Pro Tyr Glu Tyr Leu Arg Pro Ser Met
645 650 655

Val Glu Asn Ser Val Ala Ile
660

<210> 90
<211> 5487
<212> DNA
<213> Rattus norvegicus

<220>
<221> CDS
<222> (1)..(4794)

<400> 90
atg att ccc tca tac cca gtt gtc ctc cat gga cat gtg gcc act ctg 48
Met Ile Pro Ser Tyr Pro Val Val Leu His Gly His Val Ala Thr Leu
1 5 10 15

tgc cag cag ttt tta aag gag ctg aac act cac att aga gcc acc aag 96
Cys Gln Gln Phe Leu Lys Glu Leu Asn Thr His Ile Arg Ala Thr Lys
20 25 30

ggg tgc ttt ctg gat ggc ctt gaa gcc acc acg cgg tat gag tcc acg 144
Gly Cys Phe Leu Asp Gly Leu Glu Ala Thr Thr Arg Tyr Glu Ser Thr
35 40 45

gcc aag tcc aag act tca gat gga cat act caa tct gag aac agc ccc 192
Ala Lys Ser Lys Thr Ser Asp Gly His Thr Gln Ser Glu Asn Ser Pro
50 55 60

ttt gac ccc ttc tac aca gaa gtc tgt cgg tct gct gct ctg cag atg 240
Phe Asp Pro Phe Tyr Thr Glu Val Cys Arg Ser Ala Ala Leu Gln Met
65 70 75 80

gta ctc tct tgt cca ccg ctg gtc ctg ggt tac act gac aag gtt ccc 288
Val Leu Ser Cys Pro Pro Leu Val Leu Gly Tyr Thr Asp Lys Val Pro
85 90 95

agt gtc tct gaa gct gtt cat gtt ctg tca cat aga aca cag ttc agg 336
Ser Val Ser Glu Ala Val His Val Leu Ser His Arg Thr Gln Phe Arg
100 105 110

gcc agc cat gta cag ttc cac tgg ggg aaa cac cgt gct agg cac gtg 384
Ala Ser His Val Gln Phe His Trp Gly Lys His Arg Ala Arg His Val
115 120 125

gac cac aac tca ggc cca ata cac acg tca tct ctc ctg atg gaa act 432
Asp His Asn Ser Gly Pro Ile His Thr Ser Ser Leu Leu Met Glu Thr
130 135 140

gcg cat tgt aat ctt gct ttt cct ttc tgg atc tcc cca cag aga gaa 480
Ala His Cys Asn Leu Ala Phe Pro Phe Trp Ile Ser Pro Gln Arg Glu
145 150 155 160

```
ctt gat gcc acc gca aca gta ttg gca aac agg caa gat gag agc gaa      528
Leu Asp Ala Thr Ala Thr Val Leu Ala Asn Arg Gln Asp Glu Ser Glu
                165                 170                 175

cag tcc aga aag cgg ctc att gag cag agc cga gaa ttc aag aag aac      576
Gln Ser Arg Lys Arg Leu Ile Glu Gln Ser Arg Glu Phe Lys Lys Asn
            180                 185                 190

act cca gag gat tta cgc aag cag gta gca cca ctg ctc aag agc ttc      624
Thr Pro Glu Asp Leu Arg Lys Gln Val Ala Pro Leu Leu Lys Ser Phe
        195                 200                 205

caa ggg gag att gat gca ctg agt aaa agg agc aaa gaa gca gaa gca      672
Gln Gly Glu Ile Asp Ala Leu Ser Lys Arg Ser Lys Glu Ala Glu Ala
    210                 215                 220

gcc ttc ttg act gtg tac aag aga cta att gat gtt cca gat ccc gta      720
Ala Phe Leu Thr Val Tyr Lys Arg Leu Ile Asp Val Pro Asp Pro Val
225                 230                 235                 240

cca gcc ctg gac ctc ggg caa cag ctg gaa ata aaa gtg cag cgt cta      768
Pro Ala Leu Asp Leu Gly Gln Gln Leu Glu Ile Lys Val Gln Arg Leu
                245                 250                 255

cac gac att gaa aca gag aac cag aaa ctt agg gaa aca cta gaa gag      816
His Asp Ile Glu Thr Glu Asn Gln Lys Leu Arg Glu Thr Leu Glu Glu
            260                 265                 270

tac aac aag gag ttt gct gaa gtg aaa aac caa gag gtt acg ata aaa      864
Tyr Asn Lys Glu Phe Ala Glu Val Lys Asn Gln Glu Val Thr Ile Lys
        275                 280                 285

gca ctt aag gag aaa atc cga gaa tac gag cag acc ctg aag agt cag      912
Ala Leu Lys Glu Lys Ile Arg Glu Tyr Glu Gln Thr Leu Lys Ser Gln
    290                 295                 300

gcc gag acc atc gct ctg gag aag gag cag aag cta caa aat gat ttt      960
Ala Glu Thr Ile Ala Leu Glu Lys Glu Gln Lys Leu Gln Asn Asp Phe
305                 310                 315                 320

gca gag aag gag aga aag ctg caa gag aca cag atg tcc acc acc tca     1008
Ala Glu Lys Glu Arg Lys Leu Gln Glu Thr Gln Met Ser Thr Thr Ser
                325                 330                 335

aag ctg gag gaa gct gag cac aaa ctc cag act ctg caa aca gcc ctg     1056
Lys Leu Glu Glu Ala Glu His Lys Leu Gln Thr Leu Gln Thr Ala Leu
            340                 345                 350

gaa aaa act cga aca gaa tta ttt gac ctg aaa acc aaa tac gat gaa     1104
Glu Lys Thr Arg Thr Glu Leu Phe Asp Leu Lys Thr Lys Tyr Asp Glu
        355                 360                 365

gaa acc act gca aat ggg cct ggg gtg gag aag gag ggt cgt gga aca     1152
Glu Thr Thr Ala Asn Gly Pro Gly Val Glu Lys Glu Gly Arg Gly Thr
    370                 375                 380

cct tct aga cca cca ggg cag agt tca gtg aag aca agt tta aat tgc     1200
Pro Ser Arg Pro Pro Gly Gln Ser Ser Val Lys Thr Ser Leu Asn Cys
385                 390                 395                 400

gat gaa aag cta gat ccc ttc tac cta agg gcc gac gag atc gag atg     1248
Asp Glu Lys Leu Asp Pro Phe Tyr Leu Arg Ala Asp Glu Ile Glu Met
                405                 410                 415

att atg act gac ctt gaa cga gcc aac cag agg gca gag gtg gca cag     1296
Ile Met Thr Asp Leu Glu Arg Ala Asn Gln Arg Ala Glu Val Ala Gln
            420                 425                 430

aga gag gca gag act tta agg gaa cag ctt tca tcg gcc aac cac tct     1344
Arg Glu Ala Glu Thr Leu Arg Glu Gln Leu Ser Ser Ala Asn His Ser
        435                 440                 445

ctc caa ctg gcc tcg cag atc cag aag gct cca gat gtg gag cag gcc     1392
Leu Gln Leu Ala Ser Gln Ile Gln Lys Ala Pro Asp Val Glu Gln Ala
    450                 455                 460
```

```
ata gag gtg ctg acc cga tcc agc ctg gaa gta gag ttg gct gcc aaa     1440
Ile Glu Val Leu Thr Arg Ser Ser Leu Glu Val Glu Leu Ala Ala Lys
465                 470                 475                 480

gag cgg gag att gcc cag ctg gtg gaa gat gtg cag cga ctc cag gcc     1488
Glu Arg Glu Ile Ala Gln Leu Val Glu Asp Val Gln Arg Leu Gln Ala
                485                 490                 495

agc ctc acc aag ctg cgt gag aat tcc gcc agc cag atc tca cag ctg     1536
Ser Leu Thr Lys Leu Arg Glu Asn Ser Ala Ser Gln Ile Ser Gln Leu
            500                 505                 510

gag cag cag ctg aac gcc aag aat agc aca ctc aaa gac tca acc aag     1584
Glu Gln Gln Leu Asn Ala Lys Asn Ser Thr Leu Lys Asp Ser Thr Lys
        515                 520                 525

ccc ctg gag gtt tta ctc ctg gag aag aac cgc tcg ctg cag tcc gag     1632
Pro Leu Glu Val Leu Leu Leu Glu Lys Asn Arg Ser Leu Gln Ser Glu
    530                 535                 540

aat gcc acg ctg cgc atc tcc aac agt gac ctg agc gga aag ttt gca     1680
Asn Ala Thr Leu Arg Ile Ser Asn Ser Asp Leu Ser Gly Lys Phe Ala
545                 550                 555                 560

cta aac tct ctt ctc cag cga cag cta atg cag tcc ttc tac tcc aag     1728
Leu Asn Ser Leu Leu Gln Arg Gln Leu Met Gln Ser Phe Tyr Ser Lys
                565                 570                 575

gcc atg cag gaa gcc gga agc aca agc acc att ttt tca aca ggt cct     1776
Ala Met Gln Glu Ala Gly Ser Thr Ser Thr Ile Phe Ser Thr Gly Pro
            580                 585                 590

tac agc aca aac tcc ata tct tcc cca agt cca tta caa caa agc cca     1824
Tyr Ser Thr Asn Ser Ile Ser Ser Pro Ser Pro Leu Gln Gln Ser Pro
        595                 600                 605

gat gta aat ggc atg gcc cca tct ccc agc caa tca gaa agt gct ggg     1872
Asp Val Asn Gly Met Ala Pro Ser Pro Ser Gln Ser Glu Ser Ala Gly
    610                 615                 620

agc atc tca gag ggt gag gaa att gat aca gca gaa atc gcc cgg cag     1920
Ser Ile Ser Glu Gly Glu Glu Ile Asp Thr Ala Glu Ile Ala Arg Gln
625                 630                 635                 640

gtc aaa gag caa ctg atc aag cac aac att gga cag cgc att ttt gga     1968
Val Lys Glu Gln Leu Ile Lys His Asn Ile Gly Gln Arg Ile Phe Gly
                645                 650                 655

cat tac gtc ttg gga cta tct caa ggg tct gtg agt gag att ctg gcc     2016
His Tyr Val Leu Gly Leu Ser Gln Gly Ser Val Ser Glu Ile Leu Ala
            660                 665                 670

cgg ccg aag ccc tgg aat aag ctg act gtc cgt ggc aag gag cca ttc     2064
Arg Pro Lys Pro Trp Asn Lys Leu Thr Val Arg Gly Lys Glu Pro Phe
        675                 680                 685

cac aaa atg aag cag ttc ttg tct gat gag cag aac atc ctg gca ctt     2112
His Lys Met Lys Gln Phe Leu Ser Asp Glu Gln Asn Ile Leu Ala Leu
    690                 695                 700

cga agc atc caa ggc aga cag aga gag aat cca ggc cag agc ctg aac     2160
Arg Ser Ile Gln Gly Arg Gln Arg Glu Asn Pro Gly Gln Ser Leu Asn
705                 710                 715                 720

aga ctc ttt cag gaa gta ccg aaa cga aga aat ggg tct gaa ggt aac     2208
Arg Leu Phe Gln Glu Val Pro Lys Arg Arg Asn Gly Ser Glu Gly Asn
                725                 730                 735

atc acg acc cgg atc cga gca tct gag act ggt tct gat gaa gcc atc     2256
Ile Thr Thr Arg Ile Arg Ala Ser Glu Thr Gly Ser Asp Glu Ala Ile
            740                 745                 750

aag tcc atc cta gaa caa gcc aag agg gaa ctc caa gtg cag aaa acc     2304
Lys Ser Ile Leu Glu Gln Ala Lys Arg Glu Leu Gln Val Gln Lys Thr
        755                 760                 765
```

```
gct gag cca gtc cag gcg tct tcc aca gcc agc agt ggg aac tcc gac      2352
Ala Glu Pro Val Gln Ala Ser Ser Thr Ala Ser Ser Gly Asn Ser Asp
    770                 775                 780

gat gcc atc cgc tcc atc cta cag cag gcc cgc cgg gaa atg gag gcc      2400
Asp Ala Ile Arg Ser Ile Leu Gln Gln Ala Arg Arg Glu Met Glu Ala
785                 790                 795                 800

cag cag gct gcc ctt gac ccc gcc tta aag cca gca cca ctg tcc cag      2448
Gln Gln Ala Ala Leu Asp Pro Ala Leu Lys Pro Ala Pro Leu Ser Gln
                805                 810                 815

ccc gac ctc acc atc ctc aac ccc aag ctc ctg tct gcc tca ccc atg      2496
Pro Asp Leu Thr Ile Leu Asn Pro Lys Leu Leu Ser Ala Ser Pro Met
            820                 825                 830

tct act gtg tcc act tac ccc cct ctt gcc atc tcc ctg aag aaa acc      2544
Ser Thr Val Ser Thr Tyr Pro Pro Leu Ala Ile Ser Leu Lys Lys Thr
        835                 840                 845

cct gca gca cct gag gcc agt acc tca gcc ttg ccc agt gcc cca gcc      2592
Pro Ala Ala Pro Glu Ala Ser Thr Ser Ala Leu Pro Ser Ala Pro Ala
    850                 855                 860

ctc aaa aaa gag gct cag gat gca ccc aca ctg gac cca cca gga tca      2640
Leu Lys Lys Glu Ala Gln Asp Ala Pro Thr Leu Asp Pro Pro Gly Ser
865                 870                 875                 880

gct gat gct acc ccg ggg gta ctg agg ccg gtg aag aat gag cta gtc      2688
Ala Asp Ala Thr Pro Gly Val Leu Arg Pro Val Lys Asn Glu Leu Val
                885                 890                 895

cgt ggc agc acc tgg aag gat cct tgg tgg aac cct gta cag cct gaa      2736
Arg Gly Ser Thr Trp Lys Asp Pro Trp Trp Asn Pro Val Gln Pro Glu
            900                 905                 910

aga aga aat ctt acc act tcc gaa gag acc aag gcc gat gaa aca aat      2784
Arg Arg Asn Leu Thr Thr Ser Glu Glu Thr Lys Ala Asp Glu Thr Asn
        915                 920                 925

gca agt ggg aaa gaa aag aca ggc agc agc caa cct cgg gct gag cgc      2832
Ala Ser Gly Lys Glu Lys Thr Gly Ser Ser Gln Pro Arg Ala Glu Arg
    930                 935                 940

agc cag ctt cag gga ccc tcg gcg aca gca gag tac tgg aaa gag tgg      2880
Ser Gln Leu Gln Gly Pro Ser Ala Thr Ala Glu Tyr Trp Lys Glu Trp
945                 950                 955                 960

ccc aac gct gag tcc cca tac tcc cag agc tca gag ctg agc ctc act      2928
Pro Asn Ala Glu Ser Pro Tyr Ser Gln Ser Ser Glu Leu Ser Leu Thr
                965                 970                 975

gga gcc agc cgc agt gag aca ccc cag aat agc cca ctg ccc tct tcc      2976
Gly Ala Ser Arg Ser Glu Thr Pro Gln Asn Ser Pro Leu Pro Ser Ser
            980                 985                 990

cca att gtg ccc atg gca aag ccg  gcc aag cct tcc gtg  ccc ccg ctg    3024
Pro Ile Val Pro Met Ala Lys Pro  Ala Lys Pro Ser Val  Pro Pro Leu
        995                 1000                 1005

act cct  gag cag tat gag gtc  tac atg tat cag gaa  gtg gac acc      3069
Thr Pro  Glu Gln Tyr Glu Val  Tyr Met Tyr Gln Glu  Val Asp Thr
    1010                 1015                 1020

atc gag  ctc act cgg cag gtc  aaa gag aag ttg gcc  aaa aat ggc      3114
Ile Glu  Leu Thr Arg Gln Val  Lys Glu Lys Leu Ala  Lys Asn Gly
    1025                 1030                 1035

att tgc  cag agg atc ttt ggg  gag aag gta ctg ggc  ctg tcc cag      3159
Ile Cys  Gln Arg Ile Phe Gly  Glu Lys Val Leu Gly  Leu Ser Gln
    1040                 1045                 1050

ggc agc  gtc agc gac atg ctc  tca agg cca aaa cca  tgg agc aaa      3204
Gly Ser  Val Ser Asp Met Leu  Ser Arg Pro Lys Pro  Trp Ser Lys
    1055                 1060                 1065
```

```
ctg acc cag aaa ggc cga gaa ccc ttc atc cgg atg cag ctc tgg     3249
Leu Thr Gln Lys Gly Arg Glu Pro Phe Ile Arg Met Gln Leu Trp
    1070                1075                1080

ctg aat gga gag ctg ggc caa ggt gtt ctg cct gtg caa ggg caa     3294
Leu Asn Gly Glu Leu Gly Gln Gly Val Leu Pro Val Gln Gly Gln
    1085                1090                1095

caa caa gga cca gtc ctt cac tca gtg acg tct ctg cag gac ccc     3339
Gln Gln Gly Pro Val Leu His Ser Val Thr Ser Leu Gln Asp Pro
    1100                1105                1110

ctc cag cag ggc tgt gtg agt tca gaa agc act cca aag acc tct     3384
Leu Gln Gln Gly Cys Val Ser Ser Glu Ser Thr Pro Lys Thr Ser
    1115                1120                1125

gcc agc tgc agc cct gcc cct gag tcc cca atg agt tcc agc gaa     3429
Ala Ser Cys Ser Pro Ala Pro Glu Ser Pro Met Ser Ser Ser Glu
    1130                1135                1140

tct gtg aag agt ctc act gag ctg gtc caa cag ccc tgt ccc acc     3474
Ser Val Lys Ser Leu Thr Glu Leu Val Gln Gln Pro Cys Pro Thr
    1145                1150                1155

att gag acc agt aaa gaa ggc aaa cca cca gaa ccc agt gac cca     3519
Ile Glu Thr Ser Lys Glu Gly Lys Pro Pro Glu Pro Ser Asp Pro
    1160                1165                1170

ccc acc tca gac tcc caa ccc aca aca ccg ctg cct ctc tct gga     3564
Pro Thr Ser Asp Ser Gln Pro Thr Thr Pro Leu Pro Leu Ser Gly
    1175                1180                1185

cac tca gcc ctc agc atc caa gaa tta gta gcc atg tct cct gag     3609
His Ser Ala Leu Ser Ile Gln Glu Leu Val Ala Met Ser Pro Glu
    1190                1195                1200

ctg gac acc tat ggc ata acc aag agg gtc aaa gag gtg ctg acg     3654
Leu Asp Thr Tyr Gly Ile Thr Lys Arg Val Lys Glu Val Leu Thr
    1205                1210                1215

gac aac aac ctc ggt cag cgc tta ttt gga gag acc att ctg ggg     3699
Asp Asn Asn Leu Gly Gln Arg Leu Phe Gly Glu Thr Ile Leu Gly
    1220                1225                1230

ctc acc cag ggt tct gtc tcc gac ctc ctt gcc cgc cca aag ccc     3744
Leu Thr Gln Gly Ser Val Ser Asp Leu Leu Ala Arg Pro Lys Pro
    1235                1240                1245

tgg cat aag ctt agt ctg aaa gga cgg gag ccc ttt gtc cgc atg     3789
Trp His Lys Leu Ser Leu Lys Gly Arg Glu Pro Phe Val Arg Met
    1250                1255                1260

cag cta tgg ctc aac gac ccc aac aat gtg gag aag ctg atg gat     3834
Gln Leu Trp Leu Asn Asp Pro Asn Asn Val Glu Lys Leu Met Asp
    1265                1270                1275

atg aag cgg atg gag aag aaa gcc tac atg aag agg cga cac agc     3879
Met Lys Arg Met Glu Lys Lys Ala Tyr Met Lys Arg Arg His Ser
    1280                1285                1290

tct gtc agt gac agt cag cct tgt gaa ccc ccc tct gtt ggt atc     3924
Ser Val Ser Asp Ser Gln Pro Cys Glu Pro Pro Ser Val Gly Ile
    1295                1300                1305

gac tat agc cag ggt gcc agc ccc cag cca caa cac cag ctg aag     3969
Asp Tyr Ser Gln Gly Ala Ser Pro Gln Pro Gln His Gln Leu Lys
    1310                1315                1320

aaa cct cga gtg gtc ctg gct cca gag gag aag gaa gca ctg aag     4014
Lys Pro Arg Val Val Leu Ala Pro Glu Glu Lys Glu Ala Leu Lys
    1325                1330                1335

cga gca tat cag cag aag cca tac cca tca cca aaa acc atc gag     4059
Arg Ala Tyr Gln Gln Lys Pro Tyr Pro Ser Pro Lys Thr Ile Glu
    1340                1345                1350
```

```
gag ctt gcc aca caa ctc aac ctg aag act agc acc gtc atc aac         4104
Glu Leu Ala Thr Gln Leu Asn Leu Lys Thr Ser Thr Val Ile Asn
    1355                1360                1365

tgg ttc cat aat tac agg tct cgg atc cgc aga gaa ctg ttc att         4149
Trp Phe His Asn Tyr Arg Ser Arg Ile Arg Arg Glu Leu Phe Ile
    1370                1375                1380

gag gag att caa gcc gga agc cag ggc cag gcc ggt gcc agc gac         4194
Glu Glu Ile Gln Ala Gly Ser Gln Gly Gln Ala Gly Ala Ser Asp
    1385                1390                1395

tca cct tca gct cga agt agc cgg gct gca ccc agc tct gaa ggc         4239
Ser Pro Ser Ala Arg Ser Ser Arg Ala Ala Pro Ser Ser Glu Gly
    1400                1405                1410

gac agt tgt gac ggc gtg gaa gcc gct gac acc gag gag cca ggt         4284
Asp Ser Cys Asp Gly Val Glu Ala Ala Asp Thr Glu Glu Pro Gly
    1415                1420                1425

ggc aac ata gtg gcc acc aag tct cag gga ggg ccg gca gag gtg         4329
Gly Asn Ile Val Ala Thr Lys Ser Gln Gly Gly Pro Ala Glu Val
    1430                1435                1440

acc gca gcc ccg gca gac cgg gaa gaa gcg acg cag ccg gcc gag         4374
Thr Ala Ala Pro Ala Asp Arg Glu Glu Ala Thr Gln Pro Ala Glu
    1445                1450                1455

aag gcg aag gca cag cca cta tcc tcg ggg acc cca gga caa gac         4419
Lys Ala Lys Ala Gln Pro Leu Ser Ser Gly Thr Pro Gly Gln Asp
    1460                1465                1470

gac ggt gaa gac gca ggc cgg tct agg cca cct cct gag ggt ctt         4464
Asp Gly Glu Asp Ala Gly Arg Ser Arg Pro Pro Pro Glu Gly Leu
    1475                1480                1485

gct gac gcc cca gcg ccc gtg cca aac ctc gcc gca ccc gcg gcc         4509
Ala Asp Ala Pro Ala Pro Val Pro Asn Leu Ala Ala Pro Ala Ala
    1490                1495                1500

gga gag gac gcc gct acc tca gcc act gcc cca gcc atg gcc act         4554
Gly Glu Asp Ala Ala Thr Ser Ala Thr Ala Pro Ala Met Ala Thr
    1505                1510                1515

gag gcc cct ggg gcg gcc agg gcc ggc cct gcc gag agg agt tct         4599
Glu Ala Pro Gly Ala Ala Arg Ala Gly Pro Ala Glu Arg Ser Ser
    1520                1525                1530

gcg ttg cca agc acc agc gcg cct gcc aac gcg cct gcg cga agg         4644
Ala Leu Pro Ser Thr Ser Ala Pro Ala Asn Ala Pro Ala Arg Arg
    1535                1540                1545

cct agc tct ctg cag agc ctc ttt ggt ctg cct gag gca gcg ggc         4689
Pro Ser Ser Leu Gln Ser Leu Phe Gly Leu Pro Glu Ala Ala Gly
    1550                1555                1560

gcc cgg gac aac ccg gtg cgg aag aag aag gcc gcg aac ttg aac         4734
Ala Arg Asp Asn Pro Val Arg Lys Lys Lys Ala Ala Asn Leu Asn
    1565                1570                1575

agc atc atc cac cgc ctg gag aag gct gcc agc cgg gag gag ccc         4779
Ser Ile Ile His Arg Leu Glu Lys Ala Ala Ser Arg Glu Glu Pro
    1580                1585                1590

att gaa tgg gag ttc tgagggcgcc gggcctctgg gcctgaccag cagggcctcg     4834
Ile Glu Trp Glu Phe
    1595

ccgggctagc caggcctact tgcgcctagc aggtcccccca ggcctagtca gcactggcta  4894

gtggtgggct ggggcaggcg ggtgcgtgga gggctgtgct gacccgaggc ctggacgtgt   4954

tgcgcactta ccgccctgcg ggccacaggg caaaatcgcc ataggccaag gtgcatatag   5014

aaaacaaagg agcattaagc ccaatctatg ttgtgttttc aaggaagaaa acggaaatgt   5074
```

gtagtcaagc ttttttgtac cctgaagtgt ttttttttt taattgccct aagtgatttc 5134

cacaggttct ggaataactc tttacagctt ttgccttgcg ccctcctctt tcgtgtgggc 5194

tttaaaaaaa aaaaaaatca aacccacata ttaaaagggg gctttttatc tgccatctaa 5254

tggcttcaga gcgataatac actattatct tcttaaacca ggaagggggg gattttttca 5314

gaaaaattaa aaaagaaagt ttttgtagct gttcagttgc cactaagaga ttgcacagtc 5374

aaaccgactc taaacacact agtttggatt cctaaatatt ttcaagaaaa gaatcttctc 5434

gtttgaaact ttgaattaaa ataaaacaca tttactccac atatttttta aca 5487

<210> 91
<211> 1598
<212> PRT
<213> Rattus norvegicus

<400> 91

Met Ile Pro Ser Tyr Pro Val Val Leu His Gly His Val Ala Thr Leu
1 5 10 15

Cys Gln Gln Phe Leu Lys Glu Leu Asn Thr His Ile Arg Ala Thr Lys
20 25 30

Gly Cys Phe Leu Asp Gly Leu Glu Ala Thr Thr Arg Tyr Glu Ser Thr
35 40 45

Ala Lys Ser Lys Thr Ser Asp Gly His Thr Gln Ser Glu Asn Ser Pro
50 55 60

Phe Asp Pro Phe Tyr Thr Glu Val Cys Arg Ser Ala Ala Leu Gln Met
65 70 75 80

Val Leu Ser Cys Pro Pro Leu Val Leu Gly Tyr Thr Asp Lys Val Pro
85 90 95

Ser Val Ser Glu Ala Val His Val Leu Ser His Arg Thr Gln Phe Arg
100 105 110

Ala Ser His Val Gln Phe His Trp Gly Lys His Arg Ala Arg His Val
115 120 125

Asp His Asn Ser Gly Pro Ile His Thr Ser Ser Leu Leu Met Glu Thr
130 135 140

Ala His Cys Asn Leu Ala Phe Pro Phe Trp Ile Ser Pro Gln Arg Glu
145 150 155 160

Leu Asp Ala Thr Ala Thr Val Leu Ala Asn Arg Gln Asp Glu Ser Glu
165 170 175

Gln Ser Arg Lys Arg Leu Ile Glu Gln Ser Arg Glu Phe Lys Lys Asn
180 185 190

Thr Pro Glu Asp Leu Arg Lys Gln Val Ala Pro Leu Leu Lys Ser Phe
195 200 205

Gln Gly Glu Ile Asp Ala Leu Ser Lys Arg Ser Lys Glu Ala Glu Ala
210 215 220

Ala Phe Leu Thr Val Tyr Lys Arg Leu Ile Asp Val Pro Asp Pro Val
225 230 235 240

Pro Ala Leu Asp Leu Gly Gln Gln Leu Glu Ile Lys Val Gln Arg Leu
245 250 255

His Asp Ile Glu Thr Glu Asn Gln Lys Leu Arg Glu Thr Leu Glu Glu
260 265 270

Tyr Asn Lys Glu Phe Ala Glu Val Lys Asn Gln Glu Val Thr Ile Lys
275 280 285

Ala Leu Lys Glu Lys Ile Arg Glu Tyr Glu Gln Thr Leu Lys Ser Gln
290 295 300

Ala Glu Thr Ile Ala Leu Glu Lys Glu Gln Lys Leu Gln Asn Asp Phe
305 310 315 320

Ala Glu Lys Glu Arg Lys Leu Gln Glu Thr Gln Met Ser Thr Thr Ser
325 330 335

Lys Leu Glu Glu Ala Glu His Lys Leu Gln Thr Leu Gln Thr Ala Leu
340 345 350

Glu Lys Thr Arg Thr Glu Leu Phe Asp Leu Lys Thr Lys Tyr Asp Glu
355 360 365

Glu Thr Thr Ala Asn Gly Pro Gly Val Glu Lys Glu Gly Arg Gly Thr
370 375 380

Pro Ser Arg Pro Pro Gly Gln Ser Ser Val Lys Thr Ser Leu Asn Cys
385 390 395 400

Asp Glu Lys Leu Asp Pro Phe Tyr Leu Arg Ala Asp Glu Ile Glu Met
405 410 415

Ile Met Thr Asp Leu Glu Arg Ala Asn Gln Arg Ala Glu Val Ala Gln
420 425 430

Arg Glu Ala Glu Thr Leu Arg Glu Gln Leu Ser Ser Ala Asn His Ser
435 440 445

Leu Gln Leu Ala Ser Gln Ile Gln Lys Ala Pro Asp Val Glu Gln Ala
450 455 460

Ile Glu Val Leu Thr Arg Ser Ser Leu Glu Val Glu Leu Ala Ala Lys
465 470 475 480

Glu Arg Glu Ile Ala Gln Leu Val Glu Asp Val Gln Arg Leu Gln Ala
485 490 495

Ser Leu Thr Lys Leu Arg Glu Asn Ser Ala Ser Gln Ile Ser Gln Leu
500 505 510

Glu Gln Gln Leu Asn Ala Lys Asn Ser Thr Leu Lys Asp Ser Thr Lys
515 520 525

Pro Leu Glu Val Leu Leu Leu Glu Lys Asn Arg Ser Leu Gln Ser Glu
530 535 540

Asn Ala Thr Leu Arg Ile Ser Asn Ser Asp Leu Ser Gly Lys Phe Ala
545 550 555 560

Leu Asn Ser Leu Leu Gln Arg Gln Leu Met Gln Ser Phe Tyr Ser Lys
565 570 575

Ala Met Gln Glu Ala Gly Ser Thr Ser Thr Ile Phe Ser Thr Gly Pro
580 585 590

Tyr Ser Thr Asn Ser Ile Ser Ser Pro Ser Pro Leu Gln Gln Ser Pro
595 600 605

Asp Val Asn Gly Met Ala Pro Ser Pro Ser Gln Ser Glu Ser Ala Gly
610 615 620

Ser Ile Ser Glu Gly Glu Glu Ile Asp Thr Ala Glu Ile Ala Arg Gln
625 630 635 640

Val Lys Glu Gln Leu Ile Lys His Asn Ile Gly Gln Arg Ile Phe Gly
645 650 655

His Tyr Val Leu Gly Leu Ser Gln Gly Ser Val Ser Glu Ile Leu Ala
660 665 670

Arg Pro Lys Pro Trp Asn Lys Leu Thr Val Arg Gly Lys Glu Pro Phe
675 680 685

His Lys Met Lys Gln Phe Leu Ser Asp Glu Gln Asn Ile Leu Ala Leu
690 695 700

Arg Ser Ile Gln Gly Arg Gln Arg Glu Asn Pro Gly Gln Ser Leu Asn
705 710 715 720

Arg Leu Phe Gln Glu Val Pro Lys Arg Arg Asn Gly Ser Glu Gly Asn
725 730 735

Ile Thr Thr Arg Ile Arg Ala Ser Glu Thr Gly Ser Asp Glu Ala Ile
740 745 750

Lys Ser Ile Leu Glu Gln Ala Lys Arg Glu Leu Gln Val Gln Lys Thr
755 760 765

Ala Glu Pro Val Gln Ala Ser Ser Thr Ala Ser Ser Gly Asn Ser Asp
770 775 780

Asp Ala Ile Arg Ser Ile Leu Gln Gln Ala Arg Arg Glu Met Glu Ala
785 790 795 800

Gln Gln Ala Ala Leu Asp Pro Ala Leu Lys Pro Ala Pro Leu Ser Gln
805 810 815

Pro Asp Leu Thr Ile Leu Asn Pro Lys Leu Leu Ser Ala Ser Pro Met
820 825 830

Ser Thr Val Ser Thr Tyr Pro Pro Leu Ala Ile Ser Leu Lys Lys Thr
835 840 845

Pro Ala Ala Pro Glu Ala Ser Thr Ser Ala Leu Pro Ser Ala Pro Ala
850 855 860

Leu Lys Lys Glu Ala Gln Asp Ala Pro Thr Leu Asp Pro Pro Gly Ser
865 870 875 880

Ala Asp Ala Thr Pro Gly Val Leu Arg Pro Val Lys Asn Glu Leu Val
885 890 895

Arg Gly Ser Thr Trp Lys Asp Pro Trp Trp Asn Pro Val Gln Pro Glu
900 905 910

Arg Arg Asn Leu Thr Thr Ser Glu Glu Thr Lys Ala Asp Glu Thr Asn
915 920 925

Ala Ser Gly Lys Glu Lys Thr Gly Ser Ser Gln Pro Arg Ala Glu Arg
930 935 940

Ser Gln Leu Gln Gly Pro Ser Ala Thr Ala Glu Tyr Trp Lys Glu Trp
945 950 955 960

Pro Asn Ala Glu Ser Pro Tyr Ser Gln Ser Ser Glu Leu Ser Leu Thr
965 970 975

Gly Ala Ser Arg Ser Glu Thr Pro Gln Asn Ser Pro Leu Pro Ser Ser
980 985 990

Pro Ile Val Pro Met Ala Lys Pro Ala Lys Pro Ser Val Pro Pro Leu
995 1000 1005

Thr Pro Glu Gln Tyr Glu Val Tyr Met Tyr Gln Glu Val Asp Thr
1010 1015 1020

Ile Glu Leu Thr Arg Gln Val Lys Glu Lys Leu Ala Lys Asn Gly
1025 1030 1035

Ile Cys Gln Arg Ile Phe Gly Glu Lys Val Leu Gly Leu Ser Gln
1040 1045 1050

Gly Ser Val Ser Asp Met Leu Ser Arg Pro Lys Pro Trp Ser Lys
1055 1060 1065

Leu Thr Gln Lys Gly Arg Glu Pro Phe Ile Arg Met Gln Leu Trp
1070 1075 1080

Leu Asn Gly Glu Leu Gly Gln Gly Val Leu Pro Val Gln Gly Gln
1085 1090 1095

Gln Gln Gly Pro Val Leu His Ser Val Thr Ser Leu Gln Asp Pro
1100 1105 1110

Leu Gln Gln Gly Cys Val Ser Ser Glu Ser Thr Pro Lys Thr Ser
1115 1120 1125

Ala Ser Cys Ser Pro Ala Pro Glu Ser Pro Met Ser Ser Ser Glu
1130 1135 1140

Ser Val Lys Ser Leu Thr Glu Leu Val Gln Gln Pro Cys Pro Thr
1145 1150 1155

Ile Glu Thr Ser Lys Glu Gly Lys Pro Pro Glu Pro Ser Asp Pro
1160 1165 1170

Pro Thr Ser Asp Ser Gln Pro Thr Thr Pro Leu Pro Leu Ser Gly
1175 1180 1185

His Ser Ala Leu Ser Ile Gln Glu Leu Val Ala Met Ser Pro Glu
1190 1195 1200

Leu Asp Thr Tyr Gly Ile Thr Lys Arg Val Lys Glu Val Leu Thr
1205 1210 1215

Asp Asn Asn Leu Gly Gln Arg Leu Phe Gly Glu Thr Ile Leu Gly
1220 1225 1230

Leu Thr Gln Gly Ser Val Ser Asp Leu Leu Ala Arg Pro Lys Pro
1235 1240 1245

Trp His Lys Leu Ser Leu Lys Gly Arg Glu Pro Phe Val Arg Met
1250 1255 1260

Gln Leu Trp Leu Asn Asp Pro Asn Asn Val Glu Lys Leu Met Asp
1265 1270 1275

Met Lys Arg Met Glu Lys Lys Ala Tyr Met Lys Arg Arg His Ser
1280 1285 1290

Ser Val Ser Asp Ser Gln Pro Cys Glu Pro Pro Ser Val Gly Ile
1295 1300 1305

Asp Tyr Ser Gln Gly Ala Ser Pro Gln Pro Gln His Gln Leu Lys
1310 1315 1320

Lys Pro Arg Val Val Leu Ala Pro Glu Glu Lys Glu Ala Leu Lys
1325 1330 1335

Arg Ala Tyr Gln Gln Lys Pro Tyr Pro Ser Pro Lys Thr Ile Glu
1340 1345 1350

Glu Leu Ala Thr Gln Leu Asn Leu Lys Thr Ser Thr Val Ile Asn
1355 1360 1365

Trp Phe His Asn Tyr Arg Ser Arg Ile Arg Arg Glu Leu Phe Ile
1370 1375 1380

Glu Glu Ile Gln Ala Gly Ser Gln Gly Gln Ala Gly Ala Ser Asp
1385 1390 1395

Ser Pro Ser Ala Arg Ser Ser Arg Ala Ala Pro Ser Ser Glu Gly
1400 1405 1410

Asp Ser Cys Asp Gly Val Glu Ala Ala Asp Thr Glu Glu Pro Gly
1415 1420 1425

Gly Asn Ile Val Ala Thr Lys Ser Gln Gly Gly Pro Ala Glu Val
1430 1435 1440

Thr Ala Ala Pro Ala Asp Arg Glu Glu Ala Thr Gln Pro Ala Glu
1445 1450 1455

Lys Ala Lys Ala Gln Pro Leu Ser Ser Gly Thr Pro Gly Gln Asp
1460 1465 1470

Asp Gly Glu Asp Ala Gly Arg Ser Arg Pro Pro Pro Glu Gly Leu
1475 1480 1485

Ala Asp Ala Pro Ala Pro Val Pro Asn Leu Ala Ala Pro Ala Ala
1490 1495 1500

Gly Glu Asp Ala Ala Thr Ser Ala Thr Ala Pro Ala Met Ala Thr
1505 1510 1515

Glu Ala Pro Gly Ala Ala Arg Ala Gly Pro Ala Glu Arg Ser Ser
1520 1525 1530

Ala Leu Pro Ser Thr Ser Ala Pro Ala Asn Ala Pro Ala Arg Arg
1535 1540 1545

Pro Ser Ser Leu Gln Ser Leu Phe Gly Leu Pro Glu Ala Ala Gly
1550 1555 1560

Ala Arg Asp Asn Pro Val Arg Lys Lys Lys Ala Ala Asn Leu Asn
1565 1570 1575

Ser Ile Ile His Arg Leu Glu Lys Ala Ala Ser Arg Glu Glu Pro
1580 1585 1590

Ile Glu Trp Glu Phe
1595

<210> 92
<211> 794
<212> DNA
<213> Rattus norvegicus

<220>
<221> CDS
<222> (50)..(682)

<400> 92
tggatgctgt gagggcggcg gcactggtta ctgcgtgtgg cggcccgcg atg aag ccg 58
Met Lys Pro
1

ccc att tca ata caa gca agc gag ttt gat tct tca gac gaa gag cct 106
Pro Ile Ser Ile Gln Ala Ser Glu Phe Asp Ser Ser Asp Glu Glu Pro
5 10 15

gct gac gac gag cag act cca att caa att tca tgg cta cct ctg tca 154
Ala Asp Asp Glu Gln Thr Pro Ile Gln Ile Ser Trp Leu Pro Leu Ser
20 25 30 35

```
cga gtg aat tgt tct cag ttt ctc ggt tta tgt gct ctt cca ggt tgt      202
Arg Val Asn Cys Ser Gln Phe Leu Gly Leu Cys Ala Leu Pro Gly Cys
                40                  45                  50

aaa ttt aaa gat gtt aga aga aac att caa aaa gat aca gag gaa cta      250
Lys Phe Lys Asp Val Arg Arg Asn Ile Gln Lys Asp Thr Glu Glu Leu
            55                  60                  65

aag agc tct ggg atc caa gac gta ttt gtt ttc tgc acc aga ggg gaa      298
Lys Ser Ser Gly Ile Gln Asp Val Phe Val Phe Cys Thr Arg Gly Glu
        70                  75                  80

ctg tca aaa tat aga gtc cca aac ctt ctg gac ctc tac caa caa tat      346
Leu Ser Lys Tyr Arg Val Pro Asn Leu Leu Asp Leu Tyr Gln Gln Tyr
    85                  90                  95

gga att gtc acc cat cat cat ccg atc cca gat gga ggg act ccc gac      394
Gly Ile Val Thr His His His Pro Ile Pro Asp Gly Gly Thr Pro Asp
100                 105                 110                 115

att ggc agc tgc tgg gaa atc atg gag gag ctg gcc acc tgc ctt aaa      442
Ile Gly Ser Cys Trp Glu Ile Met Glu Glu Leu Ala Thr Cys Leu Lys
                120                 125                 130

aac aac cga aaa acc ctg ata cac tgt tat gga gga ctt gga aga tct      490
Asn Asn Arg Lys Thr Leu Ile His Cys Tyr Gly Gly Leu Gly Arg Ser
            135                 140                 145

tgt ctt gct gct tgt ctc ctc cta tac ttg tct gac tca att tca cca      538
Cys Leu Ala Ala Cys Leu Leu Leu Tyr Leu Ser Asp Ser Ile Ser Pro
        150                 155                 160

cag caa gcc ata gac agc ctt cga gat gtc agg gga tct ggg gcg ata      586
Gln Gln Ala Ile Asp Ser Leu Arg Asp Val Arg Gly Ser Gly Ala Ile
    165                 170                 175

cag acc atc aag caa tat aac tat ctt cat gaa ttc cgg gac aag tta      634
Gln Thr Ile Lys Gln Tyr Asn Tyr Leu His Glu Phe Arg Asp Lys Leu
180                 185                 190                 195

gct gcc tat ctg tca tca aga gat tca ctg tca aga tcc gtg tcc aga      682
Ala Ala Tyr Leu Ser Ser Arg Asp Ser Leu Ser Arg Ser Val Ser Arg
                200                 205                 210

taaggaagag cccagacacg cagccgcagc tgaagttcca agttctctag cgtaatctgt    742

attggagtgc tactgccatc ctgaacataa atgttatgtg cagacttcta aa            794


<210>  93
<211>  211
<212>  PRT
<213>  Rattus norvegicus

<400>  93

Met Lys Pro Pro Ile Ser Ile Gln Ala Ser Glu Phe Asp Ser Ser Asp
1               5                   10                  15

Glu Glu Pro Ala Asp Asp Glu Gln Thr Pro Ile Gln Ile Ser Trp Leu
            20                  25                  30

Pro Leu Ser Arg Val Asn Cys Ser Gln Phe Leu Gly Leu Cys Ala Leu
        35                  40                  45

Pro Gly Cys Lys Phe Lys Asp Val Arg Arg Asn Ile Gln Lys Asp Thr
    50                  55                  60

Glu Glu Leu Lys Ser Ser Gly Ile Gln Asp Val Phe Val Phe Cys Thr
65                  70                  75                  80
```

```
Arg Gly Glu Leu Ser Lys Tyr Arg Val Pro Asn Leu Leu Asp Leu Tyr
                85                  90                  95

Gln Gln Tyr Gly Ile Val Thr His His His Pro Ile Pro Asp Gly Gly
            100                 105                 110

Thr Pro Asp Ile Gly Ser Cys Trp Glu Ile Met Glu Glu Leu Ala Thr
        115                 120                 125

Cys Leu Lys Asn Asn Arg Lys Thr Leu Ile His Cys Tyr Gly Gly Leu
    130                 135                 140

Gly Arg Ser Cys Leu Ala Ala Cys Leu Leu Leu Tyr Leu Ser Asp Ser
145                 150                 155                 160

Ile Ser Pro Gln Gln Ala Ile Asp Ser Leu Arg Asp Val Arg Gly Ser
                165                 170                 175

Gly Ala Ile Gln Thr Ile Lys Gln Tyr Asn Tyr Leu His Glu Phe Arg
            180                 185                 190

Asp Lys Leu Ala Ala Tyr Leu Ser Ser Arg Asp Ser Leu Ser Arg Ser
        195                 200                 205

Val Ser Arg
    210


<210>  94
<211>  426
<212>  DNA
<213>  Rattus norvegicus


<220>
<221>  CDS
<222>  (1)..(423)

<400>  94
atg tcg gag gtg cgg ctg cca ccg ctg cgc gcc cta gac gac ttt gtc       48
Met Ser Glu Val Arg Leu Pro Pro Leu Arg Ala Leu Asp Asp Phe Val
1               5                   10                  15

ctg ggg tct gca cgt ttg gca gct cct gat cta ggc gat cca caa cga       96
Leu Gly Ser Ala Arg Leu Ala Ala Pro Asp Leu Gly Asp Pro Gln Arg
            20                  25                  30

tgg tgt cac cgc gtc atc aac aac ctc ctc tac tac caa acc aat tac      144
Trp Cys His Arg Val Ile Asn Asn Leu Leu Tyr Tyr Gln Thr Asn Tyr
        35                  40                  45

ctt ctc tgc ttt ggc atc agt ctc gct ctg gcc ggt cac cct gct gcc      192
Leu Leu Cys Phe Gly Ile Ser Leu Ala Leu Ala Gly His Pro Ala Ala
    50                  55                  60

tgc ctg gct gca gta ctt gcc gtt agc ctc ttc att ctc tgg gcc gtg      240
Cys Leu Ala Ala Val Leu Ala Val Ser Leu Phe Ile Leu Trp Ala Val
65                  70                  75                  80

ggc ggc gct ttc acc ttc ctg ctc tgc atc aca gcg cct gtg ctt ttg      288
Gly Gly Ala Phe Thr Phe Leu Leu Cys Ile Thr Ala Pro Val Leu Leu
                85                  90                  95

atc ctg ctg cat gct tca ctg cgc ctg aga aac ctt aaa aac aag att      336
Ile Leu Leu His Ala Ser Leu Arg Leu Arg Asn Leu Lys Asn Lys Ile
            100                 105                 110
```

```
gag aac aag atc gag agc atc ggt cta aag cgg aca cca atg gga ctg      384
Glu Asn Lys Ile Glu Ser Ile Gly Leu Lys Arg Thr Pro Met Gly Leu
        115                 120                 125

ctt cta gag gca ctg gga cag gag cag gaa gct gga tcc tag              426
Leu Leu Glu Ala Leu Gly Gln Glu Gln Glu Ala Gly Ser
    130                 135                 140
```

<210> 95
<211> 141
<212> PRT
<213> Rattus norvegicus

<400> 95

```
Met Ser Glu Val Arg Leu Pro Pro Leu Arg Ala Leu Asp Asp Phe Val
1               5                   10                  15

Leu Gly Ser Ala Arg Leu Ala Ala Pro Asp Leu Gly Asp Pro Gln Arg
            20                  25                  30

Trp Cys His Arg Val Ile Asn Asn Leu Leu Tyr Tyr Gln Thr Asn Tyr
        35                  40                  45

Leu Leu Cys Phe Gly Ile Ser Leu Ala Leu Ala Gly His Pro Ala Ala
    50                  55                  60

Cys Leu Ala Ala Val Leu Ala Val Ser Leu Phe Ile Leu Trp Ala Val
65                  70                  75                  80

Gly Gly Ala Phe Thr Phe Leu Leu Cys Ile Thr Ala Pro Val Leu Leu
                85                  90                  95

Ile Leu Leu His Ala Ser Leu Arg Leu Arg Asn Leu Lys Asn Lys Ile
            100                 105                 110

Glu Asn Lys Ile Glu Ser Ile Gly Leu Lys Arg Thr Pro Met Gly Leu
        115                 120                 125

Leu Leu Glu Ala Leu Gly Gln Glu Gln Glu Ala Gly Ser
    130                 135                 140
```

<210> 96
<211> 2210
<212> DNA
<213> Rattus norvegicus

<220>
<221> CDS
<222> (213)..(497)

<400> 96

```
ggaaacctca tggccaagtt gtagaaaaaa tagttgaagt gtgcctatct tggagcggag      60

cccctccctt ctccagccgc gcggagtggg agccctaggt gcaaccaagt agccgggttc     120

acggtccaga agctggtgac cctttcctac ccaccccacc ccaccccccgc ctcagtgcgc    180

cccaccgcgt ttccccctgc cgccgcgtgg cc atg agc agc ccc gtc aga acg       233
                                    Met Ser Ser Pro Val Arg Thr
                                    1               5

cca cct cct gag cgc ctg gag ccc aac cca ggg atg tcc tat gcc gtt      281
Pro Pro Pro Glu Arg Leu Glu Pro Asn Pro Gly Met Ser Tyr Ala Val
        10                  15                  20
```

```
atg gag att gcc atc att gca gct gtg atc act gcg gtg gcc ttg gtc      329
Met Glu Ile Ala Ile Ile Ala Ala Val Ile Thr Ala Val Ala Leu Val
    25                  30                  35

ctg gtc tgc ctc ctc ttt ctc atg ctt cgc tac ctg tac agg cac aag      377
Leu Val Cys Leu Leu Phe Leu Met Leu Arg Tyr Leu Tyr Arg His Lys
40                  45                  50                  55

ggc act tac tac acc aat gag gcc aaa ggc acg gag ttt gca gaa agt      425
Gly Thr Tyr Tyr Thr Asn Glu Ala Lys Gly Thr Glu Phe Ala Glu Ser
                60                  65                  70

gcg gat gca gct ctg caa agt gac cct gcc ctc cag gat gcc ggg gac      473
Ala Asp Ala Ala Leu Gln Ser Asp Pro Ala Leu Gln Asp Ala Gly Asp
            75                  80                  85

acc agc aag aag gaa tac ttt atc tgaggggtag cagacttgac atccctgcaa     527
Thr Ser Lys Lys Glu Tyr Phe Ile
        90                  95

gacgcttctg gctgcaggag agaaagtacc cccgctgtgt gccaccatta tcagcaccag    587

gaagccctca agggcacccc aagactctta ctgcagaaca ctgggtgcct gccaggaaat    647

cgatacagat gggaggaatc aaggtgcagc gtacctctcc cagggactca ttggccaccc    707

aataccattg gctccgcaag gaagggagaa catctggatg cacaggtctc cctgggcaat    767

gcaggtgtcg tctcactagc atgaaaatcc tcctcagtgt gtgtgaggtg gtttggacct    827

gggagtaagt tgtatggaag tgggacatct ggaagctgcc tgaagataaa ggttccaagt    887

gtcaatcatt gacatttggg aagactagga ggtgccacag ctaaaggca ccttcgtttc     947

ccatcaacgc agctcaagat gactacagat aagtttgaga cgtaactgag cattctatat   1007

caaatggcac tacgcttaag tgtttgggaa acataaatgc cacccatcgc tggcttttct   1067

ccatatagca ttgtgtctat gtgcgcggga caaggactgt ctagttctgc caaaagaaga   1127

aagggaaggg tgacctttcc cctcactagt accagcttat atgaaaccaa cggaaagagg   1187

ctacccagta atcaggccag gggatgaagc actggtttct tgatacacag tttttaaaag   1247

gagagctgtg ttcatcaact gtgagtgaag tccgtcatgc cagagtccca gtgaaaatgc   1307

ctctatgtta taagagactg caggtcccca gacctggctt tggctcctct ttcttggtct   1367

taggacctta agccatgtat ttgacctctc cagctttaat gttggcgttt tcaagtggaa   1427

gtgacactaa attgtgttac ctctggttct taactcccag cattgtgggc aggagcagaa   1487

gcatcagaca tggggtgtta actgtattcc agtgtcagta gcatactaga tacatggtta   1547

gcctatgttg gccaataaca attcatcttt gcaccacctc taagagcctc ctgagaagtc   1607

gctaatttaa aattaataac agggtgaata tgagctaatt agccaacccc ttccaggtaa   1667

gatttccagc ggttctgcag tcactcacaa gactttgagt cattgctaca tttaaagacc   1727

atccctcagg agccccgtca gcagcgctgt ctccacctag tagccacatc gtggtccctg   1787

ggagactaat gagattgcaa gctcagtcaa gtatggccgc caccagccga aggagcttca   1847

attcctcaga atgtcgtggg gaagagaagc agtgattcag ttataacaag gagttataat   1907

ggtcaagtgc cagatgaccc agactggagg atgtctatgt tctcacctat gaagtaaaag   1967

gctggcttca gttgacaccc actaatctag agagacctta tctgaacccc tagtggatgc   2027

ttgaaaccca aggcagtatt gaacccettt gtatttggct tttctgcaca tacttatgtt   2087

aagatttaat ttataaatta ggcacagtaa gagattaact gcagtaacta acagcacaat   2147

ggcataatta gaactataaa gctaaaataa aagattcttg aaactaaaaa aaaaaaaaaa   2207
```

aaa 2210

<210> 97
<211> 95
<212> PRT
<213> Rattus norvegicus

<400> 97

Met Ser Ser Pro Val Arg Thr Pro Pro Pro Glu Arg Leu Glu Pro Asn
1 5 10 15

Pro Gly Met Ser Tyr Ala Val Met Glu Ile Ala Ile Ile Ala Ala Val
20 25 30

Ile Thr Ala Val Ala Leu Val Leu Val Cys Leu Leu Phe Leu Met Leu
35 40 45

Arg Tyr Leu Tyr Arg His Lys Gly Thr Tyr Tyr Thr Asn Glu Ala Lys
50 55 60

Gly Thr Glu Phe Ala Glu Ser Ala Asp Ala Ala Leu Gln Ser Asp Pro
65 70 75 80

Ala Leu Gln Asp Ala Gly Asp Thr Ser Lys Lys Glu Tyr Phe Ile
85 90 95

<210> 98
<211> 2743
<212> DNA
<213> Rattus norvegicus

<220>
<221> CDS
<222> (1)..(2424)

<400> 98
atg tca gga ctg aga gag tct aag cac att ttt aaa agc ttt gca ggg 48
Met Ser Gly Leu Arg Glu Ser Lys His Ile Phe Lys Ser Phe Ala Gly
1 5 10 15

cca tac tgt gtg tgt gac aac ggt aag gac gta gac gct cct gga gat 96
Pro Tyr Cys Val Cys Asp Asn Gly Lys Asp Val Asp Ala Pro Gly Asp
20 25 30

tat aac cag tgc ctg cag ctc tat cgg agg agg gga cta act gaa cga 144
Tyr Asn Gln Cys Leu Gln Leu Tyr Arg Arg Arg Gly Leu Thr Glu Arg
35 40 45

ctt gct gac tgt gaa ggg gcc atc ttg gct tct caa gcc acg gac aca 192
Leu Ala Asp Cys Glu Gly Ala Ile Leu Ala Ser Gln Ala Thr Asp Thr
50 55 60

aag agc cgg gac ctg tgt gga gtc cag gat gct gga gct gct gat ctg 240
Lys Ser Arg Asp Leu Cys Gly Val Gln Asp Ala Gly Ala Ala Asp Leu
65 70 75 80

tac aat gat gtg ggc aac ttg ggg gtt gag gaa atg cac ccc agg tta 288
Tyr Asn Asp Val Gly Asn Leu Gly Val Glu Glu Met His Pro Arg Leu
85 90 95

gtt agt cct ggg ttc tcc tat cca gtg ctg tca gta cta gtt tat acg 336
Val Ser Pro Gly Phe Ser Tyr Pro Val Leu Ser Val Leu Val Tyr Thr
100 105 110

ctc tgg tta aat caa gtg tat gac ttt aaa agg cag aca ggt tcc ctt 384
Leu Trp Leu Asn Gln Val Tyr Asp Phe Lys Arg Gln Thr Gly Ser Leu

```
        115                 120                 125

aga gag cca cca ggg ctc ggc cga ggc gtc gcg cct gcg cag tcg gcc     432
Arg Glu Pro Pro Gly Leu Gly Arg Gly Val Ala Pro Ala Gln Ser Ala
    130                 135                 140

gtt cgg gca tgc gcg ggg gct gtg gag cgt cgc tcg gtg aag tct cgc     480
Val Arg Ala Cys Ala Gly Ala Val Glu Arg Arg Ser Val Lys Ser Arg
145                 150                 155                 160

gag agt gag cgc cgt ggt gga ggc acc cgc ttc ccc ggt gtc cct gcg     528
Glu Ser Glu Arg Arg Gly Gly Gly Thr Arg Phe Pro Gly Val Pro Ala
                165                 170                 175

ggc ccg gcg ctt ggc ttc agc ttc ttc cct ctg tat cta cgc ttc cca     576
Gly Pro Ala Leu Gly Phe Ser Phe Phe Pro Leu Tyr Leu Arg Phe Pro
            180                 185                 190

cgc gag ttc ccc tta ggc gca cgc ccg ggc tcc cgc agg atg ggg gac     624
Arg Glu Phe Pro Leu Gly Ala Arg Pro Gly Ser Arg Arg Met Gly Asp
        195                 200                 205

aac act agc ccc atc agc gtg atc ctg gtg agc tcg ggc agc agg ggc     672
Asn Thr Ser Pro Ile Ser Val Ile Leu Val Ser Ser Gly Ser Arg Gly
    210                 215                 220

aat aag ctg ctc ttc agg tat ccc ttc cag agg agc cag gag cac ccc     720
Asn Lys Leu Leu Phe Arg Tyr Pro Phe Gln Arg Ser Gln Glu His Pro
225                 230                 235                 240

gcg tcc cag aca act gtg ctg atc agg ggt ttc cta gac gtg ata ctt     768
Ala Ser Gln Thr Thr Val Leu Ile Arg Gly Phe Leu Asp Val Ile Leu
                245                 250                 255

gac act gta tca gac tta ctg cgc tca gcc tac tat ttc caa gat gga     816
Asp Thr Val Ser Asp Leu Leu Arg Ser Ala Tyr Tyr Phe Gln Asp Gly
            260                 265                 270

cga atg ttt gtt cct ctt tat aaa cca cgt agc aga tac gct gtc aac     864
Arg Met Phe Val Pro Leu Tyr Lys Pro Arg Ser Arg Tyr Ala Val Asn
        275                 280                 285

aac acg gga gag cat gtt gat gac cag gat ggt gac tcc agg ttc tcg     912
Asn Thr Gly Glu His Val Asp Asp Gln Asp Gly Asp Ser Arg Phe Ser
    290                 295                 300

gat gct att ctg gca aca att ttg gca acc aaa tct gaa atg tgc ggc     960
Asp Ala Ile Leu Ala Thr Ile Leu Ala Thr Lys Ser Glu Met Cys Gly
305                 310                 315                 320

caa aag ttt gag ctg aag att gat aat gta cgg ttt gtg ggg cac cca    1008
Gln Lys Phe Glu Leu Lys Ile Asp Asn Val Arg Phe Val Gly His Pro
                325                 330                 335

aca ctg ctg cag cat gct ctg ggg cag gtc tcc aaa aca gat cca tct    1056
Thr Leu Leu Gln His Ala Leu Gly Gln Val Ser Lys Thr Asp Pro Ser
            340                 345                 350

cca aag cgg gaa gca ccg acc atg atc ctt ttt aat gtg gtg ttt gca    1104
Pro Lys Arg Glu Ala Pro Thr Met Ile Leu Phe Asn Val Val Phe Ala
        355                 360                 365

ctg agg gcc aat gct gac ccg tca gtg ata aac tgt ctg cac acc ttg    1152
Leu Arg Ala Asn Ala Asp Pro Ser Val Ile Asn Cys Leu His Thr Leu
    370                 375                 380

tcc cgg cgc att gct acc gtc ctg caa cat gag gag cgc cgc tgc cag    1200
Ser Arg Arg Ile Ala Thr Val Leu Gln His Glu Glu Arg Arg Cys Gln
385                 390                 395                 400

tac ctc aca cgg gag gcc aag ctg atc tta gca ctt cag gat gag gtg    1248
Tyr Leu Thr Arg Glu Ala Lys Leu Ile Leu Ala Leu Gln Asp Glu Val
                405                 410                 415

tct gct atg gcc gat gca aat gaa ggt cct cag tcc cca ttc cac cac    1296
Ser Ala Met Ala Asp Ala Asn Glu Gly Pro Gln Ser Pro Phe His His
```

420 425 430

atc ctg ccc aag tgc aag ctg gct agg gac ctc aaa gaa gct tat gac 1344
Ile Leu Pro Lys Cys Lys Leu Ala Arg Asp Leu Lys Glu Ala Tyr Asp
435 440 445

agc ctg tgc aca tct ggt gta gta cgg ctt cac att aac agc tgg cta 1392
Ser Leu Cys Thr Ser Gly Val Val Arg Leu His Ile Asn Ser Trp Leu
450 455 460

gaa gtg agc ttc tgt ctt ccc cac aag atc cac tat gca gct tca agt 1440
Glu Val Ser Phe Cys Leu Pro His Lys Ile His Tyr Ala Ala Ser Ser
465 470 475 480

ctg atc cct cct gag gct att gaa cgg agc ctg aaa gcc atc cgc ccc 1488
Leu Ile Pro Pro Glu Ala Ile Glu Arg Ser Leu Lys Ala Ile Arg Pro
485 490 495

tac cat gct ttg cta ctg ctc agt gac gag aag tct cta ttg agt gag 1536
Tyr His Ala Leu Leu Leu Leu Ser Asp Glu Lys Ser Leu Leu Ser Glu
500 505 510

ctt ccc att gac tgc tcc ccg gct ctg gtg cgg gtg atc aag acc acg 1584
Leu Pro Ile Asp Cys Ser Pro Ala Leu Val Arg Val Ile Lys Thr Thr
515 520 525

tct gct gtg aag aac cta cag cag cta gcc cag gat gct gat ctg gcc 1632
Ser Ala Val Lys Asn Leu Gln Gln Leu Ala Gln Asp Ala Asp Leu Ala
530 535 540

ttg ctg cag gtc ttc cag ctt gca gct cac ctg gtg tac tgg ggc aag 1680
Leu Leu Gln Val Phe Gln Leu Ala Ala His Leu Val Tyr Trp Gly Lys
545 550 555 560

gcc gtc atc atc tat cca ctg tgt gag aac aac gtc tat gtc ctg tct 1728
Ala Val Ile Ile Tyr Pro Leu Cys Glu Asn Asn Val Tyr Val Leu Ser
565 570 575

ccc aat gcc agt gtg tgt ctg tac tct cca cta gct gag cag ttc tcc 1776
Pro Asn Ala Ser Val Cys Leu Tyr Ser Pro Leu Ala Glu Gln Phe Ser
580 585 590

cgc cag ttc ccg tct cat gac ctg cca tct gtc ctg gcc aag ttt tcc 1824
Arg Gln Phe Pro Ser His Asp Leu Pro Ser Val Leu Ala Lys Phe Ser
595 600 605

ttg ccc gtt tct tta tcg gag ttc agg agc ccc ctg gcc ccc cct gca 1872
Leu Pro Val Ser Leu Ser Glu Phe Arg Ser Pro Leu Ala Pro Pro Ala
610 615 620

cag gag acc cag ctc atc cag atg gtg gtg tgg atg ctg cag cgc cgg 1920
Gln Glu Thr Gln Leu Ile Gln Met Val Val Trp Met Leu Gln Arg Arg
625 630 635 640

ctg ctc atc cag ctg cat acc tat gtt tgc cta atg gca tca ccc agt 1968
Leu Leu Ile Gln Leu His Thr Tyr Val Cys Leu Met Ala Ser Pro Ser
645 650 655

gag gag gag ccc cga cct cga gag gat gac gtc ccc ttc aca gcc cga 2016
Glu Glu Glu Pro Arg Pro Arg Glu Asp Asp Val Pro Phe Thr Ala Arg
660 665 670

gtt ggt ggc cgc agc ctc agc aca ccc aat gct cta agc ttt ggc tcc 2064
Val Gly Gly Arg Ser Leu Ser Thr Pro Asn Ala Leu Ser Phe Gly Ser
675 680 685

cca acc agc agt gac gac atg acc ctc acc agc ccc agt atg gac aac 2112
Pro Thr Ser Ser Asp Asp Met Thr Leu Thr Ser Pro Ser Met Asp Asn
690 695 700

tcc agt gca gag ctg ctc ccc agt ggg gac tca cca ctg aac aaa cgg 2160
Ser Ser Ala Glu Leu Leu Pro Ser Gly Asp Ser Pro Leu Asn Lys Arg
705 710 715 720

atg aca gag aac ctg ctg gcc agc ctc tca gag cat gag cgg gct gct 2208
Met Thr Glu Asn Leu Leu Ala Ser Leu Ser Glu His Glu Arg Ala Ala

```
                725                 730                 735

atc ctc aat gtg ccc gca gcc caa aac cct gag gac ctc cgt atg ttt      2256
Ile Leu Asn Val Pro Ala Ala Gln Asn Pro Glu Asp Leu Arg Met Phe
            740                 745                 750

gcc agg ctc ctt cac tac ttc cgt ggc cgc cac cat ctg gag gag atc      2304
Ala Arg Leu Leu His Tyr Phe Arg Gly Arg His His Leu Glu Glu Ile
        755                 760                 765

atg tac aat gag aac aca cgg cgt tcc cag ctg ctc atg ctc ttt gac      2352
Met Tyr Asn Glu Asn Thr Arg Arg Ser Gln Leu Leu Met Leu Phe Asp
    770                 775                 780

aaa ttc cgc agc gtg ctg gtg gtg acc acc cat gag gac ccc gtt att      2400
Lys Phe Arg Ser Val Leu Val Val Thr Thr His Glu Asp Pro Val Ile
785                 790                 795                 800

gct gtc ttc cag gca ctg ctc aca tgagcccagg tggcatgctg agtgtgtgga     2454
Ala Val Phe Gln Ala Leu Leu Thr
                805

tggaaacaac ctcccctctg ctgcagagtt cctgactggc cttagggga taggcaaccc    2514

tagcctggca gaactgctcc ctgtgtcggt cgtcagtctg gtcagaagca gctatggggc   2574

aaccattgtt gggctttgtc ttttccctga aattctagcc cagacctcat atttactgtt   2634

agctaaggat gaccttgaac tcctgatctt actgcctgtc ctccaagtgc tatgactaca   2694

gatgtgtgcc atccatgtca cttttaaaaa atatatttat ttattttta               2743
```

<210> 99
<211> 808
<212> PRT
<213> Rattus norvegicus

<400> 99

```
Met Ser Gly Leu Arg Glu Ser Lys His Ile Phe Lys Ser Phe Ala Gly
1               5                   10                  15

Pro Tyr Cys Val Cys Asp Asn Gly Lys Asp Val Asp Ala Pro Gly Asp
            20                  25                  30

Tyr Asn Gln Cys Leu Gln Leu Tyr Arg Arg Arg Gly Leu Thr Glu Arg
        35                  40                  45

Leu Ala Asp Cys Glu Gly Ala Ile Leu Ala Ser Gln Ala Thr Asp Thr
    50                  55                  60

Lys Ser Arg Asp Leu Cys Gly Val Gln Asp Ala Gly Ala Ala Asp Leu
65                  70                  75                  80

Tyr Asn Asp Val Gly Asn Leu Gly Val Glu Glu Met His Pro Arg Leu
                85                  90                  95

Val Ser Pro Gly Phe Ser Tyr Pro Val Leu Ser Val Leu Val Tyr Thr
            100                 105                 110

Leu Trp Leu Asn Gln Val Tyr Asp Phe Lys Arg Gln Thr Gly Ser Leu
        115                 120                 125

Arg Glu Pro Pro Gly Leu Gly Arg Gly Val Ala Pro Ala Gln Ser Ala
    130                 135                 140
```

Val Arg Ala Cys Ala Gly Ala Val Glu Arg Arg Ser Val Lys Ser Arg
145 150 155 160

Glu Ser Glu Arg Arg Gly Gly Gly Thr Arg Phe Pro Gly Val Pro Ala
165 170 175

Gly Pro Ala Leu Gly Phe Ser Phe Phe Pro Leu Tyr Leu Arg Phe Pro
180 185 190

Arg Glu Phe Pro Leu Gly Ala Arg Pro Gly Ser Arg Arg Met Gly Asp
195 200 205

Asn Thr Ser Pro Ile Ser Val Ile Leu Val Ser Ser Gly Ser Arg Gly
210 215 220

Asn Lys Leu Leu Phe Arg Tyr Pro Phe Gln Arg Ser Gln Glu His Pro
225 230 235 240

Ala Ser Gln Thr Thr Val Leu Ile Arg Gly Phe Leu Asp Val Ile Leu
245 250 255

Asp Thr Val Ser Asp Leu Leu Arg Ser Ala Tyr Tyr Phe Gln Asp Gly
260 265 270

Arg Met Phe Val Pro Leu Tyr Lys Pro Arg Ser Arg Tyr Ala Val Asn
275 280 285

Asn Thr Gly Glu His Val Asp Asp Gln Asp Gly Asp Ser Arg Phe Ser
290 295 300

Asp Ala Ile Leu Ala Thr Ile Leu Ala Thr Lys Ser Glu Met Cys Gly
305 310 315 320

Gln Lys Phe Glu Leu Lys Ile Asp Asn Val Arg Phe Val Gly His Pro
325 330 335

Thr Leu Leu Gln His Ala Leu Gly Gln Val Ser Lys Thr Asp Pro Ser
340 345 350

Pro Lys Arg Glu Ala Pro Thr Met Ile Leu Phe Asn Val Val Phe Ala
355 360 365

Leu Arg Ala Asn Ala Asp Pro Ser Val Ile Asn Cys Leu His Thr Leu
370 375 380

Ser Arg Arg Ile Ala Thr Val Leu Gln His Glu Glu Arg Arg Cys Gln
385 390 395 400

Tyr Leu Thr Arg Glu Ala Lys Leu Ile Leu Ala Leu Gln Asp Glu Val
405 410 415

Ser Ala Met Ala Asp Ala Asn Glu Gly Pro Gln Ser Pro Phe His His
420 425 430

Ile Leu Pro Lys Cys Lys Leu Ala Arg Asp Leu Lys Glu Ala Tyr Asp
435 440 445

Ser Leu Cys Thr Ser Gly Val Val Arg Leu His Ile Asn Ser Trp Leu
450 455 460

Glu Val Ser Phe Cys Leu Pro His Lys Ile His Tyr Ala Ala Ser Ser
465 470 475 480

Leu Ile Pro Pro Glu Ala Ile Glu Arg Ser Leu Lys Ala Ile Arg Pro
485 490 495

Tyr His Ala Leu Leu Leu Leu Ser Asp Glu Lys Ser Leu Leu Ser Glu
500 505 510

Leu Pro Ile Asp Cys Ser Pro Ala Leu Val Arg Val Ile Lys Thr Thr
515 520 525

Ser Ala Val Lys Asn Leu Gln Gln Leu Ala Gln Asp Ala Asp Leu Ala
530 535 540

Leu Leu Gln Val Phe Gln Leu Ala Ala His Leu Val Tyr Trp Gly Lys
545 550 555 560

Ala Val Ile Ile Tyr Pro Leu Cys Glu Asn Asn Val Tyr Val Leu Ser
565 570 575

Pro Asn Ala Ser Val Cys Leu Tyr Ser Pro Leu Ala Glu Gln Phe Ser
580 585 590

Arg Gln Phe Pro Ser His Asp Leu Pro Ser Val Leu Ala Lys Phe Ser
595 600 605

Leu Pro Val Ser Leu Ser Glu Phe Arg Ser Pro Leu Ala Pro Pro Ala
610 615 620

Gln Glu Thr Gln Leu Ile Gln Met Val Val Trp Met Leu Gln Arg Arg
625 630 635 640

Leu Leu Ile Gln Leu His Thr Tyr Val Cys Leu Met Ala Ser Pro Ser
645 650 655

Glu Glu Glu Pro Arg Pro Arg Glu Asp Asp Val Pro Phe Thr Ala Arg
660 665 670

Val Gly Gly Arg Ser Leu Ser Thr Pro Asn Ala Leu Ser Phe Gly Ser
675 680 685

Pro Thr Ser Ser Asp Asp Met Thr Leu Thr Ser Pro Ser Met Asp Asn
690 695 700

Ser Ser Ala Glu Leu Leu Pro Ser Gly Asp Ser Pro Leu Asn Lys Arg
705 710 715 720

Met Thr Glu Asn Leu Leu Ala Ser Leu Ser Glu His Glu Arg Ala Ala
725 730 735

Ile Leu Asn Val Pro Ala Ala Gln Asn Pro Glu Asp Leu Arg Met Phe
740 745 750

Ala Arg Leu Leu His Tyr Phe Arg Gly Arg His His Leu Glu Glu Ile
755 760 765

Met Tyr Asn Glu Asn Thr Arg Arg Ser Gln Leu Leu Met Leu Phe Asp
770 775 780

Lys Phe Arg Ser Val Leu Val Val Thr Thr His Glu Asp Pro Val Ile
785 790 795 800

Ala Val Phe Gln Ala Leu Leu Thr
805

<210> 100
<211> 694
<212> DNA
<213> Rattus norvegicus

<220>
<221> misc_feature
<222> (660)..(660)
<223> n is a, c, g, or t

<400> 100
tttttttttt ttttttttga ggttgcctga atcacggggt ttaagagaga agcgttgggt 60

tgggggctca caaccattat aagaagggaa ctaagaaagg tttcaacaag cagataagcc 120

aaggtgcaat gtccagtagg aatggcaggc cccgcccctt tccctgctct ctgggagtcc 180

aacctgggga aggagctggc aggccccggg tcccctcagt tggcagagtt acactgtagg 240

atttgctgag ggcaaagag cttcctgaca gccgggatct ctataggctg ccccagcact 300

gagtagttct ctttgttgtc tgcagtctgg gctgaagact tcttaaagta gcacaggcgg 360

caaacagagt ggtacttgtc cgctccgcca atcacctcca cctctttctc caggcccagc 420

ctcttggtgt aggaagcttc tcgaaagcac tccatgcaca cagcggtgag cttcaccaca 480

ctctcggcca agggcaccag gttcaagatg ctgccgaaag ccttcctctg gaaggttccg 540

tccagcgccg ccacaatcac tgtcttgcct gtattggcca tcgtttcaca gaaatccaca 600

atgtcaggga aaaactgccc ttcatcgatg ccaatgacag ccacacccaa ggcctcctgn 660

gccacatcct tgagcatgca ggccggcagt gcat 694

<210> 101
<211> 637
<212> DNA
<213> Rattus norvegicus

<400> 101
agctgtaaaa acaacattta ttactttata atattgagaa tgagtatttg ggccaaaaca 60

atgggaatgc catacaaata attttttcat cttatatttc aaaacctact acagatatta 120

cactgttttt taattgattc acctgtattt gatttgggaa aaatacattg ggcaggggac 180

acaaaatatc acacaatagc actaggaaat gtctggaaaa taaggggagt tgtggattag 240

gatcatgtta gctctcatac aggacatatt tcagttatga taaaggaaga attgaagcaa 300

tcgctggatc ccagcaacat tccttcagca tctcatctac tcctactttc tctcaaataa 360

tctaagatct ctggccttct gttcataaat actccttgac tctgtttttg atgagattac 420

ttctgaacgc agttttcttt ctcttcaaag gaccaagact tgagtgtcac actgctgaga 480

attgtcccag gttgtgtcca atatcctcct agcactctcc attgtgaatc gtatcaagta 540

```
gaaaccacct attcccatgt ttgctctcca caactcaagt ccagaaagtc tcaggtacat    600
gcattctgtg gcagagacca gctccttgct tgctcaa                              637
```

## Description

### Technical Field

The present invention relates to a method for determining phospholipidosis and tools therefor. More particularly, the present invention relates to a method for determining phospholipidosis (e.g., drug-induced phospholipidosis etc.) with expression variations of blood marker genes as indices, and reagents, kits and the like for the detection of the blood marker genes.

### Background Art

Phospholipidosis (hereinafter sometimes to be abbreviated as "PLsis") is defined to be a phenomenon where phospholipid accumulates in excess in the cell. It is caused by abnormal homeostasis of lipid metabolism including that induced hereditarily, as well as many pharmaceutical agents such as antidepressants, antianginal drugs, antimalarial drugs, anti-anorectic drugs, hypolipidemic drugs and the like or metabolites thereof. In PLsis, phospholipid is mainly accumulated in lysosome and a cyclic or elliptic myelin-like structure (lamellar body) is observed electron microscopically. While the expression mechanism of toxicity has not been completely elucidated, it is considered to be caused by 1) inhibition of lysosomal enzyme (mainly phospholipid degradation enzyme (phospholipase)) activity by compound, 2) inhibition of transport pathway involved in phospholipid metabolism by compound, 3) inhibition of degradation of complex by the formation of a complex of compound and phospholipid, 4) promotion of synthesis of phospholipid by compound and the like.

Many of the PLsis-inducing compounds have a structure comprising, in a molecule, a hydrophobic domain and a positively charged hydrophilic domain in combination (cationic amphiphilic drug; CAD). In recent years, along with the progress of genome analysis, the value of orphan receptors as drug discovery targets has been recognized and the development of agonists or antagonists against the receptors is being undertaken. However, because such compounds act on receptors, they mostly have CAD structures and an increasing number of incidents have been experienced where expression of PLsis prevents development of pharmaceutical products. Moreover, the pharmaceutical products that have already been approved include those reported to induce PLsis as a side effect. Therefore, the development of an efficient evaluation or prediction system for PLsis-inducibility of a drug has been urgently desired.

Incidentally, microarray technology for simultaneously monitoring expression of several thousand to several tens of thousand kinds of mRNAs (comprehensive gene expression analysis, transcriptomics) has been actively used in various kinds of fields of medicine and biology. In the field of toxicology, too, this technology has been utilized for the elucidation of the mechanism of toxicity expression and the study of toxicity prediction, and is expected to be a new study field called toxicogenomics. Toxicity phenomenon is considered to accompany not only independent changes in one or several genes, but also integral variations where many genes are correlated, such as interaction of genes, cascade and the like. Based thereon, it is expected that the behavior of molecules involved in toxicity expression can be comprehensively perceived by the use of a technique of microarray capable of analysis at transcriptome level.

The present inventors previously analyzed comprehensively gene expression in human cultured cells exposed to a known PLsis-inducing compound using microarray, identified and reported marker genes that showed remarkably varying expression on inducing PLsis (Sawada, H. et al., Toxicol. Sci., 83: 282-92 (2005)). Since an in vitro screening system using cultured cells can evaluate many samples in a short time using a small amount of a compound, it is a superior tool for rapidly predicting the presence or absence of PLsis-inducibility and efficiently optimizing a structure in the initial step of drug discovery when synthesis of the compound is restricted.

On the other hand, the results in an in vitro evaluation system do not always reflect the in vivo toxicity expression. For example, a compound that induces PLsis in cells by in vitro screening can be said to have a PLsis-inducing potential. However, the compound may not show toxicity when it is rapidly metabolized in vivo and the like, and vice versa. Alternatively, when the concentration at which the efficacy is exhibited is sufficiently lower than the concentration at which the toxicity is expressed, staging up to the preclinical or clinical stage is possible.

Therefore, construction of an in vivo evaluation system capable of rapidly and conveniently determining the presence or absence of the expression of drug-induced PLsis due to the administration of a compound in the later stage of development or after placing on the market has been demanded. In such an evaluation system, since experiment animals or volunteers for clinical trial or patients are the subjects, collection of a sample for an analysis is desirably as noninvasive as possible. On the other hand, since utilization of transcriptomics requires a cell-containing sample, the sample for an analysis is preferably blood, lymphocyte and the like.

When a biological sample such as blood, lymph fluid and the like is used as an analysis target, a novel and more suitable marker gene (genes), which is different from marker genes used for in vitro evaluation systems, may be found (e.g., see Rockett, J.C. et al., Toxicol. Sci., 69: 49-59 (2002)). However, there is no report relating to a particular marker gene capable of highly accurately determining the PLsis-inducibility in an in vivo evaluation system.

**Disclosure Of The Invention**

It is an object of the present invention to provide an *in vivo* evaluation system of PLsis, which comprises identifying a gene showing varying expression in blood and the like in correlation with PLsis expression, or a PLsis marker gene, and using expression variation of the gene as an index.

In an attempt to solve the aforementioned problems, the present inventors have performed comprehensive analysis of gene expression using a microarray with, as a sample, the blood from rats administrated with various known PLsis-inducing compounds for 3 days or 7 days. As a result, the present inventors have succeeded in identifying genes that showed remarkably varying expression for all of these compounds for each administration period. In addition, for many compounds, most of these genes showed expression variation by administration for 3 days and administration for 7 days. The present inventors further studied based on these findings and completed the present invention.

Accordingly, the present invention provides

[1] a reagent for determining phospholipidosis in a mammal, which comprises a nucleic acid capable of hybridizing to a nucleic acid having a base sequence shown by any of SEQ ID NO n (wherein n is an odd number between 1 and 57, an integer number between 59 and 63, an even number between 64 and 98, 100 or 101) under high stringent conditions and/or a nucleic acid capable of hybridizing to a nucleic acid having a base sequence complementary to the base sequence under high stringent conditions;

[2] a kit for determining phospholipidosis in a mammal, which comprises two or more reagents containing a nucleic acid capable of hybridizing to a transcription product of a gene showing varying expression in correlation with expression of phospholipidosis under high stringent conditions and/or a nucleic acid capable of hybridizing to a nucleic acid having a base sequence complementary to the transcription product under high stringent conditions, wherein,

(a) at least one reagent is the reagent of the above-mentioned [1], and

(b) when two or more reagents of the above-mentioned [1] are contained, each reagent can detect expression of different genes;

[3] a method for determining phospholipidosis in a mammal, which comprises detecting expression variation of one or more genes showing expression variation in correlation with phospholipidosis expression, in a sample from a mammal, wherein at least one gene has the same or substantially the same base sequence as the base sequence shown by any of SEQ ID NO n (wherein n is an odd number between 1 and 57, an integer number between 59 and 63, an even number between 64 and 98, 100 or 101);

[4] the method of the above-mentioned [3], wherein the mammal is administered with a compound or exposed to the compound, and phospholipidosis is caused by the compound;

[5] the method of the above-mentioned [3], wherein the mammal is human;

[6] the method of the above-mentioned [3], wherein the mammal is rat, mouse, dog or monkey; and

[7] the method of the above-mentioned [3], wherein the sample is blood or lymphocyte; and the like.

The method for determining PLsis of the present invention is characterized by detection of expression variation of a PLsis marker gene in a sample from a mammal, and affords a superior effect in that the expression of PLsis can be determined noninvasively and with good precision, as compared to conventional in vivo toxicity tests and the like.

**Best Mode for Embodying the Invention**

The present invention provides a reagent for determining PLsis containing a nucleic acid capable of detecting the expression of a gene showing varying expression in correlation with PLsis expression (i.e., PLsis marker gene). As used herein, by "varying expression in correlation with PLsis expression" is meant a statistically significant tendency toward substantial increase or decrease in the expression, when one or more mammalian tissues show histopathological finding of PLsis. By the "substantial increase or decrease" is meant an increase to not less than 1.5-fold of the normal state or a decrease to not more than 2/3 of the normal state, and by the "substantially no variation" is meant an expression level of 2/3 to 1.5-fold of the normal state.

Specifically, as a PLsis marker gene detected by the reagent of the present invention, 54 kinds of genes (including 7 kinds of EST) having the same base sequences or substantially the same base sequences as shown in SEQ ID NO n (wherein n is an odd number between 1 and 57, an integer number between 59 and 63, an even number between 64 and 98, 100 or 101) can be mentioned. As used herein, by the "substantially the same base sequences" is meant base sequences capable of hybridizing to nucleic acids having complementary strand sequences of base sequences shown in SEQ ID NO n (wherein n is an odd number between 1 and 57, an integer number between 59 and 63, an even number between 64 and 98, 100 or 101) under high stringent conditions, wherein the proteins encoded thereby are the same or substantially the same proteins encoded by the base sequences shown in said SEQ ID NOs. The "high stringent conditions" refers to the conditions under which nucleic acids having complementary strand sequences of base sequences shown in SEQ ID NO n (wherein n is an odd number between 1 and 57, an integer number between 59 and 63, an even number between 64 and 98, 100 or 101) can hybridize with nucleic acids having base sequences showing complementarity of not less than about 70%, preferably not less than about 80%, more preferably not less than about 90%, particularly preferably not less than about 95%, in the overlapping regions, and, for example, a sodium concentration of about 19-40 mM, preferably about 19-20 mM, a temperature of about 50-70°C, preferably about 60-65°C, and particularly preferably, a sodium concentration of about 19 mM and a temperature of about 65°C can be mentioned. Those skilled in this field can easily obtain desired stringency by suitably changing a salt concentration of the hybridization solution, a temperature of hybridization reaction, a probe concentration, a probe length, a mismatch number, a hybridization reaction time, a salt concentration of the washing solution, a washing temperature, and the like.

The "substantially the same protein" refers to a protein having an amino acid sequence showing not less than about 60%, preferably not less than about 70%, more preferably not less than about 80%, particularly preferably not less than about 90%, most preferably not less than about 95%, homology to the amino acid sequence shown in SEQ ID NO m (wherein m is an even number between 2 and 58, or an odd number between 65 and 99) and the same level of activity as the protein having the amino acid sequence shown in the above-mentioned SEQ ID NOs. Alternatively, when a PLsis marker gene has the same or substantially the same base sequence as the base sequence shown in SEQ ID NO n (wherein n is an integer number between 59 and 63, 100 or 101), the "substantially the same protein" refers to a protein having a partial amino acid sequence showing not less than about 60%, preferably not less than about 70%, more preferably not less than about 80%, particularly preferably not less than about 90%, most preferably not less than about 95%, homology to the partial amino acid sequence encoded by the base sequence shown in SEQ ID NO n (wherein n is an integer number between 59 and 63, 100 or 101) and the same level of activity as the protein having the partial amino acid sequence shown in the above-mentioned.

The "same level of activity" means that the activity is qualitatively the same (e.g., physiologically or pharmacologically), and preferably, quantitatively equivalent (e.g., 0.5- to 2-fold), but may be different. As long as the homology conditions of amino acid sequence are satisfied, moreover, other quantitative elements such as molecular weight and the like may be different.

With regard to the amino acid sequence, a "homology" means a proportion (%) of the same amino acid residue and analogous amino acid residue to the whole amino acid residues overlapped in the optimal alignment (preferably, the algorithm is such that a gap can be introduced into one or both of the sequences for an optimal alignment) where two amino acid sequences are aligned using a mathematic algorithm known in the technical field. The "analogous amino acid" means amino acids having similar physiochemical properties, and, for example, the amino acids are classified into groups such as an aromatic amino acid (Phe, Trp, Tyr), an aliphatic amino acid (Ala, Leu, Ile, Val), a polar amino acid (Gln, Asn), a basic amino acid (Lys, Arg, His), an acidic amino acid (Glu, Asp), an amino acid having a hydroxy group (Ser, Thr) and an amino acid having a small side-chain (Gly, Ala, Ser, Thr, Met). Substitution by such analogous amino acids is expected not to change the phenotype of proteins (i.e., conservative amino acid substitution). Specific examples of the conservative amino acid substitution are known in this technical field and described in various literatures (e.g., see Bowie et al., Science, 247: 1306-1310 (1990)).

The homology of the amino acid sequence in the present specification can be calculated using homology calculation algorithm NCBI BLAST (National Center for Biotechnology Information Basic Local Alignment Search Tool) under the following conditions (expectancy=10; allowing gap; matrix=BLOSUM62; filtering=OFF). Other Algorithms to determine a homology of amino acid sequence include, for example, the algorithm as described in Karlin et al., Proc. Natl. Acad. Sci. USA, 90: 5873-5877 (1993) [the algorithm is incorporated into NBLAST and XBLAST programs (version 2.0) (Altschul et al., Nucleic Acids Res., 25: 3389-3402 (1997))], the algorithm as described in Needleman et al., J. Mol. Biol., 48: 444-453 (1970) [the algorithm is incorporated into a GAP program in a GCG software package], the algorithm as described in Myers and Miller, CABIOS, 4: 11-17 (1988) [the algorithm is incorporated into an ALIGN program (version 2.0) which is a part of a CGC sequence alignment software package], the algorithm as described in Pearson et al., Proc. Natl. Acad. Sci. USA, 85: 2444-2448 (1988) [the algorithm is incorporated into a FASTA program in a GCG software package], and the like, which can be preferably used in a similar manner.

More preferably, an amino acid sequence substantially the same as the amino acid sequence shown in SEQ ID NO m (wherein m is an even number between 2 and 58, or an odd number between 65 and 99) is an amino acid sequence having not less than about 60%, preferably not less than about 70%, more preferably not less than about 80%, homology to the amino acid sequence shown in any of said SEQ ID NOs; a partial amino acid sequence substantially the same as the partial amino acid sequence encoded by the base sequence shown in SEQ ID NO n (wherein n is an integer number between 59 and 63, 100 or 101) is a partial amino acid sequence having not less than about 60%, preferably not less than about 70%, more preferably not less than about 80%, homology to the partial amino acid sequence encoded by the base sequence shown in any of said SEQ ID NOs.

**[0020]** The protein having such homology includes, for example, a protein containing 1) an amino acid sequence shown in SEQ ID NO m (wherein m is an even number between 2 and 58, or an odd number between 65 and 99) (or a partial amino acid sequence encoded by the base sequence shown in SEQ ID No n (wherein n is an integer number between 59 and 63, 100 or 101)) wherein one or more (preferably about 1-30, more preferably about 1-10, particularly preferably several (1-5)) amino acids have been deleted, 2) an amino acid sequence shown in SEQ ID NO m (wherein m is an even number between 2 and 58, or an odd number between 65 and 99) (or a partial amino acid sequence encoded by the base sequence shown in SEQ ID No n (wherein n is an integer number between 59 and 63, 100 or 101)) wherein one or more (preferably about 1-30, more preferably about 1-10, particularly preferably several (1-5)) amino acids have been added, 3) an amino acid sequence shown in SEQ ID NO m (wherein m is an even number between 2_and 58, or an odd number between 65 and 99) (or a partial amino acid sequence encoded by the base sequence shown in SEQ ID NO n (wherein n is an integer number between 59 and 63, 100 or 101)) wherein one or more (preferably about 1-30, more preferably about 1-10, particularly preferably several (1-5)) amino acids have been inserted, 4) an amino acid sequence shown in SEQ ID NO m (wherein m is an even number between 2 and 58, or an odd number between 65 and 99) (or a partial amino acid sequence encoded by the base sequence shown in SEQ ID NO n (wherein n is an integer number between 59 and 63, 100 or 101)) wherein one or more (preferably about 1-30, more preferably about 1-10, particularly preferably several (1-5)) amino acids have been substituted by other amino acids, or 5) an amino acid sequence wherein the above-mentioned sequences have been combined, and the like.

When the amino acid sequence is inserted, deleted or substituted as mentioned above, the position of the insertion, deletion or substitution is not particularly limited.

More specifically, as a gene having a substantially the same base sequence as the base sequence shown in SEQ ID NO n (wherein n is an odd number between 1 and 57, an integer number between 59 and 63, an even number between 64 and 98, 100 or 101), allele variants of a rat gene having the base sequence shown in any of these SEQ ID NOs, orthologs of the gene in mammals other than rat (e.g., human, monkey, bovine, horse, swine, sheep, goat, dog, cat, rabbit, hamster, guinea pig, mouse etc.) and the like can be mentioned.

The base sequences of PLsis marker genes from rat of the present invention [i.e., base sequences shown in SEQ ID NO n (wherein n is an odd number between 1 and 57, an integer number between 59 and 63, an even number between 64 and 98, 100 or 101)] are all known, and published in the GenBank database under the accession Nos. of 1)NM_031355, 3)NM_053359, 5)NM_017051, 7)NM_013052, 9)NM_053372, 11)NM_053843, 13)NM_001004202, 15) NM_012512(or AW916647), 17)NM_019186, 19)NM_024139, 21)XM_215858(or AA891920), 23)NM_012778(or L07268), 25)XM_213793(or AI412863), 27)NM_057114, 29)NM_053800, 31)NM_022391, 33)XM_213921(or BE099979), 35)L38482, 37)NM_001007742(or BI275880), 39)XM_214451(or AI600237), 41)XM_226624(or BI291626), 43)NM_022597, 45)NM_145184, 47)NM_053719, 49)NM_017073, 51)NM_053582, 53)NM_138881, 55)XM_340803 (or BI303656), 57)XM_213333(or AI171327), 59)AI029175, 60)BM392055, 61)BI288013, 62)AI172141, 63)AI600030, 64)NM_012904, 66)NM_053598, 68)NM_053554, 70)NM_053822, 72)NM_053587, 74)NM_001012345(or XM_ 341887), 76)NM_022205, 78)NM_031512, 80)XM_223508, 82)NM_053373, 84)NM_012992, 86)NM_001009717, 88) NM_031010, 90)XM_341053, 92)XM_214152, 94)XM_346274, 96)NM_001013233(or XM_344660), 98)XM_340780, 100)BM389006 and 101)AA799328, respectively.

Moreover, as human orthologs corresponding to them, in sequence, 1)NM_005662, 3)NM_004045, 5)NM_ 000636, 7)NM_003405, 9)NM_003064, 11)NM_000569(NM_000570 as variant), 13)(a human gene corresponding to NM_001004202 is not known), 15)AK026463(NM_004048 as variant), 17)NM_005738(NM_212460 as variant), 19)NM_ 007236, 21)NM_013248, 23)NM_000385(NM_198098 as variant), 25)NM_016433, 27)NM_002574(NM_181696, NM_ 181697 as variant), 29)NM_003229, 31)NM_004219, 33)NM_003851 (similar to both XM_213921 and AI029175), 35) NM_004359, 37)NM_016172, 39)NM_004280, 41)NM_012081, 43)NM_001908(NM_147780, NM_147781, NM_ 147782, NM_147783 as variant), 45)NM_006313, 47)NM_198449, 49)NM_002065, 51)NM_022164, 53)NM_080657, 55)NM_024632, 57)NM_152766, 59)NM_003851(similar to both XM_213921, AI029175), 60)NM_018571, 61)NM_ 015982, 62)NM_000037(NM_020475, NM_020476, NM_020477 as variant), 63) (a human gene corresponding to AI600030 is not known), 64)NM_000700, 66)NM_019094(NM_199040 as variant), 68)NM_001008660(NM_007166 as variant), 70)NM_002964, 72)NM_002965, 74)NM_032564, 76)NM_001008540(NM_003467 as variant), 78)NM_ 000576, 80)NM_012161(NM_033535 as variant), 82)NM_005091, 84)NM_002520(NM_199185 as variant), 86)NM_ 052972, 88)NM_001140, 90)NM_001913(NM_181500, NM_181552 as variant), 92)NM_005192, 94)NM_007213, 96) NM_002101(NM_016815 as variant), 98)NM_012075 and 100)NM_003258(101) a human gene corresponding to AA799328 is not known) can be mentioned.

A gene having the same or substantially the same base sequence as the base sequence shown in SEQ ID NO:1 (hereinafter sometimes to be abbreviated as "vdac3") encodes a protein at the opening of a voltage-dependent ion channel of mitochondrial outer membrane that possibly functions for the transport of adenine nucleotides (voltage-dependent anion channel 3).

A gene having the same or substantially the same base sequence as the base sequence shown in SEQ ID NO:3 (hereinafter sometimes to be abbreviated as "atoxl") encodes copper binding protein that mediates the intracellular copper transport and homeostasis (ATX1 (antioxidant protein 1) homolog 1 (yeast)).

A gene having the same or substantially the same base sequence as the base sequence shown in SEQ ID NO:5 (hereinafter sometimes to be abbreviated as "sod2") encodes mitochondrial enzyme that converts superoxide to hydrogen peroxide (superoxide dismutase 2, mitochondrial).

A gene having the same or substantially the same base sequence as the base sequence shown in SEQ ID NO:7 (hereinafter sometimes to be abbreviated as "ywhah") encodes a protein, which is an apoptosis inhibitor and activator of tyrosine hydroxylase and tryptophan hydroxylase (tyrosine 3-monooxygenase/tryptophan 5-monooxygenase activation protein, eta polypeptide).

A gene having the same or substantially the same base sequence as the base sequence shown in SEQ ID NO:9 (hereinafter sometimes to be abbreviated as "slpi") encodes an inhibitor of a protease such as trypsin, cathepsin G and neutrophil elastase and the like (secretory leukocyte protease inhibitor).

A gene having the same or substantially the same base sequence as the base sequence shown in SEQ ID NO:11 (hereinafter sometimes to be abbreviated as "fcgr3") encodes a protein, which is bound with immunoglobulin and initiates various immune responses (Fc receptor, IgG, low affinity III).

A gene having the same or substantially the same base sequence as the base sequence shown in SEQ ID NO:13 (hereinafter sometimes to be abbreviated as "cc16") encodes CC chemokine that promotes migration and humectant of inflammatory cell in rat (chemokine (C-C motif) ligand 6). However, the corresponding human gene is not known.

A gene having the same or substantially the same base sequence as the base sequence shown in SEQ ID NO:15 (hereinafter sometimes to be abbreviated as "B2m") encodes a component of MHC class I antigen that acts on transepithelial transport of IgG (Beta-2 microglobulin).

A gene having the same or substantially the same base sequence as the base sequence shown in SEQ ID NO:17 (hereinafter sometimes to be abbreviated as "arl4") encodes GTP binding protein that plays a key role in the vesicular transport and protein secretion (ADP-ribosylation-like 4).

A gene having the same or substantially the same base sequence as the base sequence shown in SEQ ID NO:19 (hereinafter sometimes to be abbreviated as "chp") encodes $Ca^{2+}$binding protein that plays a role in the membrane transport (calcium binding protein p22).

A gene having the same or substantially the same base sequence as the base sequence shown in SEQ ID NO:21 encodes a protein similar to NXT1, an NTF2-related transport protein (Similar to NTF2-related export protein NXT1 (LOC296219), mRNA).

A gene having the same or substantially the same base sequence as the base sequence shown in SEQ ID NO:23 (hereinafter sometimes to be abbreviated as "aqp1") encodes a protein constituting a water channel (aquaporin 1 (channel-forming integral protein, 28kDa)).

A gene having the same or substantially the same base sequence as the base sequence shown in SEQ ID NO:25 encodes a protein similar to a glycolipid transfer protein, which is involved in the intermembrane transport of glycosphingolipid, glycoglycerolipid (Similar to Glycolipid transfer protein (LOC288707), mRNA).

A gene having the same or substantially the same base sequence as the base sequence shown in SEQ ID NO:27 (hereinafter sometimes to be abbreviated as "prdx1") encodes an antioxidant having peroxydase activity induced by oxidative stress (peroxiredoxin 1).

A gene having the same or substantially the same base sequence as the base sequence shown in SEQ ID NO:29 (hereinafter sometimes to be abbreviated as "txn") encodes a protein involved in the response to UV and oxidative stress, which decreases a reactive oxygen intermediate (thioredoxin).

A gene having the same or substantially the same base sequence as the base sequence shown in SEQ ID NO:31 (hereinafter sometimes to be abbreviated as "pttg") is a premalignant gene, which is downregulated in the response to serum starvation, and promotes cell growth and angiogenesis (pituitary tumor-transforming 1).

A gene having the same or substantially the same base sequence as the base sequence shown in SEQ ID NO:33 encodes a protein similar to cellular repressor CREG of E1A-stimulated gene involved in transcriptional regulation relating to cell growth or differentiation (Similar to cellular repressor of E1A-stimulated genes CREG (LOC289185), mRNA).

A gene having the same or substantially the same base sequence as the base sequence shown in SEQ ID NO:35 encodes an enzyme, which is conjugated with ubiquitin, and regulates transfer from G1 phase to S phase and chromosome alignment (similar to cell division cycle 34; ubiquitin-conjugating enzyme E2-32 KDA complementing; ubiquitin carrier protein; ubiquitin-protein ligase (LOC299602), mRNA).

A gene having the same or substantially the same base sequence as the base sequence shown in SEQ ID NO:37 (hereinafter sometimes to be abbreviated as "ubadc1") encodes a protein assumed to be an intracellular signal molecule capable of regulating the growth of endothelial cell (ubiquitin associated domain containing 1).

A gene having the same or substantially the same base sequence as the base sequence shown in SEQ ID NO:39 encodes a protein similar to eukaryotic translation elongation factor 1ε1 (similar to eukaryotic translation elongation factor 1 epsilon 1 (LOC291057), mRNA).

A gene (EST) having the same or substantially the same base sequence as the base sequence shown in SEQ ID NO:41 encodes a protein similar to RNA polymerase II elongation factor ELL2 (Similar to RNA polymerase II elongation factor ELL2 (LOC309918), mRNA).

A gene having the same or substantially the same base sequence as the base sequence shown in SEQ ID NO:43 (hereinafter sometimes to be abbreviated as "ctsb") encodes cysteine protease belonging to the papain family derived from lysosome (cathepsin B).

A gene having the same or substantially the same base sequence as the base sequence shown in SEQ ID NO:45 (hereinafter sometimes to be abbreviated as "usp15") encodes an enzyme belonging to the ubiquitin specific cysteine protease family (ubiquitin specific protease 15).

A gene having the same or substantially the same base sequence as the base sequence shown in SEQ ID NO:47 (hereinafter sometimes to be abbreviated as "emb") encodes a transmembrane protein, which is a cell adhesion molecule (embigin).

A gene having the same or substantially the same base sequence as the base sequence shown in SEQ ID NO:49 (hereinafter sometimes to be abbreviated as "glu1") encodes glutamine synthetase (glutamine synthetase 1).

A gene having the same or substantially the same base sequence as the base sequence shown in SEQ ID NO:51 (hereinafter sometimes to be abbreviated as "1cn7") encodes cathepsin B associated protein inactive as a catalyst, which is assumed to be an extracellular matrix protein (lipocalin 7) .

A gene having the same or substantially the same base sequence as the base sequence shown in SEQ ID NO:53 (hereinafter sometimes to be abbreviated as "best5") encodes an antivirus protein, which is induced by interferons (Best 5 protein).

A gene having the same or substantially the same base sequence as the base sequence shown in SEQ ID NO:55 encodes a protein similar to a transcription corepressor Sin3A-associated protein (SAP30) related to histone deacetylase (Similar to Sin3A associated protein p30-like).

A gene having the same or substantially the same base sequence as the base sequence shown in SEQ ID NO:57 encodes a protein similar to hypothetical protein MGC40107 (Similar to hypothetical protein MGC40107 (LOC287442), mRNA).

A gene (EST) having the same or substantially the same base sequence as the base sequence shown in SEQ ID NO:59 has a sequence similar to mRNA of cellular repressor CREG of a gene stimulated by E1A involved in the transcriptional regulation relating to the cell growth or differentiation (Similar to cellular repressor of E1A-stimulated genes CREG (LOC289185), mRNA).

A gene (EST) having the same or substantially the same base sequence as the base sequence shown in SEQ ID NO:60 has a sequence similar to mRNA of an antiapoptotic protein, which potentiates the activation of JNK1 (Similar to amyotrophic lateral sclerosis 2 (juvenile) chromosome region, candidate 2).

A gene (EST) having the same or substantially the same base sequence as the base sequence shown in SEQ ID NO:61 has a sequence similar to mRNA of RNA binding protein involved in the translational repression of mRNA in germ cell (Similar to germ cell specific Y-box binding protein).

A gene (EST) having the same or substantially the same base sequence as the base sequence shown in SEQ ID NO:62 has a sequence similar to mRNA of cytoskeletal anchoring protein Ankyrin1 that adheres cytoskeletal element to a plasma membrane (Similar to Ankyrin 1).

The function of a gene (EST) having the same or substantially the same base sequence as the base sequence shown in SEQ ID NO:63 is unknown.

A gene having the same or substantially the same base sequence as the base sequence shown in SEQ ID NO:64 (hereinafter sometimes to be abbreviated as "anxa1") encodes calcium-dependent phospholipid binding protein that inhibits phospholipase A2 (annexinA1).

A gene having the same or substantially the same base sequence as the base sequence shown in SEQ ID NO:66 (hereinafter sometimes to be abbreviated as "nudt4") encodes diphosphoinositol polyphosphate phosphohydrolase possibly involved in the metabolism of inositol phosphate (nudix (nucleoside diphosphate linked moiety X)-type motif 4).

A gene having the same or substantially the same base sequence as the base sequence shown in SEQ ID NO:68 (hereinafter sometimes to be abbreviated as "picalm") encodes a protein, which is bound with a clathrin heavy chain and acts on endocytosis (Phosphatidylinositol binding clathrin assembly protein).

A gene having the same or substantially the same base sequence as the base sequence shown in SEQ ID NO:70 (hereinafter sometimes to be abbreviated as "S100a8") encodes a protein, which is a member of the S100 family, forms a complex with S100A9, and mediates arachidonic acid secretion and leukocyte supplementation to a site of inflammation (S100 calcium binding protein A8 (calgranulin A)).

A gene having the same or substantially the same base sequence as the base sequence shown in SEQ ID NO:72 (hereinafter sometimes to be abbreviated as "S100a9") encodes a protein, which is a member of the S100 family, forms a complex with S100A8, and mediates arachidonic acid secretion and leukocyte supplementation to a site of inflammation (S100 calcium binding protein A9 (calgranulin B)).

A gene having the same or substantially the same base sequence as the base sequence shown in SEQ ID NO:74 (hereinafter sometimes to be abbreviated as "dgat2") encodes a member of the diacylglycerol acyltransferase family, which catalyzes triacylglycerol biosynthesis (Diacylglycerol O-acyltransferase homolog 2 (mouse)).

A gene having the same or substantially the same base sequence as the base sequence shown in SEQ ID NO:76 (hereinafter sometimes to be abbreviated as "cxcr4") encodes GPCR, which is bound with CXC chemokine (Chemokine (C-X-C motif) receptor 4).

A gene having the same or substantially the same base sequence as the base sequence shown in SEQ ID NO:78 (hereinafter sometimes to be abbreviated as "illb") encodes cytokine that regulates defense reaction and inflammation reaction (interleukin 1 beta).

A gene having the same or substantially the same base sequence as the base sequence shown in SEQ ID NO:80 (hereinafter sometimes to be abbreviated as "fb×15") encodes assumed subunit of SCF ubiquitin ligase involved in proteolysis (F-box and leucine-rich repeat protein 5 (predicted)).

A gene having the same or substantially the same base sequence as the base sequence shown in SEQ ID NO:82 (hereinafter sometimes to be abbreviated as "pglyrp1") encodes a protein, which is bound with peptidoglycan and gram-positive bacterium and is involved in congenital immunity (peptidoglycan recognition protein 1).

A gene having the same or substantially the same base sequence as the base sequence shown in SEQ ID NO:84 (hereinafter sometimes to be abbreviated as "npm1") encodes a protein, which is a nucleic acid binding ribonuclease possibly involved in ribosome assembly and is a molecular chaperon (nucleophosmin 1).

A gene having the same or substantially the same base sequence as the base sequence shown in SEQ ID NO:86 encodes a serum protein (Similar to leucine-rich alpha-2-glycoprotein) containing plural leucine-rich repeats.

A gene having the same or substantially the same base sequence as the base sequence shown in SEQ ID NO:88 (hereinafter sometimes to be abbreviated as "alox15") encodes an enzyme that converts arachidonic acid to 15-hydroperoxyeicosatetraenoic acid and lipoxin A4 (arachidonate 12-lipoxygenase).

A gene having the same or substantially the same base sequence as the base sequence shown in SEQ ID NO:90 (hereinafter sometimes to be abbreviated as "cut11") encodes a protein (Cut-like 1 (Drosophila)) that acts on the cell cycle of mitosis, and possibly regulates an immune response to apoptosis-inducing stimulation.

A gene having the same or substantially the same base sequence as the base sequence shown in SEQ ID NO:92 encodes tyrosine-serine phosphatase that inhibits the progression of cell cycle (cyclin-dependent kinase inhibitor 3 (predicted)).

A gene having the same or substantially the same base sequence as the base sequence shown in SEQ ID NO:94 encodes a protein, which is a member of the prenylated Rab acceptor (PRA1) family and has moderate similarity to a glutamic acid transporter (Similar to DXIm×39e protein).

A gene having the same or substantially the same base sequence as the base sequence shown in SEQ ID NO:96 encodes an erythrocyte transmembrane sialoglycoprotein, which forms a triple complex with Epb4.1 and Mpp1 and possibly plays a role in the regulation of cell shape (Similar to glycophorin C isoform 2).

A gene having the same or substantially the same base sequence as the base sequence shown in SEQ ID NO:98 encodes a member of uncharacterized protein UPF0171 family (similar to CGTHBA protein (-14 gene protein) (predicted)).

A gene (EST) having the same or substantially the same base sequence as the base sequence shown in SEQ ID NO:100 has a sequence similar to mRNA of cytoplasmic enzyme that synthesizes thymidine acid for DNA synthesis (similar to thymidine kinase 1).

The function of a gene (EST) having the same or substantially the same base sequence as the base sequence shown in SEQ ID NO:101 is unknown.

The genes having the same or substantially the same base sequence as the base sequences shown in SEQ ID NOs: 1, 3, 5, 7, 9, 11, 13, 15, 64, 66, 68, 70, 72, 74, 76, 78, 80, 82, 84 and 86 show increased in vivo expression in correlation with PLsis expression in some organ (see Example 2, Table 3 and Example 4, Table 7 to be described below), and the genes having the same or substantially the same base sequence as the base sequences shown in SEQ ID NOs: 17, 19, 21, 23, 25, 27, 29, 31, 33, 35, 37, 39, 41, 43, 45, 47, 49, 51, 53, 55, 57, 59 - 63, 88, 90, 92, 94, 96, 98, 100 and 101 (see Example 2, Table 4 and Example 4, Table 8 to be described below) show decreased expression in correlation with PLsis expression.

As a nucleic acid capable of detecting the expression of a PLsis marker gene contained in the reagent for determining PLsis of the present invention (hereinafter sometimes to be abbreviated as "the reagent of the present invention"), for example, a nucleic acid (probe) capable of hybridizing to a transcription product of a PLsis marker gene, an oligonucleotide set capable of functioning as a primer amplifying a part or whole of the transcription product and the like can be mentioned. That is, as the nucleic acid, for example, a nucleic acid capable of hybridizing to a nucleic acid having a base sequence shown by SEQ ID NO n (wherein n is any of an odd number between 1 and 57, an integer number between 59 and 63, an even number between 64 and 98, 100 or 101) (sense strand = coding strand) under high stringent conditions, and/or a nucleic acid capable of hybridizing to a nucleic acid having a base sequence complementary to the base sequence (antisense strand = non-coding strand) under high stringent conditions can be preferably mentioned. Being "capable of hybridizing under high stringent conditions" means as defined above. The nucleic acid may be a DNA or RNA, or a DNA/RNA chimera. Preferably, DNA can be mentioned.

The nucleic acid to be used as a probe may be double strand or single strand. In the case of a double-strand, it may be a double-strand DNA, a double-strand RNA or a DNA:RNA hybrid. In the case of a single strand, a sense strand (e.g., in case of cDNA, cRNA) or an antisense strand (e.g., in case of mRNA, cDNA) can be selected for use according to the sample to be used. The length of the nucleic acid is not particularly limited as long as it can specifically hybridize to a target nucleic acid and, for example, it is not less than about 15 bases, preferably not less than about 30 bases. The nucleic acid is preferably labeled with a labeling agent to enable detection or quantitation of a target nucleic acid.

As examples of the labeling agent, a radioisotope, an enzyme, a fluorescent substance, a luminescent substance and the like can be used. Examples of the radioisotope include [$^{32}P$], [$^{3}H$], [$^{14}C$] and the like. As the enzyme, those that are stable and high in specific activity are preferred; for example, β-galactosidase, β-glucosidase, alkaline phosphatase, peroxidase, malate dehydrogenase and the like can be used. As examples of the fluorescent substance, fluorescamine, fluorescein isothiocyanate and the like can be used. As examples of the luminescent substance, luminol, luminol derivative, luciferin, lucigenin and the like can be used. Furthermore, a biotin-(strepto)avidin system can also be used for binding of a probe with a labeling agent. For immobilization of a nucleic acid to be a probe on a solid phase, a nucleic acid in a sample can be labeled using a labeling agent similar to those mentioned above.

An oligonucleotide set to be used as a primer is not particularly limited as long as it can specifically hybridize to each of a base sequence shown in each SEQ ID NO (sense strand) and a base sequence complementary thereto (antisense strand), and amplify a DNA fragment sandwiched therebetween and, for example, a set of oligo DNAs designed to have a length of about 15 to about 100 bases, preferably about 15 to about 50 bases, and amplify about 100 bp to several kbp DNA fragments can be mentioned.

For quantitative analysis of PLsis marker gene expression using a trace amount of an RNA sample, competitive RT-PCR or real-time RT-PCR is preferably used. Competitive RT-PCR is a method for calculating the amount of an object DNA by carrying out a competitive amplification reaction in a reaction mixture in the co-presence of a known amount of other template nucleic acid, which can be amplified by a primer set capable of amplifying the object DNA as a competitor, and comparing the amounts of the amplification products. When using competitive RT-PCR, therefore, the reagent of the present invention can further contain, besides the above-mentioned primer set, a nucleic acid which is amplified by the primer set to produce an amplification product distinguishable from the object DNA (e.g., amplification product different from the object DNA in size, amplification product showing different migration pattern by a restriction enzyme treatment and the like). This competitor nucleic acid may be a DNA or an RNA. In the case of a DNA, cDNA is synthesized from an RNA sample by a reverse transcription reaction and a competitor is added to perform PCR, and in the case of an RNA, it may be added to an RNA sample from the start to perform RT-PCR. In the latter case, an absolute amount of the original mRNA can also be assumed since the efficiency of the reverse transcription reaction is taken into consideration.

On the other hand, real-time RT-PCR does not require electrophoresis, since the amplification amount by PCR can be monitored real-time, and the expression of a PLsis marker gene can be analyzed more rapidly. Generally, monitoring is performed using various fluorescent reagents. These include reagents (intercalator) emitting fluorescence by binding to double stranded DNA such as SYBR Green I, ethidium bromide and the like, nucleic acids usable as the above-mentioned probes (the nucleic acid hybridizes to the target nucleic acid within amplification region), wherein the both ends are respectively modified with a fluorescent substance (e.g., FAM, HEX, TET, FITC etc.) and a quenching substance (e.g., TAMRA, DABCYL etc.) and the like.

The nucleic acid functionable as a probe capable of detecting the expression of a PLsis marker gene can be obtained by amplifying a nucleic acid having a desired length by PCR using the above-mentioned primer set capable of amplifying a part or whole of a transcription product of the gene and using cDNA or genomic DNA from any cell (e.g., hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, or interstitial cell, or corresponding precursor cell, stem cell or cancer cell thereof, and the like) of a mammal (e.g., human, monkey, bovine, horse, swine, sheep, goat, dog, cat, rabbit, hamster, guinea pig, mouse, rat and the like), or any tissue where such cells are present (e.g., brain, any portion of the brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, lung, gastrointestinal tract (e.g., large intestine, small intestine), blood vessel, heart, thymus, spleen, submandibular gland, peripheral blood, prostate, testicle, ovary, placenta, uterus, bone, joint, adipose tissue, skeletal muscle, and the like) as a template, or cloning the above-mentioned PLsis marker gene or cDNA from cDNA or genomic DNA library derived from the aforementioned cell or tissue by colony or plaque hybridization and the like and, where necessary, treating them with a restriction enzyme and the like to give a fragment having a suitable length. The hybridization can be carried out according to the method described in, for example, Molecular Cloning, 2nd. ed. (mentioned above) and the like. When a commercially available library is used, hybridization can be carried out according to the method described in the instruction manual attached to the library. Alternatively, the nucleic acid can also be obtained by chemically synthesizing a part or whole of the base sequence and/or its complementary strand sequence based on the information of a base sequences shown in SEQ ID NO n (wherein n is an odd number between 1 and 57, an integer number between 59 and 63, an even number between 64 and 98, 100 or 101) using a commercially available DNA/RNA automatic synthesizer and the like. In addition, a chip with a solid phased nucleic acid can also be prepared by direct *in situ* (on chip) synthesis of the nucleic acid on a solid phase such as silicone, glass and the like.

The nucleic acid functionable as a primer capable of amplifying a part or whole of a transcription product of a PLsis marker gene can be obtained by chemically synthesizing a part of a base sequence and its complementary strand sequence, based on the information of the base sequences shown in SEQ ID NO n (wherein n is an odd number between 1 and 57, an integer number between 59 and 63, an even number between 64 and 98, 100 or 101), using a commercially available DNA/RNA automatic synthesizer and the like.

The nucleic acid capable of detecting the expression of a PLsis marker gene can be provided as a solid in a dry state or in the form of an alcohol precipitate, or in a dissolution state in water or a suitable buffer (e.g., TE buffer etc.). When used as a labeled probe, the nucleic acid can be provided after labeling with any of the above-mentioned labeling substances, or provided independently of a labeling substance and labeled when in use.

Alternatively, the nucleic acid can be immobilized on a suitable solid phase. The solid phase is exemplified by, but not limited to, glass, silicone, plastic, nitrocellulose, nylon, polyvinylidene difluoride and the like. The immobilizing means is exemplified by, but not limited to, a method comprising previously introducing a functional group such as amino group; aldehyde group, SH group, biotin and the like into a nucleic acid, introducing, onto a solid phase, a functional group (e.g., aldehyde group, amino group, SH group, streptavidin and the like) reactive with the nucleic acid, and crosslinking the solid phase and the nucleic acid with covalent bond between these functional groups, or immobilizing a polyanionic nucleic acid with electrostatic bond using a polycation-coated solid phase and the like.

One preferable embodiment of a nucleic acid probe immobilized on a solid phase is ArrayPlate™ etc. provided by High Throughput Genomics, Inc. ArrayPlate™ is a 96 well plate immobilized with various nucleic acid probes regularly disposed in the bottom of each well (e.g., 4x4 array). By the presence of a nucleic acid having one end hybridizable to a probe and the other end hybridizable to a target nucleic acid as a mediating spacer, a hybridization reaction of the probe with a target nucleic acid can be carried out in a liquid phase rather than on the solid phase surface, which enables a quantitative measurement of the target nucleic acid. Accordingly, expression variation of various PLsis marker genes can be simultaneously detected at once in a single well, and once sufficient quantitation performance is achieved, an advantage of higher efficiency than real-time PCR wherein expression variation in each marker gene is individually detected can be afforded.

The reagent of the present invention can further contain, in addition to a nucleic acid capable of detecting the expression of a PLsis marker gene, other substance necessary for the reaction for detecting the expression of the gene, which does not interfere with the reaction when preserved in coexistence. Alternatively, the reagent of the present invention can also be provided as a kit together with a separate reagent containing other substance necessary for the reaction for detecting the expression of a PLsis marker gene. For example, when the reaction for detecting the expression of a PLsis marker gene is PCR, as said other substance, for example, a reaction buffer, dNTPs, a thermostable DNA polymerase and the like can be mentioned. When competitive PCR or real-time PCR is used, a competitor nucleic acid, a fluorescent reagent (the above-mentioned intercalator, fluorescence probe etc.) and the like can be further contained.

In drug-induced PLsis, for example, it is ideal that each PLsis marker gene should show expression variation for any PLsis-inducing compound and substantially no expression variation for any PLsis non-inducing compound, though, in fact, such results cannot be obtained all the time. Therefore, when expression of each marker gene is used as a single index, emergence of a certain extent of false-positive and false-negative compounds is generally inevitable. However, by examining expression variation of plural PLsis marker genes, the determination accuracy can be further improved.

Accordingly, the present invention also provides a kit for determining PLsis, which contains two or more reagents containing a nucleic acid capable of detecting a PLsis marker gene in combination. The nucleic acid to be contained in each reagent can detect PLsis marker genes different from each other. Moreover, at least one of the reagents contains the above-mentioned reagent of the present invention, namely, a nucleic acid capable of detecting a gene having a base sequence the same or substantially the same as the base sequence shown by any of SEQ ID NO n (wherein n is an odd number between 1 and 57, an integer number between 59 and 63, an even number between 64 and 98, 100 or 101). More preferably, a kit that detects any 2 or more, further preferably 3 or more, still more preferably 4 or more, particularly preferably 5 or more, and most preferably 6 or more, of the above-mentioned 54 PLsis marker genes can be mentioned.

As the nucleic acid capable of detecting a PLsis marker gene possibly contained in a reagent other than the reagent of the present invention, which is contained as the constituent member of the kit of the present invention, is not particularly limited and, for example, a nucleic acid capable of detecting a PLsis marker gene other than the above-mentioned 54 PLsis marker genes, such as a gene encoding a lysosomal enzyme, a gene encoding a lipid metabolism (e.g., cholesterol synthesis, fatty acid elongation, unsaturated fatty acid synthesis etc.)-related protein, a gene encoding a transport (e.g., fatty acid transport, protein transport, amino acid transport etc.)-related protein, a gene encoding a cell growth-related protein, a gene encoding a protease or protease inhibitor, a gene encoding an amino acid metabolism-related protein and the like can be mentioned. More specifically, a nucleic acid capable of detecting human genes having base sequences registered in the GenBank database under the ID Nos. of NM_000859, AL518627, NM_002130, AA639705, BC005807, AF116616, NM_025225, D80010, NM_001731, AW134535, NM_004354, AF135266, AC007182, NM_003832, NM_019058, AB040875, AA488687, NM_018687, NM_021158, BG231932, NM_000235, AA873600, AF096304, AW150953, NM_001360, AC001305, NM_024090, NM_006214, NM_024108, NM_021980, AF003934, NM_000596, U15979, M92934, NM_002087, AK023348, NM_002773, NM_000131, BC003169, NM_002217, NM_003122, NM_001673, NM_000050, U08024, NM_003167, BC005161, AF162690, AW517464, AF116616, NM_017983, NM_016061, BE966922, BE552428, NM_012445, NM_000792, NM_015930, NM_021800, NM_005980, NM_000565, AB033025, AL110298, NM_006931, NM_001955, NM_003897, AA778684, NM_001283, NM_012242, AI934469, NM_003186 and NM_002450 (see above Sawada, H. et al., *Toxicol. Sci., 83*: 282-92 (2005)), orthologues thereof in other mammals and the like can be mentioned.

The nucleic acid contained in each reagent constituting the kit is particularly preferably so constructed as to detect expression of a PLsis marker gene by the same method (e.g., northern blot, dot blot, DNA array technique, quantitative RT-PCR etc.).

The constitution of the kit of the present invention is exemplified by, but not limited to, one wherein the above-mentioned reagents are separately provided [e.g., when nucleic acid functions as a labeled probe (particularly dot blot analysis), a primer for PCR (particularly real-time quantitative PCR) etc.], one wherein nucleic acids capable of detecting the expression of different PLsis marker genes are contained in a single reagent [e.g., when nucleic acid functions as PCR (particularly, when each marker gene can be distinguished by the size of an amplification product and the like), a labeled probe (particularly, when each marker gene can be distinguished by the size of a transcription product by northern blot analysis) and the like], one wherein nucleic acids capable of detecting the expression of different PLsis marker genes are immobilized in separate regions of a single solid phase [e.g., when nucleic acid functions as a probe for hybridization to label cRNA etc., and the like] and the like.

The present invention also provides a method for determining PLsis in a mammal, comprising detecting expression variation of one or more genes showing varying expression in correlation with PLsis expression in a sample from the mammal. Examples of the mammal include, but not limited to, human, monkey, bovine, horse, swine, sheep, goat, dog, cat, rabbit, hamster, guinea pig, mouse, rat and the like. While preferable animal species can be appropriately selected depending on the object, for example, when the PLsis-inducibility of a drug candidate compound is to be evaluated, human is preferably used in the clinical stage, and monkey, rat, mouse, dog and the like are preferably used in the preclinical stage.

As PLsis in mammals, drug-induced PLsis (encompassing any embodiment such as PLsis caused by administration of a pharmaceutical agent or a veterinary drug or a candidate compound thereof, PLsis caused by accidental ingestion of toxin or exposure to a chemical substance present in the environment etc.), PLsis associated with hereditary phospholipid dysbolism, other disease and the like can be mentioned. Thus, as a mammal to be the test target in the method of the present invention, a test subject (experimental animal) to whom a pharmaceutical agent (or veterinary drug) candidate compound is to be administered, a patient (patient animal) to whom a pharmaceutical agent (or veterinary drug) possibly expressing PLsis has been administered, human or other mammal (possibly) exposed to a chemical substance and the like capable of inducing PLsis and the like, a mammal affected or possibly affected (in the future) with a hereditary or nonhereditary disease possibly inducing PLsis and the like can be mentioned.

A method of exposing a mammal to a test compound for determination of a drug-induced PLsis by a pharmaceutical agent (or veterinary drug) candidate compound is not particularly limited. For example, the test compound can be administered in the form of a solid, a semi-solid, a liquid, an aerosol and the like orally or parenterally (e.g., intravenously, intramuscularly, intraperitoneally, intraarterially, subcutaneously, intradermally, airway etc.). The dose of the test compound varies depending on the kind of the compound, animal species, body weight, administration form and the like and, for example, an amount within the range where the animal can survive and necessary for the target cell to be exposed to the highest concentration of the test compound, at which the cell can survive, for more than a given time and the like can be mentioned. The dose can be administered in one to several portions. While the time from the administration to the sample collection varies depending on the kind of a marker gene used, the *in vivo* kinetics of the test compound and the like, in general, it is appropriately selected from the range of about 3 hr to about 8 weeks, preferably about 2 to about 14 days from first dose.

As a sample from a mammalian, any cell of a mammal (e.g., human, monkey, bovine, horse, swine, sheep, goat, dog, cat, rabbit, hamster, guinea pig, mouse, rat and the like), (e.g., hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, interstitial cell, or corresponding precursor cell, stem cell or cancer cell thereof, and the like), or any tissue where such cells are present (e.g., brain, any portion of the brain (e.g., olfactory bulb, amygdaloid nucleus, basal ganglia, hippocampus, thalamus, hypothalamus, cerebral cortex, medulla oblongata, cerebellum), spinal cord, eyeball, hypophysis, stomach, pancreas, kidney, liver, gonad, thyroid, gall-bladder, bone marrow, adrenal gland, skin, lung, gastrointestinal tract (e.g., large intestine, small intestine), blood vessel, heart, thymus, spleen, submandibular gland, peripheral blood, prostate, testicle, ovary, placenta, uterus, bone, joint, adipose tissue, skeletal muscle, and the like), and the like can be mentioned. Blood (e.g., peripheral blood), lymphocyte and the like are particularly preferable since rapid and convenient collection is possible, it is less-invasive to animals, and the like.

The PLsis marker gene whose expression variation is examined by the determination method of the present invention is not particularly limited as long as at least one of them has a base sequence the same or substantially the same as the base sequence shown by any of SEQ ID NO: n (wherein n is an odd number between 1 and 57, an integer number between 59 and 63, an even number between 64 and 98, 100 or 101). Preferably, a method for using any two or more, preferably three or more, more preferably four or more, particularly preferably five or more, most preferably six or more, of the above-mentioned 54 PLsis marker genes as detection target can be mentioned.

As the PLsis marker gene other than the above-mentioned 54 genes which can be the detection target in the determination method of the present invention, for example, a gene encoding a lysosomal enzyme, a gene encoding a lipid metabolism (e.g., cholesterol synthesis, fatty acid elongation, unsaturated fatty acid synthesis etc.)-related protein, a gene encoding a transport (e.g., fatty acid transport, protein transport, amino acid transport etc.)-related protein, a gene encoding a cell growth-related protein, a gene encoding a protease or protease inhibitor, a gene encoding an amino acid metabolism-related protein and the like can be mentioned. More specifically, human genes having base sequences registered in the GenBank database under the ID Nos. of NM_014960, NM_000859, AL518627, NM_002130, AA639705, BC005807, AF116616, NM_025225, U47674, D80010, NM_001731, AW134535, NM_004354, AF135266, AC007182, NM_003832, NM_019058, AB040875, AA488687, NM_018687, NM_021158, BG231932, NM_024307, NM_000235, AA873600, D63807, AF096304, AW150953, NM_001360, NM_021969, AC001305, NM_024090, NM_001443, NM_006214, NM_024108, NM_021980, NM_002151, AF003934, NM_000596, U15979, M92934, NM_002087, AK023348, NM_002773, NM_000131, BC003169, NM_002217, NM_003122, NM_001673, NM_000050, NM_001085, U08024, NM_003167, BC005161, AF162690, AW517464, AF116616, NM_017983, AL136653, NM_016061, BE966922, BE552428, NM_022823, NM_012445, NM_000792, NM_015930, NM_021800, NM_005980, NM_000565, AB033025, NM_006931, AL110298, NM_006931, NM_001955, NM_003897, NM_003186, AA778684, NM_001283, NM_012242, AI934469, NM_003186 and NM_002450 (see above Sawada, H. et al., Toxicol. Sci., 83: 282-92 (2005)), orthologues thereof in other mammals and the like can be mentioned.

The expression of a PLsis marker gene in a sample from a mammal can be examined by preparing an RNA (e.g., total RNA, mRNA) fraction from the sample, and detecting a transcription product of the marker gene contained in the fraction. An RNA fraction can be prepared by a known means such as guanidine-CsCl ultracentrifugation method, AGPC method and the like. A high purity total RNA can be prepared rapidly and conveniently from a trace sample using a commercially available RNA extraction kit (e.g., RNeasy Mini Kit; manufactured by QIAGEN etc.). As a means for detecting a transcription product of a PLsis marker gene in an RNA fraction, for example, a method using hybridization (northern blot, dot blot, DNA chip analysis etc.), a method using PCR (RT-PCR, competitive PCR, real-time PCR etc.) and the like can be mentioned. Since expression variation of a PLsis marker gene can be detected rapidly and conveniently with high quantitation performance from a trace sample, quantitative PCR such as competitive PCR, real-time PCR and the like is preferable, and since expression variation of multiple marker genes can be detected at once and quantitation performance can be improved by the selection of a detection method and the like, DNA chip analysis is preferable.

In the case of northern blot or dot blot hybridization, a PLsis marker gene can be detected using the above-mentioned reagent or kit of the present invention containing a nucleic acid to be used as a labeled probe. In the case of Northern hybridization, therefore, an RNA fraction prepared as mentioned above is separated by gel electrophoresis, transferred to a membrane such as nitrocellulose, nylon, polyvinylidene difluoride and the like, hybridized under the above-mentioned "high stringent conditions" in a hybridization buffer containing the reagent of the present invention or each reagent contained in the kit of the present invention, and the label amount bonded to the membrane by a suitable method is measured for each band, whereby the expression amount of each PLsis marker gene can be measured. In the case of dot blot, too, a membrane spotted with an RNA fraction is subjected to a hybridization reaction in the same manner (conducted for each PLsis marker gene), and the label amount of the spot is measured, whereby the expression amount of each marker gene can be measured.

In the case of DNA chip analysis (solid phased probe described for the above-mentioned reagent of the present invention), for example, cDNA harboring a suitable promoter such as T7 promoter and the like is synthesized by reverse transcription reaction from the RNA fraction prepared as mentioned above, and cRNA is synthesized using an RNA polymerase (where labeled cRNA is obtained by using, as a substrate, a mononucleotide labeled with biotin and the like). The labeled cRNA is contacted with the above-mentioned solid phased probe to allow hybridization reaction, and the label amount bonded to each probe on the solid phase is measured, whereby the expression amount of each PLsis marker gene can be measured. This method is advantageous in terms of rapidness and convenience as the number of PLsis marker genes (i.e., probes to be solid phased) to be detected increases.

In a preferable embodiment of the prediction method of the present invention, as a method for detecting the expression of a PLsis marker gene, quantitative PCR is used. As quantitative PCR, for example, competitive PCR, real-time PCR and the like can be mentioned. Since electrophoresis after amplification reaction is not necessary, real-time PCR is more superior in rapid performance.

In the case of competitive PCR, a known amount of a competitor nucleic acid is used, which can be amplified by said primer set, and after amplification, can be distinguished from an amplification product of a target nucleic acid (i.e., a transcription product of a PLsis marker gene), based on different amplification size, different migration pattern of restriction enzyme-treated fragment and the like in addition to the primer set described for the above-mentioned reagent of the present invention. Since amplification occurs competitively where a target nucleic acid and a competitor nucleic acid compete for a primer, the amount ratio of the amplification products reflects the amount ratio of the original templates. The competitor nucleic acid may be DNA or RNA. In the case of DNA, cDNA is synthesized from an RNA fraction prepared as mentioned above by reverse transcription reaction, and then PCR is performed in the coexistence of the reagent of the present invention and a competitor, and in the case of RNA, a competitor is added to an RNA fraction to allow reverse transcription reaction, and the reagent of the present invention is further added to perform PCR.

In real-time PCR, the amplification amount is monitored in real-time using a fluorescent reagent, and an apparatus integrally comprising a thermal cycler and a spectrofluorophotometer is necessary. Such apparatus is commercially available. There are several methods depending on the fluorescent reagent to be used and, for example, intercalator method, TaqMan™ probe method, Molecular Beacon method and the like can be mentioned. In any case, cDNA is synthesized by reverse transcription reaction from an RNA fraction prepared as mentioned above, and the reagent of the present invention and a fluorescent reagent (probe) called intercalator, TaqMan™probe or Molecular Beacon probe are added to PCR reaction system. Since intercalator binds to a synthesized double stranded DNA and emits fluorescence upon irradiation of excitation light, the amount of an amplification product can be monitored by measuring the intensity of fluorescence, based on which the amount of original template cDNA can be assumed. The TaqMan™ probe is an oligonucleotide capable of hybridizing to an amplification region of the target nucleic acid, which has both ends modified by a fluorescent substance and a quenching substance, respectively. It hybridizes to a target nucleic acid during annealing but is prohibited from emitting fluorescence by the presence of the quenching substance, and emits fluorescence when decomposed by the exonuclease activity of DNA polymerase during elongation, which releases the fluorescent substance. Accordingly, by measuring fluorescence intensity, the amount of the amplification product can be monitored, based on which the amount of original template cDNA can be assumed. The Molecular Beacon probe is an oligonucleotide capable of hybridizing to an amplification region of a target nucleic acid and having a hairpin type secondary structure, which has both ends modified by a fluorescent substance and a quenching substance, respectively. When it has a hairpin structure, it does not emit fluorescence due to the presence of a quenching substance, and emits fluorescence when the distance between the fluorescent substance and the quenching substance grows upon hybridization to the target nucleic acid during annealing. Therefore, the amount of the amplification product can be monitored by measuring the fluorescence intensity, based on which the amount of original template cDNA can be assumed.

Alternatively, expression of PLs is marker gene in a sample from a mammal can be determined by preparing a protein fraction from the sample and detecting a translation product (i.e., marker protein) of the marker gene in the fraction. Detection of a marker protein can be performed by an immunological measurement method (e.g., ELISA, FIA, RIA, Western blot etc.) using an antibody to each protein, and can also be performed by measuring the physiological activity of a protein showing measurable physiological activity of enzyme and the like, by a known method for each marker protein. Alternatively, a marker protein can be detected by mass spectrometry such as MALDI-TOFMS and the like.

The antibody to each marker protein can be obtained according to a general technique used for the production of a polyclonal antibody or monoclonal antibody, which uses, as a sensitizing antigen, a protein or partial peptide thereof, or a salt thereof having the same or substantially the same amino acid sequence as the amino acid sequence shown in SEQ ID NO: m (wherein m is an even number between 2 and 58, or an odd number between 65 and 99), or a protein or partial peptide thereof, or a salt thereof having the same or substantially the same partial amino acid sequence as the partial amino acid sequence encoded by each base sequence shown in SEQ ID NO: n (wherein n is an integer number between 59 and 63, 100 or 101).

In the determination method of the present invention, the standard according to which the presence or absence of a PLsis expression is judged is not particularly limited as long as the determination results based on the standard have sufficient reliability for use as a diagnostic system (compound evaluation system in clinical or preclinical tests) for the pathology. For example, (1) a method for determining PLsis positive when expression of all PLsis marker genes to be the detection target increases or decreases (where "expression substantially increases or decreases" means as defined above), and determining PLsis negative when expression of any of the PLsis marker genes does not substantially change (where "expression does not substantially change" means as defined above), (2) a method for determining PLsis negative when expression of all PLsis marker genes to be the detection target does not substantially change, and determining PLsis positive when expression of any of PLsis marker genes substantially increases or decreases, (3) a method for determining PLsis positive when expression of not less than a certain number (e.g., 2-(n-1) genes) out of PLsis marker genes in the number of n to be the detection target substantially increases or decreases and the like can be mentioned. However, the above-mentioned method (1) is defective in that the frequency of appearance of false-positive can be reduced, but the frequency of appearance of false-negative increases and a considerable number of PLsis expressions may be overlooked. On the other hand, according to the method of (2), the frequency of appearance of false-negative can be reduced, but the frequency of appearance of false-positive increases, which possibly eliminates potential compounds in, for example, clinical or preclinical tests, and the room of development of pharmaceutical products and the like may be narrowed.

As diagnostic criteria for avoiding the above-mentioned problems and determining PLsis expression with higher precision, for example, use of Phospholipidosis mRNA score (to be referred to as the "average variation rate" in the present specification) described in above Sawada, H. et al., *Toxicol. Sci., 83*: 282-92 (2005) can be mentioned. The "average variation rate" is defined as follows. That is, an expression amount is measured for each marker gene when mammal is and is not exposed to a test compound and upon exposure, when the expression amount increased, its magnification (e.g., 2 when increased two-fold) is taken as an expression variation rate (X) of each gene, and when the expression amount decreased, an inverse number of its magnification (e.g., 2 when decreased to 1/2) is taken as an expression variation rate (X) of each gene, and an average value of the expression variation rate of the total marker genes (n genes) is defined to be an average variation rate (following formula).

$$\text{Average variation rate} = m_1 X_1 + m_2 X_2 + \cdots + m_n X_n$$

$$(m_1 + m_2 + \cdots + m_n = 1)$$

wherein $m_i$ (i=1-n) shows the weight of each gene. While the weight is not particularly limited, it is preferably $m_i \times n=0.2\text{-}5$, for example, it is the same weight for all ($m_i =1/n$) .

For example, a mammal is exposed to each of 2 or more (preferably 5 or more, more preferably 10 or more, more preferably 15 or more) known PLsis-inducing compounds and 2 or more (preferably 5 or more, more preferably 10 or more, more preferably 15 or more) known PLsis-noninducing compounds, in the sample from the mammal, expression variation of one or more PLsis marker genes selected (at least one of them is among the above-mentioned 54 marker genes of the present invention) is detected, and the average variation rate of expression of the marker gene and the presence or absence of actual PLsis expression are compared. The presence or absence of actual PLsis expression can be determined using vacuolated lymphocyte ratio of the peripheral blood from a mammal, or histopathological finding in one or more from various tissues (e.g., foam cell humectant in the lung and mesenteric lymph node, vacuolation of hepatocyte or bile duct epithelial cell in the liver, vacuolation of lymphocyte or foam cell humectant in the spleen, vacuolation of renal tubule epithelial cell in the kidney, vacuolation of cerebellar Purkinje cell in the brain) as an index.

As a result of comparison, an average variation rate affording correct determination of the presence or absence of the PLsis-inducibility of the above-mentioned known PLsis-inducing and non-inducing compounds with the probability of not less than about 70%, preferably not less than about 80%, more preferably not less than about 90%, particularly preferably not less than about 95%, is determined and used as a standard value. It is more preferable to study the validity of the standard value determined in this way by comparing average variation rate of PLsis marker gene expression and the presence or absence of actual PLsis expression in the same manner, using different known PLsis-inducing and noninducing compounds. When the average variation rate value affording accurate determination of the presence or absence of PLsis expression with higher probability by combining the determination results of the compounds studied anew is obtained, the standard value only needs to be amended.

Examples of other preferable diagnostic criteria applicable to the determination method of the present invention include, but are not limited to, those according to the diagnostic criteria of hepatotoxicity/nephrotoxicity-inducing potential by a compound as described in WO 02/10453 and WO 02/095000.

Abbreviations for bases, amino acids and the like used in the present specification are based on abbreviations specified by the IUPAC-IUB Commission on Biochemical Nomenclature or abbreviations in common use in relevant fields. Some examples are given below. When an enantiomer may be present in amino acid, it is of the L-configuration, unless otherwise stated.
DNA: Deoxyribonucleic acid
cDNA: Complementary deoxyribonucleic acid
A: Adenine
T: Thymine
G: Guanine
C: Cytosine
RNA: Ribonucleic acid
mRNA: Messenger ribonucleic acid
dATP: Deoxyadenosine triphosphate
dTTP: Deoxythymidine triphosphate
dGTP: Deoxyguanosine triphosphate
dCTP: Deoxycytidine triphosphate
ATP: Adenosine triphosphate
EDTA: Ethylenediaminetetraacetic acid
SDS: Sodium dodecyl sulfate
Gly: Glycine
Ala: Alanine
Val: Valine
Leu: Leucine
Ile: Isoleucine
Ser: Serine
Thr: Threonine
Cys: Cysteine
Met: Methionine
Glu: Glutamic acid
Asp: Aspartic acid
Lys: Lysine
Arg: Arginine
His: Histidine
Phe: Phenylalanine
Tyr: Tyrosine
Trp: Tryptophan
Pro: Proline
Asn: Asparagine
Gln: Glutamine
pGlu: Pyrroglutamic acid
Sec: Selenocysteine

The present invention is explained in more detail in the following by referring to Examples, which are mere examples and do not limit the scope of the present invention in any way.

**Example 1**

6 kinds of known PLsis-inducing compounds (amiodarone:1000 mg/kg/day, tamoxifen:1000 mg/kg/day, chloroquine:250 mg/kg/day, perhexiline:600 mg/kg/day, fluoxetine:300 mg/kg/day and quinacrine:200 mg/kg/day) were administered by gavage to 5-week-old Crj:CD(SD)IGS rats (Charles River Japan Inc., produced in closed environment) at a dosing liquid volume of 10 mL/kg/day once daily for 3 days (4 days for the negative control (0.5 w/v% methylcellulose solution administration)).

After completion of the administration, in all animals, livers, kidneys, spleens, lungs and mesenteric lymph nodes were sampled, with additional organs and tissues sampled for respective test compounds, i.e., adrenal glands, pituitary glands and eyeballs for tamoxifen, brains and femoral muscles for quinacrine, brains and eyeballs for chloroquine, brains and skins for perhexiline, and brains for fluoxetine; these sampled tissues were examined for histopathological changes by conventional methods of optical microscopy and electron microscopy. In addition, a blood smear sample (Giemsa stain sample) was prepared, and the vacuolated lymphocyte ratio of the peripheral blood was measured with observation under a microscope.

The results for histopathological changes and vacuolated lymphocyte ratio in the PLsis-inducing compound administration group are shown in Table 1. In all histopathological tests in the compound administration group, changes considered to be attributable to PLsis were observed. Simultaneously, the vacuolated ratio of lymphocyte was found to be high in a vacuolated lymphocyte test of peripheral blood.

Table 1

| Vacuolated lymphocyte ratio and histopathological finding in PLsis-inducing compound administrated rat | | | | | | | |
|---|---|---|---|---|---|---|---|
| Compound | Dose (mg/kg/ day) | Vacuolated lymphocyte ratio (%) | Histopathological findings (PLsis) | | | | |
| | | | Lung | Lymph node | Liver | Spleen | Others |
| Amiodarone | 1000 | 40↑ | + | + | - | + | - |
| Tamoxifen | 1000 | 40↑ | + | + | + | - | - |
| Chloroquine | 250 | 66↑ | - | + | - | + | - |
| Perhexiline | 600 | 52↑ | + | + | - | + | - |
| Fluoxetine | 300 | 31↑ | + | + | + | - | brain |
| Quinacrine | 200 | 32↑ | + | + | + | - | - |
| ↑: high value; +: PLsis lesion; - : no PLsis lesion | | | | | | | |

**Example 2**

At 24 hr after completion of the oral administration for 3 consecutive days in Example 1, the blood was collected from the compound administration group and the control (non-administration) group (3 rats per group), which was pooled and subjected to GeneChip analysis.

After collection using PAXgene Blood RNA Tube (PreAnalytiX), the blood was stood for 2 hr or more, and cryopreserved at -80°C. Total RNA was extracted using a PAXgene Blood RNA Kit (PreAnalytiX). A sample for GeneChip analysis was prepared and analyzed according to the protocol provided by Affymetrix Inc. cDNA synthesis reaction was carried out using a SuperScript Choice system (Invitrogen), then biotin-labeled cRNA synthesis reaction was carried out using Enzo BioArray HighYield RNA Transcript Labeling Kit (Affymetrix), after which cRNA was fragmented and a labeled sample was prepared. The labeled sample was hybridized to Rat Expression Array 230A (RAE230A, Affymetrix), the array was stained, washed and scanned by GeneChip system (Affymetrix), and the following three image data were obtained from one sheet of array.

<u>Image data</u>

1. data of staining by a method omitting fluorescence amplification reaction by antibody
2. data of staining by the method described in a manual for once performing fluorescence amplification reaction by antibody
3. data of staining by a method for twice performing fluorescence amplification reaction by antibody

From the image data, measurement value (Signal), numerical data such as Signal Log Ratio and the like and determination data such as Detection, Change and the like were obtained using MicroarraySuite5.0 (parameter: Default value). Fold Change was calculated from Signal Log Ratio of the image data 2 using the conversion equation of Fold Change = $2^{\text{Signal Log Ratio}}$.

Then, using a DNA microarray data analysis soft, GeneSpring (Silicon Genetics), the probe set* that showed variation by compound addition was extracted according to the following criteria, and used as a variation probe set. (*In GeneChip analysis using RAE230A, 11 sites per one measurement target sequence are measured, and the measurement results at 11 sites are combined to give one numerical value and determination data. RAE230A contains 15924 combinations of 11 site measurement sets, and each measurement set is referred to as a probe set.)

<u>Extraction criteria of variation probe set</u>

A probe set wherein two or more of the above-mentioned three image data satisfy the following three conditions was taken as a variation probe set.

1. A probe set judged as Increase or Decrease by MicroarraySuite5.0
2. A probe set whose higher measurement value (Signal) was judged as Presence by MicroarraySuite5.0
3. A probe set wherein Signal Log Ratio of treatment group shows 0.6 or above or -0.6 or below, based on the control group

The annotation information of the gene name, function, sequence and the like of the probe set was obtained from HumanPSD (Incyte), NetAffx (database relating to target mounted on GeneChip, Affymetrix).

The probe sets with expression level variation were extracted and the number is summarized in Table 2.

Table 2

| Number of expression variation probe sets in rat administrated with PLsis-inducing compound | | | |
|---|---|---|---|
| Compound | Dose (mg/kg/day) | Increased expression | Decreased expression |
| Amiodarone | 1000 | 613 | 282 |
| Tamoxifen | 1000 | 258 | 94 |
| Chloroquine | 250 | 382 | 318 |
| Perhexiline | 600 | 310 | 179 |
| Fluoxetine | 300 | 372 | 391 |
| Quinacrine | 200 | 240 | 392 |

In all 6 compounds, expression variation was observed as follows:

increase: 9 probe sets (8 genes)(Table 3),
decrease: 27 probe sets (26 genes) (Table 4).

Table 3 Probe set with increase of expression in all of six PLsis-inducing compound 3-day administration groups

| Category | Affymetrix No. | Accession No. | Gene name | Gene symbol | Function | Corresponding human gene | a | b |
|---|---|---|---|---|---|---|---|---|
| transport system | 1386902_at | NM_031355 | voltage-dependent anion channel 3 | Vdac3 | protein at opening of voltage-dependent ion channel of mitochondrial outer membrane that possibly functions for transport of adenine nucleotides | NM_005662 | 0 | |
| transport system /antioxidant action | 1367945_at | NM_053359 | ATX1 (antioxidant protein 1) homolog 1 (yeast) | Atox1 | copper binding protein that mediates intracellular copper transport and homeostasis | NM_004045 | 0 | |
| antioxidant action | 1370172_at | NM_017051 | superoxide dismutase 2, mitochondrial | Sod2 | mitochondrial enzyme that converts superoxide to hydrogen peroxide | NM_000636 | 0 | |
| cell cycle-growth-death | 1367693_at | NM_013052 | tyrosine 3-monooxygenase/ tryptophan 5-monooxygenase activation protein, eta polypeptide | Ywhah | apoptosis inhibitor and activator of tyrosine hydroxylase and tryptophan hydroxylase | NM_003405 | 0 | |
| protease inhibitor | 1367998_at | NM_053372 | secretory leukocyte protease inhibitor | Slpi | inhibitor of protease such as trypsin, cathepsin G and neutrophil elastase and the like | NM_003064 | 6 | |

(continued)

| Category | Affymetrix No. | Accession No. | Gene name | Gene symbol | Function | Corresponding human gene | a | b |
|---|---|---|---|---|---|---|---|---|
| immune response | 1367850_at | NM_053843 | Fc receptor, IgG, low affinity III | Fcgr3 | binds with immunoglobulin and initiates various immune responses | NM_000569, NM_ 000570 | 3 | |
| | 1398246_s_at | NM_053843 | Fc receptor, IgG, low affinity III | Fcgr3 | binds with immunoglobulin and initiates various immune responses | NM_000569 NM_ 000570 | 4 | |
| | 1389123_at | NM_001004 202 | chemokine (C-C motif) ligand 6 | cc16 | CC chemokine that promotes migration and infiltration of inflammatory cell | none | 6 | |
| | 1371440_at | NM_012512 | Beta-2 microglobulin | B2m | component of MHC class I antigen that acts on transepithelial transport of IgG | AK026463, NM_ 004048 | 1 | AW916647 |

a) number of compounds showing increase in the expression even in 7-day administration group
b) Accession No. of EST containing partial sequence

Table 4 Probe set with decrease of expression in all of six PLsis-inducing compound 3-day administration groups

| Category | Affymetrix No. | Accession No. | Gene name | Gene symbol | Correspon-Function | ding human gene | a | b |
|---|---|---|---|---|---|---|---|---|
| Transport system | 1367960_at | NM_ 019186 | ADP-ribosylation-like 4 | Ar14 | vesicular transport and secretion associated GTP binding protein | NM_005738, NM_ 212460 | | |
| | 1371469_at | NM_024139 | calcium binding protein p22 | Chp | membrane transport associated Ca2+ binding protein | NM_007236 | 4 | |
| | 1387388 at | NM 024139 | calcium binding protein p22 | Chp | membrane transport associated Ca2+ binding protein | NM_007236 | 5 | |
| | 1371908_ at | XM_ 215858 | Similar to NTF2-related export protein NXT1 (LOC296219), mRNA | --- | functions in nuclear transport pathway | NM_ 013248 NM_ 013248 | 4 | AA891920 |
| | 1387651_at | NM_012778 | aquaporin 1 (channel-forming integral protein, 28kDa) | Aqp1 | water channel | NM_000385, NM_ 198098 | 4 | L07268 |
| | 1388480_at - | XM_ 213793 - | Similar to Glycolipid transfer protein (LOC288707), mRNA | --- | intermembrane transport of glycosphingolipid, glycoglycerolipid | NM_016433 | 2 | AI412863 |
| Antioxidant action | 1367613_at | NM_057114 | peroxiredoxin 1 | Prdx1 | antioxidant having peroxydase activity induced by oxidative stress | NM_002574, NM_ 181696, NM_181697 | 2 | |
| | 1398839_at | NM_053800 | thioredoxin | Txn | involved in response to UV and oxidative stress, decreases reactive oxygen intermediate | NM_003329 | 3 | |

(continued)

| Category | Affymetrix No. | Accession No. | Gene name | Gene symbol | Correspon-Function | ding human gene | a | b |
|---|---|---|---|---|---|---|---|---|
| cell cycle· growth· death | 1367780_at | NM_022391 | pituitary tumor-transforming 1 | Pttg1 | premalignant gene, downregulated in response to serum starvation, promotes cell growth and angiogenesis | NM_004219 | 4 | |
| | 1375428_at | XM_213921 | Similar to cellular repressor of E1A-stimulated genes CREG (LOC289185), mRNA | --- | involved in transcriptional regulation in cell growth or differentiation | NM_003851 | 5 | BE099979 |
| | 1381968_at | AI029175 | Similar to cellular repressor of E1A-stimulated genes CREG (LOC289185), mRNA | --- | involved in transcriptional regulation in cell growth or differentiation | NM_003851 | 5 | |
| | 1375896_at | BM392055 | Similar to amyotrophic lateral sclerosis 2 (juvenile) chromosome region, candidate 2 | --- | antiapoptopic protein potentiating activation of JNK1 | NM_018571 | 3 | |
| | 1388286_a_ at | L38482 | Similar to cell division cycle 34; ubiquitin-conjugating enzyme E2-32 KDA complementing; ubiquitin carrier protein; ubiquitin-protein ligase | --- | ubiquitin conjugating enzyme conjugating enzyme that regulates transfer from G1 phase to S phase and chromosome alignment | NM_ 004359 | 1 | |

(continued)

| Category | Affymetrix No. | Accession No. | Gene name | Gene symbol | Correspon-Function | ding human gene | a | b |
|---|---|---|---|---|---|---|---|---|
| | 1388387_at | NM_001007 742 | ubiquitin associated domain containing 1 | Ubadc1 | putative intracellular signal molecule capable of regulating growth of endothelial cell | NM_016172 | 3 | BI275880 |

a) number of compounds showing decrease in the expression even in 7-day administration group
b) other Accession No. of the same gene or Accession No. of EST containing partial sequence

Table 4 (continued) Probe set with decrease of expression in all of six PLsis-inducing compound 3-day administration groups

| Category | Affymetrix No. | Accession No. | Gene name | Gene symbol | Function | Corresponding human gene | a | b |
|---|---|---|---|---|---|---|---|---|
| RNA transcript | 1373200_at | AI600237 | Similar to eukaryotic translation elongation factor 1 epsilon 1 (LOC291057), mRNA | --- | translation | NM_004280 | 3 | |
| | 1389680_at | XM_226624 | Similar to RNA polymerase II elongation factor ELL2 (LOC309918), mRNA | --- | member of RNA polymerase II elongation factor ELL2 family | NM-012081 | 5 | BI291626 |
| | 1388914_at | XM_214451 | Similar to germ cell specific Y-box binding protein | --- | RNA binding protein, involved in translational repression of mRNA in germ cell | NM_015982 | 4 | BI288013 |
| protease | 1367646_at | NM_022597 | cathepsin B | Ctsb | cysteine protease belonging to papain family derived from lysosome | NM_001908, NM_ 147780, NM_147781, NM_147782, NM_ 147783 | 1 | |
| | 1370460_at | NM_145184 | ubiqutin specific protease 15 | Usp 15 | enzyme belonging to ubiquitin Specific cysteine protease family | NM_006313 | 3 | |
| cytoskeleton | 1368541_at | NM_053719 | embigin | Emb | transmembrane protein, cell adhesion molecule | NM_198449 | 3 | |
| | 1389398_at | AI172141 | Similar to Ankyrin 1 | --- | cytoskeletal anchoring protein that adheres cytoskeletal element to plasma membrane | NM_000037, NM_ 020475, NM_020476, NM_020477 | 1 | |
| | 1367632_at | NM_017073 | glutamine synthetase 1 | Glu1 | glutamine synthetase | NM_002065 | 0 | |

(continued)

| Category | Affymetrix No. | Accession No. | Gene name | Gene symbol | Function | Corresponding human gene | a | b |
|---|---|---|---|---|---|---|---|---|
| Others | 1367650_at | NM_053582 | lipocalin 7 | Lcn7 | putative extracellular matrix protein, cathepsin B associated protein inactive as catalyst | NM_022164 | 5 | |
| | 1370913_at | NM_138881 | Best5 protein | Best5 | responses to interferon, similar to human viperin | NM_080657 | 3 | |
| | 1372191_at | XM_340803 | Similar to Sin3A associated protein p30-like | --- | similar to transcription corepressor Sin3A associated protein (SAP30) related to histone deacetylase | NM_024632 | 3 | BI303656 |
| Unknown | 1375526_at | XM_213333 | Similar to hypothetical protein MGC40107 (LOC287442), mRNA | --- | | NM_152766 | 6 | AI171327 |
| | 1388493_at | AI600030 | --- | --- | | none | 3 | |

a) number of compounds showing decrease in the expression even in 7-day administration group
b) other Accession No. of the same gene or Accession No. of EST containing partial sequence

**Example 3**

6 kinds of known PLsis-inducing compounds (amiodarone:300 mg/kg/day, tamoxifen:100 mg/kg/day, chloroquine:75 mg/kg/day, perhexiline:200 mg/kg/day, imipramine:100 mg/kg/day and quinacrine:60 mg/kg/day, negative control:0.5 w/v% methylcellulose solution) were administered by gavage to 5-week-old Crj:CD(SD)IGS rats (Charles River Japan Inc., produced in closed environment) at a dosing liquid volume of 10 mL/kg/day once daily for 7 days.

After completion of the administration, in all animals, livers, kidneys, spleens, lungs and mesenteric lymph nodes were sampled, these sampled tissues were examined for histopathological changes by conventional methods of optical microscopy and electron microscopy. In addition, a blood smear sample (Giemsa stain sample) was prepared, and the vacuolated lymphocyte ratio of the peripheral blood was measured with observation under a microscope.

The results for histopathological changes and vacuolated lymphocyte ratio in the PLsis-inducing compound administration group are shown in Table 5. In all the histopathological tests in the compound administration group, changes considered to be attributable to Plsis were observed. Simultaneously, the vacuolated ratio of lymphocyte was found to be high in a vacuolated lymphocyte test of peripheral blood.

Table 5

| Vacuolated lymphocyte ratio and histopathological finding in PLsis-inducing compound administrated rat | | | | | | | |
|---|---|---|---|---|---|---|---|
| Compound | Dose (mg/ kg/ day) | Vacuolated lymphocyte ratio (%) | Histopathological findings (PLsis) | | | | |
| | | | Lung | Liver | Spleen | Kidney | Lymph node |
| Negative control | 0 | 0.0 | - | - | - | - | - |
| Amiodarone | 300 | 31.5↑ | ++ | - | - | - | ++ |
| Imipramine | 100 | 12.3↑ | + | ++ | - | - | - |
| Tamoxifen | 100 | 10.3↑ | + | - | - | - | ++ |
| Quinacrine | 60 | 16.0↑ | + | ++ | + | + | - |
| Chloroquine | 75 | 16.3↑ | - | + | + | - | - |
| Perhexiline | 200 | 12.5↑ | + | - | - | - | + |
| ↑: high level ; ++: severe PLsis lesion; +: mild PLsis lesion; -: no PLsis lesion | | | | | | | |

**Example 4**

At 24 hr after completion of the oral administration for 7 consecutive days in Example 3, the blood was collected from the compound administration,group and the control (non-administration) group (4 rats per group), which was pooled and subjected to GeneChip analysis in the same manner as in Example 2.

The probe sets with expression level variation were extracted and the number is summarized in Table 6.

Table 6

| Number of expression variation probe sets in rat administrated with PLsis-inducing compound | | | |
|---|---|---|---|
| compound | Dose (mg/kg/day) | Increased expression | Decreased expression |
| Amiodarone | 300 | 515 | 457 |
| Imipramine | 100 | 123 | 320 |
| Tamoxifen | 100 | 546 | 368 |
| Quinacrine | 60 | 258 | 103 |
| Chloroquine | 75 | 310 | 179 |
| Perhexiline | 200 | 49 | 83 |

In all 6 compounds, expression variation was observed as follows:

increase: 14 probe sets (14 genes) (Table 7),
decrease: 9 probe sets (9 genes) (Table 8). Of these, expression variation was observed in all the tested 6 compounds (5 compounds were duplicated) even after 3 days of administration in chemokine (C-C motif) ligand 6 (NM_001004202), secretory leukocyte peptidase inhibitor (NM_053372) and similar to hypothetical protein MGC40107 (XM_213333).

Table 7 Probe set with increase of expression in all of six PLsis-inducing compound 7-day administration groups

| Category | Affymetrix No. | Accession No. | Gene name | Gene symbol | Function | Corresponding human gene | a |
|---|---|---|---|---|---|---|---|
| Lipid-associated | 1367614_at | NM_ 012904 | annexin A1 | Anxa1 | calcium-dependent phospholipid binding protein that inhibits phospholipase A2 | NM _000700 | 3 |
| | 1370180_ at | NM_ 053598 | nudix (nucleoside diphosphate linked moiety X)- Nudt4 type motif 4 | Nudt4 | diphosphoinositol polyphosphate phosphohydrolase possibly involved in metabolism of inositol phosphate | NM_ 019094, NM 199040 | 5 |
| | 1373877_at | NM_053554 | Phosphatidyl inositol binding clathrin assembly protein | Picalm | bound with clathrin heavy chain, acts on endocytosis | NM_001008660, NM_ 007166 | 3 |
| | 1368494_ at | NM_ 053822 | S100 calcium binding protein A8 (calgranulin A) | S100a8 | member of S100 family, forms a complex with S100A9 and mediates arachidonic acid secretion and leukocyte supplementation to a site of inflammation | NM_002964 | 4 |
| | 1387125_at | NM_053587 | S100 calcium binding protein A9 (calgranulin B) | S100a9 | member of S100 family, forms a complex with S100A8 and mediates arachidonic acid secretion and leukocyte supplementation to a site of inflammation | NM_ 002965 | 3 |

(continued)

| Category | Affymetrix No. | Accession No. | Gene name | Gene symbol | Function | Corresponding human gene | a |
|---|---|---|---|---|---|---|---|
| | 1371615_at | NM_001012345 (XM_ 341887) | Diacylglycerol 0-acyltransferase homolog 2 (mouse) | Dgat2 | member of diacylglycerol acyltransferase family, catalyzes triacylglycerol biosynthesis | NM_032564 | 0 |
| Immune reaction | 1373661_a_ at | NM_022205 | Chemokine (C-X-C motif) receptor 4 | Cxcr4 | GPCR, which is bound with CXC chemokine binding | NM_001008540, NM_ 003467 | 3 |
| | 1389123_at | NM_001004202 | chemokine (C-C motif) ligand 6 | Cc16 | CC chemokine that promotes migration and humectant of inflammatory cell | | 6 |
| | 1398256_at | NM_031512 | interleukin 1 beta | 111b | cytokine that regulates defense reaction and inflammation reaction | NM_000576 | 4 |
| | 1367998_ at | NM_ 053372 | secretory leukocyte peptidase inhibitor | Slpi | protease inhibitor such as trypsin, cathepsin G and neutrophil elastase and the like | NM_ 003064 | 6 |
| Others | 1372776_at | XM_223508 | F-box and leucine-rich 5 repeat protein 5 (predicted) | Fbx15 | putative subunit of SCF ubiquitin ligase involved in proteolysis | NM_012161, NM_ 033535 | 4 |
| | 1387422_at | NM_053373 | peptidoglycan recognition protein 1 | Pglyrp1 | Bound with peptidoglycan and gram-positive bacterium, involved in congenital involved in congenital immunity | NM_ 005091 NM_ 005091 | 2 |

(continued)

| Category | Affymetrix No. | Accession No. | Gene name | Gene symbol | Function | Corresponding human gene | a |
|---|---|---|---|---|---|---|---|
| | 1399158_a_ at | NM_012992 | nucleophosmin 1 | Npm1 | nucleic acid binding ribonuclease, molecular chaperon, possibly involved in ribosome assembly | NM_002520, NM_ 199185 | 2 |
| Unknown function | 1374626_at NM_ 001009717 | alpha-2- | Similar to leucine-rich glycoprotein | | serum protein containing plural leucine-rich repeats | NM_052972 | 5 |
| a) number of compounds showing increase in the expression even in 3-day administration group | | | | | | | |

Table 8 Probe set with decrease of expression in all of six PLsis-inducing compound 7-day administration groups

| Category | Affymetrix No. | Accession No. | Gene name | Gene symbol | Function | Corresponding human gene | a |
|---|---|---|---|---|---|---|---|
| Lipid-associated | 1387796_at | NM 031010 | arachidonate 12-lipoxygenase | Alox15 | enzyme that converts arachidonic acid to 15-hydroperoxyeicosatetraenoic acid and lipoxin A4 | NM_001140 | 3 |
| Immune reaction/cell growth-associated | 1372138_at | XM_341053 | Cut-like 1 (Drosophila) | Cut11 | acts on cell cycle of mitosis, possibly regulates immune response to apoptosis-inducing stimulation | NM_001913, NM_181500, NM_181552 | 1 |
| Cell growth-associated | 1372685_at | XM_214152 | cyclin-dependent kinase inhibitor 3 (predicted) | | tyrosine-serine phosphatase that inhibits progression of cell cycle | NM_005192 | 3 |
| Intracellular transport-associated | 132931_at | XM_346274 | Similar to DXImx39e protein | | member of prenylated Rab acceptor (PRA1) family, moderately similar to glutamic acid transporter | NM 007213 NM_007213 | 1 |
| Others | 1372273_at | NM_001013233(XM_344660) | Similar to glycophorin C isoform 2 | | erythrocyte transmembrane sialoglycoprotein, forms a triple complex with Epb4. 1 and Mpp1, possibly involved in regulation of cell shape | NM_002101, NM_016815 | 1 |
| | 1389858_at | BM389006 | similar to thymidine kinase 1 | | cytoplasmic enzyme that synthesizes thymidine acid for DNA synthesis | NM_003258 | 5 |

(continued)

| Category | Affymetrix No. | Accession No. | Gene name | Gene symbol | Function | Corresponding human gene | a |
|---|---|---|---|---|---|---|---|
| Unknown function | 1373948_at | XM_340780 | similar to CGTHBA protein (-14 gene protein) (-14 gene (predicted) | | member of uncharacterized protein UPF0171 family protein UPF0171 family | NM _012075 | 1 |
| | 1375526_at | XM_213333 | similar to hypothetical protein MGC40107 | | member of unknown function protein (DUF423) family, possible membrane protein | NM_152766 | 6 |
| | 1371970_at | AA799328 | | | | | 1 |
| a) number of compounds showing decrease in the expression even in 3-day administration group | | | | | | | |

## Industrial Applicability

The method for determining PLsis of the present invention uses, as an index, expression variation of a PLsis marker gene in blood or lymphocyte. Therefore, PLsis expression can be determined noninvasively and with high precision, as compared to conventional *in vivo* toxicity tests and the like. Accordingly, the method is useful for the toxicity evaluation of drug candidate compounds in the later stage of drug development, such as clinical or preclinical stages and the like. In addition, the method is also useful for the diagnosis of other drug-induced PLsis, and hereditary or nonhereditary diseases associated with PLsis or PLsis-like changes as pathology.

While some of the preferable embodiments of the present invention have been explained with emphasis as mentioned above, it will, however, be evident for those of ordinary skill in the art that changes may be made to the preferable embodiments and that the present invention can be practiced in a manner other than specifically described in the present specification. Therefore, the present invention includes all the modifications encompassed in the spirit and scope of the invention as set forth in the appended claims.

All the references cited herein, including patents, patent applications and publications, are hereby incorporated in their entireties by reference.

This application is based on patent application Nos. 2005-040698 filed on February 17, 2005 and 2005-128412 filed on April 26, 2005 in Japan, the contents of which are incorporated in full herein by this reference.

## Claims

1. A reagent for determining phospholipidosis in a mammal, which comprises a nucleic acid capable of hybridizing to a nucleic acid having a base sequence shown by any of SEQ ID NO n (wherein n is an odd number between 1 and 57, an integer number between 59 and 63, an even number between 64 and 98, 100 or 101) under high stringent conditions and/or a nucleic acid capable of hybridizing to a nucleic acid having a base sequence complementary to the base sequence under high stringent conditions.

2. A kit for determining phospholipidosis in a mammal, which comprises two or more reagents containing a nucleic acid capable of hybridizing to a transcription product of a gene showing varying expression in correlation with expression of phospholipidosis under high stringent conditions and/or a nucleic acid capable of hybridizing to a nucleic acid having a base sequence complementary to the transcription product under high stringent conditions, wherein,

   (a) at least one reagent is the reagent of claim 1, and
   (b) when two or more reagents of claim 1 are contained, each reagent can detect expression of different genes.

3. A method for determining phospholipidosis in a mammal, which comprises detecting expression variation of one or more genes showing expression variation in correlation with phospholipidosis expression, in a sample from a mammal, wherein at least one gene has the same or substantially the same base sequence as the base sequence shown by any of SEQ ID NO n (wherein n is an odd number between 1 and 57, an integer number between 59 and 63, an even number between 64 and 98, 100 or 101).

4. The method of claim 3, wherein the mammal is administered with a compound or exposed to the compound, and phospholipidosis is caused by the compound.

5. The method of claim 3, wherein the mammal is human.

6. The method of claim 3, wherein the mammal is rat, mouse, dog or monkey.

7. The method of claim 3, wherein the sample is blood or lymphocyte.

**INTERNATIONAL SEARCH REPORT**

International application No.
PCT/JP2006/303205

A. CLASSIFICATION OF SUBJECT MATTER
*C12N15/00*(2006.01), *C12Q1/68*(2006.01)

According to International Patent Classification (IPC) or to both national classification and IPC

B. FIELDS SEARCHED

Minimum documentation searched (classification system followed by classification symbols)
*C12N15/00*(2006.01), *C12Q1/68*(2006.01)

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
BIOSIS/MEDLINE/WPIDS(STN), GenBank/EMBL/DDBJ/GeneSeq, PubMed, JSTPlus(JOIS)

C. DOCUMENTS CONSIDERED TO BE RELEVANT

| Category* | Citation of document, with indication, where appropriate, of the relevant passages | Relevant to claim No. |
|---|---|---|
| Y | MORTUZA, G.B. et al., Characterisation of a potential biomarker of phospholipidosis from amiodarone-treated rats, Biochim.Biophys,Acta., Vol.1631, No.2, 17 March, 2003 (17.03.03), pages 136 to 146 | 1-7 |
| Y | SAWADA, H. et al., A toxicogenomic approach to drug-induced phospholipidosis: analysis of its induction mechanism and establishment of a novel in vitro screening system, Toxicol.Sci., Vol.83, No.2, 01 September, 2004 (01.09.04) (Epub.), pages 282 to 292 | 1-7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 March, 2006 (13.03.06) | 20 March, 2006 (20.03.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/303205

C (Continuation). DOCUMENTS CONSIDERED TO BE RELEVANT

| Category* | Citation of document, with indication, where appropriate, of the relevant passages | Relevant to claim No. |
|---|---|---|
| A | IDBORG-BJORKMAN, H. et al., Screening of biomarkers in rat urine using LC/electrospray ionization-MS and two-way data analysis, Anal.Chem., Vol.75, No.18, 15 September, 2003 (15.09.03), pages 4784 to 4792 | 1-7 |
| A | GERBAUX, C. et al., Hyperactivity of cathepsin B and other lysosomal enzymes in fibroblasts exposed to azithromycin, a dicationic macrolide antibiotic with exceptional tissue accumulation, FEBS Lett., Vol.394, No.3, 07 October, 1996 (07.10.96), pages 307 to 310 | 1-7 |
| A | ROCKETT, J.C. et al., DNA arrays to monitor gene expression in rat blood and uterus following 17beta-estradiol exposure: biomonitoring environmental effects using surrogate tissues, Toxicol.Sci., Vol.69, No.1, 2002.09, pages 49 to 59 | 1-7 |
| A | REASOR, M.J. et al., Drug-induced phospholipi dosis: are there functional consequences?, Exp.Biol.Med. (Maywood), 2001.10, Vol.226, No.9, pages 825 to 830 | 1-7 |
| P,X | WO 2005/052154 A1 (TAKEDA PHARMACEUTICAL CO., LTD.), 09 June, 2005 (09.06.05), All pages (particularly, pages 24 to 25) (Family: none) | 1-7 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0210453 A
- WO 02095000 A
- JP 2005040698 A
- JP 2005128412 A

### Non-patent literature cited in the description


- **SAWADA, H. et al.** *Toxicol. Sci.,* 2005, vol. 83, 282-92
- **ROCKETT, J.C. et al.** *Toxicol. Sci.,* 2002, vol. 69, 49-59
- **BOWIE et al.** *Science,* 1990, vol. 247, 1306-1310
- **KARLIN et al.** *Proc. Natl. Acad. Sci. USA,* 1993, vol. 90, 5873-5877
- **ALTSCHUL et al.** *Nucleic Acids Res.,* 1997, vol. 25, 3389-3402
- **NEEDLEMAN et al.** *J. Mol. Biol.,* 1970, vol. 48, 444-453
- **MYERS ; MILLER.** *CABIOS,* 1988, vol. 4, 11-17
- **PEARSON et al.** *Proc. Natl. Acad. Sci. USA,* 1988, vol. 85, 2444-2448